# EUROPÄISCHE PATENTANMELDUNG

(11) **EP 2 383 267 A1**
(43) Veröffentlichungstag der Anmeldung: **02.11.2011**
(21) Anmeldenummer: 11006169.4
(22) Anmeldetag: 27.09.2007
(51) Int. Cl.: C07D 401/12, C07D 401/14, C07D 403/12, C07D 471/04, C07D 409/14, C07D 403/14

(54) **Substituierte Sulfonamid-Derivate**

(30) Priorität: 29.09.2006 DE 102006046743
(62) Teilanmeldung aus: 07818500.6
(71) Anmelder: Grünenthal GmbH, 52078 Aachen (DE)
(72) Erfinder: Oberbörsch, Stefan Dr. Dr., 52078 Aachen (DE); Schunk, Stefan Dr. Dr., 52080 Aachen (DE); Reich, Melanie Dr., 52072 Aachen (DE); Hees, Sabine, 52076 Aachen (DE); Jostock, Ruth Dr., 52223 Stolberg (DE); Engels, Michael Franz-Martin, 2300 Turnhout (BE); Klees, Achim Dr., 52072 Aachen (DE); Christroph, Thomas Dr., 52080 Aachen (DE); Schiene, Klaus Dr., 41363 Jüchen (DE); Germann, Tieno Dr., 52080 Aachen (DE); Bijsterveld, Edward Dr., 6546 XL Nijmegen (NL)

(57) **Zusammenfassung**

Die Erfindung betrifft substituierte Sulfonamid-Derivate, Verfahren zu deren Herstellung, Arzneimittel enthaltend diese Verbindungen und die Verwendung von substituierten Sulfonamid-Derivaten zur Herstellung von Arzneimitteln.

## Beschreibung

Die vorliegende Erfindung betrifft substituierte Sulfonamid-Derivate, Verfahren zu deren Herstellung, Arzneimittel enthaltend diese Verbindungen und die Verwendung von substituierten Sulfonamid-Derivaten zur Herstellung von Arzneimitteln.

Im Gegensatz zur konstitutiven Expression des Bradykinin 2 Rezeptors (B2R) wird der Bradykinin 1 Rezeptor (B1 R) in den meisten Geweben nicht oder nur schwach exprimiert. Allerdings ist die Expression des B1 R auf verschiedenen Zellen induzierbar. Beispielsweise erfolgt im Verlauf von Entzündungsreaktionen eine rasche und ausgeprägte Induktion des B1 R auf neuronalen Zellen aber auch verschiedenen peripheren Zellen wie Fibroblasten, Endothelzellen, Granulozyten, Makrophagen und Lymphozyten. Somit kommt es im Verlauf von Entzündungsreaktionen zu einem switch von einer B2R zu einer B1 R Dominanz auf den beteiligten Zellen. Wesentlich an dieser B1 R Heraufregulation beteiligt sind die Zytokine Interleukin-1 (IL-1) und Tumor Nekrose Faktor alpha (TNFα) (Passos et al. J. Immunol. 2004, 172, 1839-1847). Nach Aktivierung mit spezifischen Liganden können B1R-exprimierende Zellen anschließend selbst entzündungsfördernde Zytokine wie IL-6 und IL-8 sezernieren (Hayashi et al., Eur. Respir. J. 2000, 16, 452-458). Dies führt zur Einwanderung weiterer Entzündungszellen, z.B. neutrophiler Granulozyten (Pesquero et al., PNAS 2000, 97, 8140-8145). Über diese Mechanismen kann das Bradykinin-B1 R-System zur Chronifizierung von Erkrankungen beitragen. Dies belegt eine Vielzahl tierexperimenteller Untersuchungen (Übersichten in Leeb-Lundberg et al., Pharmacol Rev. 2005, 57, 27-77 und Pesquero et al., Biol. Chem. 2006, 387, 119-126). Auch am Menschen zeigt sich eine verstärkte Expression des B1R, z.B. auf Enterozyten und Makrophagen im betroffenen Gewebe von Patienten mit entzündlichen Darmerkrankungen (Stadnicki et al., Am. J. Physiol. Gastrointest. Liver Physiol. 2005, 289, G361-366) bzw. auf T-Lymphozyten von Patienten mit Multipler Sklerose (Pratet al., Neurology. 1999;53,2087-2092) oder eine Aktivierung des Bradykinin-B2R-B1 R Systems im Verlauf von Infektionen mit Staphyloccocus aureus (Bengtson et al., Blood 2006, 108, 2055-2063). Infektionen mit Staphyloccocus aureus sind verantwortlich für Krankheitsbilder wie oberflächliche Infektionen der Haut bis hin zu septischem Schock.

Aufgrund der dargestellten pathophysiologischen Zusammenhänge ergibt sich für die Anwendung von B1 R-Antagonisten ein großes therapeutisches Potential bei akuten und insbesondere chronisch-entzündlichen Erkrankungen. Hierzu gehören Erkrankungen der Atemwege (Asthma bronchiale, Allergien, COPD/chronicobstruktive pulmonary disease, zystische Fibrose usw.), entzündliche Darmerkrankungen (Ulcerative Colitis, CD/Crohn's disease usw.), neurologische Erkrankungen (Multiple Sklerose, Neurodegeneration usw.), Entzündungen der Haut (atopische Dermatitis, Psoriasis, bakterielle Infektionen usw.) und Schleimhäute (M. Behcet, Pelvitis, Prostatitis usw.), rheumatische Erkrankungen (rheumatoide Arthritis, Osteoarthritis usw.), septischen Schock und Reperfusionssyndrom (nach Herzinfarkt, Schlaganfall).

Darüber hinaus ist das Bradykinin(Rezeptor)-System auch an der Regulation der Angiogenese beteiligt (Potential als Angiogenese-Inhibitor bei Krebs sowie Makula-Degeneration am Auge) und B1 R-knockout Mäuse sind geschützt vor der Induktion von Übergewicht durch eine besonders fettreiche Ernährung (Pesquero et al., Biol. Chem. 2006, 387, 119-126). B1R-Antagonisten eignen sich daher auch zur Behandlung von Fettleibigkeit.

B1 R-Antagonisten sind insbesondere geeignet zur Behandlung von Schmerz, insbesondere Entzündungsschmerz und neuropathischem Schmerz (Calixto et al., Br. J. Pharmacol 2004, 1-16), hier insbesondere von diabetischer Neuropathie (Gabra et al., Biol. Chem. 2006, 387, 127-143). Weiterhin eignen sie sich zur Behandlung von Migräne.

Bei der Entwicklung von B1 R-Modulatoren besteht jedoch das Problem, dass der humane und der Ratten-B1 R-Rezeptor sich so stark unterscheiden, dass viele Verbindungen, die gute B1 R-Modulatoren am humanen Rezeptor sind, nur eine schlechte oder keine Affinität zum Ratten-Rezeptor aufweisen. Dies erschwert die tierpharmakologischen Untersuchungen beträchtlich, da viele Untersuchungen normalerweise an der Ratte durchgeführt werden. Wenn jedoch keine Wirksamkeit am Ratten-Rezeptor vorhanden ist, können weder Wirkung noch Nebenwirkung an der Ratte untersucht werden. Dies hat bereits dazu geführt, dass transgene Tiere mit humanen B1-Rezeptoren für tierpharmakologische Untersuchungen erzeugt wurden (Hess et al., Biol Chem. 2006; 387(2):195-201). Die Arbeit mit transgenen Tieren ist jedoch teurer als die Arbeit mit den unveränderten Tieren. Da in der Arzneimittelentwicklung jedoch gerade die Langzeit-Toxizitätsuntersuchungen an der Ratte zu den Standarduntersuchungen gehört, diese jedoch im Falle von fehlender Wirksamkeit am Rezeptor nicht sinnvoll ist, fehlt für die Entwicklung solcher Verbindungen ein wichtiges, etabliertes Instrument zur Überprüfung der Sicherheit. Es besteht daher Bedarf an neuen B1 R-Modulatoren, wobei B1 R-Modulatoren, die sowohl an den Ratten-Rezeptor als auch an den humanen Rezeptor binden, besondere Vorteile bieten.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch B1R-Rezeptoren vermittelt werden. Diese Aufgabe wird durch die erfindungsgemäßen substituierten Sulfonamid-Derivate gelöst.

### Gegenstand der Erfindung sind substituierte Sulfonamid-Derivate der allgemeinen Formel I

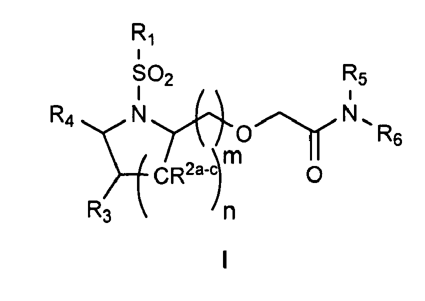

worin
n für 0, 1, 2 oder 3 steht;
m für 1 oder 2 steht;
R¹ für Aryl oder Heteroaryl steht;
R^{2a-c}, R³ und R⁴ für H stehen oder mit einem benachbarten Rest R^{2a-c}, R³ oder R⁴ einen fünf- oder sechsgliedrigen Ring bilden, der gesättigt oder ungesättigt und einfach oder mehrfach substituiert sein kann und Heteroatome aus der Gruppe N oder O enthalten kann,
R⁵ und R⁶ zusammen einen 4-8-gliedrigen Ring bilden, der gesättigt oder ungesättigt, aber nicht aromatisch sein kann, wobei der 4-8-gliedrige Ring mit einem aromatischen, gesättigten oder ungesättigten 4-10-gliedrigen Ring kondensiert sein kann, und der 4-8-gliedrige Ring und/oder der ankondensierte 4-10-gliedrige Ring mit einem basischen Rest substituiert oder kondensiert ist und mit einem weiteren basischen Rest oder Resten aus der Gruppe C₁₋₆-Alkyl, C₁₋₃-Alkyloxy, C₃₋₈-Cycloalkyl, =O, Aralkyl oder Aryl substituiert sein kann;
oder R⁵ und R⁶ zusammen einen 4-8-gliedrigen Ring bilden, der ein weiteres Heteroatom aus der Gruppe N oder O enthält und mit einem basischen oder nicht basischen Rest substituiert sein kann und in dem Fall, dass der basische oder nicht basische Substituent nicht über das weitere Heteroatom N an den 4-8-gliedrigen Ring gebunden ist, dieses weitere Heteroatom N zusätzlich mit einer C₁-₆-Alkylgruppe, einer Aryl- oder Aralkyl-Gruppe substituiert sein kann;
oder
R⁵ H, C₁₋₆-Alkyl, C₃₋₈-Cycloalkyl, Aryl oder Aralkyl bedeutet und R⁶ Aryl oder C₃₋₈-Cycloalkyl; oder einen über eine C₁₋₃-Alkylkette verknüpften Arylrest oder C₃₋₈-Cycloalkyl bedeutet, wobei der Arylrest und der C₃₋₈-Cycloalkyl-Ring mit einem 5-10-gliedrigen, ggf ein oder mehrere Heteroatome umfassenden, gesättigten oder ungesättigten Ring kondensiert sein können, und der Aryl- oder C₃₋₈-Cycloalkyl-Ring jeweils mit einem basischen Rest substituiert ist oder gegebenenfalls die Substitution mit dem basischen Rest an der verbrückenden C₁₋₃-Alkylkette erfolgt,
oder R⁵ H, C₁₋₆-Alkyl, C₃₋₈-Cycloalkyl, Aryl oder Aralkyl bedeutet und R⁶ C₄₋₈-Heterocyclyl; oder einen über eine C₁₋₄-Alkylkette verknüpften C₄₋₈-Heterocyclylrest bedeutet, wobei der Heterocyclyl-Ring an einem oder zwei Ringgliedern mit einem basischen oder nicht basischen Rest substituiert sein kann,
oder R⁵ und R⁶ unabhängig voneinander für H, Aralkyl oder einen verzweigten oder unverzweigten, gegebenenfalls einfach oder mehrfach substituierten C₁₋₁₀-Alkylrest steht, der ein bis drei Stickstoffatome enthält, wobei R⁵ und R⁶ nicht beide H bedeuten,
oder
R⁵ für H, C₁₋₆-Alkyl, C₃₋₈-Cycloalkyl, Aralkly oder Aryl steht und R⁶ für einen ggf. über eine C₁₋₄-Alkylgruppe verknüpften basischen Heteroarylrest, der einfach oder mehrfach substituiert sein kann, steht, wobei die C₁₋₄-Alkylkette mit einen basischen Rest substituiert sein kann;
wobei, soweit nichts anderes angegeben ist, die oben genannten Reste C₁₋₆-Alkyl, C₁₋₃-Alkoxy, Aralkyl, Aryl und Heteroaryl unsubstituiert, einfach oder mehrfach substituiert sein können und der Rest C₃₋₈-Cycloalkyl unsubstituiert oder an einem oder mehreren Ringgliedern einfach substituiert sein kann,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Ausdrücke "C₁₋₃-Alkyl", "C₁₋₆-Alkyl" und "C₁₋₁₀-Alkyl"umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 3 C-Atomen bzw. 1 bis 6 C-Atomen bzw. 1 bis 10 C-Atome, d.h. C₁₋₃-Alkanyle, C₂₋₃-Alkenyle und C₂₋₃-Alkinyle bzw. C₁₋₆-Alkanyle, C₂₋₆-Alkenyle und C₂₋₆-Alkinyle bzw. C₁₋₁₀-Alkanyle, C₂₋₁₀-Alkenyle und C₂₋₁₀-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, Hexyl, Heptanyl, Octanyl, Nonanyl, Decyl, Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl und Hexinyl umfasst. Besonders vorteilhaft sind Methyl, Ethyl, n-Propyl und iso-Propyl.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder an einem oder mehreren Ringgliedern einfach substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl.

Der Ausdruck "Heterocyclyl" bezeichnet im Sinne dieser Erfindung mono- oder polycyclische organische Reste, in denen zumindest ein Cyclus 1 Heteroatom oder 2, 3, 4 oder 5 gleiche oder verschiedene Heteroatome enthält, das/die ausgewählt sind aus der Gruppe bestehend aus N, O und S. Jeder Heterocycylrest kann unsubstituiert oder an einem oder mehreren Ringgliedern einfach substituiert vorliegen. Unter gesättigtem und ungesättigtem Heterocyclyl werden insbesondere monocyclische 5- oder 6-gliedrige Verbindungen mit zumindest einem Heteroatom aus der Gruppe N, O und S, wobei an diese Verbindungen ein weiterer 5- oder 6-gliedriger, gesättigter, ungesättigter oder aromatischer, Cyclus ankondensiert sein kann, der ebenfalls zumindest ein Heteroatom aus der Gruppe N, O, und S aufweisen kann. Beispiele sind die benzo- oder pyridino-kondensierten Analoga der oben genannten monocyclischen 5- oder 6-gliedrigen Verbindungen. Vorzugsweise ist ein gesättigter oder ungesättigter Heterocyclylrest ausgesucht aus der Gruppe, die Pyrrolidinyl, Piperidinyl, Piperazinyl, Pyrazolinyl, Morpholinyl, Tetrahydropyranyl, Dioxanyl, Dioxolanyl Indolinyl, Isoindolinyl,
bzw.

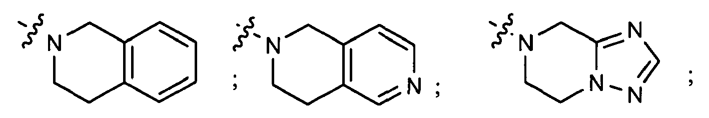

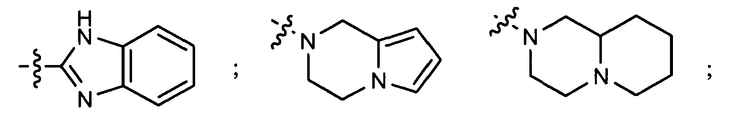

Soweit nicht anders angezeigt, kann die Substitution mit einem Heterocyclylrest über jede beliebige Position des Heterocyclylrestes erfolgen.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält.

Der Ausdruck "Heteroaryl" ist gleichbedeutend mit "aromatisches Heterocyclyl" und steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein, das dann insgesamt mehr als 7-gliedrig sein kann, vorzugsweise bis zu 14-gliedrig.. Bevorzugte Heteroatome sind Stickstoff, Sauerstoff und Schwefel. Es ist bevorzugt, dass der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Chinoxalinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des HeteroarylRestes erfolgen kann. Besonders bevorzugt ist Pyridyl.

Der Ausdruck "über C₁₋₃-Alkyl gebundenes Aryl oder Heteroaryl" bedeuten für die Zwecke der vorliegenden Erfindung, dass C₁₋₃-Alkyl und Aryl bzw. Heteroaryl die oben definierten Bedeutungen haben und der Aryl- bzw. Heteroaryl-Rest über eine C₁₋₃-Alkyl-Gruppe an die Verbindung der allgemeinen Struktur I gebunden ist. Besonders vorteilhaft im Sinne dieser Erfindung ist Phenyl, Benzyl und Phenethyl.

Der Ausdruck "Aralkyl" steht für eine mit einer Arylgruppe substituierte Alkylgruppe. Vorzugsweise wird die Aralkylgruppe ausgewählt aus der Gruppe bestehend aus Benzyl, Phenylethyl und Phenylpropyl.

Im Zusammenhang mit "Alkyl" und "Cycloalkyl" versteht man unter dem Begriff "substituiert" im Sinne dieser Erfindung die Substitution eines Wasserstoffrestes durch F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen.

Im Zusammenhang mit "gesättigtem oder ungesättigtem Heterocyclyl" versteht man unter dem Begriff substituiert die Substitution eines Wasserstoffrestes an einem oder mehreren Ringgliedern durch F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, Pyrrolinyl, Piperazinyl, Morpholinyl, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl. Insbesondere kann der an eine N-Heteroatom gebundene Wasserstoff durch eine C₁₋₆-Alkyl-Gruppe substituiert sein.

In Bezug auf "Aryl" und "Heteroaryl" bzw. "aromatischem Heterocyclyl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, CI, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, ,NHAryl; N(Aryl)₂, N(C₁₋₆-Alkyl)Aryl, Pyrrolinyl, Piperazinyl, Morpholinyl NO₂, SH, S-C₁₋₆-Alkyl, OH, O-C₁₋₆-Alkyl, O-C₁₋₆Alkyl-OH, C(=O)C₁₋₆-Alkyl, NHSO₂C₁₋₆-Alkyl, NHCOC₁₋₆-Alkyl, CO₂H, CH₂SO₂-Phenyl, CO₂-C₁-₆-Alkyl, OCF₃, CF₃, 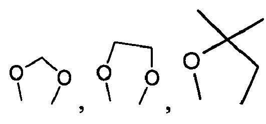 C₁₋₆-Alkyl, Pyrrolidinyl, Imidazolyl, Piperidinyl, Morpholinyl, Benzyloxy, Phenoxy, Phenyl, Pyridyl, Alkylaryl, insbesondere Benzyl, Thienyl oder Furyl; an einem oder ggf. verschiedenen Atomen, wobei ein Substituent ggf. seinerseits substituiert sein kann. Die Mehrfachsubstitution erfolgt dabei mit dem gleichen oder mit unterschiedlichen Substituenten. Für "Aryl" oder "Heteroaryl" sind dabei bevorzugte Substituenten -F, -Cl, CF₃, CH₃ oder OCH₃.

Im Rahmen der vorliegenden Erfindung bezeichnet das in den Formeln verwendete Symbol 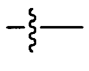 eine Verknüpfung eines entsprechenden Restes an die jeweilige übergeordnete allgemeine Struktur. Ist dieses Symbol derart in einer cyclischen oder polycyclischen, z.B. aromatischen oder anelliert aromatischen Gruppe, angeordnet, dass eine konkrete Verknüpfung an eine bestimmte Position nicht erkennbar ist, so bedeutet dies, dass eine Verknüpfung an alle Positionen der cyclischen bzw. polycyclischen Gruppe mit umfasst sein soll.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure und/oder Asparaginsäure. Besonders bevorzugt sind die Zitronensäure und die Salzsäure.

Im Sinne dieser Erfindung wird unter einem basischen Rest eine Gruppe verstanden, die unter Aufnahme von Protonen reagieren kann. Insbesondere wird darunter eine Gruppe verstanden, die mindestens einen protonierbaren Stickstoff enthält. Ein basischer Rest kann insbesondere für einen zumindest ein Stickstoffatom als Heteroatom enthaltenen, gegebenenfalls anellierten Heterocyclus stehen, wobei der Heterocyclus gegebenenfalls an einem oder mehreren Ringgliedern einfach mit C₁₋₆-Alkyl, O-C₁₋₆-Alkyl, Heterocyclyl-, OH, F, Cl, Br, J, -CN, NH₂, NH(C₁₋₆-Alkyl), N(C₁₋₆-Alkyl)₂, -NH(Aryl), -N(C₁₋₃-Alkyl)(Aryl), wobei die an diese Aminogruppen gebundenen Arylreste einfach oder mehrfach mit F, Cl, Br, CF₃, CN, OH oder OMe substituiert sein können. Bespiele solcher als basische Reste bezeichneten Heterocyclen sind Piperidin, Pyrrolidin, Azepan, Azetidin, Azocan, Pyrazin, Pyridin, Imidazol, Imidazolidin, 1,2,4-Triazol, Diazepan, Pyrimidin, Imidazolin, Piperazin, Morpholin, Chinazolin oder Chinoxalin). Weitere basische Reste im Sinne der Erfindung sind N(C₁₋₆-Alkyl)_{2,} NHC₁₋₆-Alkyl, ein mit N(C₁₋₆-Alkyl)₂ substituierter Arylrest, insbesondere Phenyl oder Naphthyl, ein mit einem 5-7-gliedrigen, zumindest ein N-Heteroatom enthaltenden Heterocyclyl, insbesondere Pyrrolindyl, Piperindinyl, 4-Methylpiperidinyl oder Morpholinyl, substituierten Aryl- oder Heteroarylrest, insbesondere Phenyl, Naphthyl, der Pyridinyl. Alle vorgenannten basischen Reste können über eine verbrückende -O-, -NH-, -NH[(-CH₂)ₚ-]-Gruppe, -N(C₁₋₃-Alkyl)[-(CH₂)p-]-Gruppe, -O-[(CH₂)p -]-Gruppe, -O-[ -(CH₂)ₚ -O-]-Gruppe, mit jeweils p = 1, 2 oder 3, oder C₁₋₃-Alkylgruppe mit der Struktur der allgemeinen Formel I verknüpft sein. Beinhaltet die verbrückende Kette ein endständiges O- oder N-Atom, so kann dieses an den basischen Rest oder an die mit dem basischen Rest zu verbindende Struktur gebunden sein. Die verbrückenden -(CH₂)p-Gruppen oder die C₁₋₃-Alkylkette können gegebenenfalls mit =O F, CI, Br, J, -CN, Phenyl oder Pyridinyl substituiert sein. Weitere Beispiele basischer Reste im Sinne der vorliegenden Erfindung sind C₁-₆-AlkylN(C₁₋₆-Alkyl)₂ oder C₁₋₆-AlkylNH(C₁₋₆-Alkyl). Für den Fall, dass der basische Rest an den aus R⁵ und R⁶ gebildeten Heterocyclus kondensiert ist, kann er für einen 6-gliedrigen, gesättigten, ungesättigten oder aromatischen, mindestens ein N-Heteroatom enthaltenden Heterocyclus stehen, vorzugsweise Pyridin oder Thiazol stehen.

Insbesondere werden unter einem basischen Rest auch ein Pyridyl-, ein Pyrrolyl, ein Imidazolyl-, ein Pyrimidinyl- oder Pyrazinylrest verstanden, der jeweils über eine C₁₋₃-Alkylkette verknüpft sein kann.

Weitere Beispiele basischer Reste sind Gruppen mit der nachfolgend dargestellten Struktur: 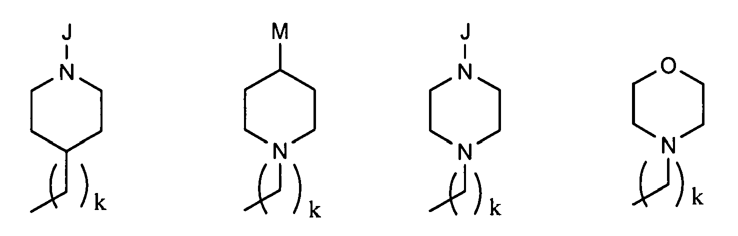 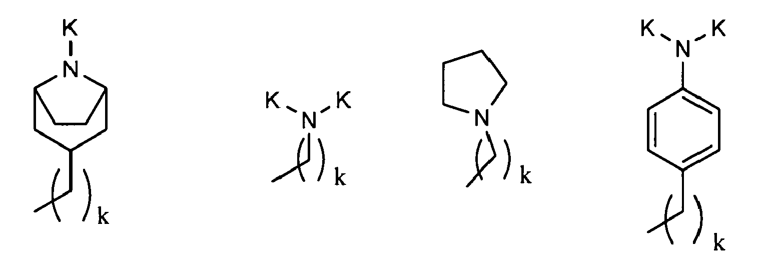 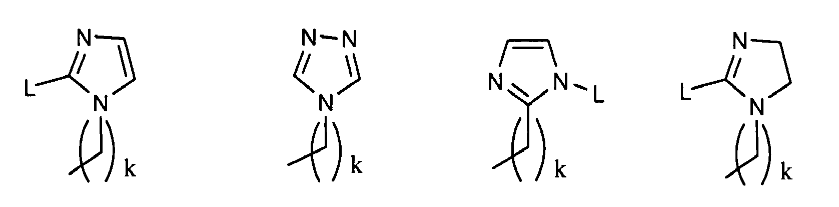 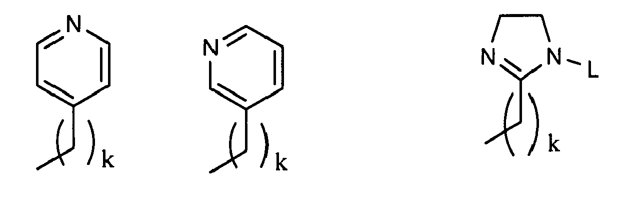 wobei
k für 0, 1 oder 2 steht
L für H oder C₁₋₆-Alkyl steht
K für C₁₋₆-Alkyl steht
M für C₁₋₆-Alkyl oder N(CH₃)₂ steht
J für 2-, 3- oder 4-Pyridyl, Phenyl, Piperidyl oder C₁₋₆-Alkyl steht

Weitere Beispiele und/oder bevorzugte Ausführungsformen basischer Reste ergeben sich aus den nachfolgenden Beschreibungen der bevorzugten erfindungsgemäßen Substanzen.

Unter einem nicht basischen Rest wird eine Gruppe verstanden, die keine basischen Eigenschaften besitzt. Insbesondere wird darunter eine Gruppe verstanden, die keinen protonierbaren Stickstoff trägt. Beispiele solcher nicht basischer Reste sind - CN, C₁₋₆-Alkyl, gegebenenfalls substituiert mit Methoxy oder C₁₋₃-Alkyloxy; oder Aryl, Heteroaryl, 3-7-gliedrige, zumindest ein Sauerstoff- oder Schwefelatom enthaltende Heterocyclen, insbesondere Tetrahydropyran oder Thiophen, jeweils unsubstituiert oder einfach oder mehrfach substituiert. Weitere Beispiele sind unsubstituiertes oder an einem oder mehreren Ringgliedern einfach substituiertes C₃₋₈-Cycloalkyl: Die vorgenannten nicht basischen Gruppen können über eine -O-, eine -O-[(CH₂)_{q} -]-bzw. -[(CH₂)_{q} -]-O-Gruppe, -O-[ -(CH₂)_{q} -O-]-Gruppe oder eine verbrückende C₁₋₃-Alkylgruppe mit der Struktur der allgemeinen Formel I verknüpft sein, wobei die - (CH₂)_{q}-Kette bzw. die Alkylkette jeweils mit =O substituiert sein kann und q = 1, 2 oder 3 gilt. Die Substituenten der nicht basischen Gruppen Aryl, Heteroaryl, 3-7-gliedrigen Heterocyclus und C₃₋₈-Cycloalkyl sind vorzugsweise ausgewählt aus F, Cl, Br, I, CN, NO₂ Aralkyl, SH, S-C₁₋₆-Alkyl, OH, O-C₁₋₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 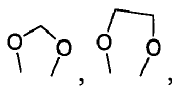 C₁₋₆-Alkyl

Bevorzugt sind Reste aus der Gruppe Aryl, Heteroaryl, C₃₋₈-Cycloalkyl, jeweils unsubstituiert oder wie oben beschrieben substituiert, die über eine C₁₋₃-Alkylkette mit der Struktur der allgemeinen Formel I verknüpft sein können, wobei die Alkylkette mit =O substituiert sein kann. Weiterhin bevorzugt sind C₁₋₆-Alkyl, gegebenenfalls substituiert mit Methoxy; C₁₋₃-Alkyloxy.

In einer bevorzugten Ausführungsform der Erfindung steht C₄₋₈-Heterocyclyl im Zusammenhang mit R⁶ für einen gesättigten oder ungesättigten, 4-8-gliedrigen cyclischen Rest, der 1, 2, 3, 4 oder 5 gleiche oder verschiedene Heteroatome im Ringsystem aufweisen kann, steht, wobei die Heteroatome vorzugsweise ausgesucht sind aus der Gruppe N, O und S und wobei sowohl die Bindung des Heterocycylrestes an die allgemeine Grundstruktur gemäß der Formel I als auch die gegebenenfalls vorliegende Substitution mit den basischen oder nicht basischen Gruppen an jedem beliebigen Ringglied vorliegen kann. Vorzugsweise ist der C₄₋₈-Heterocyclylrest hier ausgesucht aus der Gruppe bestehend aus Piperidinyl, 2,6-Dimethypiperidinyl, Piperazinyl, Morpholinyl, Pyrrolidinyl, Tetrahydropyranyl, und Tetrahydrofuranyl.

In einer anderen bevorzugten Ausführungsform der Erfindung steht ein basischer Heteroaryl-Rest im Zusammenhang mit R⁶ für einen 5-10 gliedrigen, kondensierten oder nicht-kondensierten Heteroatom-haltigen Rest steht, der zumindest ein Stickstoffatom als Heteroatom enthält und wobei sowohl die Bindung des Heteroarylrestes an die allgemeine Grundstruktur gemäß der Formel I bzw. die Bindung des Heteroarylrestes an die verbrückende C₁₋₄-Alkylgruppe als auch die gegebenenfalls vorhandene Substitution an jedem beliebigen Ringglied des Heteroarylrestes vorliegen kann. Vorzugsweise handelt es sich bei dem basischen Heteroaryl um Pyrrolyl, Pyrazolyl, Imidazolyl, Pyridinyl, Pyrazinyl, Pyridazinyl, Pyrimidinyl, Indolyl, Indazolyl, Benzoimidazolyl und Chinolinyl, Chinoxalinyl, Chinazolinyl.

In einer anderen bevorzugten Ausführungsform der Erfindung steht im Zusammenhang mit R⁶ ein an eine Arylgruppe ankondensierter 5-10 gliedriger, aromatischer oder ungesättigter, gegebenenfalls ein oder mehrere Heteroatome enthaltender Ring für einen Ring ausgewählt aus der Gruppe: 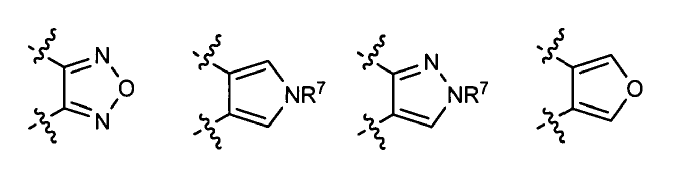 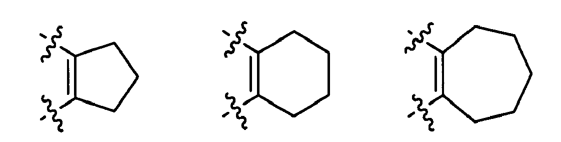 wobei R⁷ für H oder C₁₋₆-Alkyl steht,

In einer weiteren bevorzugten Ausführungsform der Erfindung steht im Zusammenhang mit R⁶ ein an einen C₃₋₈-Cycloalkyl-Ring ankondensierter 5-10-gliedriger, gesättigter oder ungesättigter, gegebenenfalls ein oder mehrere Heteroatome enthaltender Ring für einen Ring der ausgewählt ist aus der Gruppe: Phenyl, Pyridinyl, Cyclopentan, Cyclohexan oder Cycloheptan.

In einer weiteren bevorzugten Variante der vorliegenden Erfindung steht ein an den durch R⁵ und R⁶ gebildeten 4-8 gliedrigen Ring ankondensierter, aromatischer, ungesättigter oder gesättigter 4-10 gliedriger Ring für einen Ring, der ausgesucht ist aus der Gruppe bestehend aus: C₄-₁₀-Cycloalkan, C₄-₁₀-Cycloalken oder C₆₋₁₀-Aromaten und 6-gliedrige Heteroaromaten.

Bevorzugt im Sinne dieser Erfindung sind substituierte Sulfonamid-Derivate der allgemeinen Formel I, worin R¹ für Phenyl oder Benzothiophenyl, insbesondere Phenyl steht, unsubstituiert oder einfach oder mehrfach substituiert mit C₁₋₃-Alkyloxy, C₁₋₆-Alkyl, Cl, F, I, CF₃, OCF₃, OH, SH, Aryl oder Heteroaryl, jeweils unsubstituiert oder einfach oder mehrfach substituiert.

In einer bevorzugten Ausführungsform der Erfindung steht in den erfindungsgemäßen substituierten Sulfonamid-Derivaten R¹ für Phenyl, Naphthyl, Indolyl, Benzofuranyl, Benzothiophenyl, Benzoxazoly, Benzoxadiazolyl, Pyrrolyl, Furanyl, Thiophenyl, Pyridinyl, Pyridazinyl, Pyrimidinyl, Pyrazinyl, Imidazothiazolyl, Carbazolyl, Dibenzofuranyl und Dibenzothiophenyl, vorzugsweise, Phenyl, Naphthyl, Benzothiophenyl, Benzoxadiazolyl, Thiophenyl, Pyridinyl, Imidazothiazolyl, und Dibenzofuranyl, insbesondere vorzugsweise für Phenyl, Naphthyl, und Benzothiophenyl, wobei all diese Reste unsubstituiert oder einfach oder mehrfach substituiert sein können, vorzugsweise mit C₁₋₃-Alkyloxy, C₁₋₆-Alkyl, Br, CI, F, I, CF₃, OCF₃, OH, SH, Aryl oder Heteroaryl, jeweils unsubstituiert oder einfach oder mehrfach substituiert.

In einer weiteren bevorzugten Ausführungsform der erfindungsgemäßen substituierten Sulfonamid-Derivate steht R¹ für Phenyl oder Naphthyl, insbesondere Phenyl, gegebenenfalls einfach oder mehrfach substituiert mit Methyl, Methoxy, CF₃-, Cl, Br, und/oder F.

Weiterhin besonders bevorzugt sind substituierte Sulfonamid-Derivate, worin R¹ für Phenyl, substituiert in 4-Position mit Aryl oder Heteroaryl und in 2, 3, 5 und/oder 6-Position mit Methyl, Methoxy, Cl oder F, vorzugsweise in 2- und 6-Position mit Methyl substituiert.

Ganz besonders bevorzugt sind substituierte Sulfonamid-Derivate, worin R¹ 2,6-Dimethyl-4-methoxyphenyl, 2,6-Dichlor-4-trifluormethylphenyl, 2,6-Dimethyl-4-bromophenyl, 2,6-Dichloro-4-bromophenyl, 2,4,6-Trichlorophenyl, 2,4,-Dichlorophenyl, 2,6-Dichlorophenyl, 2,3-Dichlorophenyl bedeutet.

Bevorzugt sind auch substituierte Sulfonamid-Derivate der allgemeinen Formel I, worin n 2, R⁴ und R^{2b} H bedeuten und R³ und R^{2a} gemeinsam einen sechsgliedrigen aromatischen Ring bilden
oder
n 1 und R^{2a} H bedeuten und R⁴ und R³ gemeinsam einen sechsgliedrigen aromatischen Ring bilden.

In einer weiteren bevorzugten Ausführungsform der erfindungsgemäßen substituierten Sulfonamid-Derivate stehen R^{2a-c}, R³ und R⁴ für H oder bilden mit einem benachbarten Rest R^{2a-c}, R³ oder R⁴ einen aromatischen Ring, vorzugsweise eine Benzengruppe, der gegebenenfalls einfach oder mehrfach substituiert ist, vorzugsweise mit Methyl, Methoxy, CF₃-, Cl, Br und/oder F. Besonders bevorzugt sind substituierte Sulfonamid-Derivate, worin R^{2a-c}, R³ und R⁴ für H stehen.

In einer weiteren erfindungsgemäßen Ausführungsform der substituierten Sulfonamid-Derivate steht in der (CR^{2a-C})ₙ-Gruppe n für 1 oder 2, vorzugsweise 2.

In einer weiteren bevorzugten Ausführungsform der substituierten Sulfonamid-Derivate gilt in der allgemeinen Formel I m = 1.

In weiteren bevorzugten Ausführungsformen der vorliegenden Erfindung bildet:
a) die Gruppe NR⁵R⁶ in der allgemeinen Formel I eine ringförmige Gruppe gemäß den Formeln a1, a2, a3, oder a4, 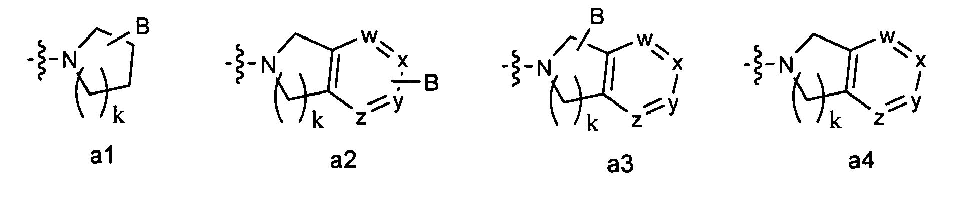
wobei
k = 0,1,2, oder 3, vorzugsweise 1 oder 2, gilt und
w, x, y und z unabhängig voneinander CH oder N darstellen, unter der Bedingung, dass höchstens zwei der Gruppen w, x, y und z gleichzeitig N darstellen und dass in der ringförmigen Gruppe gemäß der Formel a4 mindestens eine Gruppe aus w, x, y und z N darstellt. Vorzugsweise sind in den Formeln a1, a2, und a3 w, x, y, und z alle CH oder eines aus w, x, y, und z N und alle anderen CH.

In den Formeln a1, a2 und a3 stellt B einen basischen Rest dar, vorzugsweise ausgesucht aus der Gruppe bestehend aus: -NR⁸R⁹ , wobei R⁸ und R⁹ unabhängig voneinander H oder C₁₋₆-Alkyl darstellen können, und einem Rest mit der allgemeinen Formel aa1

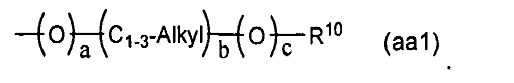

In dem Rest aa1 können a, b und c unabhängig voneinander 0 oder 1 sein, unter der Bedingung, dass wenn b gleich 0 ist, a und c nicht gleichzeitig 1 ist; und das verbrückende C₁₋₃-Alkyl kann einfach mit =O substituiert sein.

R¹⁰ stellt eine 4-10-gliedrige, aromatische, ungesättigte oder gesättigte, mono- oder polycyclische Heterocyclylgruppe dar, die 1, 2, 3 oder 4 N-Heteroatome enthalten und gegebenenfalls O und/oder S als weitere Heteroatome enthalten kann, wobei die Heterocyclylgruppe unsubstituiert oder an einem oder mehreren Ringgliedern einfach substituiert ist. Weiterhin kann R¹⁰ eine mit zumindest einer -NR¹¹R¹²-Gruppe oder einem 5- oder 6-gliedrigen, monocyclischen N-haltigen aromatischen, gesättigten oder ungesättigten, 1 oder 2 N-Heteroatome enthaltenden Heterocyclus substituierte Arylgruppe darstellt, wobei R¹¹ und R¹² unabhängig voneinander H oder C₁₋₆-Alkyl darstellen, und die Arylgruppe gegebenenfalls noch weitere Substituenten tragen kann.

Desweiteren kann R¹⁰ eine Gruppe der allgemeinen Formel aa2 darstellen: 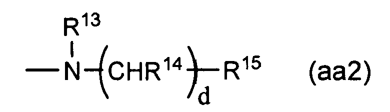 wobei
d 1, 2, oder 3 ist,
R¹³ H oder C₁₋₃-Alkyl sein kann, unabhängig für jedes Kettenglied d R¹⁴ H, oder eine gegebenenfalls substituierte Aryl- oder N-Heteroarylgruppe, vorzugsweise Phenyl, Naphthyl oder Pyridinyl, sein kann, wobei innerhalb der durch d definierten Alkylkette R¹⁴ nur einmal kein H sein kann, und R¹⁵ eine 5-7-gliedrige, gesättigte oder ungesättigte, gegebenenfalls an einem oder mehreren Ringgliedern einfach substituierte Heterocycylgruppe ist, die 1 oder 2 N-Heteroatome enthält und als weitere Heteroatome O oder S enthalten kann, vorzugsweise Morpholinyl, Piperidinyl oder 4-Methylpiperazinyl.

In bevorzugten Ausführungsformen der Erfindung ist die durch die NR⁵R⁶-Gruppe gebildete ringförmige Gruppe ausgesucht ist aus der Gruppe bestehend aus 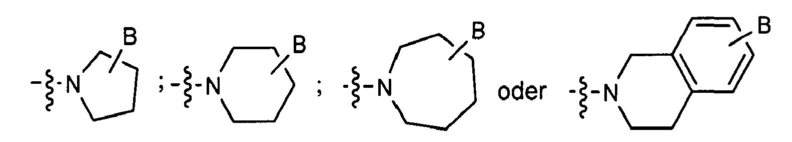 ,wobei in 4-Position mit dem basischen Rest B substituiertes Piperidin besonders bevorzugt ist,
und der basische Rest B ausgesucht ist aus der Gruppe umfassend: -N(C₁₋₆-Alkyl), vorzugsweise -N(CH₃)₂; einen Rest mit der allgemeinen Formel (aa1), wobei R¹⁰ ausgesucht ist aus der Gruppe umfassend: 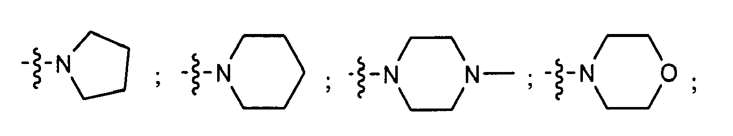 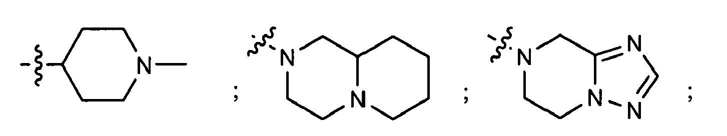 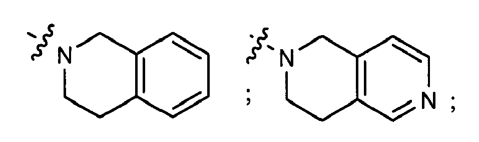 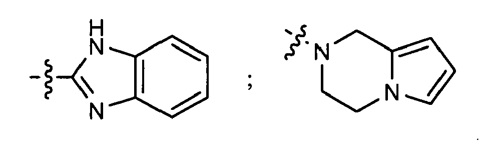 wobei diese Reste unsubstituiert oder an einem oder mehreren Ringgliedern einfach substituiert sein können, vorzugsweise mit C₁₋₆-Alkyl, insbesondere Methyl oder Ethyl, F, Cl, oder Br;
oder R¹⁰ 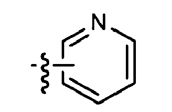 darstellt, das unsubstituiert, einfach oder mehrfach substituiert sein kann, vorzugsweise mit C₁₋₆-Alkyl, insbesondere Methyl oder Ethyl, F, Cl, Br, C₁₋₆-Alkoxy, insbesondere Methoxy, oder Phenyl;
oder
R¹⁰ für Phenyl steht, das substituiert ist mit -N(C₁₋₃-Alkyl)₂, vorzugsweise Dimethylamino oder Diethylamino, oder
für Phenyl steht, das substituiert ist mit Pyrrolidinyl, Imidazolidinyl, Dihydroimidazoly, wobei die Verknüpfung an jedem der Ringglieder des Phenyls und des Substituenten erfolgen kann,
oder
R¹⁰ für eine Gruppe der allgemeinen Formel (aa2) steht, d = 1 oder 2 ist, R¹³ H oder Methyl bedeutet, R¹⁴ für Phenyl oder Pyridinyl steht, und R¹⁵ für Morpholinyl oder 4-Methylpiperazinyl steht.

In weiteren erfindungsgemäßen Ausführungsformen ist die durch die NR⁵R⁶-Gruppe gebildete ringförmige Gruppe 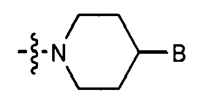 und der basische Rest B eine Gruppe mit der allgemeinen Formel (aa1); wobei, i) a = b = c = 0, ii) a = c = 0 und b = 1, iii) a = b = 0 und c = 1 oder iv) a = b = 1 und c = 0; gilt; oder der basische Rest B eine Gruppe mit der allgemeinen Formel (aa2) ist, wobei d =1 oder 2, vorzugsweise 2, gilt und R¹⁴, Pyridinyl, vorzugsweise 3-Pyridinyl, ist, und R¹⁵ Morpholinyl ist.

In weiteren bevorzugten Ausführungsformen der Erfindung stellt:
b) die Gruppe NR⁵R⁶ in der allgemeinen Formel I eine ringförmige Gruppe gemäß einer der allgemeinen Formeln b1 oder b2 dar: 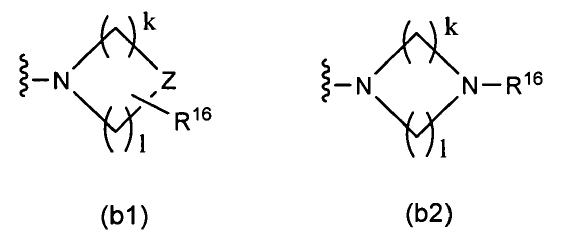 wobei
   k = 1 oder 2, vorzugsweise 2,
   I = 1, 2, oder 3, vorzugsweise 2,
   Z NR¹⁷ oder O sein kann und R¹⁷ H oder C₁₋₆-Alkyl ist,
   und R¹⁶ H oder eine Gruppe der allgemeinen Formel bb1 darstellt: 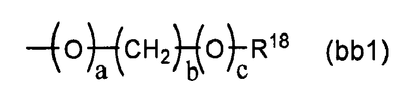
   wobei
   a und c unabhängig voneinander 0 oder 1 sind,
   b = 0, 1, 2, oder 3 gilt, mit der Maßgabe, dass wenn b = 0 ist, dann sind a und c nicht gleichzeitig 1, und wobei in der durch b definierten Alkylkette ein CH₂-Kettenglied durch C(=O) ausgetauscht sein kann,
   R¹⁸ ausgesucht ist aus der Gruppe bestehend aus unsubstituiertem, einfach oder mehrfach substituiertem Aryl oder Heteroaryl, wobei das Heteroaryl zumindest ein N-Heteroatom, vorzugsweise 1, 2 oder 3 N-Heteroatome, enthält, und O und S als weitere Heteroatome enthalten kann; gesättigtes oder ungesättigtes 5-7-gliedriges Heterocyclyl, wobei das Heterocyclyl zumindest ein Heteroatom ausgesucht aus der Gruppe N, O und S enthält, vorzugsweise 1 oder 2 N-Heteroatome, und gegebenenfalls einfach oder an mehreren Ringgliedern einfach substituiert sein kann; C₁₋₆-Alkyl, gegebenenfalls einfach oder mehrfach substituiert; C₃₋₈-Cycloalkyl, gegebenenfalls einfach oder an mehreren Ringgliedern einfach substituiert;
   und außer für die Fälle, dass i) c = 1 oder ii) b = c = 0 und a = 1 ist, R¹⁸ auch ausgesucht sein kann aus der Gruppe bestehen aus -CN und NR¹⁹R²⁰, wobei R¹⁹ und R²⁰ unabhängig voneinander H oder C₁₋₆-Alkyl sein können aber nicht gleichzeitig für H stehen.

In bevorzugten Ausführungsformen der vorliegenden Erfindung ist die durch die NR⁵R⁶-Gruppe gebildete ringförmige Gruppe ausgesucht ist aus den Gruppe bestehend aus: 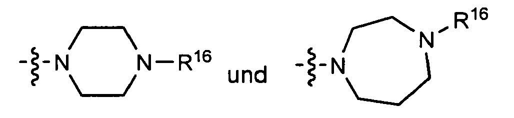 ,wobei R¹⁶ eine Gruppe der allgemeinen Formel bb1 darstellt und in der Formel bb1 (i) a = 0, b = 1, 2 oder 3 und c = 0 oder (ii) a = 0, b = 1, 2, oder 3 und c = 0 gilt. In der Formel bb1 ist R¹⁸ vorzugsweise ausgesucht aus der Gruppe bestehend aus: 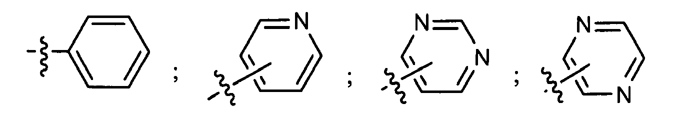 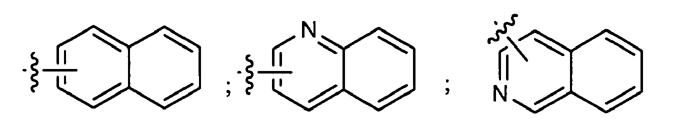 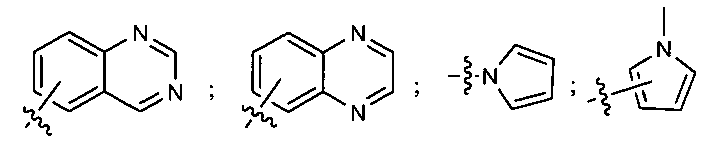 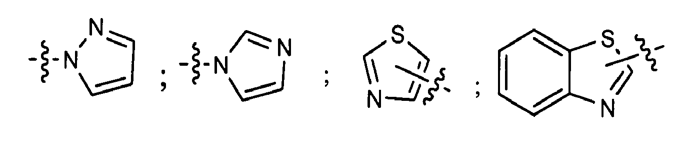 wobei diese Gruppen unsubstituiert, einfach oder mehrfach substituiert sein können, vorzugsweise mit C₁₋₃-Alkyl, insbesondere Methyl und/oder Ethyl; C₁₋₃-Alkoxy, insbesondere Methoxy; F, Cl, Br, I; -CN; CF₃; N(C₁₋₃-Alkyl)₂, NH(C₁₋₃-Alkyl), N(C₁₋₃-Alkyl)(Aryl), insbesondere N(C₁₋₃-Alkyl)(Phenyl oder Phenethyl), wobei Phenyl bzw. Phenethyl einfach oder mehrfach substituiert sein kann; Benzyl oder 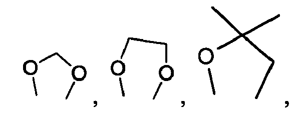 und wobei alle diese Substituenten ebenfalls einfach oder mehrfach substituiert sein können, vorzugsweise mit F, Cl, Br, -CN, -CF₃, C₁₋₃-Alkyl; Pyrrolidinyl, Piperidinyl, 4-Methylpiperindiyl oder Morpholinyl.

Desweiteren kann R¹⁸ ein Heterocyclyl darstellen, das ausgesucht ist aus der Gruppe bestehend aus: 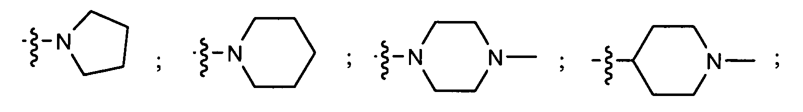 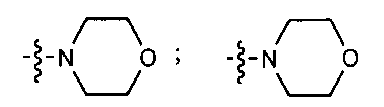 wobei diese Heterocyclylgruppen an einem oder mehreren Ringgliedern einfach substituiert sein können.

Alternativ kann R¹⁸ Cyclopentyl, Cyclohexyl, gegebenenfalls an einem oder mehreren Ringgliedern einfach substituiert, oder C₁₋₃-Alkyl, gegebenenfalls einfach oder mehrfach substituiert, darstellt.

In weiteren bevorzugten Ausführungsformen der Erfindung ist die durch die NR⁵R⁶-Gruppe gebildete ringförmige Gruppe 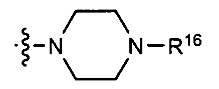 und R¹⁶ stellt eine Gruppe gemäß der oben dargestellten allgemeinen Formel bb1 dar, worin a = c = 0 und b = 0, 1 oder 2 gilt und R¹⁸ ausgesucht ist aus 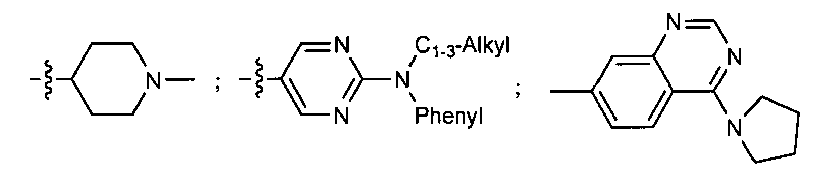 worin die Phenylgruppe substituiert sein kann, vorzugsweise einfach substituiert mit F, Cl, oder Br, insbesondere F, vorzugsweise in 4-Position,

In weiteren bevorzugten Ausführungsformen der erfindungsgemäßen Verbindungen ist:
c) in der Gruppe NR⁵R⁶ aus der allgemeinen Formel I R⁵ ausgesucht aus der Gruppe bestehend aus H und C₁₋₆-Alkyl, gegebenenfalls einfach oder mehrfach substituiert, und R⁶ ausgesucht aus Gruppen der nachfolgend dargestellten allgemeinen Formel c1 und c2: 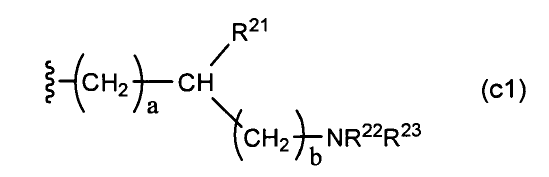 wobei in der Formel c1 folgendes gilt:
   a=0,1,oder2;
   b = 0, 1, 2, oder 3;
   R²¹ ist eine unsubstituierte oder substituierte Arylgruppe, vorzugsweise Phenyl, oder Naphthyl, beide substituiert oder unsubstituiert,
   R²² und R²³ unabhängig voneinander -H oder C₁₋₆-Alkyl darstellen oder die -NR²²R²³-Gruppe zusammen einen zumindest ein N-Heteroatom enthaltenden 5-, 6-, oder 7-gliedrigen, gesättigten oder ungesättigten Heterocyclus darstellen, der gegebenenfalls einfach oder an mehreren Ringgliedern einfach substituiert sein kann; 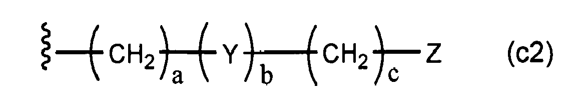
   wobei in der der Formel c2 folgendes gilt:
   a = 0, 1, 2 oder 3;
   b = 0 oder 1, mit der Maßgabe, dass wenn b = 0 auch c = 0 gilt;
   c = 0, 1, 2, oder 3
   Y für Aryl oder Heteroaryl, vorzugsweise N-Heteroatom-haltiges 5- oder 6-gliedriges Heteroaryl, gegebenenfalls substituiert; C₃₋₈-Cycloalkyl, vorzugsweise C₅₋₆-Cycloalkyl, steht;
   und
   Z eine gesättigte, ungesättigte oder aromatische, gegebenenfalls substituierte Heterocyclylgruppe darstellt, die zumindest ein N-Heteroatom enthält und als weitere Heteroatome O und/oder S enthalten kann; oder Z eine NR²⁴R²⁵-Gruppe darstellt, wobei R²⁴ und R²⁵ unabhängig voneinander H, C₁₋₆-Alkyl oder -C(=O)-(CH₂)_{d}-NR²⁶R²⁷, mit d = 1 oder 2, R²⁶ und R²⁷ unabhängig voneinander H oder C₁₋₆-Alkyl, oder die -NR²⁶R²⁷ einen 5- , 6- oder 7-gliedrigen, vorzugsweise gesättigten, Heterocyclus bilden;

In weiteren bevorzugten Ausführungsformen der erfindungsgemäßen Substanzen ist in der Gruppe NR⁵R⁶ aus der allgemeinen Formel I R⁵ ausgesucht aus der Gruppe bestehend aus H und Methyl, Ethyl, Propyl und Isopropyl, gegebenenfalls einfach oder mehrfach substituiert. R⁶ ist eine Gruppe gemäß der oben dargestellten allgemeinen Formel c1, worin:
R²¹ eine Phenyl oder Naphthylgruppe ist, die einfach oder mehrfach substituiert sein kann, und vorzugsweise unsubstituiert oder substituiert mit F, Cl, Br, I, CN, C₁₋₃-Alkyl, vorzugsweise Methyl oder Ethyl, ist,
R²² und R²³ unabhängig voneinander Methyl, Ethyl oder Propyl oder Isopropyl bedeuten oder die NR²²R²³-Gruppe zusammen einen N-Heterocyclus darstellt, der ausgesucht ist aus der Gruppe bestehend aus: 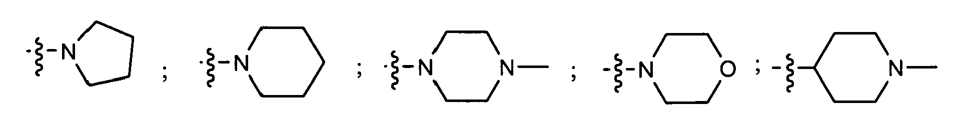
wobei diese N-Heterocyclen unsubstituiert, oder an einem oder mehreren Ringgliedern einfach substituiert sein können,
oder
R⁶ eine Gruppe der oben dargestellten allgemeinen Formel c2 darstellt, worin Y Phenyl, Naphthyl, Benzooxadiazol, Cyclopentyl, Cyclohexyl, oder Cycloheptyl darstellt, alle gegebenenfalls an einem oder mehreren Ringgliedern einfach substituiert, und
Z ausgesucht ist aus der Gruppe bestehend aus:
N(C₁₋₃-Alkyl)₂; 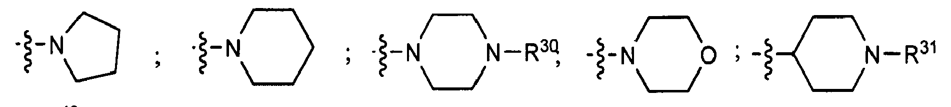 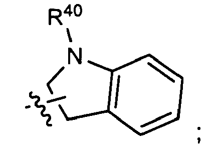 ; wobei diese Reste an einem oder mehreren Ringglieder einfach substituiert sein können, und R³⁰, R³¹ und R₄₀ H, Methyl, Ethyl, Propyl, oder Isopropyl, gegebenenfalls einfach oder mehrfach substituiert, darstellen können oder Z ausgesucht ist aus der Gruppe bestehend aus: 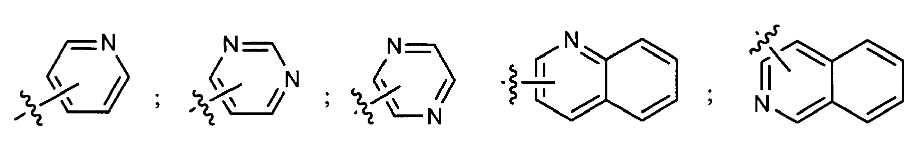 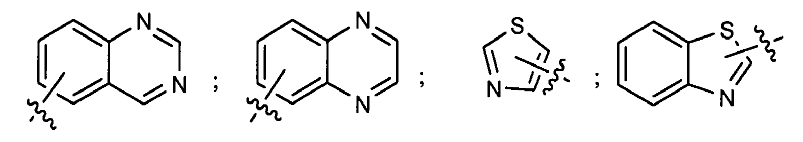 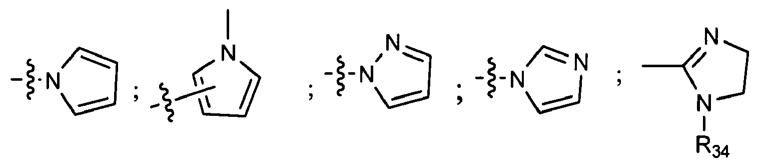 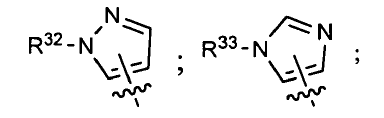 wobei R³², R³³ und R³⁴ ausgesucht sind aus H, Methyl, Ethyl, Propyl und Isopropyl, gegebenenfalls einfach oder mehrfach substituiert, und die N-Heterocyclen gegebenenfalls an einem oder mehreren Ringgliedern einfach substituiert sein können;
oder Z eine NR²⁴R²⁵-Gruppe ist, worin R²⁴ und R²⁵ unabhängig voneinander Methyl, Ethyl, Propyl oder Isopropyl darstellen oder Z eine -C(=O)-(CH₂)_{d}-NR²⁶R²⁷-Gruppe darstellt, wobei d 1 oder 2 ist und NR²⁶ R²⁷ zusammen einen Heterocyclus, ausgesucht aus 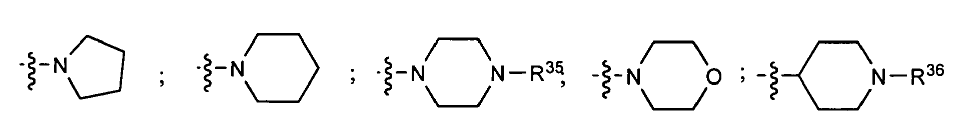 wobei die Ringglieder dieser Reste an einem oder mehreren Ringgliedern einfach substituiert sein können, und R³⁵ und R³⁶ Methyl, Ethyl, Propyl, oder Isopropyl, gegebenenfalls einfach oder mehrfach substituiert, darstellen können.

In weiteren bevorzugten Ausführungsformen der Erfindung ist in der Gruppe NR⁵R⁶ aus der allgemeinen Formel I R⁵ ausgesucht ist aus der Gruppe bestehend aus H, Methyl, Ethyl, Propyl und Isopropyl, vorzugsweise Methyl, und R⁶ stellt eine Gruppe gemäß der allgemeinen Formel c2 dar, worin a = 1 oder 2, b = 1 und c = 0 gilt, Y Phenyl oder Cyclohexyl darstellt und Z Pyrrolidinyl oder Dihydroimidazolyl darstellt.

In weiteren bevorzugten Ausführungsformen der erfindungsgemäßen Verbindungen ist:
d) in der Gruppe NR⁵R⁶ aus der allgemeinen Formel I R⁵ ausgesucht aus der Gruppe bestehend aus H, C₁₋₆-Alkyl, gegebenenfalls einfach oder mehrfach substituiert, C₃₋₈-Cycloalkyl, gegebenenfalls an einem oder mehreren Ringgliedern einfach substituiert. R⁶ ist ausgesucht ist aus Gruppen der allgemeinen Formel d1 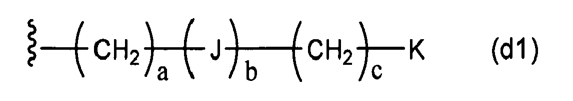 wobei in der Formel d1 folgendes gilt:
   a = 0, 1, 2, 3 oder 4;
   b = 0 oder 1, mit der Maßgabe, dass wenn b = 0 auch c = 0 gilt;
   c = 0, 1, 2 oder 3;
   J einen zumindest ein N-Heteroatom enthaltenden, vorzugsweise gesättigten oder ungesättigten 4-7-gliedrigen Heterocyclus darstellt, der gegebenenfalls an einem oder mehreren Ringgliedern einfach substituiert sein kann und K H oder eine gegebenenfalls substituierte Aryl- oder Heteroarylgruppe darstellt.

In weiteren bevorzugten Ausführungsformen der erfindungsgemäßen Verbindungen ist in der Gruppe NR⁵R⁶ aus der allgemeinen Formel I R⁵ ausgesucht ist aus der Gruppe bestehend aus H und Methyl, Ethyl, Propyl und Isopropyl, gegebenenfalls einfach oder mehrfach substituiert, und R⁶ stellt eine Gruppe gemäß der oben dargestellten allgemeinen Formel d1 dar, wobei J ausgesucht ist aus der Gruppe: 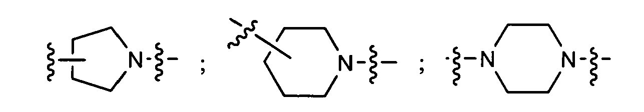 und K ausgesucht ist aus der Gruppe bestehend aus H, Phenyl, Naphthyl, Pyridinyl, alle gegebenenfalls einfach oder mehrfach substituiert.

In weiteren bevorzugten Ausführungsformen der erfindungsgemäßen Verbindungen sind:
e) in der Gruppe NR⁵R⁶ aus der allgemeinen Formel I R⁵ und R⁶ unabhängig voneinander ausgewählt sind aus der Gruppe bestehend aus H; C₁₋₆-Alkyl; Aralkyl; und -(CH₂)ᵣ-NR²⁸R²⁹, mit r = 1-6 und wobei R²⁸ und R²⁹ unabhängig voneinander ausgesucht sind aus der Gruppe bestehend aus H und C₁₋₃-Alkyl.

In weiteren bevorzugten erfindungsgemäßen Ausführungsformen ist in der Gruppe NR⁵R⁶ aus der allgemeinen Formel I:
R⁵ ausgesucht ist aus der Gruppe bestehend aus H, C₁₋₃-Alkyl, -(CH₂)ᵣ-NR²⁸R²⁹, mit r = 1-3 und wobei R²⁸ und R²⁹ C₁₋₃-Alkyl sein können, und
R⁶ ist-(CH₂)ᵣ-NR²⁸R²⁹, mit r = 1-3 und wobei R²⁸ und R²⁹ C₁₋₃-Alkyl sein können, oder R⁶ stellt eine mit einer N(C₁₋₃-Alkyl)(Aryl)-gruppe substituierte C₁₋₃-Alkylgruppe dar, wobei die Arylgruppe substituiert sein kann und Aryl vorzugsweise für Phenyl oder Naphthyl steht.

In weiteren bevorzugten Ausführungsformen der erfindungsgemäßen Verbindungen ist:
f) in der Gruppe NR⁵R⁶ aus der allgemeinen Formel I R⁵ ausgesucht aus der Gruppe bestehend aus H, C₁₋₆-Alkyl, Aryl und Aralkyl, und R⁶ eine Gruppe vom Typ -(C₁₋₄-Alkyl)ₛ-X, mit s = 0 oder 1, wobei die Gruppe X eine mindestens ein N-Heteroatom enthaltende Heteroarylgruppe ist, die gegebenenfalls einfach oder mehrfach substituiert ist und wobei in der C₁₋₄-Alkylgruppe ein H-Atom durch eine 5- oder 6-gliedrige, gesättigte, zumindest ein N-Heteroatom enthaltende Heterocyclylgruppe, die neben N noch O und/oder S als Heteroatom enthalten kann, ersetzt sein kann.

In weiteren bevorzugten erfindungsgemäßen Ausführungsformen stellt in der Gruppe NR⁵R⁶ aus der allgemeinen Formel I:
R⁵ H, Methyl, Ethyl, Propyl, Isopropyl, Phenyl oder Benzyl dar und
R⁶ eine Gruppe vom Typ (C₁₋₃-Alkyl)ₛ-X, mit s = 0 oder 1, wobei die C₁₋₃-Alkylgruppe mit einer Pyrrolidinyl, Piperidinyl, Piperazinyl oder Morpholinylgruppe substituiert sein kann und der Rest X ausgesucht ist aus der Gruppe bestehend aus: 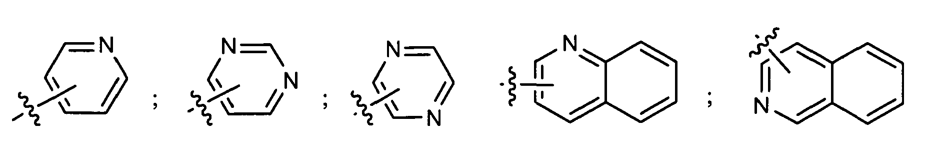 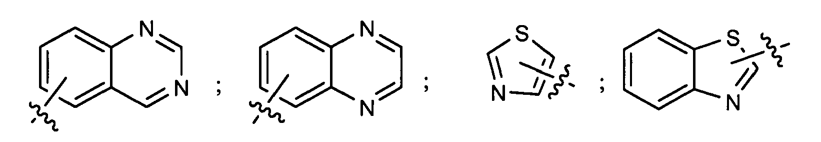 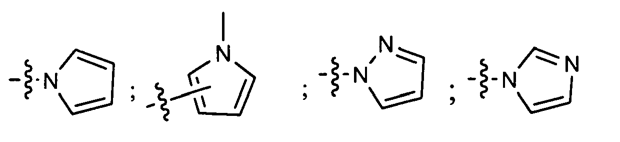 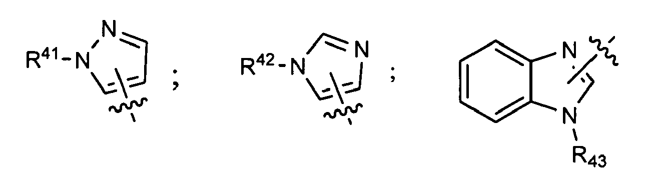
wobei R⁴¹, R⁴² und R⁴³ ausgesucht sind aus H, Methyl, Ethyl, Propyl und Isopropyl, gegebenenfalls einfach oder mehrfach substituiert, und die N-Heteroaryle gegebenenfalls einfach oder mehrfach substituiert sein können.

In weiteren bevorzugten Ausführungsformen gemäß der vorliegenden Erfindung gilt in den Verbindungen gemäß der allgemeinen Formel das:
n für 0, 1, 2 oder 3 steht;
m für 1 oder 2 steht;
R¹ für Aryl oder Heteroaryl, unsubstituiert oder einfach oder mehrfach substituiert, steht;
R^{2a-c}, R³ und R⁴ für H stehen oder mit einem benachbarten Rest R^{2a-c}, R³ oder R⁴ einen fünf- oder sechsgliedrigen Ring bilden, der gesättigt oder ungesättigt und einfach oder mehrfach substituiert sein kann und Heteroatome aus der Gruppe N oder O enthalten kann,
R⁵ und R⁶ zusammen einen 4-8-gliedrigen Ring bilden, der gesättigt oder ungesättigt, aber nicht aromatisch sein kann, mit einem basischen Rest substituiert oder kondensiert ist und mit einem weiteren basischen Rest oder Resten aus der Gruppe C₁₋₆-Alkyl, C₁₋₃-Alkyloxy, C₃₋₈-Cycloalkyl oder gegebenenfalls substituiertem Phenyl substituiert sein kann;
oder R⁵ und R⁶ zusammen einen 4-8-gliedrigen Ring bilden, der ein weiteres Heteroatom aus der Gruppe N oder O enthält und mit einem basischen oder nicht basischen Rest substituiert sein kann,
oder R⁵ H oder C₁₋₅-Alkyl bedeutet und R⁶ Aryl oder C₃₋₈-Cycloalkyl; oder einen über eine C₁₋₃-Alkylkette verknüpften Arylrest bedeutet, wobei der Aryl- oder C₃₋₈-Cycloalkyl-Ring jeweils mit einem basischen Rest substituiert ist oder gegebenenfalls die basische Substitution an der verbrückenden C₁₋₃-Alkylkette erfolgt,
oder R⁵ H oder C₁₋₅-Alkyl bedeutet und R⁶ C₄₋₈-Heterocydyl; oder einen über eine C₁-₃-Alkylkette verknüpften C₄₋₈-Heterocyclylrest bedeutet, wobei der Heterocyclyl-Ring mit einem basischen Rest oder nicht basischen Rest substituiert ist,
oder
R⁵ und R⁶ unabhängig voneinander für H oder einen verzweigten oder unverzweigten C₁₋₁₀-Alkylrest steht, der ein bis drei Stickstoffatome enthält, wobei R⁵ und R⁶ nicht beide H bedeuten,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren,
wobei vorzugsweise:
ein substituierter Aryl- oder Heteroarylrest mit F, Cl, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁-₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 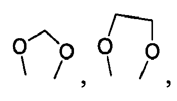 C₁₋₆-Alkyl, Phenoxy, Phenyl, Pyridyl, Thienyl oder Furyl substituiert ist,
ein substituierter Cycloalkylrest oder Alkylrest mit F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl substituiert ist,
ein basischer Rest vorzugsweise für Piperidin, Pyrrolidin, Azepan, Azetidin, Azocan, Pyridin, Imidazol, Imidazolidin, 1,2,4-Triazol, Diazepan, Pyrimidin, Imidazolin, Piperazin, N(C₁₋₆-Alkyl)₂, NHC₁₋₆-Alkyl, einem mit N(C₁₋₆-Alkyl)₂ substituierten Arylrest; wobei diese Reste alle über eine C₁₋₃-Alkylgruppe mit der Struktur der allgemeinen Formel I verknüpft sein können, die C₁₋₃-Alkylkette gegebenenfalls mit =O substituiert ist und die übrigen Reste ihrerseits mit C₁₋₆-Alkyl substituiert sein können; C₁₋₆-AlkylN(C₁₋₆-Alkyl)₂ oder C₁₋₆-AlkylNH(C₁₋₆-Alkyl), steht
und ein nicht basischer Rest vorzugsweise für Aryl, Heteroaryl, jeweils unsubstituiert oder einfach oder mehrfach substituiert mit F, Cl, Br, I, CN, , NO₂, SH, S-C₁₋₆-Alkyl, OH, O-C₁₋₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 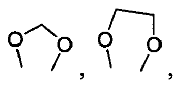 C₁₋₆-Alkyl; die über eine C₁₋₃-Alkylkette mit der Struktur der allgemeinen Formel verknüpft sein können, wobei die Alkylkette mit =O substituiert sein kann; C₁₋₆-Alkyl, gegebenenfalls substituiert mit Methoxy oder C₁₋₃-Alkyloxy; oder C₃₋₈-Cycloalkyl steht.

Weiterhin bevorzugt im Sinne dieser Erfindung sind substituierte Sulfonamid-Derivate der allgemeinen Formel I, worin R⁵ und R⁶ zusammen einen 4-8-gliedrigen Ring bilden, der gesättigt oder ungesättigt, aber nicht aromatisch sein kann, und mit einem basischen Rest aus der Gruppe 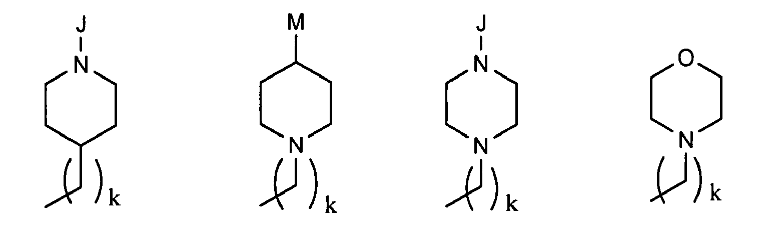 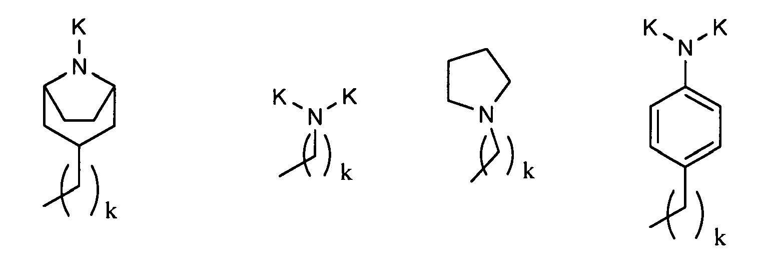 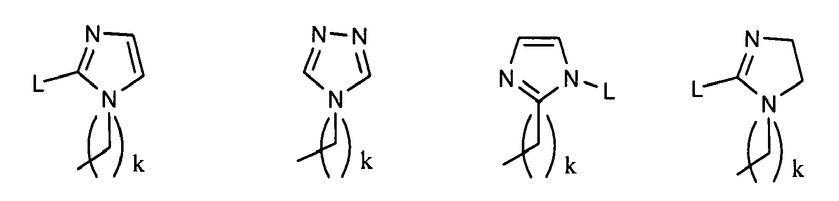 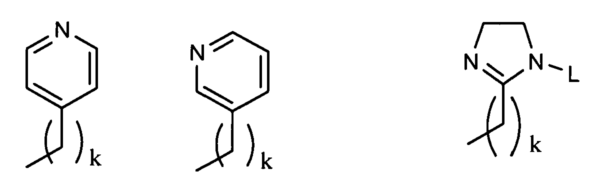 wobei
k für 0, 1 oder 2 steht
L für H oder C₁₋₆-Alkyl steht
K für C₁₋₆-Alkyl steht
M für C₁₋₆-Alkyl oder N(CH₃)₂ steht
J für 2-, 3- oder 4-Pyridyl, Phenyl, Piperidyl oder C₁₋₆-Alkyl steht substituiert ist.

Besonders bevorzugt sind substituierte Sulfonamid-Derivate, worin die Gruppe 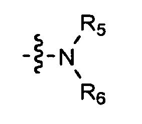 für Piperidin, Pyrrolidin oder Azepan, substituiert mit gegebenenfalls über C₁₋₃-Alkyl verknüpftem Piperidin, Pyrrolidin, Azepan, Piperazin oder Diazepan, unsubstituiert oder einfach substituiert mit Methyl oder Ethyl, mit der Maßgabe, dass die Verknüpfung zwischen zwei C-Atomen oder einem C- und einem N-Atom erfolgt, nicht aber zwischen zwei N-Atomen;
insbesondere
für einen Rest aus der Gruppe 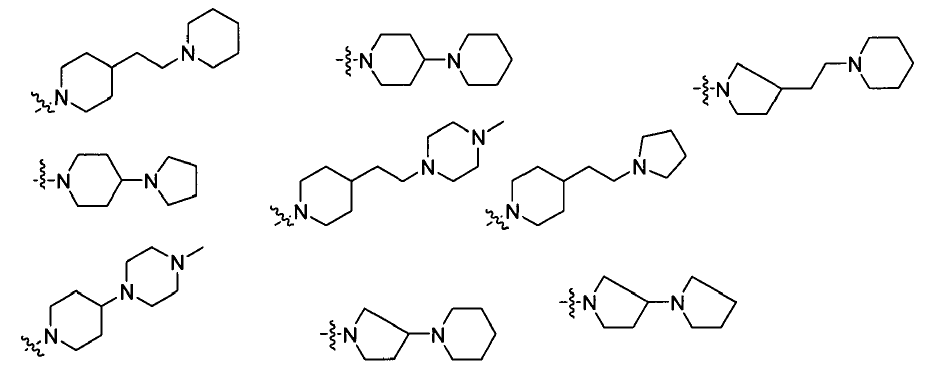 steht.

Bevorzugt sind auch substituierte Sulfonamid-Derivate der allgemeinen Formel I, worin R⁵ und R⁶ zusammen einen 4-8-gliedrigen Ring bilden, der ein weiteres Heteroatom aus der Gruppe N oder O enthält und mit einem basischen Rest aus der Gruppe 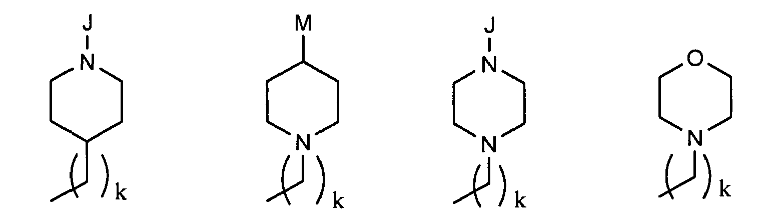 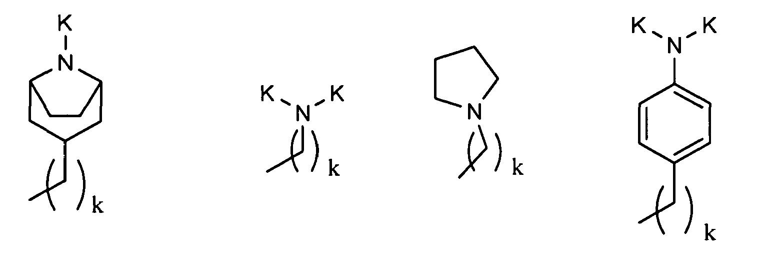 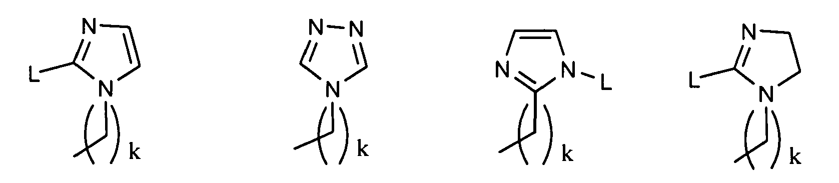 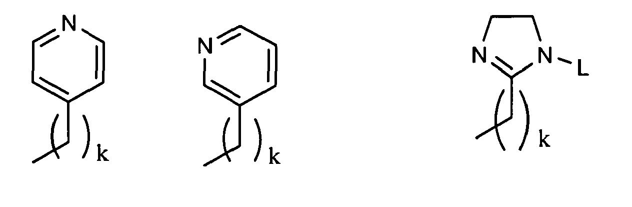 wobei
k für 0, 1 oder 2 steht
L für H oder C₁₋₆-Alkyl steht
K für C₁₋₆-Alkyl steht
M für C₁₋₆-Alkyl oder N(CH₃)₂ steht
J für 2-, 3- oder 4-Pyridyl, Phenyl, Piperidyl oder C₁₋₆-Alkyl steht
oder einem nicht basischen Rest aus der Gruppe Aryl, Heteroaryl, jeweils unsubstituiert oder einfach oder mehrfach substituiert mit F, Cl, Br, I, CF₃, OCH₃, C₁-₆-Alkyl, C₃₋₈-Cycloalkyl, unsubstituiert oder einfach substituiert mit F, Cl, Br, I, CF₃, OCH₃, C₁₋₆-Alkyl; die über eine C₁₋₃-Alkylkette mit der Struktur der allgemeinen

Formel verknüpft sein können, wobei die Alkylkette mit =O substituiert sein kann; C₁₋₆-Alkyl oder C₁₋₃-Alkyloxy;
substituiert sein kann.

Besonders bevorzugt sind substituierte Sulfonamid-Derivate, worin die Gruppe 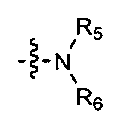 für Piperazin oder Diazepan, substituiert mit gegebenenfalls über C₁₋₃-Alkyl gebundenem Phenyl, unsubstituiert oder einfach oder mehrfach substituiert mit 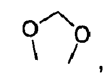 Methyl, Methoxy, F, Cl, Br, CF₃ oder CN; (CH₂)₂OCH₃; substituiert mit gegebenenfalls über C₁₋₃-Alkyl gebundenem Cyclohexyl oder Cyclopentyl; Pyrrolidin, Piperazin, Piperidin, unsubstituiert oder einfach substituiert mit Methyl oder Ethyl, jeweils verknüpft über eine C₁₋₃-Alkylgruppe; oder Pyrrolidin, Piperazin, Piperidin, unsubstituiert oder einfach substituiert mit Methyl oder Ethyl, mit der Maßgabe, dass die Verknüpfung zwischen zwei C-Atomen oder einem C- und einem N-Atom erfolgt, nicht aber zwischen zwei N-Atomen; oder mit (CH₂)ₐN(CH₃)₂ mit a = 2, 3; steht,
insbesondere für einen Rest aus der Gruppe 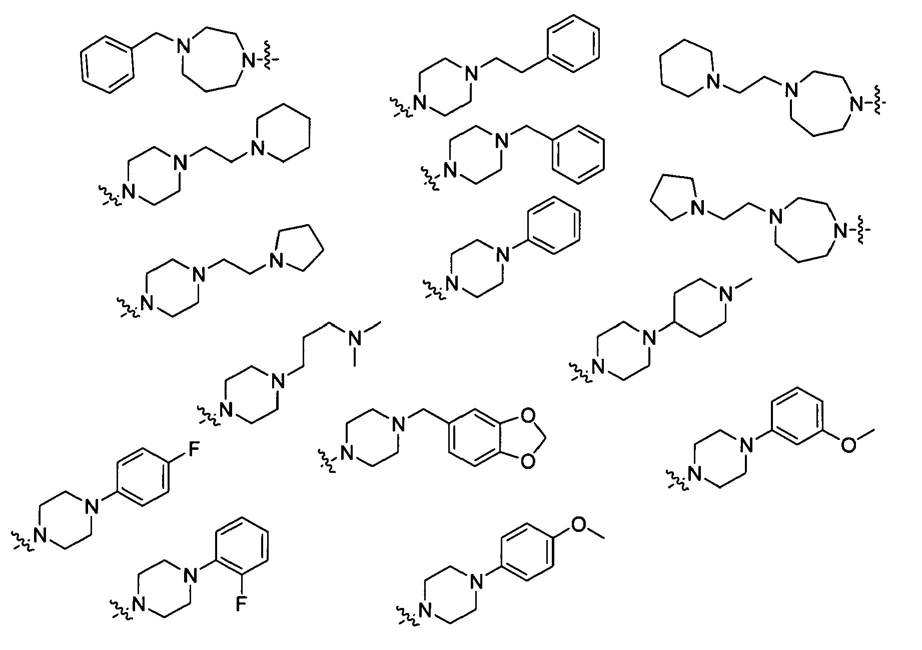 steht.

Darüber hinaus bevorzugt sind substituierte Sulfonamid-Derivate der allgemeinen Formel I, worin R⁵ H bedeutet und R⁶ Aryl oder C₃₋₈-Cycloalkyl; oder einen über eine C₁₋₃-Alkylkette verknüpften Arylrest bedeutet, wobei der Aryl- oder C₃₋₈-Cycloalkyl-Ring jeweils mit mindestens einem basischen Rest aus der Gruppe 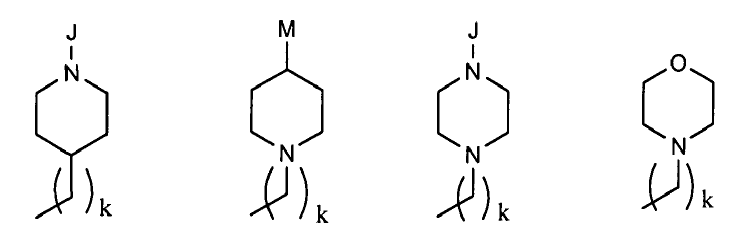 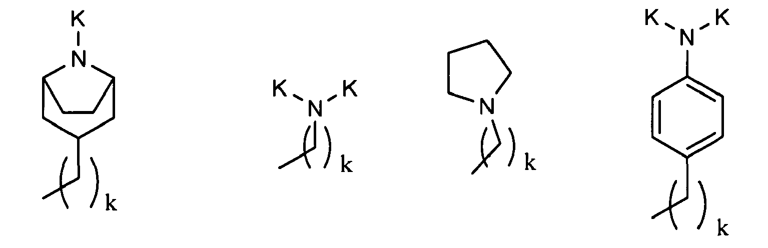 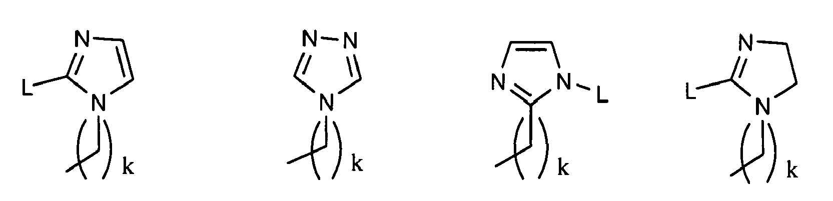 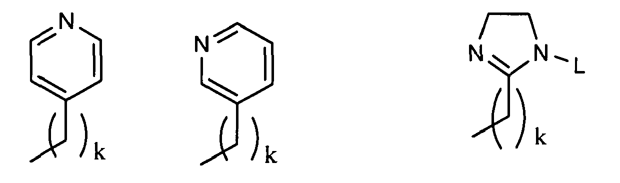 wobei
k für 0, 1 oder 2 steht
L für H oder C₁₋₆-Alkyl steht
K für C₁₋₆-Alkyl steht
M für C₁₋₆-Alkyl oder N(CH₃)₂ steht
J für 2-, 3- oder 4-Pyridyl, Phenyl, Piperidyl oder C₁₋₆-Alkyl steht
substituiert ist.

Besonders bevorzugt sind substituierte Sulfonamid-Derivate der allgemeinen Formel I, worin R⁵ H oder CH₃ und R⁶ Benzyl oder Phenethyl substituiert mit Pyrrolidin, Piperazin, Morpholin oder Piperidin, wobei diese Reste ihrerseits unsubstituiert oder einfach substituiert mit Methyl oder Ethyl und gegebenenfalls über eine C₁₋₃-Alkylkette verknüpft sind, wobei der Benzyl- oder Phenethyl-Rest gegebenenfalls mit einem weiteren basischen Rest substituiert ist,
insbesondere R⁶ 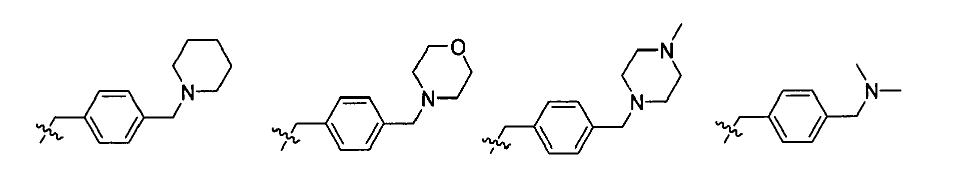 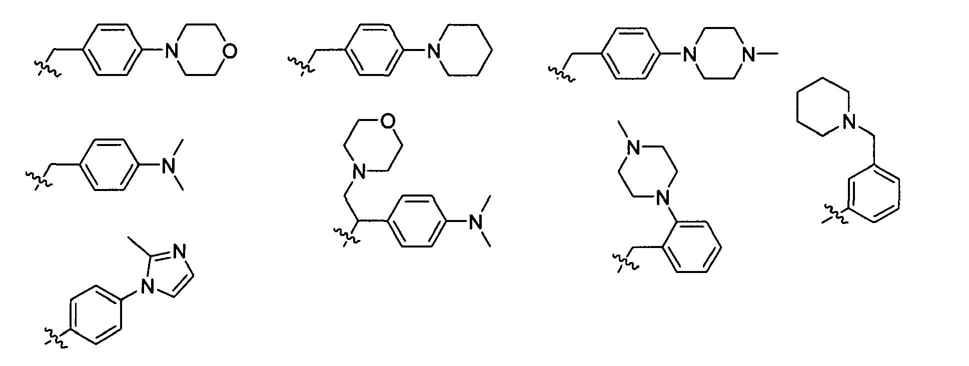 bedeutet.

Bevorzugt sind auch substituierte Sulfonamid-Derivate, worin R⁵ H oder C₁₋₅-Alkyl bedeutet und R⁶ C₄₋₈-Heterocyclyl, wobei der Heterocyclyl-Rest über ein C-Atom mit der Struktur der allgemeinen Formel I verknüpft ist; oder einen über eine C₁₋₃-Alkylkette verknüpften C₄₋₈-Heterocyclylrest bedeutet, wobei der Heterocyclyl-Ring substituiert ist mit einem basischen Rest aus der Gruppe 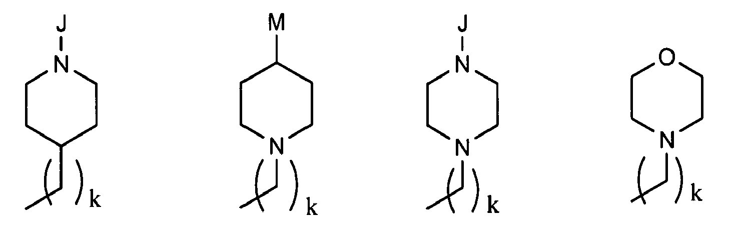 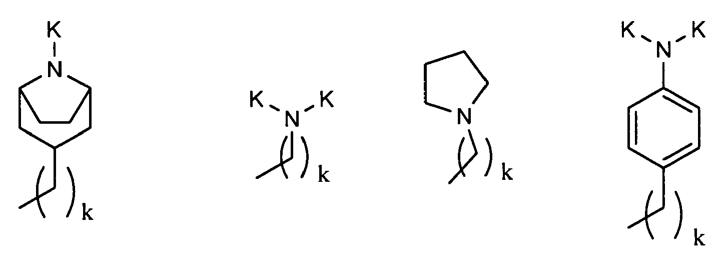 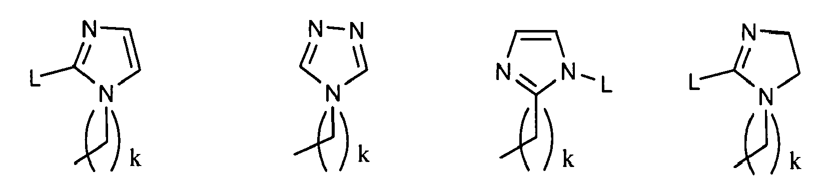 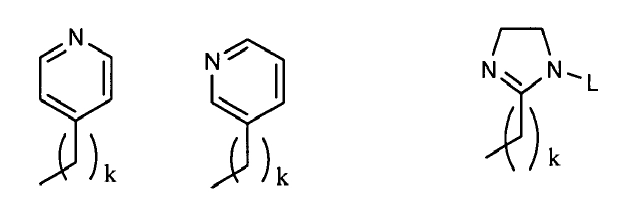 wobei
k für 0, 1 oder 2 steht
L für H oder C₁₋₆-Alkyl steht
K für C₁₋₆-Alkyl steht
M für C₁₋₆-Alkyl oder N(CH₃)₂ steht
J für 2-, 3- oder 4-Pyridyl, Phenyl, Piperidyl oder C₁₋₆-Alkyl steht
oder einem nicht basischen Rest aus der Gruppe Aryl, Heteroaryl, jeweils unsubstituiert oder einfach oder mehrfach substituiert mit F, Cl, Br, I, CF₃, OCH₃, C₁-₆-Alkyl, C₃₋₈-Cycloalkyl, unsubstituiert oder einfach oder mehrfach substituiert mit F, Cl, Br, I, CF₃, OCH₃, C₁₋₆-Alkyl; die über eine C₁₋₃-Alkylkette mit der Struktur der allgemeinen Formel verknüpft sein können, wobei die Alkylkette mit =O substituiert sein kann; C₁₋₆-Alkyl oder C₁₋₃-Alkyloxy;
substituiert ist.

Besonders bevorzugt sind substituierte Sulfonamid-Derivate, worin R⁵ H, Methyl oder Ethyl bedeutet und R⁶ für Piperidin, Pyrrolidin, Azepan; über eine C₁₋₃-Alkylkette verknüpftes Piperidin, Pyrrolidin, Azepan, Diazepan oder Piperazin; jeweils substituiert mit über eine C₁₋₃-Alkylkette verknüpftem Phenyl, Pyrrolidin, Piperazin, Morpholin oder Piperidin, unsubstituiert oder einfach substituiert mit Methyl oder Ethyl.

Ganz besonders bevorzugt sind substituierte Sulfonamid-Derivate, worin die Gruppe 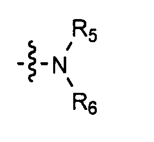 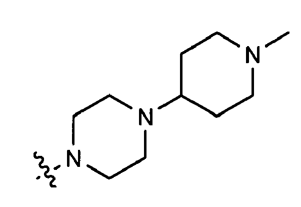 bedeutet.

Ganz besonders bevorzugt sind substituierte Sulfonamid-Derivate aus der Gruppe
- **1**: 2-[1-(4-Methoxy-2,6-dimethyl-phenylsulfonyl)-pyrrolidin-2-ylmethoxy]-N-(4-piperidin-1-ylmethyl-benzyl)-acetamid
- **2**: 2-[1-(4-Methoxy-2,6-dimethyl-phenylsulfonyl)-piperidin-2-ylmethoxy]-N-(4-piperidin-1-ylmethyl-benzyl)-acetamid
- **3**: 2-[1-(4-Methoxy-2,6-dimethyl-phenylsulfonyl)-piperidin-2-ylmethoxy]-N-[4-(4-methyl-piperazin-1-yl)-benzyl]-acetamid
- **4**: N-(4-Piperidin-1-ylmethyl-benzyl)-2-[1-(2,4,6-trichlor-phenylsulfonyl)-piperidin-2-ylmethoxy]-acetamid
- **5**: 1-[4-(1-Methyl-piperidin-4-yl)-piperazin-1-yl]-2-[2-(2,4,6-trimethyl-phenylsulfonyl)-1,2,3,4-tetrahydro-isochinolin-3-ylmethoxy]-ethanon
- **6**: 1-(4-Benzyl-[1,4]diazepan-1-yl)-2-[1-(naphthyl-1-sulfonyl)-piperidin-2-ylmethoxy]-ethanon
- **7**: 2-[1-(4-Methoxy-2,6-dimethyl-phenylsulfonyl)-piperidin-2-ylmethoxy]-N-[4-(4-methyl- piperazin-1-ylmethyl)-benzyl]-acetamid
- **8**: 2-[1-(4-Methoxy-2,6-dimethyl-phenylsulfonyl)-piperidin-2-ylmethoxy]-1-[4-(1-methyl-piperidin-4-yl)-piperazin-1-yl]-ethanon
- **9**: 1-(1,4'-bipiperidin-1'-yl)-2-((2-(2,4-dichlorphenylsulfonyl)-1,2,3,4-tetrahydroisochinolin-3-yl)methoxy)ethanon
- **10**: N-(4-Piperidin-1-ylmethyl-benzyl)-2-[1-(2,4,6-trichlor-phenylsulfonyl)-pyrrolidin-2-ylmethoxy]-acetamid
- **11**: N-(4-Dimethylaminomethyl-benzyl)-2-[1-(4-methoxy-2,6-dimethyl-phenylsulfonyl)-piperidin-2-ylmethoxy]-acetamid
- **12**: N-(1-Benzyl-piperidin-3-ylmethyl)-2-[1-(2,4,6-trimethyl-phenylsulfonyl)-piperidin-2-ylmethoxy]-acetamid
- **13**: N-[4-(4-Methyl-piperazin-1-yl)-benzyl]-2-[1-(2,4,6-trichlor-phenylsulfonyl)-piperidin-2-ylmethoxy]-acetamid
- **14**: 2-[1-(4-Methoxy-2,6-dimethyl-phenylsulfonyl)-piperidin-2-ylmethoxy]-N-(4-piperidin-1-yl-benzyl)-acetamid
- **15**: N-[4-(4-Methyl-piperazin-1-ylmethyl)-benzyl]-2-[1-(2,4,6-trichlor-phenylsulfonyl)-piperidin-2-ylmethoxy]-acetamid
- **16**: N-(1-Benzyl-piperidin-3-ylmethyl)-2-[1-(3,4-dichlor-phenylsulfonyl)-2,3-dihydro-1 H-indol-2-ylmethoxy]-acetamid
- **17**: 2-((2-(2,4-dichlorphenylsulfonyl)-1,2,3,4-tetrahydroisochinolin-3-yl)methoxy)-1-(4-(pyrrolidin-1-yl)piperidin-1-yl)ethanon
- **18**: 2-[1-(4-Methoxy-2,6-dimethyl-phenylsulfonyl)-pyrrolidin-2-ylmethoxy]-N-[4-(4-methyl-piperazin-1-yl)-benzyl]-acetamid
- **19**: 2-((1-(mesitylsulfonyl)piperidin-2-yl)methoxy)-1-(4-(pyrrolidin-1-yl)piperidin-1-yl)ethanon
- **20**: 2-((1-(3,4-dichlorphenylsulfonyl)piperidin-2-yl)methoxy)-1-(4-(2-(piperidin-1-yl)ethyl)piperidin-1-yl)ethanon
- **21**: 2-[1-(4-Methoxy-2,6-dimethyl-phenylsulfonyl)-piperidin-2-ylmethoxy]-N-(4-morpholin-4-yl-benzyl)-acetamid
- **22**: 1-[4-(1-Methyl-piperidin-4-yl)-piperazin-1-yl]-2-[1-(3-trifluormethyl-phenylsulfonyl)-piperidin-2-ylmethoxy]-ethanon
- **23**: 2-((1-(naphthalen-1-ylsulfonyl)piperidin-2-yl)methoxy)-1-(4-(pyrrolidin-1-yl)piperidin-1-yl)ethanon
- **24**: 2-[1-(4-Methoxy-2,6-dimethyl-phenylsulfonyl)-piperidin-2-ylmethoxy]-N-(4-morpholin-4-ylmethyl-benzyl)-acetamid
- **25**: 1-[4-(3-Dimethylamino-propyl)-piperazin-1-yl]-2-[2-(2,4,6-trimethyl-phenylsulfonyl)-1,2,3,4-tetrahydro-isochinolin-3-ylmethoxy]-ethanon
- **26**: 2-[1-(3,4-Dichlor-phenylsulfonyl)-piperidin-2-ylmethoxy]-1-(4-pyrrolidin-1-yl-piperidin-1-yl)-ethanon
- **27**: 1-(2-Piperidin-1-ylmethyl-pyrrolidin-1-yl)-2-[1-(2,4,6-trimethyl-phenylsulfonyl)-piperidin-2-ylmethoxy]-ethanon
- **28**: 2-[1-(3,4-Dichlor-phenylsulfonyl)-piperidin-2-ylmethoxy]-1-[4-(4-methyl-piperazin-1-yl)-piperidin-1-yl]-ethanon
- **29**: N-[4-(4-Methyl-piperazin-1-yl)-benzyl]-2-[1-(2,4,6-trichlor-phenylsulfonyl)-pyrrolidin-2-ylmethoxy]-acetamid
- **30**: N-(4-Morpholin-4-ylmethyl-benzyl)-2-[1-(2,4,6-trichlor-phenylsulfonyl)-piperidin-2-ylmethoxy]-acetamid
- **31**: 1-(4-Benzyl-[1,4]diazepan-1-yl)-2-[1-(2,4,6-trimethyl-phenylsulfonyl)-piperidin-2-ylmethoxy]-ethanon
- **32**: 2-[1-(3,4-Dichlor-phenylsulfonyl)-piperidin-2-ylmethoxy]-1-[4-(1-methyl-piperidin-4-yl)-piperazin-1-yl]-ethanon
- **33**: N-[3-(4-Methyl-piperazin-1-yl)-benzyl]-2-[2-(2,4,6-trimethyl-phenylsulfonyl)-1,2,3,4-tetrahydro-isochinolin-3-ylmethoxy]-acetamid
- **34**: N-(1-Benzyl-piperidin-3-ylmethyl)-2-[1-(naphthyl-1-sulfonyl)-piperidin-2-ylmethoxy]-acetamid
- **35**: N-(4-Dimethylaminomethyl-benzyl)-2-[1-(4-methoxy-2,6-dimethyl-phenylsulfonyl)-pyrrolidin-2-ylmethoxy]-acetamid
- **36**: 2-((1-(3,4-dichlorphenylsulfonyl)pyrrolidin-2-yl)methoxy)-1-(4-(2-(piperidin-1-yl)ethyl)piperidin-1-yl)ethanon
- **37**: N-(4-Dimethylaminomethyl-benzyl)-2-[1-(2,4,6-trichlor-phenylsulfonyl)-piperidin-2-ylmethoxy]-acetamid
- **38**: 2-((2-(3,4-dichlorphenylsulfonyl)-1,2,3,4-tetrahydroisochinolin-3-yl)methoxy)-1-(4-(pyrrolidin-1-yl)piperidin-1-yl)ethanon
- **39**: 1-(4-Benzo[1,3]dioxol-5-ylmethyl-piperazin-1-yl)-2-[1-(3,4-dichlor-phenylsulfonyl)-piperidin-2-ylmethoxy]-ethanon
- **40**: 2-[1-(4-Methoxy-2,6-dimethyl-phenylsulfonyl)-pyrrolidin-2-ylmethoxy]-N-[4-(4-methyl-piperazin-1-ylmethyl)-benzyl]-acetamid
- **41**: N-(1-Benzyl-piperidin-3-ylmethyl)-2-[2-(2,4,6-trimethyl-phenylsulfonyl)-1,2,3,4-tetrahydro-isochinolin-3-ylmethoxy]-acetamid
- **42**: 2-((1-(3,4-dichlorphenylsulfonyl)piperidin-2-yl)methoxy)-1-(4-(2-(piperidin-1-yl)ethyl)piperazin-1-yl)ethanon
- **43**: 2-[2-(3,4-Dichlor-phenylsulfonyl)-1,2,3,4-tetrahydro-isochinolin-3-ylmethoxy]-N,N-bis-(2-diethylamino-ethyl)-acetamid
- **44**: 1-(4-Benzo[1,3]dioxol-5-ylmethyl-piperazin-1-yl)-2-[1-(3,4-dichlor-phenylsulfonyl)-pyrrolidin-2-ylmethoxy]-ethanon
- **45**: N-[4-(4-Methyl-piperazin-1-ylmethyl)-benzyl]-2-[1-(2,4,6-trichlor-phenylsulfonyl)-pyrrolidi n-2-ylmethoxy]-acetamid
- **46**: 2-[1-(3,4-Dichlor-phenylsulfonyl)-piperidin-2-ylmethoxy]-1-[4-(2-methoxy-ethyl)-piperazin-1-yl]-ethanon
- **47**: N-Methyl-N-(2-morpholin-4-yl-1-phenyl-ethyl)-2-[1-(naphthyl-1-sulfonyl)-piperidin-2-ylmethoxy]-acetamid
- **48**: N,N-Bis-(2-diethylamino-ethyl)-2-[2-(2,4,6-trimethyl-phenylsulfonyl)-1,2,3,4-tetrahydro-isochinolin-3-ylmethoxy]-acetamid
- **49**: N-(4-Morpholin-4-ylmethyl-benzyl)-2-[1-(2,4,6-trichlor-phenylsulfonyl)-pyrrolidin-2-ylmethoxy]-acetamid
- **50**: N-(3-Morpholin-4-yl-benzyl)-2-[2-(2,4,6-trimethyl-phenylsulfonyl)-1,2,3,4-tetrahydro-isochinolin-3-ylmethoxy]-acetamid
- **51**: 2-[2-(2,4-Dichlor-phenylsulfonyl)-1,2,3,4-tetrahydro-isochinolin-3-ylmethoxy]-1-(2-piperidin-1-ylmethyl-pyrrolidin-1-yl)-ethanon
- **52**: 2-((1-(4-methoxyphenylsulfonyl)piperidin-2-yl)methoxy)-1-(4-(2-(piperidin-1- yl)ethyl)piperidin-1-yl)ethanon
- **53**: 2-[1-(3,4-Dichlor-phenylsulfonyl)-piperidin-2-ylmethoxy]-1-[4-(3-methoxy-phenyl)-piperazin-1-yl]-ethanon
- **54**: 1-(4-Cyclohexylmethyl-piperazin-1-yl)-2-[1-(3,4-dichlor-phenylsulfonyl)-pyrrolidin-2-ylmethoxy]-ethanon
- **55**: 2-[1-(4-Methoxy-2,6-dimethyl-phenylsulfonyl)-pyrrolidin-2-ylmethoxy]-N-(4-morpholin-4-ylmethyl-benzyl)-acetamid
- **56**: 2-[1-(3,4-Dichlor-phenylsulfonyl)-piperidin-2-ylmethoxy]-1-(4-phenyl-piperazin-1-yl)-ethanon
- **57**: 1-(4-Benzyl-piperidin-1-yl)-2-[1-(3,4-dichlor-phenylsulfonyl)-piperidin-2-ylmethoxy]-ethanon
- **58**: 1-[4-(3-Dimethylamino-propyl)-piperazin-1-yl]-2-[1-(3-trifluormethyl-phenylsulfonyl)-piperidin-2-ylmethoxy]-ethanon
- **59**: 2-[1-(3,4-Dichlor-phenylsulfonyl)-piperidin-2-ylmethoxy]-1-[4-(4-fluor-phenyl)-piperazin-1-yl]-ethanon
- **60**: 2-[1-(4-Methoxy-2,6-dimethyl-phenylsulfonyl)-pyrrolidin-2-ylmethoxy]-N-(4-piperidin-1-yl-benzyl)-acetamid
- **61**: N-(4-Morpholin-4-ylmethyl-benzyl)-2-[1-(naphthyl-1-sulfonyl)-piperidin-2-ylmethoxy]-acetamid
- **62**: 2-[1-(3,4-Dichlor-phenylsulfonyl)-pyrrolidin-2-ylmethoxy]-1-[4-(1-methyl-piperidin-4-yl)-piperazin-1-yl]-ethanon
- **63**: 1-[2-(4-Dimethylamino-phenyl)-azepan-1-yl]-2-[1-(naphthyl-1-sulfonyl)-piperidin-2-ylmethoxy]-ethanon
- **64**: 2-[1-(naphthyl-1-sulfonyl)-piperidin-2-ylmethoxy]-1-(2-piperidin-1-ylmethyl-pyrrolidin-1-yl)-ethanon
- **65**: 2-((2-(2,4-dichlorphenylsulfonyl)-1,2,3,4-tetrahydroisochinolin-3-yl)methoxy)-1-(4-morpholinopiperidin-1-yl)ethanon
- **66**: 1-(3-Dimethylamino-pyrrolidin-1-yl)-2-[1-(naphthyl-1-sulfonyl)-piperidin-2-ylmethoxy]-ethanon
- **67**: 1-(4-Benzyl-[1,4]diazepan-1-yl)-2-[2-(2,4,6-trimethyl-phenylsulfonyl)-1,2,3,4-tetrahydro-isochinolin-3-ylmethoxy]-ethanon
- **68**: 1-(4-Cyclohexylmethyl-piperazin-1-yl)-2-[1-(3,4-dichlor-phenylsulfonyl)-piperidin-2-ylmethoxy]-ethanon
- **69**: 2-((2-(4-methoxyphenylsulfonyl)-1,2,3,4-tetrahydroisochinolin-3-yl)methoxy)-1-(4-(2-(piperidin-1-yl)ethyl)piperidin-1-yl)ethanon
- **70**: N-(1-Benzyl-piperidin-3-ylmethyl)-2-[1-(3,4-dichlor-phenylsulfonyl)-piperidin-2-ylmethoxy]-acetamid
- **71**: N-(4-Piperidin-1-yl-benzyl)-2-[1-(2,4,6-trichlor-phenylsulfonyl)-pyrrolidin-2-ylmethoxy]-acetamid
- **72**: 2-((1-(3,4-dichlorphenylsulfonyl)pyrrolidin-2-yl)methoxy)-1-(4-(2-(piperidin-1-yl)ethyl)piperazin-1-yl)ethanon
- **73**: N-(4-Dimethylaminomethyl-benzyl)-2-[1-(2,4,6-trichlor-phenylsulfonyl)-pyrrolidin-2-ylmethoxy]-acetamid
- **74**: N-(4-Piperidin-1-yl-benzyl)-2-[1-(2,4,6-trichlor-phenylsulfonyl)-piperidin-2-ylmethoxy]-acetamid
- **75**: N-[2-(4-Methyl-piperazin-1-yl)-benzyl]-2-[2-(2,4,6-trimethyl-phenylsulfonyl)-1,2,3,4-tetrahydro-isochinolin-3-ylmethoxy]-acetamid
- **76**: N-(3-Piperidin-1-ylmethyl-phenyl)-2-[1-(2,4,6-trimethyl-phenylsulfonyl)-piperidin-2-ylmethoxy]-acetamid
- **77**: 1-[4-(3-Methoxy-phenyl)-piperazin-1-yl]-2-[1-(2,4,6-trimethyl-phenylsulfonyl)-piperidin-2-ylmethoxy]-ethanon
- **78**: N-(1-Benzyl-pyrrolidin-3-yl)-2-[2-(2,4-dichlor-phenylsulfonyl)-1,2,3,4-tetrahydro-isochinolin-3-ylmethoxy]-N-methyl-acetamid
- **79**: 1-(4-Benzyl-[1,4]diazepan-1-yl)-2-[2-(2,4-dichlor-phenylsulfonyl)-1,2,3,4-tetrahydro-isochinolin-3-ylmethoxy]-ethanon
- **80**: N-[2-(4-Methyl-piperazin-1-yl)-benzyl]-2-[1-(2,4,6-trimethyl-phenylsulfonyl)-piperidin-2-ylmethoxy]-acetamid
- **81**: 2-[1-(3,4-Dichlor-phenylsulfonyl)-2,3-dihydro-1 H-indol-2-ylmethoxy]-N-(4-pyrrolidin-1-ylmethyl-benzyl)-acetamid
- **82**: 2-[2-(3,4-Dichlor-phenylsulfonyl)-1,2,3,4-tetrahydro-isochinolin-3-ylmethoxy]-1-(2-piperidin-1-ylmethyl-pyrrolidin-1-yl)-ethanon
- **83**: N-(4-Pyrrolidin-1-ylmethyl-benzyl)-2-[2-(2,4,6-trimethyl-phenylsulfonyl)-1,2,3,4-tetrahydro-isochinolin-3-ylmethoxy]-acetamid
- **84**: 2-[1-(3,4-Dichlor-phenylsulfonyl)-piperidin-2-ylmethoxy]-1-[4-(4-methoxy-phenyl)-piperazin-1-yl]-ethanon
- **85**: 2-[1-(3,4-Dichlor-phenylsulfonyl)-piperidin-2-ylmethoxy]-1-[4-(3-dimethylamino-propyl)-piperazin-1-yl]-ethanon
- **86**: 2-[1-(3,4-Dichlor-phenylsulfonyl)-piperidin-2-ylmethoxy]-1-[4-(2-fluor-phenyl)-piperazin-1-yl]-ethanon
- **87**: N-[4-(2-Methyl-imidazol-1-yl)-phenyl]-2-[1-(2,4,6-trimethyl-phenylsulfonyl)-piperidin-2-ylmethoxy]-acetamid
- **88**: 2-[1-(3,4-Dichlor-phenylsulfonyl)-pyrrolidin-2-ylmethoxy]-1-[4-(3-dimethylamino-propyl)-piperazin-1-yl]-ethanon
- **89**: 2-[1-(3,4-Dichlor-phenylsulfonyl)-pyrrolidin-2-ylmethoxy]-1-(4-phenyl-piperazin-1-yl)-ethanon
- **90**: 2-(2-(1-(4-Methoxyphenylsulfonyl)piperidin-2-yl)ethoxy)-1-(4-(2-(piperidin-1-yl)ethyl)piperidin-1-yl)ethanon
- **91**: 2-(2-(1-(4-methoxyphenylsulfonyl)piperidin-2-yl)ethoxy)-1-(4-phenethylpiperazin-1-yl)ethanon
- **92**: 3-((4-(2-((1-(4-Methoxy-2,6-dimethylphenylsulfonyl)piperidin-2-yl)methoxy)acetyl)piperazin-1-yl)methyl)benzonitril hydrochlorid
- **93**: 3-((4-(2-((1-(4-Methoxy-2,6-dimethylphenylsulfonyl)pyrrolidin-2-yl)methoxy)acetyl)piperazin-1-yl)methyl)benzonitril hydrochlorid
- **94**: 2-((1-(4-Methoxy-2,6-dimethylphenylsulfonyl)piperidin-2-yl)methoxy)-1-(4-(2-(pyrrolidin-1-yl)ethyl)piperidin-1-yl)ethanon hydrochlorid
- **95**: 2-((1-(4-Methoxy-2,6-dimethylphenylsulfonyl)pyrrolidin-2-yl)methoxy)-1-(4-(2-(pyrrolidin-1-yl)ethyl)piperidin-1-yl)ethanon
- **96**: 1-(3,4-Dihydro-2,6-naphthyridin-2(1 H)-yl)-2-((1-(4-methoxy-2,6-dimethylphenylsulfonyl)piperidin-2-yl)methoxy)ethanon hydrochlorid
- **97**: 2-((1-(4-methoxy-2,6-dimethylphenylsulfonyl)piperidin-2-yl)methoxy)-1-(4-(1- methylpiperidin-4-yl)piperazin-1-yl)ethanon dihydrochlorid
- **98**: 1-(4-(Dihydro-1H-pyrido[1,2-a]pyrazin-2(6H,7H,8H,9H,9aH)-yl)piperidin-1-yl)-2-((1-(4-methoxy-2,6-dimethylphenylsulfonyl)piperidin-2-yl)methoxy)ethanon
- **99**: 1-(4-(Dihydro-1H-pyrido[1,2-a]pyrazin-2(6H,7H,8H,9H,9aH)-yl)piperidin-1-yl)-2-((1-(4-methoxy-2,6-dimethylphenylsulfonyl)piperidin-2-yl)methoxy)ethanon Dihydrochlorid
- **100**: 1-(4-(3,4-Dihydro-2,6-naphthyridin-2(1H)-yl)piperidin-1-yl)-2-((1-(4-methoxy-2,6-dimethylphenylsulfonyl)piperidin-2-yl)methoxy)ethanon
- **101**: tert-Butyl 4-(2-((1-(4-methoxy-2,6-dimethylphenylsulfonyl)piperidin-2-yl)methoxy)acetyl)piperazin-1-carboxylat
- **102**: 2-((1-(4-Methoxy-2,6-dimethylphenylsulfonyl)piperidin-2-yl)methoxy)-1-(piperazin-1-yl)ethanon hydrochlorid
- **103**: 2-((1-(4-Methoxy-2,6-dimethylphenylsulfonyl)piperidin-2-yl)methoxy)-1-(4-(3-(pyridin-4-yl)-1,2,4-oxadiazol-5-yl)piperidin-1-yl)ethanon
- **104**: 2-((1-(4-Methoxy-2,6-dimethylphenylsulfonyl)piperidin-2-yl)methoxy)-1-(4-(tetrahydro-2H-pyran-4-yl)piperazin-1-yl)ethanon
- **105**: 2-((1-(4-Methoxy-2,6-dimethylphenylsulfonyl)piperidin-2-yl)methoxy)-1-(4-(4-methylcyclohexyl)piperazin-1-yl)ethanon
- **106**: 2-((1-(4-Methoxy-2,6-dimethylphenylsulfonyl)piperidin-2-yl)methoxy)-1-(4-((1-methylpiperidin-4-yl)methyl)piperazin-1-yl)ethanon dihydrochlorid
- **107**: 2-((1-(Mesitylsulfonyl)piperidin-2-yl)methoxy)-1-(4-(1-methylpiperidin-4-yl)piperazin-1-yl)ethanon
- **108**: 2-((1-(2,6-Dichlor-4-(trifluormethyl)phenylsulfonyl)piperidin-2-yl)methoxy)-1-(4-(1-methylpiperidin-4-yl)piperazin-1-yl)ethanon
- **109**: 2-((1-(2-Chlor-6-methylphenylsulfonyl)piperidin-2-yl)methoxy)-1-(4-(1-methylpiperidin-4-yl)piperazin-1-yl)ethanon
- **110**: 1-(4-(1-Methylpiperidin-4-yl)piperazin-1-yl)-2-((1-(naphthalen-1-ylsulfonyl)piperidin-2-yl)methoxy)ethanon
- **111**: 1-(4-(1-Methylpiperidin-4-yl)piperazin-1-yl)-2-((1-(naphthalen-2-ylsulfonyl)piperidin-2-yl)methoxy)ethanon
- **112**: 2-((1-(4-Methoxy-2,6-dimethylphenylsulfonyl)piperidin-2-yl)methoxy)-1-(4-(4-methylpiperazin-1-yl)piperidin-1-yl)ethanon hydrochlorid
- **113**: 2-((1-(4-Methoxy-2,6-dimethylphenylsulfonyl)piperidin-2-yl)methoxy)-1-(4-(thieno[3,2-d]pyrimidin-4-yl)piperazin-1-yl)ethanon
- **114**: 2-((1-(4-Chlor-2,5-dimethylphenylsulfonyl)piperidin-2-yl)methoxy)-1-(4-(1-methylpiperidin-4-yl)piperazin-1-yl)ethanon dihydrochlorid
- **115**: 2-((1-(4-Chlor-3-(trifluormethyl)phenylsulfonyl)piperidin-2-yl)methoxy)-1-(4-(1-methylpiperidin-4-yl)piperazin-1-yl)ethanon
- **116**: 1-(2-((1-(4-Methoxy-2,6-dimethylphenylsulfonyl)piperidin-2-yl)methoxy)acetyl)piperidin-4-on
- **117**: 1-(4-((1H-Benzo[d]imidazol-2-yl)methyl)piperazin-1-yl)-2-((1-(4-methoxy-2,6-dimethylphenylsulfonyl)piperidin-2-yl)methoxy)ethanon
- **118**: 1-(4-(1-Methylpiperidin-4-yl)piperazin-1-yl)-2-((1-(2,4,6-trichlorphenylsulfonyl)piperidin-2-yl)methoxy)ethanon
- **119**: 1-(4-(1-Methylpiperidin-4-yl)piperazin-1-yl)-2-((1-(2,4,6- triisopropylphenylsulfonyl)piperidin-2-yl)methoxy)ethanon
- **120**: 2-((1-(2,4-Dichlorphenylsulfonyl)piperidin-2-yl)methoxy)-1-(4-(1-methylpiperidin-4-yl)piperazin-1-yl)ethanon
- **121**: 2-((1-(3-Bromphenylsulfonyl)piperidin-2-yl)methoxy)-1-(4-(1-methylpiperidin-4-yl)piperazin-1-yl)ethanon
- **122**: 2-((1-(3-Bromphenylsulfonyl)piperidin-2-yl)methoxy)-1-(4-(pyridin-4-yl)piperazin-1-yl)ethanon
- **123**: 2-((1-(4-Bromphenylsulfonyl)piperidin-2-yl)methoxy)-1-(4-(1-methylpiperidin-4-yl)piperazin-1-yl)ethanon
- **124**: 2-((1-(3-Bromphenylsulfonyl)piperidin-2-yl)methoxy)-1-(4-(2-(pyrrolidin-1-yl)ethyl)piperidin-1-yl)ethanon
- **125**: 2-((1-(4-Bromphenylsulfonyl)piperidin-2-yl)methoxy)-1-(4-(2-(pyrrolidin-1-yl)ethyl)piperidin-1-yl)ethanon
- **126**: 2-((1-(4-Bromphenylsulfonyl)piperidin-2-yl)methoxy)-1-(4-(pyridin-4-yl)piperazin-1-yl)ethanon
- **127**: (S)-2-((2-(4-Methoxy-2,6-dimethylphenylsulfonyl)-1,2,3,4-tetrahydroisochinolin-3-yl)methoxy)-1-(4-(1-methylpiperidin-4-yl)piperazin-1-yl)ethanon
- **128**: 2-((1-(5-Chlor-1,3-dimethyl-1H-pyrazol-4-ylsulfonyl)piperidin-2-yl)methoxy)-1-(4-(1-methylpiperidin-4-yl)piperazin-1-yl)ethanon
- **129**: 2-((1-(6-Chlorimidazo[2,1-b]thiazol-5-ylsulfonyl)piperidin-2-yl)methoxy)-1-(4-(1-methylpiperidin-4-yl)piperazin-1-yl)ethanon
- **130**: 1-(4-Fluor-1,4'-bipiperidin-1'-yl)-2-((1-(4-methoxy-2,6-dimethylphenylsulfonyl)piperidin-2-yl)methoxy)ethanon
- **131**: 1-(4-(1-Methylpiperidin-4-yl)piperazin-1-yl)-2-((1-(3-(o-tolyloxy)phenylsulfonyl)piperidin-2-yl)methoxy)ethanon
- **133**: (S)-2-((2-(2,4-Dichlorphenylsulfonyl)-1,2,3,4-tetrahydroisochinolin-3-yl)methoxy)-1-(4-(1-methylpiperidin-4-yl)piperazin-1-yl)ethanon
- **134**: 2-((1-(4-Methoxy-2,6-dimethylphenylsulfonyl)piperidin-2-yl)methoxy)-1-(4-(4-(trifluormethyl)cyclohexyl)piperazin-1-yl)ethanon
- **135**: (S)-2-((1-(4-Methoxy-2,6-dimethylphenylsulfonyl)azetidin-2-yl)methoxy)-1-(4-(1-methylpiperidin-4-yl)piperazin-1-yl)ethanon
- **136**: 2-((1-(4-Methoxy-2,6-dimethylphenylsulfonyl)piperidin-2-yl)methoxy)-1-(4-(2-morpholin-2-(pyridin-3-yl)ethylamino)piperidin-1-yl)ethanon
- **137**: 2-((1-(Benzo[b]thiophen-3-ylsulfonyl)piperidin-2-yl)methoxy)-1-(4-(1-methylpiperidin-4-yl)piperazin-1-yl)ethanon
- **138**: 2-((1-(2-Chlor-4-(trifluormethyl)phenylsulfonyl)piperidin-2-yl)methoxy)-1-(4-(1-methylpiperidin-4-yl)piperazin-1-yl)ethanon
- **139**: 2-((1-(2-Chlorphenylsulfonyl)piperidin-2-yl)methoxy)-1-(4-(1-methylpiperidin-4-yl)piperazin-1-yl)ethanon dihydrochlorid
- **140**: (R)-2-((1-(4-Methoxy-2,3,6-trimethylphenylsulfonyl)pyrrolidin-2-yl)methoxy)-1-(4-(1-methylpiperidin-4-yl)piperazin-1-yl)ethanon dihydrochlorid
- **141**: 2-((1-(2,6-Dichlorphenylsulfonyl)piperidin-2-yl)methoxy)-1-(4-(1-methylpiperidin-4-yl)piperazin-1-yl)ethanon dihydrochlorid
- **142**: (S)-2-((2-(4-Methoxyphenylsulfonyl)-1,2,3,4-tetrahydroisochinolin-3-yl)methoxy)-1-(4-(1-methylpiperidin-4-yl)piperazin-1-yl)ethanon
- **143**: 2-((1-(4-Methoxy-2,6-dimethylphenylsulfonyl)piperidin-2-yl)methoxy)-N-(2-(1-(pyridin-4-yl)piperidin-4-yl)ethyl)acetamid
- **144**: 1-(4-(5,6-Dihydroimidazo[1,2-a]pyrazin-7(8H)-yl)piperidin-1-yl)-2-((1-(4-methoxy-2,6-dimethylphenylsulfonyl)piperidin-2-yl)methoxy)ethanon
- **145**: 1-(4-(5,6-Dihydro-[1,2,4]triazolo[1,5-a]pyrazin-7(8H)-yl)piperidin-1-yl)-2-((1-(4-methoxy-2,6-dimethylphenylsulfonyl)piperidin-2-yl)methoxy)ethanon
- **146**: 2-((1-(4-Methoxy-2,6-dimethylphenylsulfonyl)piperidin-2-yl)methoxy)-1-(4-(4-methylpiperazin-1-carbonyl)piperidin-1-yl)ethanon
- **147**: 2-((1-(4-Methoxy-2,6-dimethylphenylsulfonyl)piperidin-2-yl)methoxy)-1-(4-(pyridin-4-yl)piperazin-1-yl)ethanon
- **148**: 2-((1-(4-Brom-2,6-dimethylphenylsulfonyl)piperidin-2-yl)methoxy)-1-(4-(1-methylpiperidin-4-yl)piperazin-1-yl)ethanon
- **149**: 2-((1-(4-Brom-2,6-dimethylphenylsulfonyl)piperidin-2-yl)methoxy)-1-(4-(2-(pyrrolidin-1-yl)ethyl)piperidin-1-yl)ethanon
- **150**: 2-((1-(4-Brom-2,6-dimethylphenylsulfonyl)piperidin-2-yl)methoxy)-1-(4-(pyridin-4-yl)piperazin-1-yl)ethanon
- **151**: 2-((1-(5-Chlor-3-methylbenzo[b]thiophen-2-ylsulfonyl)piperidin-2-yl)methoxy)-1-(4-(1-methylpiperidin-4-yl)piperazin-1-yl)ethanon dihydrochlorid
- **152**: (R)-2-((1-(2-Chlor-6-methylphenylsulfonyl)pyrrolidin-2-yl)methoxy)-1-(4-(1-methylpiperidin-4-yl)piperazin-1-yl)ethanon dihydrochlorid
- **153**: 2-((1-(2,5-Bis(trifluormethyl)phenylsulfonyl)piperidin-2-yl)methoxy)-1-(4-(1-methylpiperidin-4-yl)piperazin-1-yl)ethanon
- **154**: 2-((1-(7-Chlorbenzo[c][1,2,5]oxadiazol-4-ylsulfonyl)piperidin-2-yl)methoxy)-1-(4-(1-methylpiperidin-4-yl)piperazin-1-yl)ethanon
- **155**: 2-((1-(4-Methylnaphthalen-1-ylsulfonyl)piperidin-2-yl)methoxy)-1-(4-(1-methylpiperidin-4-yl)piperazin-1-yl)ethanon dihydrochlorid
- **156**: 1-(4-(1-Methylpiperidin-4-yl)piperazin-1-yl)-2-((1-(2,4,5-trichlorphenylsulfonyl)piperidin-2-yl)methoxy)ethanon dihydrochlorid
- **157**: 2-((1-(2-Methylnaphthalen-1-ylsulfonyl)piperidin-2-yl)methoxy)-1-(4-(1-methylpiperidin-4-yl)piperazin-1-yl)ethanon dihydrochlorid
- **158**: 2-((1-(5-(Dimethylamino)naphthalen-1-ylsulfonyl)piperidin-2-yl)methoxy)-1-(4-(1-methylpiperidin-4-yl)piperazin-1-yl)ethanon dihydrochlorid
- **159**: 1-(4-(1-Methylpiperidin-4-yl)piperazin-1-yl)-2-((1-(o-tolylsulfonyl)piperidin-2-yl)methoxy)ethanon dihydrochlorid
- **160**: 2-((1-(4-Brom-2,6-dichlorphenylsulfonyl)piperidin-2-yl)methoxy)-1-(4-(1-methylpiperidin-4-yl)piperazin-1-yl)ethanon dihydrochlorid
- **161**: (S)-2-((1-(2-Chlor-6-methylphenylsulfonyl)pyrrolidin-2-yl)methoxy)-1-(4-(2-(pyrrolidin-1-yl)ethyl)piperidin-1-yl)ethanon hydrochlorid
- **162**: (S)-2-((1-(2-Chlor-6-methylphenylsulfonyl)pyrrolidin-2-yl)methoxy)-1-(4-(1-methylpiperidin-4-yl)piperazin-1-yl)ethanon dihydrochlorid
- **163**: (S)-2-((1-(2-Chlor-6-methylphenylsulfonyl)pyrrolidin-2-yl)methoxy)-1-(4-((1-methylpiperidin-4-yl)methyl)piperazin-1-yl)ethanon dihydrochlorid
- **164**: 2-(2-(1-(4-Methoxy-2,6-dimethylphenylsulfonyl)piperidin-2-yl)ethoxy)-1-(4-(1-methylpiperidin-4-yl)piperazin-1-yl)ethanon
- **165**: 2-((1-(4-Methoxy-2,6-dimethylphenylsulfonyl)piperidin-2-yl)methoxy)-1-(4-(pyridin-3- yloxy)piperidin-1-yl)ethanon
- **166**: 2-((1-(4-Methoxy-2,6-dimethylphenylsulfonyl)piperidin-2-yl)methoxy)-1-(4-(quinoxalin-6-ylmethyl)piperazin-1-yl)ethanon
- **167**: (S)-2-((1-(4-Methoxy-2,6-dimethylphenylsulfonyl)piperidin-2-yl)methoxy)-1-(4-(1-methylpiperidin-4-yl)piperazin-1-yl)ethanon
- **168**: 2-((1-(4-Methoxy-2,6-dimethylphenylsulfonyl)piperidin-2-yl)methoxy)-1-(4-(4-(pyrrolidin-1-yl)quinazolin-7-yl)piperazin-1-yl)ethanon
- **169**: 2-((1-(4-Fluor-2,6-dimethylphenylsulfonyl)piperidin-2-yl)methoxy)-1-(4-(1-methylpiperidin-4-yl)piperazin-1-yl)ethanon dihydrochlorid
- **170**: 2-((1-(2,5-Dichlorthiophen-3-ylsulfonyl)piperidin-2-yl)methoxy)-1-(4-(1-methylpiperidin-4-yl)piperazin-1-yl)ethanon dihydrochlorid
- **171**: 2-((1-(Benzo[b]thiophen-2-ylsulfonyl)piperidin-2-yl)methoxy)-1-(4-(1-methylpiperidin-4-yl)piperazin-1-yl)ethanon
- **172**: 2-((1-(2,5-Dimethylthiophen-3-ylsulfonyl)piperidin-2-yl)methoxy)-1-(4-(1-methylpiperidin-4-yl)piperazin-1-yl)ethanon
- **173**: 2-((1-(2,3-Dichlorphenylsulfonyl)piperidin-2-yl)methoxy)-1-(4-(1-methylpiperidin-4-yl)piperazin-1-yl)ethanon dihydrochlorid
- **174**: 2-((1-(4-Methoxynaphthalen-1-ylsulfonyl)piperidin-2-yl)methoxy)-1-(4-(1-methylpiperidin-4-yl)piperazin-1-yl)ethanon dihydrochlorid
- **175**: 1-(4-(1-Methylpiperidin-4-yl)piperazin-1-yl)-2-((1-(chinolin-8-ylsulfonyl)piperidin-2-yl)methoxy)ethanon dihydrochlorid
- **176**: 2-((1-(Isochinolin-5-ylsulfonyl)piperidin-2-yl)methoxy)-1-(4-(1-methylpiperidin-4-yl)piperazin-1-yl)ethanon dihydrochlorid
- **177**: (R)-2-((1-(4-Methoxy-2,6-dimethylphenylsulfonyl)piperidin-2-yl)methoxy)-1-(4-(1-methylpiperidin-4-yl)piperazin-1-yl)ethanon dihydrochlorid
- **178**: 1-(4-(1-Methylpiperidin-4-yl)piperazin-1-yl)-2-((2-(naphthalen-2-ylsulfonyl)-1,2,3,4-tetrahydroisochinolin-3-yl)methoxy)ethanon dihydrochlorid
- **179**: 1-(4-(1-Methylpiperidin-4-yl)piperazin-1-yl)-2-((1-(5,6,7,8-tetrahydronaphthalen-2-ylsulfonyl)piperidin-2-yl)methoxy)ethanon dihydrochlorid
- **180**: (S)-2-((1-(4-Methoxy-2,6-dimethylphenylsulfonyl)pyrrolidin-2-yl)methoxy)-1-(4-(1-methylpiperidin-4-yl)piperazin-1-yl)ethanon
- **181**: (S)-2-((1-(4-Methoxy-2,6-dimethylphenylsulfonyl)pyrrolidin-2-yl)methoxy)-1-(4-((1-methylpiperidin-4-yl)methyl)piperazin-1-yl)ethanon
- **182**: (S)-2-((2-(4-Methoxyphenylsulfonyl)-1,2,3,4-tetrahydroisochinolin-3-yl)methoxy)-1-(4-(2-(pyrrolidin-1-yl)ethyl)piperidin-1-yl)ethanon
- **183**: (S)-2-((2-(2,4-Dichlorphenylsulfonyl)-1,2,3,4-tetrahydroisochinolin-3-yl)methoxy)-1-(4-(2-(pyrrolidin-1-yl)ethyl)piperidin-1-yl)ethanon
- **184**: (S)-2-((2-(2,4-Dichlorphenylsulfonyl)-1,2,3,4-tetrahydroisochinolin-3-yl)methoxy)-1-(4-((1-methylpiperidin-4-yl)methyl)piperazin-1-yl)ethanon
- **185**: (S)-2-((2-(4-Methoxy-2,6-dimethylphenylsulfonyl)-1,2,3,4-tetrahydroisochinolin-3-yl)methoxy)-1-(4-((1-methylpiperidin-4-yl)methyl)piperazin-1-yl)ethanon
- **186**: (S)-2-((1-(4-Methoxy-2,6-dimethylphenylsulfonyl)piperidin-2-yl)methoxy)-1-(4-(pyridin-4-yloxy)piperidin-1-yl)ethanon hydrochlorid
- **187**: (S)-2-((2-(4-Methoxy-2,6-dimethylphenylsulfonyl)-1,2,3,4-tetrahydroisochinolin-3-yl)methoxy)-1-(4-(2-(pyrrolidin-1-yl)ethyl)piperidin-1-yl)ethanon
- **188**: 2-((1-(4-Methoxy-2,6-dimethylphenylsulfonyl)piperidin-2-yl)methoxy)-1-(4-((pyridin-4-yloxy)methyl)piperidin-1-yl)ethanon
- **189**: (S)-2-((2-(4-Methoxyphenylsulfonyl)-1,2,3,4-tetrahydroisochinolin-3-yl)methoxy)-1-(4-((1-methylpiperidin-4-yl)methyl)piperazin-1-yl)ethanon dihydrochlorid
- **190**: (S)-2-((1-(4-Methoxy-2,6-dimethylphenylsulfonyl)pyrrolidin-2-yl)methoxy)-1-(4-(2-(pyrrolidin-1-yl)ethyl)piperidin-1-yl)ethanon hydrochlorid
- **191**: 2-((1-(2-Chlornaphthalen-1-ylsulfonyl)piperidin-2-yl)methoxy)-1-(4-(1-methylpiperidin-4-yl)piperazin-1-yl)ethanon dihydrochlorid
- **192**: 2-((1-(4-Methoxy-2,6-dimethylphenylsulfonyl)pyrrolidin-2-yl)methoxy)-1-(4-(1-methylpiperidin-4-yl)piperazin-1-yl)ethanon
- **193**: N-(4-(4,5-Dihydro-1H-imidazol-2-yl)benzyl)-2-((1-(4-methoxy-2,6-dimethylphenylsulfonyl)piperidin-2-yl)methoxy)-N-methylacetamid Hydrochlorid
- **194**: 2-((1-(4-Methoxy-2,6-dimethylphenylsulfonyl)piperidin-2-yl)methoxy)-N-methyl-N-(2-(4-(pyrrolidin-1-yl)cyclohexyl)ethyl)acetamid
- **195**: 2-((1-(4-Methoxy-2,6-dimethylphenylsulfonyl)piperidin-2-yl)methoxy)-N-(3-(pyrazin-2-yloxy)benzyl)acetamid
- **196**: 2-((1-(4-Methoxy-2,6-dimethylphenylsulfonyl)piperidin-2-yl)methoxy)-N-(4-(pyrazin-2-yloxy)benzyl)acetamid
- **197**: 1-(4-((5-Chlor-2-phenyl-1H-imidazol-4-yl)methyl)piperazin-1-yl)-2-((1-(4-methoxy-2,6-dimethylphenylsulfonyl)piperidin-2-yl)methoxy)ethanon
- **198**: 1-(4-((1,5-Dimethyl-1H-pyrazol-4-yl)methyl)piperazin-1-yl)-2-((1-(4-methoxy-2,6-dimethylphenylsulfonyl)piperidin-2-yl)methoxy)ethanon
- **199**: 1-(4-((2-(Dimethylamino)pyrimidin-5-yl)methyl)piperazin-1-yl)-2-((1-(4-methoxy-2,6-dimethylphenylsulfonyl)piperidin-2-yl)methoxy)ethanon
- **200**: 1-(4-(6-Fluor-3,4-dihydroisochinolin-2(1H)-yl)piperidin-1-yl)-2-((1-(4-methoxy-2,6-dimethylphenylsulfonyl)piperidin-2-yl)methoxy)ethanon
- **201**: 2-((1-(4-Methoxy-2,6-dimethylphenylsulfonyl)piperidin-2-yl)methoxy)-1-(4-(pyridin-4-yl)piperidin-1-yl)ethanon
- **202**: 2-((1-(4-Methoxy-2,6-dimethylphenylsulfonyl)-1,2,3,4-tetrahydrochinolin-2-yl)methoxy)-1-(4-(1-methylpiperidin-4-yl)piperazin-1-yl)ethanon
- **203**: 1-(4-(1-Methylpiperidin-4-yl)piperazin-1-yl)-2-((1-(naphthalen-2-ylsulfonyl)-1,2,3,4-tetrahydrochinolin-2-yl)methoxy)ethanon
- **204**: 2-((1-(4-Methoxyphenylsulfonyl)-1,2,3,4-tetrahydrochinolin-2-yl)methoxy)-1-(4-(1-methylpiperidin-4-yl)piperazin-1-yl)ethanon
- **205**: 2-((1-(4-Methoxy-2,6-dimethylphenylsulfonyl)piperidin-2-yl)methoxy)-N-(2-(pyrazin-2-yloxy)benzyl)acetamid
- **206**: 2-((1-(4-Methoxy-2,6-dimethylphenylsulfonyl)piperidin-2-yl)methoxy)-1-(1H-pyrrol[3,4-c]pyridin-2(3H)-yl)ethanon
- **207**: 2-((1-(4-Methoxy-2,6-dimethylphenylsulfonyl)piperidin-2-yl)methoxy)-1-(2-(pyridin-3-yl)morpholin)ethanon
- **208**: 2-((1-(4-Methoxy-2,6-dimethylphenylsulfonyl)piperidin-2-yl)methoxy)-1-(3-(pyridin-3-ylmethyl)pyrrolidin-1-yl)ethanon
- **209**: N-(2-(4-(Dimethylamino)cyclohexyl)ethyl)-2-((1-(4-methoxy-2,6-dimethylphenylsulfonyl)piperidin-2-yl)methoxy)-N-methylacetamid
- **210**: 2-((1-(6-Methoxynaphthalen-2-ylsulfonyl)piperidin-2-yl)methoxy)-1-(4-(1- methylpiperidin-4-yl)piperazin-1-yl)ethanon
- **211**: 1-(4-(3,4-Dihydropyrrolo[1,2-a]pyrazin-2(1H)-yl)piperidin-1-yl)-2-((1-(4-methoxy-2,6-dimethylphenylsulfonyl)piperidin-2-yl)methoxy)ethanon
- **212**: 2-((1-(4-Methoxy-2,6-dimethylphenylsulfonyl)piperidin-2-yl)methoxy)-1-(4-(2-(1-methylpiperidin-4-yl)ethyl)piperazin-1-yl)ethanon
- **213**: 2-((1-(3,4-Dichlorphenylsulfonyl)-1,2,3,4-tetrahydroquinolin-2-yl)methoxy)-N-(2-(4-(dimethylamino)cyclohexyl)ethyl)-N-methylacetamid
- **214**: 1-(4-((2-((4-Fluorphenyl)(methyl)amino)pyrimidin-5-yl)methyl)piperazin-1-yl)-2-((1-(4-methoxy-2,6-dimethylphenylsulfonyl)piperidin-2-yl)methoxy)ethanon
- **215**: 2-((1-(4-Methoxy-2,6-dimethylphenylsulfonyl)piperidin-2-yl)methoxy)-1-(6-(4-methylpiperazin-1-yl)-3,4-dihydroisochinolin-2(1H)-yl)ethanon
- **216**: 2-((1-(3,4-Dichlorphenylsulfonyl)-1,2,3,4-tetrahydroquinolin-2-yl)methoxy)-N-methyl-N-(2-(4-(pyrrolidin-1-yl)cyclohexyl)ethyl)acetamid
- **217**: 2-((1-(4-Methoxy-2,6-dimethylphenylsulfonyl)piperidin-2-yl)methoxy)-1-(4-(pyridin-3-yl)piperidin-1-yl)ethanon
- **218**: N-((S)-1-Benzylpyrrolidin-3-yl)-N-methyl-2-((1-(2-(trifluormethyl)phenylsulfonyl)piperidin-2-yl)methoxy)acetamid
- **219**: 2-((1-(4-Methoxyphenylsulfonyl)piperidin-2-yl)methoxy)-1-(4-(3-methylbenzyl)piperazin-1-yl)ethanon
- **220**: 2-((1-(4-Methoxyphenylsulfonyl)piperidin-2-yl)methoxy)-1-(4-(4-phenethylthiazol-2-yl)piperazin-1-yl)ethanon
- **221**: 2-(2-(1-(4-Methoxyphenylsulfonyl)piperidin-2-yl)ethoxy)-1-(4-(2-(piperidin-1-yl)ethyl)piperazin-1-yl)ethanon
- **222**: 2-((1-(Mesitylsulfonyl)piperidin-2-yl)methoxy)-1-(4-(5-methyl-1H-benzo[d]imidazol-2-yl)piperidin-1-yl)ethanon
- **223**: 1-(2-((4,6-Dimethylpyridin-2-yl)methyl)piperidin-1-yl)-2-((2-(mesitylsulfonyl)-1,2,3,4-tetrahydroisoquinolin-3-yl)methoxy)ethanon
- **224**: 1-(2-(5-Brompyridin-3-yl)piperidin-1-yl)-2-((1-(3-(trifluormethyl)phenylsulfonyl)piperidin-2-yl)methoxy)ethanon
- **225**: 1-(1,4'-Bipiperidin-1'-yl)-2-((1-(mesitylsulfonyl)piperidin-2-yl)methoxy)ethanon
- **226**: 2-((1-(Mesitylsulfonyl)piperidin-2-yl)methoxy)-1-(2-(pyridin-2-ylmethyl)pyrrolidin-1-yl)ethanon
- **227**: 2-((1-(Mesitylsulfonyl)piperidin-2-yl)methoxy)-1-(2-(6-methoxypyridin-3-yl)piperidin-1-yl)ethanon
- **228**: 1-(2-((5-Ethylpyridin-2-yl)methyl)piperidin-1-yl)-2-((1-(mesitylsulfonyl)piperidin-2-yl)methoxy)ethanon
- **229**: 2-((1-(Mesitylsulfonyl)piperidin-2-yl)methoxy)-1-(2-((3-methylpyridin-2-yl)methyl)piperidin-1-yl)ethanon
- **230**: 1-(4-(4-Methylpiperazin-1-yl)piperidin-1-yl)-2-((1-(naphthen-1-ylsulfonyl)piperidin-2-yl)methoxy)ethanon
- **231**: 2-((1-(Mesitylsulfonyl)piperidin-2-yl)methoxy)-1-(4-(4-methylpiperazin-1-yl)piperidin-1-yl)ethanon
- **232**: 2-((1-(Mesitylsulfonyl)piperidin-2-yl)methoxy)-1-(4-((1-methylpiperidin-4-yl)methyl)piperazin-1-yl)ethanon
- **233**: 2-((2-(2,4-Dichlorphenylsulfonyl)-1,2,3,4-tetrahydroisoquinolin-3-yl)methoxy)-1-(4-(4-methylpiperazin-1-yl)piperidin-1-yl)ethanon
- **234**: 1-(4-(4-Benzylthiazol-2-yl)piperazin-1-yl)-2-((1-(mesitylsulfonyl)piperidin-2-yl)methoxy)ethanon
- **235**: 1-(1,4'-Bipiperidin-1'-yl)-2-((1-(naphthen-1-ylsulfonyl)piperidin-2-yl)methoxy)ethanon
- **236**: 2-((1-(4-Methoxy-2,6-dimethylphenylsulfonyl)piperidin-2-yl)methoxy)-1-(3-(pyridin-2-yl)pyrrolidin-1-yl)ethanon
- **237**: 2-((1-(4-Methoxy-2,6-dimethylphenylsulfonyl)piperidin-2-yl)methoxy)-1-(3-(pyridin-3-yl)pyrrolidin-1-yl)ethanon
- **238**: 2-((1-(4-Methoxy-2,6-dimethylphenylsulfonyl)piperidin-2-yl)methoxy)-1-(3-(pyridin-4-yl)pyrrolidin-1-yl)ethanon
- **239**: 2-((1-(4-Methoxy-2,6-dimethylphenylsulfonyl)piperidin-2-yl)methoxy)-N-methyl-N-(2-(pyridin-4-yl)ethyl)acetamid
- **240**: 1-(2-((4,6-Dimethylpyridin-2-yl)methyl)pyrrolidin-1-yl)-2-((1-(naphthen-1-ylsulfonyl)piperidin-2-yl)methoxy)ethanon
- **241**: 2-((1-(4-Methoxy-2,6-dimethylphenylsulfonyl)piperidin-2-yl)methoxy)-N-methyl-N-(1-phenyl-2-(pyrrolidin-1-yl)ethyl)acetamid
- **242**: 2-((1-(4-Methoxy-2,6-dimethylphenylsulfonyl)piperidin-2-yl)methoxy)-1-(4-methylpiperazin-1-yl)ethanon
- **243**: N-(2-(1H-Indol-3-yl)ethyl)-2-((1-(4-methoxy-2,6-dimethylphenylsulfonyl)piperidin-2-yl)methoxy)acetamid
- **244**: 2-((1-(4-Methoxy-2,6-dimethylphenylsulfonyl)piperidin-2-yl)methoxy)-N-(2-methylquinolin-4-yl)acetamid
- **245**: 1-(4-(2-Ethoxyethyl)piperazin-1-yl)-2-((2-(mesitylsulfonyl)-1,2,3,4-tetrahydroisoquinolin-3-yl)methoxy)ethanon
- **246**: 2-((2-(Mesitylsulfonyl)-1,2,3,4-tetrahydroisoquinolin-3-yl)methoxy)-1-(4-(2-methoxyethyl)piperazin-1-yl)ethanon
- **247**: 2-((1-(4-Methoxy-2,6-dimethylphenylsulfonyl)piperidin-2-yl)methoxy)-1-(2-(pyridin-2-yl)pyrrolidin-1-yl)ethanon
- **248**: 2-((1-(4-Methoxy-2,6-dimethylphenylsulfonyl)piperidin-2-yl)methoxy)-N-(2-(4-methylpiperazin-1-yl)ethyl)acetamid
- **249**: 2-((1-(4-Methoxy-2,6-dimethylphenylsulfonyl)piperidin-2-yl)methoxy)-N-(2-morpholin-2-(4-(trifluormethyl)phenyl)ethyl)acetamid
- **250**: N-(5-(Dimethylamino)pentyl)-2-((1-(4-methoxy-2,6-dimethylphenylsulfonyl)piperidin-2-yl)methoxy)acetamid
- **251**: N-(2-(2-Chlorphenyl)-2-morpholinoethyl)-2-((1-(4-methoxy-2,6-dimethylphenylsulfonyl)piperidin-2-yl)methoxy)acetamid
- **252**: 2-((1-(4-Methoxy-2,6-dimethylphenylsulfonyl)piperidin-2-yl)methoxy)-N-(2-(4-methoxyphenyl)-2-(pyrrolidin-1-yl)ethyl)acetamid
- **253**: 1-(4-(3,5-Dimethoxyphenyl)piperazin-1-yl)-2-((1-(2,4,6-trichlorphenylsulfonyl)piperidin-2-yl)methoxy)ethanon
- **254**: 1-(4-(2-(Diisopropylamino)ethyl)piperazin-1-yl)-2-((1-(2,4,6-trichlorphenylsulfonyl)piperidin-2-yl)methoxy)ethanon
- **255**: N-(1-Benzylpiperidin-4-yl)-N-cyclopropyl-2-((1-(2,4,6-trichlorphenylsulfonyl)piperidin-2-yl)methoxy)acetamid
- **256**: N-(2-Morpholino-2-(pyridin-3-yl)ethyl)-2-((1-(2,4,6-trichlorphenylsulfonyl)piperidin-2-yl)methoxy)acetamid
- **257**: N-(1-Ethyl-1H-pyrazol-5-yl)-2-((1-(4-methoxy-2,6-dimethylphenylsulfonyl)piperidin-2-yl)methoxy)acetamid
- **258**: N-(Isoquinolin-5-yl)-2-((1-(4-methoxy-2,6-dimethylphenylsulfonyl)piperidin-2-yl)methoxy)acetamid
- **259**: N-(2-(Dimethylamino)ethyl)-2-((1-(4-methoxy-2,6-dimethylphenylsulfonyl)piperidin-2-yl)methoxy)acetamid
- **260**: N-(3-(2,6-Dimethylpiperidin-1-yl)propyl)-2-((1-(4-methoxy-2,6-dimethylphenylsulfonyl)piperidin-2-yl)methoxy)acetamid
- **261**: N-(2-(Dimethylamino)-1-phenylethyl)-2-((1-(4-methoxy-2,6-dimethylphenylsulfonyl)piperidin-2-yl)methoxy)acetamid
- **262**: 2-((1-(4-Methoxy-2,6-dimethylphenylsulfonyl)piperidin-2-yl)methoxy)-N-(1-phenyl-2-(pyrrolidin-1-yl)ethyl)acetamid
- **263**: 2-((1-(4-Methoxy-2,6-dimethylphenylsulfonyl)piperidin-2-yl)methoxy)-N-(2-(4-methylpiperazin-1-yl)-1-phenylethyl)acetamid
- **264**: N-(3-Morpholinophenyl)-2-((2-(phenylsulfonyl)-1,2,3,4-tetrahydroisoquinolin-3-yl)methoxy)acetamid
- **265**: 2-((2-(2,4-Dichlorphenylsulfonyl)-1,2,3,4-tetrahydroisoquinolin-3-yl)methoxy)-N-(3-morpholinophenyl)acetamid
- **266**: 2-((1-(3,4-Dichlorphenylsulfonyl)piperidin-2-yl)methoxy)-1-(2-(piperidin-1-ylmethyl)morpholino)ethanon
- **267**: 2-((1-(4-Methoxy-2,6-dimethylphenylsulfonyl)pyrrolidin-2-yl)methoxy)-1-(4-(4-methylpiperazin-1-yl)piperidin-1-yl)ethanon
- **268**: 2-((1-(4-Methoxy-2,6-dimethylphenylsulfonyl)pyrrolidin-2-yl)methoxy)-1-(4-(2-(piperidin-1-yl)ethyl)piperidin-1-yl)ethanon
- **269**: 1-(4-(2-(2,5-Dimethyl-1H-pyrrol-1-yl)ethyl)piperazin-1-yl)-2-((1-(4-methoxy-2,6-dimethylphenylsulfonyl)pyrrolidin-2-yl)methoxy)ethanon
- **270**: 1-(4-(2-(Diisopropylamino)ethyl)piperazin-1-yl)-2-((1-(4-methoxy-2,6-dimethylphenylsulfonyl)pyrrolidin-2-yl)methoxy)ethanon
- **271**: 2-((1-(4-Methoxy-2,6-dimethylphenylsulfonyl)pyrrolidin-2-yl)methoxy)-1-(2-(pyridin-2-yl)pyrrolidin-1-yl)ethanon
- **272**: 2-((1-(4-Methoxy-2,6-dimethylphenylsulfonyl)pyrrolidin-2-yl)methoxy)-1-(3-(pyridin-3-yl)pyrrolidin-1-yl)ethanon
- **273**: 2-((1-(4-Methoxy-2,6-dimethylphenylsulfonyl)pyrrolidin-2-yl)methoxy)-1-(3-(pyridin-4-yl)pyrrolidin-1-yl)ethanon
- **274**: 2-((1-(4-Methoxy-2,6-dimethylphenylsulfonyl)pyrrolidin-2-yl)methoxy)-1-(4-(pyridin-4-yl)piperazin-1-yl)ethanon
- **275**: 2-((2-(4-Methoxy-2,3,6-trimethylphenylsulfonyl)-1,2,3,4-tetrahydroisoquinolin-3-yl)methoxy)-1-(4-(4-methylpiperazin-1-yl)piperidin-1-yl)ethanon
- **276**: 2-((2-(4-Methoxy-2,3,6-trimethylphenylsulfonyl)-1,2,3,4-tetrahydroisoquinolin-3-yl)methoxy)-1-(4-(2-(piperidin-1-yl)ethyl)piperidin-1-yl)ethanon
- **277**: 2-((2-(4-Methoxy-2,3,6-trimethylphenylsulfonyl)-1,2,3,4-tetrahydroisoquinolin-3-yl)methoxy)-1-(2-((6-methylpyridin-2-yl)methyl)piperidin-1-yl)ethanon
- **278**: 2-((1-(2,6-Dichlorphenylsulfonyl)piperidin-2-yl)methoxy)-1-(4-(4-methylpiperazin-1- yl)piperidin-1-yl)ethanon
- **279**: 2-((1-(2,6-Dichlorphenylsulfonyl)piperidin-2-yl)methoxy)-1-(4-(2-(piperidin-1-yl)ethyl)piperidin-1-yl)ethanon
- **280**: 2-((1-(2,6-Dichlorphenylsulfonyl)piperidin-2-yl)methoxy)-1-(4-(2-(2,5-dimethyl-1H-pyrrol-1-yl)ethyl)piperazin-1-yl)ethanon
- **281**: 2-((1-(2,6-Dichlorphenylsulfonyl)piperidin-2-yl)methoxy)-1-(4-(2-(diisopropylamino)ethyl)piperazin-1-yl)ethanon
- **282**: 2-((1-(2,6-Dichlorphenylsulfonyl)piperidin-2-yl)methoxy)-1-(3-(pyridin-4-yl)pyrrolidin-1-yl)ethanon
- **283**: 2-((1-(2,6-Dichlorphenylsulfonyl)piperidin-2-yl)methoxy)-1-(4-(pyridin-4-yl)piperazin-1-yl)ethanon
- **284**: 2-((1-(2,6-Dichlorphenylsulfonyl)piperidin-2-yl)methoxy)-1-(2-((6-methylpyridin-2-yl)methyl)piperidin-1-yl)ethanon
- **285**: 2-((1-(2,6-Dichlorphenylsulfonyl)pyrrolidin-2-yl)methoxy)-1-(4-(4-methylpiperazin-1-yl)piperidin-1-yl)ethanon
- **286**: 2-((1-(2,6-Dichlorphenylsulfonyl)pyrrolidin-2-yl)methoxy)-1-(4-(2-(piperidin-1-yl)ethyl)piperidin-1-yl)ethanon
- **287**: 2-((1-(2,6-Dichlorphenylsulfonyl)pyrrolidin-2-yl)methoxy)-1-(4-(2-(2,5-dimethyl-1H-pyrrol-1-yl)ethyl)piperazin-1-yl)ethanon
- **288**: 2-((1-(4-Methoxy-2,6-dimethylphenylsulfonyl)piperidin-2-yl)methoxy)-1-(4-(3-methoxyphenyl)piperazin-1-yl)ethanon
- **289**: 1-(4-(4-Fluorphenyl)piperazin-1-yl)-2-((1-(4-methoxy-2,6-dimethylphenylsulfonyl)piperidin-2-yl)methoxy)ethanon
- **290**: 2-((1-(4-Methoxy-2,6-dimethylphenylsulfonyl)piperidin-2-yl)methoxy)-1-(4-(4-methoxyphenyl)piperazin-1-yl)ethanon
- **291**: 1-(4-Isopropylpiperazin-1-yl)-2-((1-(4-methoxy-2,6-dimethylphenylsulfonyl)piperidin-2-yl)methoxy)ethanon
- **292**: 2-((2-(Mesitylsulfonyl)-1,2,3,4-tetrahydroisoquinolin-3-yl)methoxy)-1-(4-(4-methylpiperazin-1-yl)piperidin-1-yl)ethanon
- **293**: 2-((1-(3,4-Dichlorphenylsulfonyl)piperidin-2-yl)methoxy)-N-(3-(pyrrolidin-1-ylmethyl)phenyl)acetamid
- **294**: N-(4-(Dimethylamino)butyl)-2-((1-(2,4,6-trichlorphenylsulfonyl)pyrrolidin-2-yl)methoxy)acetamid
- **295**: 1-(4-Ethylpiperazin-1-yl)-2-((1-(2,4,6-trichlorphenylsulfonyl)pyrrolidin-2-yl)methoxy)ethanon
- **296**: 1-(4-(Pyrrolidin-1-yl)piperidin-1-yl)-2-((1-(2,4,6-trichlorphenylsulfonyl)pyrrolidin-2-yl)methoxy)ethanon
- **297**: 1-(4-Morpholinopiperidin-1-yl)-2-((1-(2,4,6-trichlorphenylsulfonyl)pyrrolidin-2-yl)methoxy)ethanon
- **298**: 1-(1,4'-Bipiperidin-1'-yl)-2-((1-(2,4,6-trichlorphenylsulfonyl)pyrrolidin-2-yl)methoxy)ethanon
- **299**: 2-((1-(4-Methoxy-2,6-dimethylphenylsulfonyl)pyrrolidin-2-yl)methoxy)-N-(4-(pyrrolidin-1-ylmethyl)benzyl)acetamid
- **300**: N-((1-Benzylpiperidin-3-yl)methyl)-2-((1-(4-methoxy-2,6-dimethylphenylsulfonyl)pyrrolidin-2-yl)methoxy)acetamid
- **301**: 2-((1-(4-Methoxy-2,6-dimethylphenylsulfonyl)pyrrolidin-2-yl)methoxy)-N-(4-(4-methylpiperazin-1-yl)phenyl)acetamid
- **302**: 2-((1-(4-Methoxy-2,6-dimethylphenylsulfonyl)pyrrolidin-2-yl)methoxy)-N-(4-(4-methylpiperazin-1-yl)benzyl)acetamid
- **303**: N-((S)-1-Benzylpyrrolidin-3-yl)-2-((1-(4-methoxy-2,6-dimethylphenylsulfonyl)pyrrolidin-2-yl)methoxy)-N-methylacetamid
- **304**: 2-((1-(4-Methoxy-2,6-dimethylphenylsulfonyl)pyrrolidin-2-yl)methoxy)-N-methyl-N-(2-morpholino-1-phenylethyl)acetamid
- **305**: 2-((1-(4-Methoxy-2,6-dimethylphenylsulfonyl)pyrrolidin-2-yl)methoxy)-1-(4-(pyridin-2-yl)piperazin-1-yl)ethanon
- **306**: 2-((1-(4-Methoxy-2,6-dimethylphenylsulfonyl)pyrrolidin-2-yl)methoxy)-1-(4-(3-methoxyphenyl)piperazin-1-yl)ethanon
- **307**: 1-(4-(4-Fluorphenyl)piperazin-1-yl)-2-((1-(4-methoxy-2,6-dimethylphenylsulfonyl)pyrrolidin-2-yl)methoxy)ethanon
- **308**: 2-((1-(4-Methoxy-2,6-dimethylphenylsulfonyl)pyrrolidin-2-yl)methoxy)-1-(4-(4-methoxyphenyl)piperazin-1-yl)ethanon
- **309**: 2-((1-(4-Methoxy-2,6-dimethylphenylsulfonyl)pyrrolidin-2-yl)methoxy)-1-(4-(pyrimidin-2-yl)piperazin-1-yl)ethanon
- **310**: 2-((1-(4-Mhoxy-2,6-dimethylphenylsulfonyl)pyrrolidin-2-yl)methoxy)-1-(4-phenethylpiperazin-1-yl)ethanon
- **311**: 1-(4-(5-Chlor-2-methylphenyl)piperazin-1-yl)-2-((1-(4-methoxy-2,6-dimethylphenylsulfonyl)pyrrolidin-2-yl)methoxy)ethanon
- **312**: 2-(4-(2-((1-(4-Methoxy-2,6-dimethylphenylsulfonyl)pyrrolidin-2-yl)methoxy)acetyl)piperazin-1-yl)nicotinonitrile
- **313**: 2-((1-(2,6-Dichlorphenylsulfonyl)piperidin-2-yl)methoxy)-N-(4-(pyrrolidin-1-ylmethyl)benzyl)acetamid
- **314**: N-((1-Benzylpiperidin-3-yl)methyl)-2-((1-(2,6-dichlorphenylsulfonyl)piperidin-2-yl)methoxy)acetamid
- **315**: N-((S)-1-Benzylpyrrolidin-3-yl)-2-((1-(2,6-dichlorphenylsulfonyl)piperidin-2-yl)methoxy)-N-methylacetamid
- **316**: 2-((1-(2,6-Dichlorphenylsulfonyl)piperidin-2-yl)methoxy)-1-(4-(pyridin-2-yl)piperazin-1-yl)ethanon
- **317**: 2-((1-(2,6-Dichlorphenylsulfonyl)piperidin-2-yl)methoxy)-1-(4-(3-methoxyphenyl)piperazin-1-yl)ethanon
- **318**: 2-((1-(2,6-Dichlorphenylsulfonyl)piperidin-2-yl)methoxy)-1-(4-(4-fluorphenyl)piperazin-1-yl)ethanon
- **319**: 2-((1-(2,6-Dichlorphenylsulfonyl)piperidin-2-yl)methoxy)-1-(4-(4-methoxyphenyl)piperazin-1-yl)ethanon
- **320**: 2-((1-(2,6-Dichlorphenylsulfonyl)piperidin-2-yl)methoxy)-1-(4-phenethylpiperazin-1-yl)ethanon
- **321**: 1-(4-(5-Chlor-2-methylphenyl)piperazin-1-yl)-2-((1-(2,6-dichlorphenylsulfonyl)piperidin-2-yl)methoxy)ethanon
- **322**: 2-((1-(2,6-Dichlorphenylsulfonyl)pyrrolidin-2-yl)methoxy)-N-(4-(pyrrolidin-1-ylmethyl)benzyl)acetamid
- **323**: N-((1-Benzylpiperidin-3-yl)methyl)-2-((1-(2,6-dichlorphenylsulfonyl)pyrrolidin-2- yl)methoxy)acetamid
- **324**: 2-((1-(2,6-Dichlorphenylsulfonyl)pyrrolidin-2-yl)methoxy)-1-(4-(3-methoxyphenyl)piperazin-1-yl)ethanon
- **325**: 2-((1-(2,6-Dichlorphenylsulfonyl)pyrrolidin-2-yl)methoxy)-1-(4-(4-methoxyphenyl)piperazin-1-yl)ethanon
- **326**: 2-((1-(2,6-Dichlorphenylsulfonyl)pyrrolidin-2-yl)methoxy)-1-(4-phenethylpiperazin-1-yl)ethanon
- **327**: N-((1-Benzylpiperidin-3-yl)methyl)-2-((1-(2,4,6-trichlorphenylsulfonyl)pyrrolidin-2-yl)methoxy)acetamid
- **328**: 1-(4-(4-Methoxyphenyl)piperazin-1-yl)-2-((1-(2,4,6-trichlorphenylsulfonyl)pyrrolidin-2-yl)methoxy)ethanon
- **329**: 1-(4-Phenethylpiperazin-1-yl)-2-((1-(2,4,6-trichlorphenylsulfonyl)pyrrolidin-2-yl)methoxy)ethanon
- **330**: 1-(4-(4-Methylpiperazin-1-yl)piperidin-1-yl)-2-((1-(2,4,6-trichlorphenylsulfonyl)pyrrolidin-2-yl)methoxy)ethanon
- **331**: 1-(4-(2-(Piperidin-1-yl)ethyl)piperidin-1-yl)-2-((1-(2,4,6-trichlorphenylsulfonyl)pyrrolidin-2-yl)methoxy)ethanon
- **332**: 1-(4-(2-(2,5-Dimethy)-1H-pyrrol-1-yl)ethyl)piperazin-1-yl)-2-((1-(2,4,6-trichlorphenylsulfonyl)pyrrolidin-2-yl)methoxy)ethanon
- **333**: 1-(4-(2-(Diisopropylamino)ethyl)piperazin-1-yl)-2-((1-(2,4,6-trichlorphenylsulfonyl)pyrrolidin-2-yl)methoxy)ethanon
- **334**: N-(1-Benzylpiperidin-4-yl)-N-cyclopropyl-2-((1-(2,4,6-trichlorphenylsulfonyl)pyrrolidin-2-yl)methoxy)acetamid
- **335**: 1-(4-(Pyridin-4-yl)piperazin-1-yl)-2-((1-(2,4,6-trichlorphenylsulfonyl)pyrrolidin-2-yl)methoxy)ethanon
- **336**: 1-(1,4'-Bipiperidin-1'-yl)-2-((1-(2,6-dichlorphenylsulfonyl)piperidin-2-yl)methoxy)ethanon
- **337**: 1-(1,4'-Bipiperidin-1'-yl)-2-((1-(2,6-dichlorphenylsulfonyl)pyrrolidin-2-yl)methoxy)ethanon
- **338**: N-(2-(Piperidin-1-yl)ethyl)-2-((1-(2,4,6-trichlorphenylsulfonyl)pyrrolidin-2-yl)methoxy)acetamid
- **339**: N-(2-(Pyrrolidin-1-yl)ethyl)-2-((1-(2,4,6-trichlorphenylsulfonyl)pyrrolidin-2-yl)methoxy)acetamid
- **340**: 1-(4-(3,5-Dimethoxyphenyl)piperazin-1-yl)-2-((1-(4-methoxy-2,6-dimethylphenylsulfonyl)piperidin-2-yl)methoxy)ethanon
- **341**: 1-(4-(2-(Diisopropylamino)ethyl)piperazin-1-yl)-2-((1-(4-methoxy-2,6-dimethylphenylsulfonyl)piperidin-2-yl)methoxy)ethanon
- **342**: N-(1-Benzylpiperidin-4-yl)-N-cyclopropyl-2-((1-(4-methoxy-2,6-dimethylphenylsulfonyl)piperidin-2-yl)methoxy)acetamid
- **343**: 2-((1-(4-Methoxy-2,6-dimethylphenylsulfonyl)piperidin-2-yl)methoxy)-N-(2-(pyrrolidin-1-yl)ethyl)acetamid
- **344**: 2-((1-(4-Methoxy-2,6-dimethylphenylsulfonyl)piperidin-2-yl)methoxy)-N-(3-morpholinopropyl)acetamid
- **345**: 2-((1-(4-Methoxy-2,6-dimethylphenylsulfonyl)piperidin-2-yl)methoxy)-N-(pyridin-3-ylmethyl)acetamid
- **346**: 1-(4-(3-Chlorphenyl)piperazin-1-yl)-2-((1-(4-methoxy-2,6-dimethylphenylsulfonyl)pyrrolidin-2-yl)methoxy)ethanon
- **347**: 1-(4-(3,4-Dimethylphenyl)piperazin-1-yl)-2-((1-(4-methoxy-2,6-dimethylphenylsulfonyl)pyrrolidin-2-yl)methoxy)ethanon
- **348**: 1-(4-(3,4-Dichlorphenyl)piperazin-1-yl)-2-((1-(4-methoxy-2,6-dimethylphenylsulfonyl)pyrrolidin-2-yl)methoxy)ethanon
- **349**: 1-(4-(3,4-Dichlorbenzyl)piperazin-1-yl)-2-((1-(4-methoxy-2,6-dimethylphenylsulfonyl)pyrrolidin-2-yl)methoxy)ethanon
- **350**: 1-(4-(4-Brombenzyl)piperazin-1-yl)-2-((1-(4-methoxy-2,6-dimethylphenylsulfonyl)pyrrolidin-2-yl)methoxy)ethanon
- **351**: 2-((1-(4-Methoxy-2,6-dimethylphenylsulfonyl)pyrrolidin-2-yl)methoxy)-1-(4-(2,4,6-trimethylbenzyl)piperazin-1-yl)ethanon
- **352**: 1-(4-(4-Chlorbenzyl)piperazin-1-yl)-2-((1-(4-methoxy-2,6-dimethylphenylsulfonyl)pyrrolidin-2-yl)methoxy)ethanon
- **353**: 2-((1-(4-Methoxy-2,6-dimethylphenylsulfonyl)pyrrolidin-2-yl)methoxy)-1-(4-(4-methylbenzyl)piperazin-1-yl)ethanon
- **354**: 1-(4-(3-Chlorphenyl)piperazin-1-yl)-2-((1-(2,6-dichlorphenylsulfonyl)pyrrolidin-2-yl)methoxy)ethanon
- **355**: 2-((1-(2,6-Dichlorphenylsulfonyl)pyrrolidin-2-yl)methoxy)-1-(4-(3,4-dimethylphenyl)piperazin-1-yl)ethanon
- **356**: 1-(4-(3,4-Dichlorphenyl)piperazin-1-yl)-2-((1-(2,6-dichlorphenylsulfonyl)pyrrolidin-2-yl)methoxy)ethanon
- **357**: 1-(4-(3,4-Dichlorbenzyl)piperazin-1-yl)-2-((1-(2,6-dichlorphenylsulfonyl)pyrrolidin-2-yl)methoxy)ethanon
- **358**: 1-(4-(4-Brombenzyl)piperazin-1-yl)-2-((1-(2,6-dichlorphenylsulfonyl)pyrrolidin-2-yl)methoxy)ethanon
- **359**: 1-(4-(4-Chlorbenzyl)piperazin-1-yl)-2-((1-(2,6-dichlorphenylsulfonyl)pyrrolidin-2-yl)methoxy)ethanon
- **360**: 2-((1-(2,6-Dichlorphenylsulfonyl)pyrrolidin-2-yl)methoxy)-1-(4-(4-methylbenzyl)piperazin-1-yl)ethanon
- **361**: N-Benzyl-N-(2-(dimethylamino)ethyl)-2-((1-(4-methoxy-2,6-dimethylphenylsulfonyl)piperidin-2-yl)methoxy)acetamid
- **362**: 1-(4-Benzyl-1,4-diazepan-1-yl)-2-((1-(4-methoxy-2,6-dimethylphenylsulfonyl)piperidin-2-yl)methoxy)ethanon
- **363**: 1-((R)-3-(Dimethylamino)pyrrolidin-1-yl)-2-((1-(4-methoxy-2,6-dimethylphenylsulfonyl)piperidin-2-yl)methoxy)ethanon
- **364**: N-((S)-1-Benzylpyrrolidin-3-yl)-2-((1-(4-methoxy-2,6-dimethylphenylsulfonyl)piperidin-2-yl)methoxy)-N-methylacetamid
- **365**: N-(1-Benzylpyrrolidin-3-yl)-2-((1-(4-methoxy-2,6-dimethylphenylsulfonyl)piperidin-2-yl)methoxy)acetamid
- **366**: N-(3-(1H-Imidazol-1-yl)propyl)-2-((1-(4-methoxy-2,6-dimethylphenylsulfonyl)piperidin-2-yl)methoxy)acetamid
- **367**: N-(4-(Dimethylamino)benzyl)-N-isopropyl-2-((1-(4-methoxy-2,6-dimethylphenylsulfonyl)piperidin-2-yl)methoxy)acetamid
- **368**: 2-((1-(4-Methoxy-2,6-dimethylphenylsulfonyl)piperidin-2-yl)methoxy)-N-methyl-N-(2- morpholino-1-phenylethyl)acetamid
- **369**: 2-((1-(4-Methoxy-2,6-dimethylphenylsulfonyl)piperidin-2-yl)methoxy)-1-(4-(pyridin-2-yl)piperazin-1-yl)ethanon
- **370**: N-(Isoquinolin-1-yl)-2-((1-(4-methoxy-2,6-dimethylphenylsulfonyl)piperidin-2-yl)methoxy)acetamid
- **371**: 2-((1-(4-Methoxy-2,6-dimethylphenylsulfonyl)piperidin-2-yl)methoxy)-N-(1-methyl-1H-benzo[d]imidazol-2-yl)acetamid
- **372**: 2-((1-(4-Methoxy-2,6-dimethylphenylsulfonyl)piperidin-2-yl)methoxy)-N-(pyrimidin-4-yl)acetamid
- **373**: 2-((1-(4-Methoxy-2,6-dimethylphenylsulfonyl)piperidin-2-yl)methoxy)-1-(4-phenethylpiperazin-1-yl)ethanon
- **374**: 2-((1-(4-Methoxy-2,6-dimethylphenylsulfonyl)piperidin-2-yl)methoxy)-1-((S)-2-(pyrrolidin-1-ylmethyl)pyrrolidin-1-yl)ethanon
- **375**: 2-((1-(4-Methoxy-2,6-dimethylphenylsulfonyl)piperidin-2-yl)methoxy)-1-(4-methyl-1,4-diazepan-1-yl)ethanon
- **376**: 1-(4-(3-Chlor-5-(trifluormethyl)pyridin-2-yl)piperazin-1-yl)-2-((1-(4-methoxy-2,6-dimethylphenylsulfonyl)piperidin-2-yl)methoxy)ethanon
- **377**: N-Benzyl-N-(2-(dimethylamino)ethyl)-2-((1-(4-methoxy-2,6-dimethylphenylsulfonyl)piperidin-2-yl)methoxy)acetamid
- **378**: 1-(4-(3,4-Dichlorphenyl)piperazin-1-yl)-2-((1-(4-methoxy-2,6-dimethylphenylsulfonyl)piperidin-2-yl)methoxy)ethanon
- **379**: N-(2-(Dimethylamino)ethyl)-2-((1-(4-methoxy-2,6-dimethylphenylsulfonyl)piperidin-2-yl)methoxy)-N-methylacetamid
- **380**: N-(2-(Diethylamino)ethyl)-N-ethyl-2-((1-(4-methoxy-2,6-dimethylphenylsulfonyl)piperidin-2-yl)methoxy)acetamid
- **381**: 1-(4-(3,4-Dichlorbenzyl)piperazin-1-yl)-2-((1-(4-methoxy-2,6-dimethylphenylsulfonyl)piperidin-2-yl)methoxy)ethanon
- **382**: 1-(4-(4-Brombenzyl)piperazin-1-yl)-2-((1-(4-methoxy-2,6-dimethylphenylsulfonyl)piperidin-2-yl)methoxy)ethanon
- **383**: 2-((1-(4-Methoxy-2,6-dimethylphenylsulfonyl)piperidin-2-yl)methoxy)-1-(4-(2,4,6-trimethylbenzyl)piperazin-1-yl)ethanon
- **384**: 1-(4-(4-Chlorbenzyl)piperazin-1-yl)-2-((1-(4-methoxy-2,6-dimethylphenylsulfonyl)piperidin-2-yl)methoxy)ethanon
- **385**: 2-((1-(4-Methoxy-2,6-dimethylphenylsulfonyl)piperidin-2-yl)methoxy)-1-(4-(4-methylbenzyl)piperazin-1-yl)ethanon
- **386**: 2-((1-(4-Methoxy-2,6-dimethylphenylsulfonyl)piperidin-2-yl)methoxy)-1-(4-(4-methoxybenzyl)piperazin-1-yl)ethanon
- **387**: 1-(4-(2-Fluorbenzyl)piperazin-1-yl)-2-((1-(4-methoxy-2,6-dimethylphenylsulfonyl)piperidin-2-yl)methoxy)ethanon
- **388**: 2-((1-(4-Methoxy-2,6-dimethylphenylsulfonyl)piperidin-2-yl)methoxy)-1-(4-methyl-2-phenylpiperazin-1-yl)ethanon
- **389**: 1-(3-(Pyridin-3-yl)pyrrolidin-1-yl)-2-((1-(2,4,6-trichlorphenylsulfonyl)piperidin-2-yl)methoxy)ethanon
- **390**: 1-(3-(Pyridin-4-yl)pyrrolidin-1-yl)-2-((1-(2,4,6-trichlorphenylsulfonyl)piperidin-2-yl)methoxy)ethanon
- **391**: N-Methyl-N-(2-(pyridin-4-yl)ethyl)-2-((1-(2,4,6-trichlorphenylsulfonyl)piperidin-2-yl)methoxy)acetamid
- **392**: N-Methyl-N-(1-phenyl-2-(pyrrolidin-1-yl)ethyl)-2-((1-(2,4,6-trichlorphenylsulfonyl)piperidin-2-yl)methoxy)acetamid
- **393**: 1-(4-Methylpiperazin-1-yl)-2-((1-(2,4,6-trichlorphenylsulfonyl)piperidin-2-yl)methoxy)ethanon
- **394**: N-(Pyridin-4-ylmethyl)-2-((1-(2,4,6-trichlorphenylsulfonyl)piperidin-2-yl)methoxy)acetamid
- **395**: 2-((1-(4-Methoxy-2,6-dimethylphenylsulfonyl)piperidin-2-yl)methoxy)-N-(2-morpholinopyridin-3-yl)acetamid
- **396**: 2-((1-(4-Methoxy-2,6-dimethylphenylsulfonyl)piperidin-2-yl)methoxy)-N-(6-morpholinopyridin-3-yl)acetamid
- **397**: 2-((1-(4-Methoxy-2,6-dimethylphenylsulfonyl)piperidin-2-yl)methoxy)-1-(4-(5-methyl-1H-benzo[d]imidazol-2-yl)piperidin-1-yl)ethanon
- **398**: 1-(2-(4-(Dimethylamino)phenyl)pyrrolidin-1-yl)-2-((1-(4-methoxy-2,6-dimethylphenylsulfonyl)piperidin-2-yl)methoxy)ethanon
- **399**: 2-((1-(4-Methoxy-2,6-dimethylphenylsulfonyl)piperidin-2-yl)methoxy)-1-(2-(piperidin-1-ylmethyl)pyrrolidin-1-yl)ethanon
- **400**: 1-(2-(4-(Dimethylamino)phenyl)azepan-1-yl)-2-((1-(4-methoxy-2,6-dimethylphenylsulfonyl)piperidin-2-yl)methoxy)ethanon
- **401**: N-(2-(4-Benzylpiperazin-1-yl)ethyl)-2-((1-(4-methoxy-2,6-dimethylphenylsulfonyl)piperidin-2-yl)methoxy)acetamid
- **402**: 2-((1-(4-Methoxy-2,6-dimethylphenylsulfonyl)piperidin-2-yl)methoxy)-1-(4-(4-phenylthiazol-2-yl)piperazin-1-yl)ethanon
- **403**: 2-((1-(2,6-Dichlorphenylsulfonyl)piperidin-2-yl)methoxy)-1-(4-(3,4-dimethylphenyl)piperazin-1-yl)ethanon
- **404**: 1-(4-(3,4-Dichlorphenyl)piperazin-1-yl)-2-((1-(2,6-dichlorphenylsulfonyl)piperidin-2-yl)methoxy)ethanon
- **405**: 1-(4-(3,4-Dichlorbenzyl)piperazin-1-yl)-2-((1-(2,6-dichlorphenylsulfonyl)piperidin-2-yl)methoxy)ethanon
- **406**: 1-(4-(4-Brombenzyl)piperazin-1-yl)-2-((1-(2,6-dichlorphenylsulfonyl)piperidin-2-yl)methoxy)ethanon
- **407**: 1-(4-(4-Chlorbenzyl)piperazin-1-yl)-2-((1-(2,6-dichlorphenylsulfonyl)piperidin-2-yl)methoxy)ethanon
- **408**: 2-((1-(2,6-Dichlorphenylsulfonyl)piperidin-2-yl)methoxy)-1-(4-(4-methylbenzyl)piperazin-1-yl)ethanon
- **409**: 2-((1-(4-Methoxy-2,6-dimethylphenylsulfonyl)piperidin-2-yl)methoxy)-N-(4-(pyrrolidin-1-yl)phenyl)acetamid
- **410**: 3-(4-(2-((1-(4-Methoxy-2,6-dimethylphenylsulfonyl)piperidin-2-yl)methoxy)acetyl)piperazin-1-yl)propanenitrile
- **411**: N-(3-(4-Menzylpiperazin-1-yl)propyl)-2-((1-(4-methoxy-2,6-dimethylphenylsulfonyl)piperidin-2-yl)methoxy)acetamid
- **412**: N-(3-(4-Ethylpiperazin-1-yl)propyl)-2-((1-(4-methoxy-2,6-dimethylphenylsulfonyl)piperidin-2-yl)methoxy)acetamid
- **413**: 2-((1-(4-Methoxy-2,6-dimethylphenylsulfonyl)piperidin-2-yl)methoxy)-N-(3-(piperidin- 1-yl)propyl)acetamid
- **414**: N-((1-Benzylpyrrolidin-3-yl)methyl)-2-((1-(4-methoxy-2,6-dimethylphenylsulfonyl)piperidin-2-yl)methoxy)acetamid
- **415**: N-((1-Benzylpiperidin-3-yl)methyl)-2-((1-(4-methoxy-2,6-dimethylphenylsulfonyl)piperidin-2-yl)methoxy)acetamid
- **416**: 2-((1-(4-Methoxy-2,6-dimethylphenylsulfonyl)piperidin-2-yl)methoxy)-N-(2-(5-methyl-1H-pyrazol-1-yl)ethyl)acetamid
- **417**: 2-((1-(4-Methoxy-2,6-dimethylphenylsulfonyl)piperidin-2-yl)methoxy)-N-(4-(2-(1-methyl-1H-benzo[d]imidazol-2-yl)ethyl)phenyl)acetamid
- **418**: 2-((1-(4-Methoxy-2,6-dimethylphenylsulfonyl)piperidin-2-yl)methoxy)-N-(3-(piperidin-1-ylmethyl)phenyl)acetamid
- **419**: 2-((1-(4-Methoxy-2,6-dimethylphenylsulfonyl)piperidin-2-yl)methoxy)-N-(4-(piperidin-1-ylmethyl)phenyl)acetamid
- **420**: N-(4-((1H-Imidazol-1-yl)methyl)phenyl)-2-((1-(4-methoxy-2,6-dimethylphenylsulfonyl)piperidin-2-yl)methoxy)acetamid
- **421**: 2-((1-(4-Methoxy-2,6-dimethylphenylsulfonyl)piperidin-2-yl)methoxy)-N-(4-((4-methylpiperidin-1-yl)methyl)phenyl)acetamid
- **422**: N-((1-Benzylpiperidin-3-yl)methyl)-2-((1-(4-methoxyphenylsulfonyl)indolin-2-yl)methoxy)acetamid
- **423**: 2-((1-(4-Methoxyphenylsulfonyl)indolin-2-yl)methoxy)-N-(3-(piperidin-1-ylmethyl)phenyl)acetamid
- **424**: N-(3-(Ethyl(phenyl)amino)propyl)-2-((1-(4-methoxy-2,6-dimethylphenylsulfonyl)piperidin-2-yl)methoxy)acetamid
- **425**: 2-((1-(4-Methoxy-2,6-dimethylphenylsulfonyl)piperidin-2-yl)methoxy)-N-(2-(pyrazin-2-yl)ethyl)acetamid
- **426**: N-(1-(1H-pyrazol-1-yl)propan-2-yl)-2-((1-(4-methoxy-2,6-dimethylphenylsulfonyl)piperidin-2-yl)methoxy)acetamid
- **427**: 2-((1-(4-Methoxy-2,6-dimethylphenylsulfonyl)piperidin-2-yl)methoxy)-N-(4-(2-methyl-1 H-imidazol-1-yl)phenyl)acetamid
- **428**: 2-((1-(4-Methoxy-2,6-dimethylphenylsulfonyl)piperidin-2-yl)methoxy)-N-(2-(piperidin-1-yl)benzyl)acetamid
- **429**: 2-((1-(4-Methoxy-2,6-dimethylphenylsulfonyl)piperidin-2-yl)methoxy)-N-(3-(piperidin-1-yl)phenyl)acetamid
- **430**: 2-((1-(4-Methoxy-2,6-dimethylphenylsulfonyl)piperidin-2-yl)methoxy)-N-(3-(piperidin-1-yl)benzyl)acetamid
- **431**: 2-((1-(4-Methoxy-2,6-dimethylphenylsulfonyl)piperidin-2-yl)methoxy)-N-(2-(4-methylpiperazin-1-yl)phenyl)acetamid
- **432**: 2-((1-(4-Methoxy-2,6-dimethylphenylsulfonyl)piperidin-2-yl)methoxy)-N-(2-(4-methylpiperazin-1-yl)benzyl)acetamid
- **433**: 2-((1-(4-Methoxy-2,6-dimethylphenylsulfonyl)piperidin-2-yl)methoxy)-N-(3-(4-methylpiperazin-1-yl)benzyl)acetamid
- **434**: 2-((1-(4-Methoxy-2,6-dimethylphenylsulfonyl)piperidin-2-yl)methoxy)-N-(4-(4-methylpiperazin-1-yl)benzyl)acetamid
- **435**: 2-((1-(4-Methoxy-2,6-dimethylphenylsulfonyl)piperidin-2-yl)methoxy)-N-(2-morpholinobenzyl)acetamid
- **436**: 2-((1-(4-Methoxy-2,6-dimethylphenylsulfonyl)piperidin-2-yl)methoxy)-N-(3-morpholinophenyl)acetamid
- **437**: 2-((1-(4-Methoxy-2,6-dimethylphenylsulfonyl)piperidin-2-yl)methoxy)-N-(3-morpholinobenzyl)acetamid
- **438**: 2-((1-(4-Methoxy-2,6-dimethylphenylsulfonyl)piperidin-2-yl)methoxy)-N-(4-morpholinophenyl)acetamid
- **439**: 2-((1-(4-Methoxy-2,6-dimethylphenylsulfonyl)piperidin-2-yl)methoxy)-N-(4-morpholinobenzyl)acetamid
- **440**: 2-((1-(2,6-Dichlorphenylsulfonyl)piperidin-2-yl)methoxy)-N-(2-(piperidin-1-yl)ethyl)acetamid
- **441**: 2-((1-(2,6-Dichlorphenylsulfonyl)piperidin-2-yl)methoxy)-N-(4-(dimethylamino)butyl)acetamid
- **442**: 2-((1-(2,6-Dichlorphenylsulfonyl)piperidin-2-yl)methoxy)-1-(4-(2-fluorphenyl)piperazin-1-yl)ethanon
- **443**: 2-((1-(2,6-Dichlorphenylsulfonyl)piperidin-2-yl)methoxy)-1-(4-(pyrrolidin-1-yl)piperidin-1-yl)ethanon
- **444**: 1-(4-(2-Fluorbenzyl)piperazin-1-yl)-2-((1-(4-methoxy-2,6-dimethylphenylsulfonyl)pyrrolidin-2-yl)methoxy)ethanon
- **445**: 2-((1-(4-Methoxy-2,6-dimethylphenylsulfonyl)pyrrolidin-2-yl)methoxy)-1-(4-methyl-2-phenylpiperazin-1-yl)ethanon
- **446**: 2-((1-(4-Methoxy-2,6-dimethylphenylsulfonyl)pyrrolidin-2-yl)methoxy)-N-methyl-N-(2-(4-methylpiperazin-1-yl)-1-phenylethyl)acetamid
- **447**: 2-((1-(4-Methoxy-2,6-dimethylphenylsulfonyl)pyrrolidin-2-yl)methoxy)-1-(2-(pyridin-2-ylmethyl)pyrrolidin-1-yl)ethanon
- **448**: 2-((1-(4-Methoxy-2,6-dimethylphenylsulfonyl)pyrrolidin-2-yl)methoxy)-1-(2-((6-methylpyridin-2-yl)methyl)pyrrolidin-1-yl)ethanon
- **449**: 2-((1-(4-Methoxy-2,6-dimethylphenylsulfonyl)pyrrolidin-2-yl)methoxy)-1-(2-(pyridin-2-ylmethyl)piperidin-1-yl)ethanon
- **450**: 2-((1-(4-Methoxy-2,6-dimethylphenylsulfonyl)pyrrolidin-2-yl)methoxy)-N-methyl-N-(1-phenyl-2-(pyrrolidin-1-yl)ethyl)acetamid
- **451**: 2-((1-(2,6-Dichlorphenylsulfonyl)pyrrolidin-2-yl)methoxy)-1-(4-(4-methoxybenzyl)piperazin-1-yl)ethanon
- **452**: 2-((1-(2,6-Dichlorphenylsulfonyl)pyrrolidin-2-yl)methoxy)-1-(4-(2-(dimethylamino)ethyl)piperazin-1-yl)ethanon
- **453**: 2-((1-(2,6-Dichlorphenylsulfonyl)pyrrolidin-2-yl)methoxy)-1-(2-(pyridin-4-ylmethyl)piperidin-1-yl)ethanon
- **454**: 2-((1-(2,6-Dichlorphenylsulfonyl)piperidin-2-yl)methoxy)-1-(4-(2-fluorbenzyl)piperazin-1-yl)ethanon
- **455**: 2-((1-(2,6-Dichlorphenylsulfonyl)piperidin-2-yl)methoxy)-1-(4-methyl-2-phenylpiperazin-1-yl)ethanon
- **456**: 2-((1-(2,6-Dichlorphenylsulfonyl)piperidin-2-yl)methoxy)-1-(2-(pyridin-2-ylmethyl)pyrrolidin-1-yl)ethanon
- **457**: 2-((1-(2,6-Dichlorphenylsulfonyl)piperidin-2-yl)methoxy)-1-(2-((4,6-dimethylpyridin-2-yl)methyl)pyrrolidin-1-yl)ethanon
- **458**: 2-((1-(2,6-Dichlorphenylsulfonyl)piperidin-2-yl)methoxy)-1-(2-((6-methylpyridin-2- yl)methyl)pyrrolidin-1-yl)ethanon
- **459**: N-(2-(Pyrrolidin-1-yl)ethyl)-2-((1-(2,4,6-trichlorphenylsulfonyl)piperidin-2-yl)methoxy)acetamid
- **460**: 1-(4-Benzylpiperazin-1-yl)-2-((1-(4-methoxy-2,6-dimethylphenylsulfonyl)pyrrolidin-2-yl)methoxy)ethanon
- **461**: 2-((1-(4-Methoxy-2,6-dimethylphenylsulfonyl)pyrrolidin-2-yl)methoxy)-1-(4-phenylpiperazin-1-yl)ethanon
- **462**: 1-(4-(Benzo[d][1,3]dioxol-5-ylmethyl)piperazin-1-yl)-2-((1-(4-methoxy-2,6-dimethylphenylsulfonyl)pyrrolidin-2-yl)methoxy)ethanon
- **463**: N-Benzyl-2-((1-(4-methoxy-2,6-dimethylphenylsulfonyl)pyrrolidin-2-yl)methoxy)-N-(4-methylpyridin-2-yl)acetamid
- **464**: N-Benzyl-2-((1-(4-methoxy-2,6-dimethylphenylsulfonyl)pyrrolidin-2-yl)methoxy)-N-(6-methylpyridin-2-yl)acetamid
- **465**: 2-((1-(4-Methoxy-2,6-dimethylphenylsulfonyl)pyrrolidin-2-yl)methoxy)-1-(4-(3-(trifluormethyl)phenyl)piperazin-1-yl)ethanon
- **466**: N-Ethyl-2-((1-(4-methoxy-2,6-dimethylphenylsulfonyl)pyrrolidin-2-yl)methoxy)-N-(pyridin-4-ylmethyl)acetamid
- **467**: 2-((1-(4-Methoxy-2,6-dimethylphenylsulfonyl)pyrrolidin-2-yl)methoxy)-1-(4-methyl-1,4-diazepan-1-yl)ethanon
- **468**: 2-((1-(4-Methoxy-2,6-dimethylphenylsulfonyl)pyrrolidin-2-yl)methoxy)-1-(4-(5-(trifluormethyl)pyridin-2-yl)piperazin-1-yl)ethanon
- **469**: 1-(4-(3-Chlor-5-(trifluormethyl)pyridin-2-yl)piperazin-1-yl)-2-((1-(4-methoxy-2,6-dimethylphenylsulfonyl)pyrrolidin-2-yl)methoxy)ethanon
- **470**: 2-((1-(4-Methoxy-2,6-dimethylphenylsulfonyl)pyrrolidin-2-yl)methoxy)-N-(4-(2-(1-methyl-1 H-benzo[d]imidazol-2-yl)ethyl)phenyl)acetamid
- **471**: 2-((1-(4-Methoxy-2,6-dimethylphenylsulfonyl)pyrrolidin-2-yl)methoxy)-N-(3-(piperidin-1-ylmethyl)phenyl)acetamid
- **472**: N-(4-(2-(1H-Benzo[d]imidazol-2-yl)ethyl)phenyl)-2-((1-(4-methoxy-2,6-dimethylphenylsulfonyl)pyrrolidin-2-yl)methoxy)acetamid
- **473**: 2-((1-(4-Methoxy-2,6-dimethylphenylsulfonyl)pyrrolidin-2-yl)methoxy)-N-(2-(4-methylpiperazin-1-yl)benzyl)acetamid
- **474**: 2-((1-(4-Methoxy-2,6-dimethylphenylsulfonyl)pyrrolidin-2-yl)methoxy)-1-(4-(5-methyl-1 H-benzo[d]imidazol-2-yl)piperidin-1-yl)ethanon
- **475**: 1-(2-(4-(Dimethylamino)phenyl)azepan-1-yl)-2-((1-(4-methoxy-2,6-dimethylphenylsulfonyl)pyrrolidin-2-yl)methoxy)ethanon
- **476**: 1-((R)-3-(Dimethylamino)pyrrolidin-1-yl)-2-((1-(4-methoxy-2,6-dimethylphenylsulfonyl)pyrrolidin-2-yl)methoxy)ethanon
- **477**: N-(1-Benzylpiperidin-4-yl)-2-((1-(4-methoxy-2,6-dimethylphenylsulfonyl)pyrrolidin-2-yl)methoxy)acetamid
- **478**: 1-(4-Benzylpiperazin-1-yl)-2-((1-(2,5-dichlorthiophen-3-ylsulfonyl)pyrrolidin-2-yl)methoxy)ethanon
- **479**: 2-((2-(4-Methoxy-2,3,6-trimethylphenylsulfonyl)-1,2,3,4-tetrahydroisoquinolin-3-yl)methoxy)-1-(4-phenylpiperazin-1-yl)ethanon
- **480**: 1-(4-Benzylpiperazin-1-yl)-2-((1-(2,6-dichlorphenylsulfonyl)piperidin-2-yl)methoxy)ethanon
- **481**: 2-((1-(2,6-Dichlorphenylsulfonyl)piperidin-2-yl)methoxy)-1-(4-(3-(trifluormethyl)phenyl)piperazin-1-yl)ethanon
- **482**: 2-((1-(2,6-Dichlorphenylsulfonyl)piperidin-2-yl)methoxy)-N-ethyl-N-(pyridin-4-ylmethyl)acetamid
- **483**: 2-((1-(2,6-Dichlorphenylsulfonyl)piperidin-2-yl)methoxy)-1-(4-methyl-1,4-diazepan-1-yl)ethanon
- **484**: 2-((1-(2,6-Dichlorphenylsulfonyl)piperidin-2-yl)methoxy)-1-(4-(5-(trifluormethyl)pyridin-2-yl)piperazin-1-yl)ethanon
- **485**: 1-(4-(3-Chlor-5-(trifluormethyl)pyridin-2-yl)piperazin-1-yl)-2-((1-(2,6-dichlorphenylsulfonyl)piperidin-2-yl)methoxy)ethanon
- **486**: N-Benzyl-2-((1-(2,6-dichlorphenylsulfonyl)piperidin-2-yl)methoxy)-N-(pyridin-2-yl)acetamid
- **487**: 2-((1-(2,6-Dichlorphenylsulfonyl)piperidin-2-yl)methoxy)-1-(4-(5-methyl-1H-benzo[d]imidazol-2-yl)piperidin-1-yl)ethanon
- **488**: N-(1-Benzylpiperidin-4-yl)-2-((1-(2,6-dichlorphenylsulfonyl)piperidin-2-yl)methoxy)acetamid
- **489**: N-Benzyl-2-((1-(2,6-dichlorphenylsulfonyl)piperidin-2-yl)methoxy)-N-(2-(dimethylamino)ethyl)acetamid
- **490**: 1-(2-((5-Ethylpyridin-2-yl)methyl)pyrrolidin-1-yl)-2-((1-(4-methoxy-2,6-dimethylphenylsulfonyl)pyrrolidin-2-yl)methoxy)ethanon
- **491**: 2-((1-(4-Methoxy-2,6-dimethylphenylsulfonyl)pyrrolidin-2-yl)methoxy)-1-(4-(2-methoxyphenyl)piperazin-1-yl)ethanon
- **492**: 1-(4-(Cyclohexylmethyl)piperazin-1-yl)-2-((1-(4-methoxy-2,6-dimethylphenylsulfonyl)pyrrolidin-2-yl)methoxy)ethanon
- **493**: N-(3-Chlor-4-morpholinophenyl)-2-((1-(4-methoxy-2,6-dimethylphenylsulfonyl)pyrrolidin-2-yl)methoxy)acetamid
- **494**: 2-((1-(4-Methoxy-2,6-dimethylphenylsulfonyl)pyrrolidin-2-yl)methoxy)-N-(1-methyl-1H-benzo[d]imidazol-5-yl)acetamid
- **495**: N-(2-(Dimethylamino)-2-(4-(trifluormethyl)phenyl)ethyl)-2-((1-(4-methoxy-2,6-dimethylphenylsulfonyl)pyrrolidin-2-yl)methoxy)acetamid
- **496**: 2-((1-(4-Methoxy-2,6-dimethylphenylsulfonyl)pyrrolidin-2-yl)methoxy)-N-(2-(pyrrolidin-1-yl)-2-(4-(trifluormethyl)phenyl)ethyl)acetamid
- **497**: 2-((1-(2,6-Dichlorphenylsulfonyl)piperidin-2-yl)methoxy)-1-(2-(pyridin-3-yl)pyrrolidin-1-yl)ethanon
- **498**: 2-((1-(2,6-Dichlorphenylsulfonyl)piperidin-2-yl)methoxy)-1-(4-(2-methoxyphenyl)piperazin-1-yl)ethanon
- **499**: 1-(4-(Cyclohexylmethyl)piperazin-1-yl)-2-((1-(2,6-dichlorphenylsulfonyl)piperidin-2-yl)methoxy)ethanon
- **500**: 2-((1-(4-Methoxy-2,6-dimethylphenylsulfonyl)pyrrolidin-2-yl)methoxy)-N-(2-morpholino-1-phenylethyl)acetamid
- **501**: 2-((1-(4-Methoxy-2,6-dimethylphenylsulfonyl)pyrrolidin-2-yl)methoxy)-N-(1-methyl-1H-indazol-6-yl)acetamid
- **502**: N-(2-(2-Chlorphenyl)-2-(pyrrolidin-1-yl)ethyl)-2-((1-(4-methoxy-2,6-dimethylphenylsulfonyl)pyrrolidin-2-yl)methoxy)acetamid
- **503**: 1-(4-(3,5-Dimethoxyphenyl)piperazin-1-yl)-2-((1-(4-methoxy-2,6- dimethylphenylsulfonyl)pyrrolidin-2-yl)methoxy)ethanon
- **504**: 2-((1-(4-Methoxy-2,6-dimethylphenylsulfonyl)pyrrolidin-2-yl)methoxy)-N-(1-methyl-1H-benzo[d]imidazol-2-yl)acetamid
- **505**: 2-((1-(4-Methoxy-2,6-dimethylphenylsulfonyl)pyrrolidin-2-yl)methoxy)-N-(1-phenyl-2-(pyrrolidin-1-yl)ethyl)acetamid
- **506**: 3-(4-(2-((1-(2,6-Dichlorphenylsulfonyl)piperidin-2-yl)methoxy)acetyl)piperazin-1-yl)propanenitrile
- **507**: N-Benzyl-2-((1-(4-methoxy-2,6-dimethylphenylsulfonyl)piperidin-2-yl)methoxy)-N-(4-methylpyridin-2-yl)acetamid
- **508**: N-Benzyl-2-((1-(4-methoxy-2,6-dimethylphenylsulfonyl)piperidin-2-yl)methoxy)-N-(6-methylpyridin-2-yl)acetamid
- **509**: 2-((1-(4-Methoxy-2,6-dimethylphenylsulfonyl)piperidin-2-yl)methoxy)-1-(4-(3-(trifluormethyl)phenyl)piperazin-1-yl)ethanon
- **510**: N-ethyl-2-((1-(4-methoxy-2,6-dimethylphenylsulfonyl)piperidin-2-yl)methoxy)-N-(pyridin-4-ylmethyl)acetamid
- **511**: 1-(4-(3-Chlorphenyl)piperazin-1-yl)-2-((1-(4-methoxy-2,6-dimethylphenylsulfonyl)piperidin-2-yl)methoxy)ethanon
- **512**: 1-(4-(3,4-Dimethylphenyl)piperazin-1-yl)-2-((1-(4-methoxy-2,6-dimethylphenylsulfonyl)piperidin-2-yl)methoxy)ethanon
- **513**: 2-(4-(2-((1-(4-Methoxy-2,6-dimethylphenylsulfonyl)piperidin-2-yl)methoxy)acetyl)piperazin-1-yl)nicotinonitrile
- **514**: 2-((1-(4-Methoxy-2,6-dimethylphenylsulfonyl)piperidin-2-yl)methoxy)-1-(4-(5-(trifluormethyl)pyridin-2-yl)piperazin-1-yl)ethanon
- **515**: N-Benzyl-2-((1-(2,5-dichlorthiophen-3-ylsulfonyl)piperidin-2-yl)methoxy)-N-(6-methylpyridin-2-yl)acetamid
- **516**: 1-(4-(2-Fluorphenyl)piperazin-1-yl)-2-((1-(4-methoxy-2,6-dimethylphenylsulfonyl)pyrrolidin-2-yl)methoxy)ethanon
- **517**: 1-(4-Ethylpiperazin-1-yl)-2-((1-(4-methoxy-2,6-dimethylphenylsulfonyl)pyrrolidin-2-yl)methoxy)ethanon
- **518**: 2-((1-(4-Methoxy-2,6-dimethylphenylsulfonyl)pyrrolidin-2-yl)methoxy)-1-(4-(pyrrolidin-1-yl)piperidin-1-yl)ethanon
- **519**: 2-((1-(4-Methoxy-2,6-dimethylphenylsulfonyl)pyrrolidin-2-yl)methoxy)-1-(4-morpholinopiperidin-1-yl)ethanon
- **520**: 1-(1,4'-Bipiperidin-1'-yl)-2-((1-(4-methoxy-2,6-dimethylphenylsulfonyl)pyrrolidin-2-yl)methoxy)ethanon
- **521**: N-(3-(4-Benzylpiperazin-1-yl)propyl)-2-((1-(4-methoxy-2,6-dimethylphenylsulfonyl)pyrrolidin-2-yl)methoxy)acetamid
- **522**: 2-((1-(4-Methoxy-2,6-dimethylphenylsulfonyl)pyrrolidin-2-yl)methoxy)-N-(3-(piperidin-1-yl)propyl)acetamid
- **523**: 2-((1-(4-Methoxy-2,6-dimethylphenylsulfonyl)pyrrolidin-2-yl)methoxy)-N-(4-(pyrrolidin-1-yl)butyl)acetamid
- **524**: 2-((1-(4-Methoxy-2,6-dimethylphenylsulfonyl)piperidin-2-yl)methoxy)-1-(4-(4-methylpiperazin-1-yl)piperidin-1-yl)ethanon
- **525**: N-(3-(1H-Benzo[d]imidazol-2-yl)propyl)-2-((1-(4-methoxy-2,6-dimethylphenylsulfonyl)piperidin-2-yl)methoxy)acetamid
- **526**: 1-(4-(Benzo[d]thiazol-2-yl)piperazin-1-yl)-2-((1-(4-methoxy-2,6-dimethylphenylsulfonyl)piperidin-2-yl)methoxy)ethanon
- **527**: 2-((1-(2,6-Dichlorphenylsulfonyl)piperidin-2-yl)methoxy)-N-(4-(2-methyl-1H-imidazol-1-yl)phenyl)acetamid
- **528**: 2-((1-(2,6-Dichlorphenylsulfonyl)piperidin-2-yl)methoxy)-1-(2-(4-(dimethylamino)phenyl)pyrrolidin-1-yl)ethanon
- **529**: 1-(4-Benzyl-1,4-diazepan-1-yl)-2-((1-(2,6-dichlorphenylsulfonyl)piperidin-2-yl)methoxy)ethanon
- **530**: 2-((1-(2,6-Dichlorphenylsulfonyl)piperidin-2-yl)methoxy)-N-(4-(dimethylamino)benzyl)-N-isopropylacetamid
- **531**: 2-(4-(2-((1-(4-Methoxy-2,6-dimethylphenylsulfonyl)pyrrolidin-2-yl)methoxy)acetyl)-1,4-diazepan-1-yl)nicotinonitrile
- **532**: 2-((1-(4-Methoxy-2,6-dimethylphenylsulfonyl)piperidin-2-yl)methoxy)-N-methyl-N-(2-(piperidin-1-yl)ethyl)acetamid
- **533**: 2-((1-(4-Methoxy-2,6-dimethylphenylsulfonyl)piperidin-2-yl)methoxy)-1-(4-(2-(piperidin-1-yl)ethyl)piperidin-1-yl)ethanon
- **534**: 2-((1-(4-Methoxy-2,6-dimethylphenylsulfonyl)piperidin-2-yl)methoxy)-1-(2-(2-morpholinoethyl)piperidin-1-yl)ethanon
- **535**: 2-((1-(4-Methoxy-2,6-dimethylphenylsulfonyl)piperidin-2-yl)methoxy)-N-(5-methyl-1H-pyrazol-3-yl)acetamid
- **536**: 2-((1-(4-Methoxy-2,6-dimethylphenylsulfonyl)piperidin-2-yl)methoxy)-N-(5-phenyl-1 H-pyrazol-3-yl)acetamid
- **537**: N-(3-(4-Chlorphenyl)-1H-pyrazol-5-yl)-2-((1-(4-methoxy-2,6-dimethylphenylsulfonyl)piperidin-2-yl)methoxy)acetamid
- **538**: N-(4-(3,4-Dimethoxyphenyl)-1H-pyrazol-5-yl)-2-((1-(4-methoxy-2,6-dimethylphenylsulfonyl)piperidin-2-yl)methoxy)acetamid
- **539**: 2-((1-(4-Methoxy-2,6-dimethylphenylsulfonyl)piperidin-2-yl)methoxy)-N-(3-(thiophen-2-yl)-1H-pyrazol-5-yl)acetamid
- **540**: 2-((1-(4-Methoxy-2,6-dimethylphenylsulfonyl)piperidin-2-yl)methoxy)-N-(5-(2-methoxyphenyl)-1H-pyrazol-3-yl)acetamid
- **541**: 2-((1-(4-Methoxy-2,6-dimethylphenylsulfonyl)piperidin-2-yl)methoxy)-N-(5-(3-methoxyphenyl)-1H-pyrazol-3-yl)acetamid
- **542**: N-(5-(3-Fluorphenyl)-1H-pyrazol-3-yl)-2-((1-(4-methoxy-2,6-dimethylphenylsulfonyl)piperidin-2-yl)methoxy)acetamid
- **543**: 2-((1-(4-Methoxy-2,6-dimethylphenylsulfonyl)piperidin-2-yl)methoxy)-N-(5-(4-methoxyphenyl)-1H-pyrazol-3-yl)acetamid
- **544**: 2-((1-(4-Methoxy-2,6-dimethylphenylsulfonyl)piperidin-2-yl)methoxy)-N-(3-p-tolyl-1 H-pyrazol-5-yl)acetamid
- **545**: 2-((1-(4-Methoxy-2,6-dimethylphenylsulfonyl)piperidin-2-yl)methoxy)-N-(3-phenyl-1 H-pyrazol-5-yl)acetamid
- **546**: 2-((1-(4-Methoxy-2,6-dimethylphenylsulfonyl)piperidin-2-yl)methoxy)-N-(quinolin-2-yl)acetamid
- **547**: 2-((1-(4-Methoxyphenylsulfonyl)indolin-2-yl)methoxy)-1-(4-(2-(piperidin-1-yl)ethyl)piperidin-1-yl)ethanon
- **548**: 1-(4-(2-(2,5-Dimethyl-1H-pyrrol-1-yl)ethyl)piperazin-1-yl)-2-((1-(4- methoxyphenylsulfonyl)indolin-2-yl)methoxy)ethanon
- **549**: 2-((1-(2,6-Dichlorphenylsulfonyl)piperidin-2-yl)methoxy)-N-(4-(morpholinomethyl)benzyl)acetamid
- **550**: 2-((1-(2,6-Dichlorphenylsulfonyl)piperidin-2-yl)methoxy)-1-(4-(4-(trifluormethyl)phenyl)piperazin-1-yl)ethanon
- **551**: 2-((1-(2,6-Dichlorphenylsulfonyl)piperidin-2-yl)methoxy)-1-(4-(3,5-dichlorpyridin-2-yl)piperazin-1-yl)ethanon
- **552**: 2-((1-(2,6-Dichlorphenylsulfonyl)piperidin-2-yl)methoxy)-1-(4-(2-hydroxyethyl)piperazin-1-yl)ethanon
- **553**: 1-(4-(Benzo[d]thiazol-2-yl)piperazin-1-yl)-2-((1-(2,6-dichlorphenylsulfonyl)piperidin-2-yl)methoxy)ethanon
- **554**: 1-(4-(6-Chlorbenzo[d]thiazol-2-yl)piperazin-1-yl)-2-((1-(2,6-dichlorphenylsulfonyl)piperidin-2-yl)methoxy)ethanon
- **555**: N-(4-(4-Ethylpiperazin-1-yl)phenyl)-2-((1-(4-methoxyphenylsulfonyl)piperidin-2-yl)methoxy)acetamid
- **556**: 2-((1-(4-Methoxyphenylsulfonyl)piperidin-2-yl)methoxy)-N-(7-morpholinobenzo[c][1,2,5]oxadiazol-4-yl)acetamid
- **557**: 2-((1-(4-Methoxyphenylsulfonyl)piperidin-2-yl)methoxy)-N-(3-methyl-4-(2-(piperidin-1-yl)acetamido)phenyl)acetamid
- **558**: 2-((1-(4-Methoxyphenylsulfonyl)piperidin-2-yl)methoxy)-1-(4-(2-phenoxyethyl)piperazin-1-yl)ethanon
- **559**: 1-(4-(2-(2,5-Dimethyl-1H-pyrrol-1-yl)ethyl)piperazin-1-yl)-2-((1-(4-methoxy-2,6 dimethylphenylsulfonyl)piperidin-2-yl)methoxy)ethanon
- **560**: 1-(4-(3-(Dimethylamino)propyl)piperazin-1-yl)-2-((2-(4-methoxy-2,3,6-trimethylphenylsulfonyl)-1,2,3,4-tetrahydroisoquinolin-3-yl)methoxy)ethanon
- **561**: N,N-bis(3-(Dimethylamino)propyl)-2-((1-(4-methoxy-2,6-dimethylphenylsulfony)piperidin-2-yl)methoxy)acetamid
- **562**: 2-((2-(4-Methoxy-2,3,6-trimethylphenylsulfonyl)-1,2,3,4-tetrahydroisoquinolin-3-yl)methoxy)-1-(4-(1-methylpiperidin-4-yl)piperazin-1-yl)ethanon
- **563**: 2-((2-(4-Methoxy-2,3,6-trimethylphenylsulfonyl)-1,2,3,4-tetrahydroisoquinolin-3-yl)methoxy)-1-(4-((1-methylpiperidin-4-yl)methyl)piperazin-1-yl)ethanon
- **564**: 2-((1-(2,6-Dichlorphenylsulfonyl)piperidin-2-yl)methoxy)-1-(4-(3-(dimethylamino)propyl)piperazin-1-yl)ethanon
- **565**: 2-((1-(2,6-Dichlorphenylsulfonyl)piperidin-2-yl)methoxy)-1-(4-(1-methylpiperidin-4-yl)piperazin-1-yl)ethanon
- **566**: 2-((1-(2,6-Dichlorphenylsulfonyl)pyrrolidin-2-yl)methoxy)-1-(4-(3-(dimethylamino)propyl)piperazin-1-yl)ethanon
- **567**: 2-((1-(2,6-Dichlorphenylsulfonyl)pyrrolidin-2-yl)methoxy)-1-(4-(1-methylpiperidin-4-yl)piperazin-1-yl)ethanon
- **568**: 1-(4-Benzylpiperazin-1-yl)-2-((1-(4-chlor-2,5-dimethylphenylsulfonyl)piperidin-2-yl)methoxy)ethanon
- **569**: 2-((1-(2,6-Dichlor-4-(trifluormethyl)phenylsulfonyl)pyrrolidin-2-yl)methoxy)-1-(4-(3-(dimethylamino)propyl)piperazin-1-yl)ethanon
- **570**: 2-((1-(2,6-Dichlor-4-(trifluormethyl)phenylsulfonyl)pyrrolidin-2-yl)methoxy)-1-(4-(1-methylpiperidin-4-yl)piperazin-1-yl)ethanon
- **571**: 1-(4-(1-Methylpiperidin-4-yl)piperazin-1-yl)-2-((1-(2-(trifluormethyl)phenylsulfonyl)piperidin-2-yl)methoxy)ethanon
- **572**: 2-((1-(Benzo[b]thiophen-3-ylsulfonyl)piperidin-2-yl)methoxy)-1-(4-(4-methylpiperazin-1-yl)piperidin-1-yl)ethanon
- **573**: 2-((1-(Benzo[b]thiophen-3-ylsulfonyl)piperidin-2-yl)methoxy)-1-(4-((1-methylpiperidin-4-yl)methyl)piperazin-1-yl)ethanon
- **574**: N-((S)-1-Benzylpyrrolidin-3-yl)-2-((1-(2,6-dichlor-4-(trifluormethyl)phenylsulfonyl)piperidin-2-yl)methoxy)-N-methylacetamid
- **575**: N-(4-(Dimethylamino)benzyl)-2-((1-(2,4,6-trichlorphenylsulfonyl)pyrrolidin-2-yl)methoxy)acetamid
- **576**: N-(4-(dimethylamino)benzyl)-2-((1-(4-methoxy-2,6-dimethylphenylsulfonyl)pyrrolidin-2-yl)methoxy)acetamid
- **577**: N-(4-(Dimethylamino)benzyl)-2-((1-(4-methoxy-2,6-dimethylphenylsulfonyl)piperidin-2-yl)methoxy)acetamid
- **578**: N-(4-(Dimethylamino)benzyl)-2-((1-(2,4,6-trichlorphenylsulfonyl)piperidin-2-yl)methoxy)acetamid
- **579**: 2-((1-(4-Methoxy-2,6-dimethylphenylsulfonyl)pyrrolidin-2-yl)methoxy)-N-(4-morpholinobenzyl)acetamid
- **580**: N-(4-Morpholinobenzyl)-2-((1-(2,4,6-trichlorphenylsulfonyl)pyrrolidin-2-yl)methoxy)acetamid
- **581**: N-(4-Morpholinobenzyl)-2-((1-(2,4,6-trichlorphenylsulfonyl)piperidin-2-yl)methoxy)acetamid

in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die oben verwendete Nummerierung der einzelnen Ausführungsformen der erfindungsgemäßen Substanzen wird in den nachfolgenden Erläuterungen der vorliegenden Erfindung, insbesondere in der Beschreibung der Beispiele, beibehalten.

Die erfindungsgemäßen Verbindungen weisen eine antagonistische Wirkung an dem humanen B1R-Rezeptor oder dem B1R-Rezeptor der Ratte auf. In einer bevorzugten Ausführungsform der Erfindung weisen die erfindungsgemäßen Substanzen eine antagonistische Wirkung sowohl am humanen B1R- Rezeptor als auch am B1R-Rezeptor der Ratte auf.

Besonders bevorzugt sind Verbindungen, die im FLIPR-Assay bei einer Konzentration von 10 µM am humanen B1 R-Rezeptor und/oder am B1R-Rezeptor der Ratte eine Inhibition von mindestens 15%, 25%, 50%, 70%, 80% oder 90% aufweisen. Ganz besonders bevorzugt sind Verbindungen, die eine Inhibition am humanen B1R-Rezeptor und am B1R-Rezeptor der Ratte von mindestens 70%, insbesondere 80% und insbesondere bevorzugt 90% aufweisen.

Die agonistische bzw. antagonistische Wirkung von Substanzen kann am Bradykinin Rezeptor 1 (B1R) der Spezies Mensch und Ratte mit ektopisch exprimierenden Zelllinien (CHO K1 Zellen) und mit Hilfe eines Ca²⁺-sensitiven Farbstoffs (Fluo-4) im Fluorescent Imaging Plate Reader (FLIPR) quantifiziert werden. Die Angabe in % Aktivierung wird bezogen auf das Ca²⁺-Signal nach Zugabe von Lys-Des-Arg⁹-Bradykinin (0,5 nM), bzw. Des-Arg⁹-Bradykinin (100 nM). Antagonisten führen zu einer Unterdrückung des Ca²+-Einstroms nach der Zugabe des Agonisten. Angegeben werden % Inhibition im Vergleich zu der maximal erreichbaren Inhibition.

Die erfindungsgemäßen Substanzen wirken beispielsweise auf den im Zusammenhang mit verschiedenen Erkrankungen relevanten B1R, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Sulfonamid-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Sulfonamid-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße spirocyclische Azaindol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Sulfonamid-Derivate verzögert freisetzen. Die erfindungsgemäßen substituierten Sulfonamid -Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Sulfonamid-Derivats appliziert.

In einer bevorzugten Form des Arzneimittels liegt ein enthaltenes erfindungsgemäßes substituiertes Sulfonamid -Derivat als reines Diastereomer und/oder Enantiomer, als Razemat oder als nicht-äquimolare oder äquimolare Mischung der Diastereomere und/oder Enantiomere vor.

B1R wurde insbesondere im Schmerzgeschehen identifiziert. Entsprechend können erfindungsgemäße substituierten Sulfonamid-Derivate zur Herstellung eines Arzneimittels zur Behandlung von Schmerz, insbesondere von akutem, viszeralem, neuropathischem oder chronischem Schmerz, verwendet werden.

Ein weiterer Gegenstand der Erfindung ist daher die Verwendung eines erfindungsgemäßen substituierten Sulfonamid-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen substituierten Sulfonamid-Derivats zur Herstellung eines Arzneimittels zur Behandlung von Diabetes, Erkrankungen der Atemwege, entzündlichen Darmerkrankungen, neurologischen Erkrankungen, Entzündungen der Haut, rheumatischen Erkrankungen, septischem Schock, Reperfusionssyndrom, Fettleibigkeit und als Angiognese-Inhibitor.

Dabei kann es in einer der vorstehenden Verwendungen bevorzugt sein, wenn ein verwendetes substituiertes Sulfonamid-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis eines erfindungsgemäßen substituierten Sulfonamid-Derivats, oder eines erfindungsgemäßen Arzneimittels.

Ein weiterer Gegenstand der Erfindung ist ein Verfahren zur Herstellung der erfindungsgemäßen substituierten Sulfonamid-Derivats wie in der folgenden Beschreibung, Beispielen und Ansprüchen ausgeführt.

Die erfindungsgemäßen Verbindungen können nach dem folgenden Syntheseschema erhalten werden.

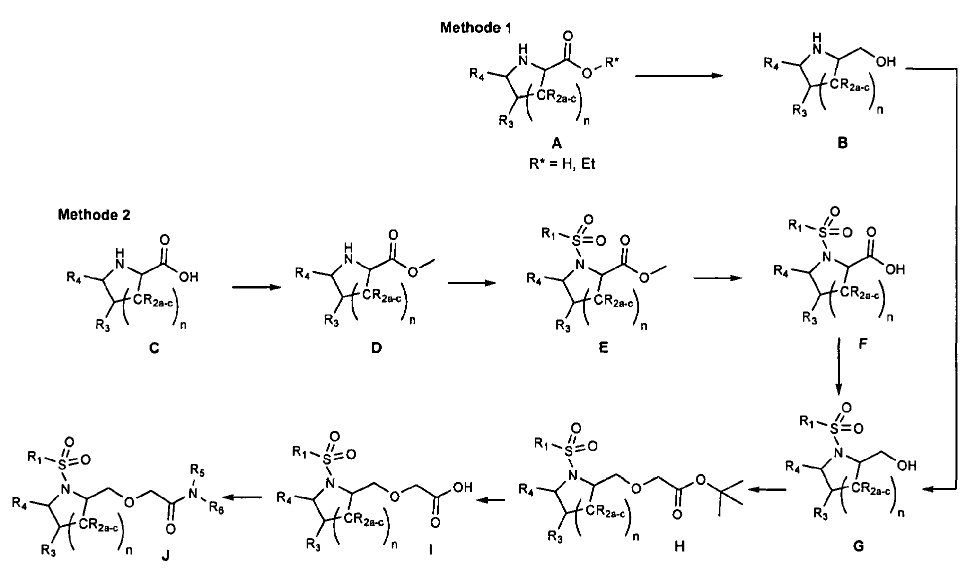

Dabei werden nach **Methode 1** die racemischen (R- und S-Konfiguration) oder enantiomerenreinen (R- oder S-Konfiguration) Aminosäuren/-säureester **A** durch eine Reduktion zu einem Aminoalkohol **B**, unter Verwendung von Metallhydriden als Reduktionsmitteln wie beispielsweise LiAlH₄, BH₃ x DMS, BH₃ x THF oder NaBH₄ in einem organischen Lösungsmittel wie THF oder Diethylether, in einem Temperaturbereich von -20°C - +100°C, bevorzugt bei 0°C - +70°C, umgesetzt. Die Aminoalkohole **B** werden in einer Sulfonylierung mit Sulfonylchloriden, -bromiden oder -pentafuorphenolaten R₁SO₂X (X = Br, Cl oder OPFP) in Gegenwart einer organischen oder anorganischen Base, beispielsweise Kaliumcarbonat, Natriumhydrogencarbonat, Diisopropylethylamin, Triethylamin, Pyridin, Diethylamin und/oder Dimethylaminopyridin, oder in Gegenwart von Tetra-n-butylammoniumchlorid und in einem organischen Lösungsmittel beispielsweise Acetonitril, Dichlormethan oder N,N-Dimethylformamid in einem Temperaturbereich von 0°C - 120 °C, zu den sulfonylierten Aminoalkoholen **G** umgesetzt.

In **Methode 2** werden die racemischen (R- und S-Konfiguration) oder enantiomerenreinen (R- oder S-Konfiguration) Aminosäuren **C** unter Verwendung wasserentziehender Reagenzien beispielsweise anorganische Säuren wie H₂SO₄ oder Phosphoroxiden oder organischen Reagenzien wie Thionylchlorid, in organische Lösungsmitteln wie THF, Diethylether, Methanol, Ethanol oder Dichlormethan in einem Temperaturbereich von 0°C bis zur Siedetemperatur des jeweiligen Lösungsmittels, bevorzugt bei 40°C, zu den Aminoestern **D** verestert und anschließend in einer Sulfonylierung mit Sulfonylchloriden oder -bromiden RSO₂X (X = Br oder Cl) in Gegenwart einer organischen oder anorganischen Base, beispielsweise Kaliumcarbonat, Natriumhydrogencarbonat, Diisopropylethylamin, Pyridin, Diethylamin oder Triethylamin und in einem organischen Lösungsmittel beispielsweise Acetonitril oder Dichlormethan, in einem Temperaturbereich von 0°C - +25°C, zu den sulfonylierten Aminoestern **E** umgesetzt, welche in einer Esterspaltung unter Verwendung von organischen Säuren, wie Trifluoressigsäure oder wässrigen anorganischen Säuren, wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, Dichlormethan, THF, Diethylether oder diese Lösungsmittel als Gemische, in einem Temperaturbereich von -20°C - +25°C, bevorzugt bei Raumtemperatur, die sulfonylierten Aminosäuren F ergeben.

Anschließend werden die sulfonylierten Aminoalkohole **G** in einer Alkylierungsreaktion mit halogenierten Esterderivaten unter Verwendung von Tetrabutylammoniumchlorid oder -bromid oder Tetrabutylammoniumhydrogensulfat in einer Phasentransferreaktion unter Verwendung eines organischen Lösungsmittels wie Toluol, Benzol oder Xylol und anorganischen Base wie Kaliumhydroxid, Natriumhydroxid, Natriumcarbonat, Natriumhydrogencarbonat, Kaliumcarbonat oder in Gegenwart einer organischen oder anorganischen Base, übliche anorganische Basen sind Metallalkoholate wie Natriummethanolat, Natriumethanolat, Kalium-tert-butylat, Lithium- oder Natriumbasen wie Lithiumdiisopropylamid, Buthyllithium, tert-Butyllithium, Natriummethylat oder Metallhydride wie Kaliumhydrid, Lithiumhydrid, Natriumhydrid; übliche organische Basen sind Diisopropylethylamin, Triethylamin, in einem organischen Lösungsmittel wie Dichlormethan, THF oder Diethylether, in einem Temperaturbereich von -20°C - +25°C, bevorzugt bei 0°C - +25°C, zu den Produkten der allgemeinen Struktur **H** umgesetzt, welche in einer Esterspaltung unter Verwendung von organischen Säuren wie Trifluoressigsäure oder wässrigen anorganischen Säuren wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, Dichlormethan, THF, Diethylether oder diese Lösungsmittel als Gemische, in einem Temperaturbereich von 0°C - +25°C, die Säurestufen der allgemeinen Formel **I** ergeben. Die Carbonsäuren **I** werden in einer Amidbildung unter Verwendung primärer oder sekundärer Amine, in Gegenwart wasserentziehender Mittel wie Natrium- oder Magnesiumsulfat, Phosphoroxid oder Reagenzien wie beispielsweise CDI, DCC, TBTU, EDCI oder Benzotriazol-1-yloxytris(dimethylamino)phosphonium Hexafluorphosphat (BOP) auch in Gegenwart von HOAt oder HOBt und einer organischen Base beispielsweise DIPEA, Pyridin oder 4-Methylmopholin in einem organischen Lösungsmittel wie THF, Dichlormethan, Diethylether, Dioxan, DMF oder Acetonitril, 0°C - + 40°C, vorzugsweise bei Raumtemperatur, zu den Endprodukten der allgemeinen Formel J umgesetzt.

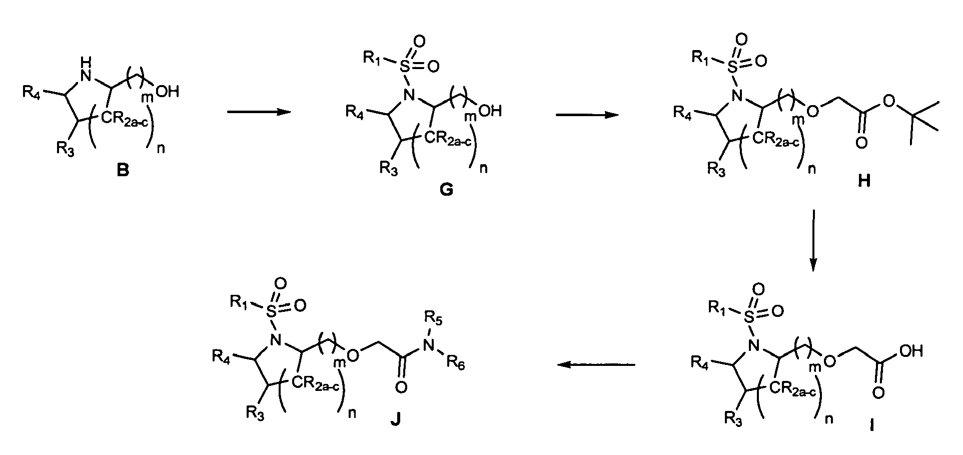

Kommerziell erhältliche Aminoalkohole werden als Bausteine **B** in das Syntheseverfahren engesetzt und, wie in Methode 1 und 2 beschrieben, zu den Endprodukten mit der allgemeinen Formel **J** umgesetzt.

Verbindungen der allgemeinen Formel I, bei denen m 2 bedeutet, können nach den gleichen Methoden aus Edukten der allgemeinen Formel K 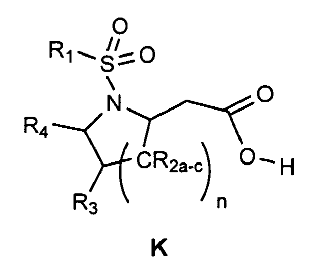 hergestellt werden. Vorzugsweise beginnt die Synthese auf der Stufe der Edukte Gₐ.

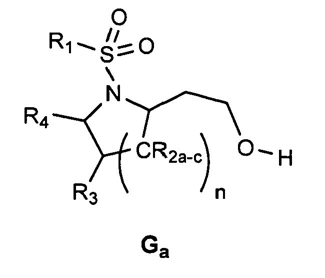

### Verfahren für die Herstellung von Verbindungen der allgemeinen Formel J_{c}

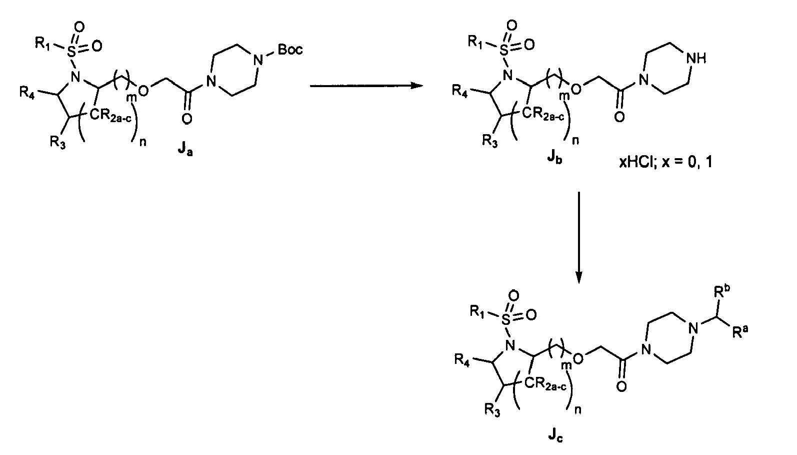

Verbindungen der allgemeinen Formel **Jₐ**, können, wie in Methode 1 und 2 beschrieben, aus kommerziell erhältlichen Aminoalkoholen welche als Bausteine **B** eingesetzt werden und schließlich in der letzten Stufe in einer Amidbildung mit tert-Butyl piperazin-1-carboxylat umgesetzt werden, hergestellt werden und unter sauren Bedingungen, in Gegenwart von beispielsweise Trifluoressigsäure, Chlorwasserstoff oder Chlortrimethylsilan/Wasser in einem organischem Lösungsmittel wie Ethylacetat, Methanol, Diethylether, Methylethylketon, 1,4-Dioxan oder diese Lösungsmittel als Gemische, in einem Temperaturbereich von 0°C - +100°C, bevorzugt bei +25°C - +80°C, zu den Produkten der allgemeinen Struktur **J_{b}** umgesetzt werden. Die Amine oder die entsprechenden Hydrochloride der allgemeinen Formel **J_{b}** können mit Aldehyden oder Ketonen in einer reduktiven Aminierung gegebenenfalls in Gegenwart einer organischen Base wie Triethylamin oder Diisopropylethylamin und in Gegenwart eines geeigneten Reduktionsmittels, beispielsweise Natriumtriacetoxyborhydrid, Natriumcyanoborhydrid oder Natriumdiacetoxyborhydrid oder diese oder ähnliche Reduktionsmittel als polymergebundene Varianten, gegebenenfalls in Gegenwart von Essigsäure, in einem organischen Lösungsmittel beispielsweise Tetrahydrofuran, Dichlormethan, 1,2-Dichlorethan oder diese Lösungsmittel als Gemische, in einem Temperaturbereich von -0 °C - +25°C zu Verbindungen der allgemeinen Formel **J_{c}** umgesetzt werden.

### Verfahren für die Herstellung von Verbindungen der allgemeinen Formel Jₑ

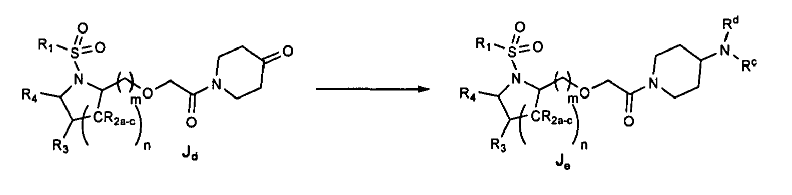

Verbindungen der allgemeinen Formel **J_{d}** können, wie in Methode 1 und 2 beschrieben, aus kommerziell erhältlichen Aminoalkoholen welche als Bausteine **B** eingesetzt werden und schließlich in der letzten Stufe in einer Amidbildung mit Piperidin-4-on umgesetzt werden, hergestellt werden. Die Ketone der allgemeinen Formel **J_{d}** können mit Aminen in einer reduktiven Aminierung gegebenenfalls in Gegenwart einer organischen Base wie Triethylamin oder Diisopropylethylamin und in Gegenwart eines geeigneten Reduktionsmittels, beispielsweise Natriumtriacetoxyborhydrid, Natriumcyanoborhydrid oder Natriumdiacetoxyborhydrid oder diese oder ähnliche Reduktionsmittel als polymergebundene Varianten, gegebenenfalls in Gegenwart von Essigsäure, in einem organischen Lösungsmittel beispielsweise Tetrahydrofuran, Dichlormethan, 1,2-Dichlorethan oder diese Lösungsmittel als Gemische, in einem Temperaturbereich von -0 °C - +25°C zu Verbindungen der allgemeinen Formel **Jₑ** umgesetzt werden.

### Verfahren für die Herstellung von Verbindungen der allgemeinen Formel Jₖ

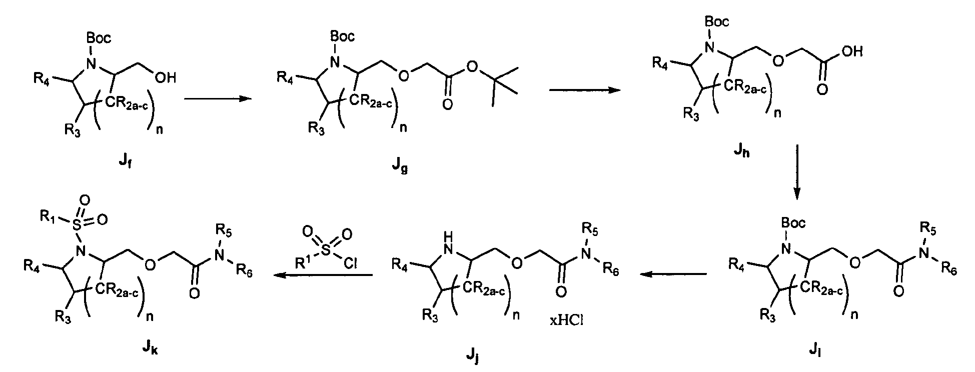

Die kommerziell erhältlichen, geschützten Aminoalkoholen der allgemeinen Formel **J_{f}** können in einer Alkylierungsreaktion mit halogenierten Esterderivaten unter Verwendung von Tetrabutylammoniumchlorid oder -bromid oder Tetrabutylammoniumhydrogensulfat in einer Phasentransferreaktion unter Verwendung eines organischen Lösungsmittels wie Toluol, Benzol oder Xylol und anorganischen Base wie Kaliumhydroxid, Natriumhydroxid, Natriumcarbonat, Natriumhydrogencarbonat, Kaliumcarbonat oder in Gegenwart einer organischen oder anorganischen Base, übliche anorganische Basen sind Metallalkoholate wie Natriummethanolat, Natriumethanolat, Kalium-tert-butylat, Lithium- oder Natriumbasen wie Lithiumdiisopropylamid, Buthyllithium, tert-Butyllithium, Natriummethylat oder Metallhydride wie Kaliumhydrid, Lithiumhydrid, Natriumhydrid; übliche organische Basen sind Diisopropylethylamin, Triethylamin, in einem organischen Lösungsmittel wie Dichlormethan, THF oder Diethylether, in einem Temperaturbereich von -20°C - +25°C, bevorzugt bei 0°C - +25°C, zu den Produkten der allgemeinen Struktur **J_{g}** umgesetzt werden, welche in einer Esterspaltung unter Verwendung von organischen Säuren wie Trifluoressigsäure oder wässrigen anorganischen Säuren wie Salzsäure oder Verwendung von wässrigen anorganischen Basen wie Lithiumhydroxid, Kaliumhydroxid, Natriumhydroxid, Natriumcarbonat, Natriumhydrogencarbonat, Kaliumcarbonat in organischen Lösungsmittel wie Methanol, Dioxan, Dichlormethan, THF, Diethylether oder diese Lösungsmittel als Gemische, in einem Temperaturbereich von 0°C - +110°C, bevorzugt bei +25°C - +90°C, die Säurestufen der allgemeinen Formel **Jₕ** ergeben. Die Carbonsäuren Jₕ können in einer Amidbildung unter Verwendung primärer oder sekundärer Amine, in Gegenwart wasserentziehender Mittel wie Natrium- oder Magnesiumsulfat, Phosphoroxid oder Reagenzien wie beispielsweise CDI, DCC, TBTU, EDCI oder Benzotriazol-1-yloxytris(dimethylamino)phosphonium Hexafluorphosphat (BOP) auch in Gegenwart von HOAt oder HOBt und einer organischen Base beispielsweise DIPEA, Pyridin oder 4-Methylmorpholin in einem organischen Lösungsmittel wie THF, Dichlormethan, Diethylether, Dioxan, DMF oder Acetonitril, 0°C - + 40°C, vorzugsweise bei Raumtemperatur, zu den Produkten der allgemeinen Formel **Jᵢ** umgesetzt werden. Die Verbindungen **Jᵢ** können entschützt werden in Gegenwart einer anorganischen oder organischen Säure wie HCl, Trifluoressigsäure oder Ameisensäure in einem organischen Lösungsmittel wie THF, Dichlormethan, Diethylether, Dioxan, MeOH oder Chloroform, 0°C - + 40°C, vorzugsweise bei +25 - +40°C, zu den Produkten der allgemeinen Formel **Jⱼ** umgesetzt. Die Amine **Jⱼ** werden in einer Sulfonylierung mit Sulfonylchloriden, - bromiden oder -pentafuorphenolaten R₁SO₂X (X = Br, Cl oder OPFP) in Gegenwart einer organischen oder anorganischen Base, beispielsweise Kaliumcarbonat, Natriumhydrogencarbonat, Diisopropylethylamin, Triethylamin, Pyridin, Diethylamin, 1,8-Diazabicyclo[5.4.0]-undec-7-en (DBU) und/oder Dimethylaminopyridin, oder in Gegenwart von Tetra-n-butylammoniumchlorid und in einem organischen Lösungsmittel beispielsweise Acetonitril, Dichlormethan, THF oder N,N-Dimethylformamid in einem Temperaturbereich von 0°C - 120 °C, vorzugsweise bei +25°C - +70°C, zu den Sulfonamiden **Jₖ** umgesetzt.

Die Trennung von Diastereomeren und/oder Enantiomeren erfolgt nach dem Fachmann bekannten Methoden, beispielsweise durch Umkristallisation, Chromatographie oder insbesondere HPLC-Chromatographie bzw. Kristallisation mit einer gegebenenfalls chiralen Säure oder Base und Trennung der Salze oder chirale HPLC-Chromatographie (Fogassy et al., Optical resolution methods, Org. Biomol. Chem 2006, 4, 3011-3030).

### Beispiele

Die Ausbeuten der hergestellten Verbindungen sind nicht optimiert.

Alle Temperaturen sind unkorrigiert.

Die Angabe "Ether" bedeutet Diethylether, "EE" Ethylacetat, "DCM" Dichlormethan, "DMF" Dimethylformamid, "DME" Dimethoxyethan "DMSO" Dimethylsulfoxid und "THF" Tetrahydrofuran. Die Angabe "Äquivatente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Die eingesetzten Chemikalien und Lösungsmittel wurden kommerziell bei den herkömmlichen Anbietern (ABCR, Acros, Acocado, Aldrich, ApolloScientific, Bachem, Bionet, Chempur, Fluka, Lancaster, Maybridge, Merck, Sigma, TCi TygerScientific etc.) erworben.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die Analytik erfolgte über HPLC-MS, Vorstufen wurden über NMR bestätigt.

### Abkürzungen

TBTU = O-(Benzotriazol-1-yl)-N,N,N',N'-tetramethyluronium tetrafluoroborat
CDI = 1,1'-Carbonyldiimidazol
DCC = Dicyclohexylcarbodiimid
EDCI = 1-ethyl-3-(3-dimethylaminopropyl)carbodiimide
HOAt = 1-Hydroxy-7-azabenzotriazole
DIPEA = N,N-Diisopropylamin
HOBt = 1-Hydroxybenzotriazol
PFP - Pentafluorphenyl

### Herstellung der Säurebausteine:

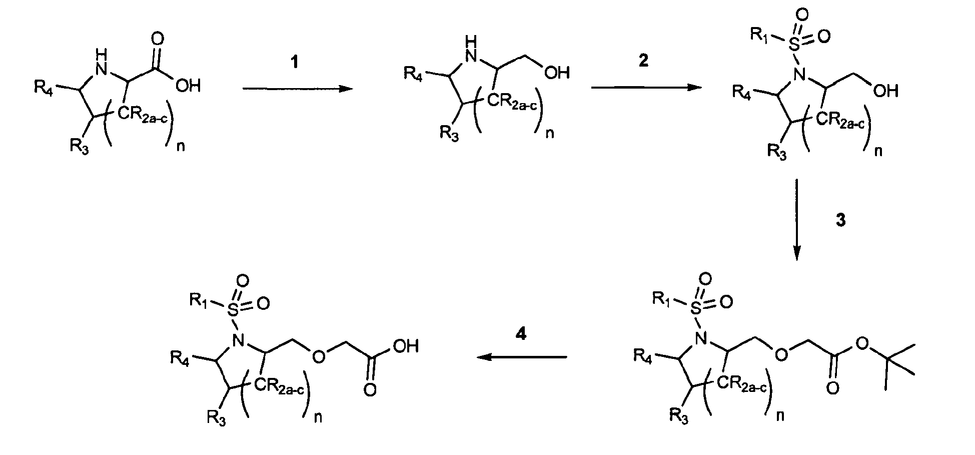

### Methode 1

1. Zu einer Suspension der cyclischen Aminosäure (100 mmol) in THF (150 ml) gab man unter Rühren sukzessive und bei einer Temperatur zwischen -10°C und RT LiAlH₄ (100ml, 1.0 M in Diethylether) unter Inertgasatmosphäre hinzu. Die Reaktionsmischung wurde für 16 h gerührt und erwärmte dabei bis auf RT. Anschließend kühlte man wieder auf 0°C ab und gab unter Rühren Ethylacetat (20 ml), Wasser (8 ml), 15%-ige wässrige NaOH (8 ml) und Wasser (20 ml) hinzu. Nach Filtration wurde der Rückstand mit Diethylether gewaschen. Das Lösungsmittel der vereinigten organischen Phasen wurde unter Vakuum entfernt und das Produkt ohne weitere Aufreinigung in die nächste Stufe eingesetzt.
2. Zu einer Lösung des Aminoalkohols (100 mmol) in CH₂Cl₂ (200 ml) wurde Et₃N (125 mmol) zugegeben und unter Verwendung eines Eisbads auf 0°C abgekühlt. Anschließend gab man das jeweilige Sulfonylchlorid (50 mmol) unverdünnt oder als Lösung in CH₂Cl₂ (100 ml) hinzu und rührte für 3 h bei RT. Nach Zugabe von 0.5 M Salzsäure (100 ml) wurde die organische Phase abgetrennt, mit Wasser gewaschen, über Na₂SO₄ getrocknet, filtriert und das Lösungsmittel unter Vakuum entfernt. Das Rohprodukt wurde ohne weitere Reinigung in der nächsten Stufe verwendet oder durch Säulenchromatographie gereinigt.
3. Zu einer Lösung des Produkts aus Stufe 2 (31 mmol) in Toluol (200 ml) wurde n-Bu₄NCl (10 mmol) zugegeben, man kühlte auf 0°C ab, gab zuerst wässrige 35%-ige NaOH (200 ml) und dann Bromessigsäure-tert.-butylester (46 mmol) tropfenweise hinzu. Die Reaktionsmischung wurde für 3 h gerührt, anschließend mit Wasser neutral gewaschen, mit Na₂SO₄ getrocknet und das organische Lösungsmittel unter Vakuum entfernt. Das Rohprodukt wurde ohne weitere Reinigung in der nächsten Stufe verwendet oder durch Säulenchromatographie gereinigt.
4. Das Produkt aus Stufe 3 (30 mmol) wurde in CH₂Cl₂ (200 ml) gelöst, mit TFA (30 ml) versetzt und für 2 h bei RT gerührt. Das Lösungsmittel wurde weitgehend unter Vakuum entfernt und das Rohprodukt durch Umkristallisation oder Chromatographie aufgereinigt.

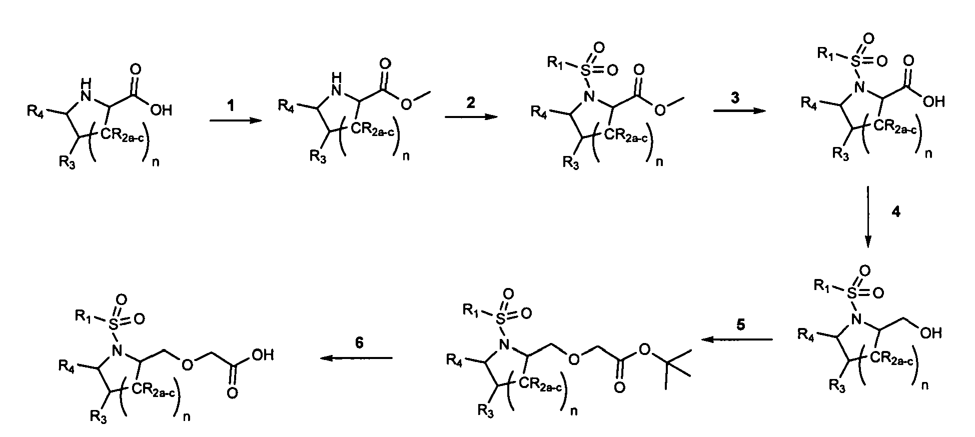

### Methode 2

1. Eine Lösung der Aminosäure (153 mmol) in Methanol (500 ml) wurde auf 0°C abgekühlt und tropfenweise mit Thionylchlorid (168 mmol, 12 ml) versetzt. Nach Erwärmung auf RT wurde die Reaktionslösung bei 40°C über Nacht erhitzt. Nach Destillation des Lösungsmittels erhielt man das Rohprodukt, welches ohne weitere Aufarbeitung in die nächste Stufe eingesetzt wurde.
2. Zu einer Lösung des Methylesters aus Stufe 1 (152 mmol) in CH₂Cl₂ (400 ml) gab man Pyridin (459 mmol) und eine Lösung des Sulfonylchlorids (153 mmol) in CH₂Cl₂ (100 ml). Die Reaktionslösung wurde über Nacht bei RT gerührt. Die Lösung wurde mit wenig CH₂Cl₂ verdünnt und nacheinander mit 0.5 M KHSO₄, gesättigter wäßriger NaHCO₃-Lösung und gesättigter wäßriger NaCl-Lösung gewaschen. Die abgetrennte organische Phase wurde über Na₂SO₄ getrocknet, das Lösungsmittel im Vakuum entfernt und das Rohprodukt mittels Säulenchromatographie aufgereinigt.
3. Zu einer Lösung des Produktes aus Stufe 2 (136 mmol) in einem Methanol/Dioxan/4 M NaOH-Gemisch im Verhältnis 15/4/1 (1020 ml, 203 mmol NaOH, 1.5 Äquivalente) gab man unter Rühren 4 M NaOH (153 ml, 610 mmol, 4.5 Äquivalente) und rührte über Nacht bei RT. Das Lösungsmittel wurde im Vakuum entfernt. Der Rückstand wurde mit Ethylacetat gelöst und mit 0.5 M KHSO₄ gewaschen. Die organische Phase wurde mit gesättigter wäßriger NaCl-Lösung gewaschen und die separierte organische Phase nach Filtration mit Na₂SO₄ getrocknet. Nach Entfernung des Lösungsmittels im Vakuum und waschen mit Diethylether erhielt man das aufgereinigte Produkt aus Stufe 3.
4. Zu einer Lösung des Produkts aus Stufe 3 (31 mmol) in THF (250 ml) gab man bei 0 °C unter Rühren BH₃ x DMS (2.0 M in THF, 31,2 ml, 63 mmol) langsam tropfenweise hinzu. Nach Rühren für 30 min bei RT ließ man die Lösung über Nacht langsam bis auf RT erwärmen. Anschließend gab man langsam solange Methanol hinzu bis kein Gas mehr freigesetzt wurde und reduzierte das Lösungsmittel unter Vakuum. Da Rohprodukt wurde über Silica filtriert und mit CH₂Cl₂/Methanol im Verhältnis 9/1 gewaschen.
5. Zu einer Lösung des Produkts aus Stufe 4 (31 mmol) in Toluol (175 ml) wurde n-Bu₄NCl (10 mmol, 2.9g) zugegeben, man kühlte auf 0°C ab, gab zuerst wässrige 35%-ige NaOH (200 ml) hinzu und dann Bromessigsäure-tert.-Butylester (48 mmol, 7 ml) tropfenweise hinzu. Die Reaktionsmischung wurde für 3 h gerührt, anschließend mit Wasser neutral gewaschen, mit Na₂SO₄ getrocknet und das organische Lösungsmittel unter Vakuum entfernt. Das Rohprodukt wurde ohne weitere Reinigung in der nächsten Stufe verwendet.
6. Das Produkt aus Stufe 5 (30 mmol) wurde in einer Mischung aus MeOH/Dioxan/4 M NaOH im Verhältnis 15/4/1 (236 ml, 47 mmol NaOH) gelöst, mit weiterer NaOH (4 M, 35 ml, 141 mmol) versetzt und über Nacht bei RT gerührt. Das Lösungsmittel wurde im Vakuum reduziert, der Rückstand mit Ethylacetat verdünnt und mit 0.5 M KHSO₄ gewaschen. Die organische Phase wurde abgetrennt, mit gesättigter wäßriger NaCl-Lösung gewaschen und mit Na₂SO₄ getrocknet. Nach Filtration wurde das Lösungsmittel im Vakuum entfernt. Das Rohprodukt wurde durch Coevaporation mit Diethylether und CH₂Cl₂ gereinigt.

### Methode 3

1. Zu einer Lösung oder Suspension des Aminoalkohols (74 mmol) in Aceton (350 ml) gab man bei RT K₂CO₃ (148 mmol) und das Sulfonylchlorid (82 mmol) und rührte bei 40 - 50°C über Nacht. Die Reaktionsmischung wurde auf RT abgekühlt und filtriert. Das Lösungsmittel des Filtrats wurde anschließend unter Vakuum entfernt. Das Rohprodukt wurde ohne weitere Aufarbeitung in die nächste Stufe eingesetzt.
2. Zu einer Lösung des Produkts aus Stufe 1 (31 mmol) in Toluol (200 ml) wurde n-Bu₄NCl (10 mmol) zugegeben, man kühlte auf 0°C ab, gab zuerst wässrige 35%ige NaOH (200 ml) und dann Bromessigsäure-tert.-butylester (46 mmol) tropfenweise hinzu. Die Reaktionsmischung wurde für 3 h gerührt, anschließend mit Wasser neutral gewaschen, mit Na₂SO₄ getrocknet und das organische Lösungsmittel unter Vakuum entfernt. Das Rohprodukt wurde ohne weitere Reinigung in der nächsten Stufe verwendet oder durch Säulenchromatographie gereinigt.

### Methoden zur Esterspaltung

a. Zu einer Lösung des *tert*-Butylesters (63 mmol) in THF (200 ml) und Methanol (200 ml) gab man 6 M NaOH (200 ml, 1200 mmol). Die Reaktionsmischung wurde bei RT gerührt. Nach 15 min - 1 h wurde das organische Lösungsmittel entfernt, der Rückstand auf 0°C abgekühlt und mit 6 M HCl (210 ml) versetzt. Die wässrige Phase wurde mit CH₂Cl₂ (200 ml) und Ethylacetat (200 ml) extrahiert. Die vereinigten organischen Phasen wurden über Na₂SO₄ getrocknet. Nach Filtration wurde das Lösungsmittel unter Vakuum entfernt und der Rückstand mit i-Propylether zweimal co-evaporiert.
b. Zu einer Suspension des tert-Butylesters (38 mmol) in 6 M NaOH (64 ml, 384 mmol) und Methanol (64 ml) gab man Dioxan (30 ml) bis eine Lösung erhalten wurde. Die Reaktionslösung wurde bei RT gerührt. Nach 15 min - 3 h wurde das organische Lösungsmittel entfernt, der Rückstand auf 0°C abgekühlt und mit 6 M HCl (200 ml) versetzt. Die wässrige Phase wurde mit CH₂Cl₂ (200 ml) extrahiert. Die vereinigten organischen Phasen wurden über Na₂SO₄ getrocknet. Nach Filtration wurde das Lösungsmittel unter Vakuum entfernt und der Rückstand mit i-Propoylether zweimal co-evaporiert.
c. Der tert-Butylester (7 mmol) wurde über Nacht bei RT in 4 M HCl in Dioxan (7 ml, 27 mmol) gerührt. Nach Entfernung des Lösungsmittels wurde der Rückstand zweimal mit i-Propylether co-evaporiert.

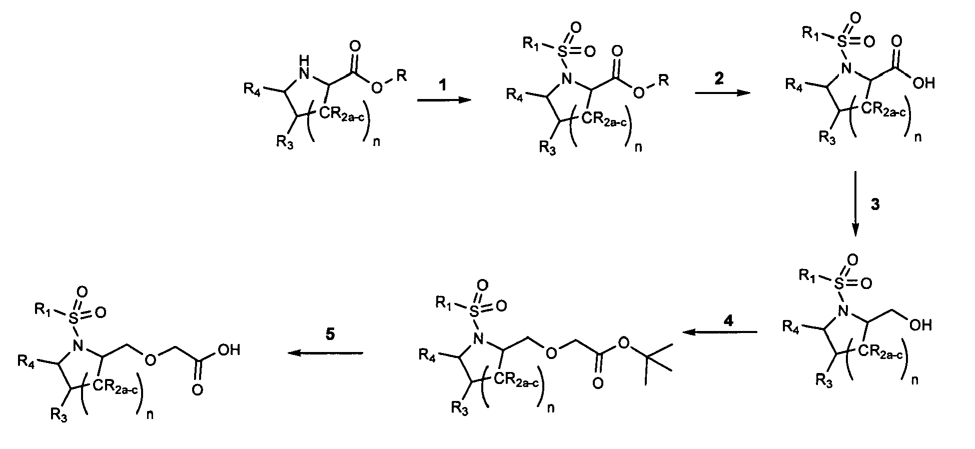

### Herstellung von 2-((1-(2,6-Dichlor-4-(trifluormethyl)phenylsulfonyl)piperidin-2-yl)methoxy)essigsäure S24

1. Zu einer Suspension von DL-Piperidin-2-carbonsäureethylester (38 mmol) in CH₂Cl₂ (150 ml) wurde Et₃N (95 mmol) hinzugegeben. Die Lösung wurde auf 0°C abgekühlt und das Sulfonylchlorid (42 mmol) in einer Lösung von CH₂Cl₂ (30 ml) langsam tropfenweise hinzugegeben und für 2 h bei RT gerührt. Die organische Phase wurde mit 1 M HCl (250 ml) und H₂O (250 ml) extrahiert. Die abgetrennte organische Phase wurde über Na₂SO₄ getrocknet. Das Lösungsmittel wurde unter Vakuum entfernt. Der Rückstand wurde mit i-Propylether co-evaporiert und das Produkt ohne weitere Aufarbeitung in die nächste Stufe eingesetzt.
2. Zu einer Lösung des Esters (38 mmol) in einem Lösungsmittelgemisch aus Methanol/Dioxan/4 M NaOH (15/4/1) (57 mmol NaOH) gab man unter Rühren bei RT 4 M NaOH (113 mmol) und rührte für 2 h. Das organische Lösungsmittel wurde unter Vakuum entfernt, der Rückstand mit Ethylacetat (300 ml) verdünnt und mit 1 M KHSO₄ (300 ml) extrahiert. Die organische Phase wurde mit gesättigter NaCl-Lösung (200 ml) gewaschen. Die abgetrennt organische Phase trocknete man über Na₂SO₄, filtrierte und entfernte das Lösungsmittel unter Vakuum. Das Produkt wurde ohne weitere Aufreinigung in die nächste Stufe eingesetzt.
3. Zur Lösung der Carbonsäure (27 mmol) in THF (135 ml) gab man unter Rühren langsam bei 0°C 2 M BH₃ x DMS in THF (82 mmol). Nach weiterer Kühlung für 30 min rührte man bei RT über Nacht. Nach Entfernung des Lösungsmittels erhielt man das Rohprodukt, welches ohne weitere Aufreinigung in die nächste Stufe eingesetzt wurde.
4. Zu einer Lösung von Bromessigsäure-tert.-butylester (40 mmol) in Toluol (100 ml) gab man n-Bu₄NCl (8.8 mmol). Die Reaktionsmischung wurde auf 0°C abgekühlt und 35%ige NaOH (150 ml) und dann tropfenweise der Alkohol (27 mmol) gelöst in Toluol (50 ml) zugegeben. Nach Rühren für 1.5 h bei RT wurde die organische Phase separiert mit Wasser (4 x 150 ml) und mit gesättigter NaCl-Lösung (150 ml) extrahiert. Die abgetrennte organische Phase trocknete man über Na₂SO₄, filtrierte und entfernte dann das Lösungsmittel unter Vakuum. Das Rohprodukt wurde über Säulenchromatographie aufgereinigt.
5. Der tert-Butylester (16 mmol) wurde über Nacht bei RT in 4 M HCl in Dioxan (70 ml, 27 mmol) bei RT gerührt. Nach Entfernung des Lösungsmittels wurde der das Rohprodukt über Säulenchromatographie aufgereinigt.

### Herstellung von 2-((1-(2,6-Dichlor-4-(trifluormethyl)phenylsulfonyl)pyrrolidin-2-yl)methoxy)essigsäure S23

1. Zu einer Suspension von DL-Pyrrolidin-2-carbonsäuremethylester Hydrochlorid (36 mmol) in CH₂Cl₂ (180 ml) wurde Et₃N (181 mmol) hinzugegeben. Die Lösung wurde auf 0°C abgekühlt und das Sulfonylchlorid (40 mmol) in einer Lösung von CH₂Cl₂ (30 ml) langsam tropfenweise hinzugegeben und für 2 h bei RT gerührt. Die organische Phase wurde mit 1 M HCl (250 ml), H₂O (250 ml) extrahiert. Die abgetrennte organische Phase wurde über Na₂SO₄ getrocknet. Das Lösungsmittel wurde unter Vakuum entfernt. Der Rückstand wurde mit i-Propylether co-evaporiert und das Produkt ohne weitere Aufarbeitung in die nächste Stufe eingesetzt.
2. Zu einer Lösung des Esters (36 mmol) in einem Lösungsmittelgemisch aus Methanol/Dioxan/4 M NaOH (15/4/1) (54 mmol NaOH) gab man unter Rühren bei RT 4 M NaOH (108 mmol) und rührte für 2 h. Das organische Lösungsmittel wurde unter Vakuum entfernt, der Rückstand mit Ethylacetat (300 ml) verdünnt und mit 1 M KHSO₄ (300 ml) extrahiert. Die organische Phase wurde mit gesättigter NaCl-Lösung (200 ml) gewaschen. Die abgetrennt organische Phase trocknete man über Na₂SO₄, filtrierte und entfernte das Lösungsmittel unter Vakuum. Das Produkt wurde ohne weitere Aufreinigung in die nächste Stufe eingesetzt.
3. Zur Lösung der Carbonsäure (28 mmol) in THF (140 ml) gab man unter Rühren langsam bei 0°C 2 M BH₃ x DMS in THF (86 mmol). Nach weiterer Kühlung für 30 min rührte man bei RT über Nacht. Nach Entfernung des Lösungsmittels erhielt man das Rohprodukt, welches ohne weitere Aufreinigung in die nächste Stufe eingesetzt wurde.
4. Zu einer Lösung von Bromessigsäure-*tert*.-butylester (42 mmol) in Toluol (100 ml) gab man *n*-Bu₄NCl (9 mmol). Die Reaktionsmischung wurde auf 0°C abgekühlt und 35%ige NaOH (150 ml) und dann tropfenweise der Alkohol (28 mmol) gelöst in Toluol (50 ml) zugegeben. Nach Rühren für 1.5 h bei RT wurde die organische Phase separiert, mit Wasser (4 x 150 ml) und mit gesättigter NaCl-Lösung (150 ml) extrahiert. Die abgetrennte organische Phase trocknete man über Na₂SO₄, filtrierte und entfernte dann das Lösungsmittel unter Vakuum. Das Rohprodukt wurde über Säulenchromatographie aufgereinigt.
5. Der *tert*-Butylester (16 mmol) wurde über Nacht bei RT in 4 M HCl in Dioxan (70 ml, 27 mmol) bei RT gerührt. Nach Entfernung des Lösungsmittels wurde der das Rohprodukt über Säulenchromatographie aufgereinigt.

### Herstellung von 2-((1-(3,4-Dichlorophenylsulfonyl)-1,2,3,4-tetrahydroquinolin-2-yl)methoxy)essigsäure S35

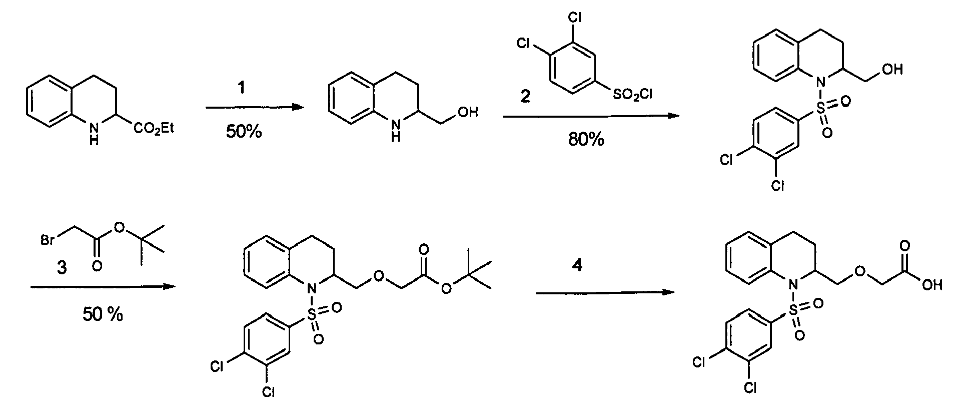

1. 1,2,3,4-Tetrahydroquinolin-2-carbonsäureethylester (25 mmol) in THF (5 ml/mol) wurde bei 0°C tropfenweise zu einer Suspension von LAH (2 äq.) in THF (50 ml) gegeben. Die Reaktionsmischung wurde 1 h bei RT gerührt und anschließend für 4 h unter Rückfluß erhitzt. Nach Zugabe von wässriger gesättigter Na₂SO₄-Lösung wurde filtriert und das organische Lösungsmittel unter Vakuum entfernt. Das Produkt wurde über Säulenchromatographie aufgereinigt (3:7 Ethylacetat/Hexan). Ausbeute: 50%
2. Zu einer auf 0°C gekühlten Suspension des Alkohols (16 mmol) in CH₂Cl₂ (5 ml/mmol) wurde Pyridin (5 äq.), DMAP (0.5 äq) and 3,4-Dichlorbenzolsulfonylchlorid (1.2 äq.) gelöst in CH₂Cl₂ (50 ml) hinzugegeben. Nach Rühren bei 0°C für 5 h wurde CH₂Cl₂ zugegeben und mit wässriger Kupfersulfatlösung, Wasser und gesättigter NaCl-Lösung gewaschen. Nach Trocknung über Natriumsulfat und Filtration wurde das Lösungsmittel unter Vakuum entfernt. Das Produkt wurde über Säulenchromatographie aufgereinigt (5:95 Ethylacetat/CH₂Cl₂). Ausbeute: 80 %
3. Zu einer auf 0°C gekühlten Suspension von NaH (2 äq.) in THF (300 ml) gab man unter Rühren tropfenweise eine Lösung des Sulfonamids (16 mmol) gelöst in THF (100 ml). Nach Rühren für 45 min bei dieser Temperatur wurde eine Lösung von Bromessigsäure-*tert*.-butylester (1.5 äq) in THF (50 ml) zugegeben. Die Reaktionsmischung wurde für 20 h auf 50°C erhitzt. Anschließend kühlte man auf 0°C ab, gab Eis hinzu und extrahierte mit Ethylacetat. Die organische Phase wurde mit wässriger gesättigter NaCl-Lösung gewaschen und über Na₂SO₄ getrocknet. Nach Filtration wurde das Lösungsmittel unter Vakuum entfernt. Das Produkt wurde über Säulenchromatographie aufgereinigt (1:9 Ethylacetat/Hexan). Ausbeute: 50 %
4. Zu einer Lösung des tert-Butylesters (1 äq.) in CH₂Cl₂ (10 ml/mmol) gab man unter Rühren TFA (13 äq.) bei einer Temperatur von 0°C hinzu. Nach Rühren für 3 h bei 0°C wurde das Lösungsmittel unter Vakuum entfernt. Das Rohprodukt wurde ohne weitere Aufarbeitung verwendet.

### Herstellung von 4-Methoxy-2,6-dimethylbenzol-1-sulfonylchlorid

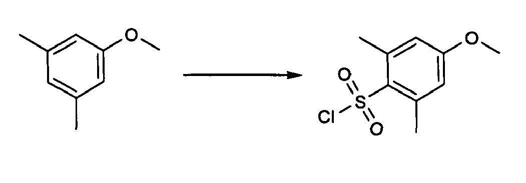

Zu einer Lösung von 3,5-Dimethylanisol (102.5 g, 753 mmol) in CH₂Cl₂ (1l) auf 0°C abgekühlt gab man Chlorsulfonsäure (251 ml, 3763 mmol) in CH₂Cl₂ (250 ml) tropfenweise hinzu. Nach 10 min wurde die Reaktionsmischung auf Eis gegeben (1l) und extrahierte mit CH₂Cl₂ (1 x 250 ml). Die organische Phase wurde mit Wasser (1 l) und mit wässriger gesättigter NaCl-Lösung (1l) gewaschen. Nach Trocknung über Na₂SO₄ und Filtration wurde das Lösungsmittel unter Vakuum entfernt. Das Produkt wurde über Säulenchromatographie aufgereinigt (Silica, Heptan/ CH₂Cl₂, 5:1). Ausbeute: 63.5g, 36%

Nach diesen Methoden wurden die folgenden Bausteine hergestellt:

| **Baustein** | **Struktur** | **Synthesemethode** |
|---|---|---|
| S1 | 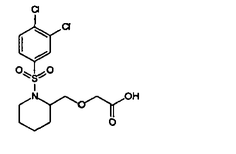 | 1 |
| S2 | 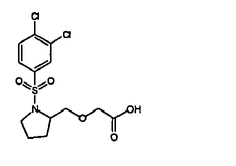 | 1 |
| S3 | 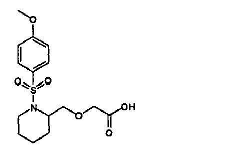 | 1 |
| S4 | 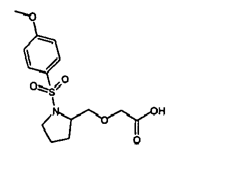 | 1 |
| S5 | 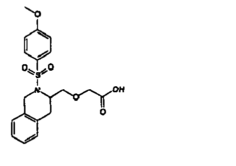 | 1 |
| S6 | 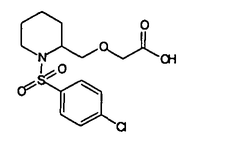 | 1 |
| S7 | 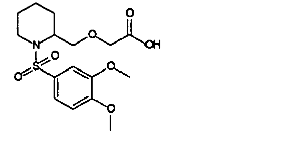 | 1 |
| S8 | 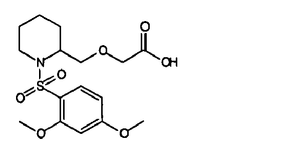 | 1 |
| S9 | 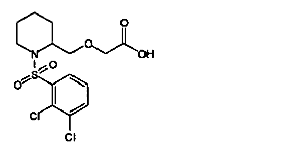 | 1 |
| S10 | 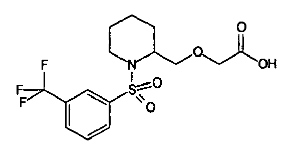 | 1 |
| S11 | 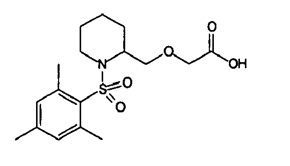 | 1 |
| S12 | 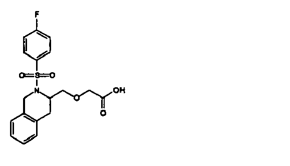 | 1 |
| S13 | 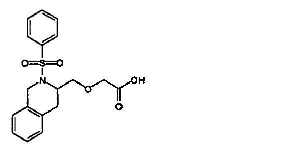 | 1 |
| S14 | 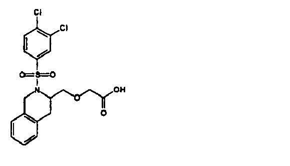 | 1 |
| S15 | 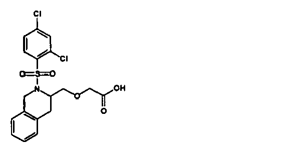 | 1 |
| S16 | 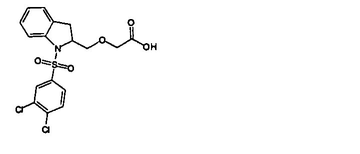 | 2 |
| S17 | 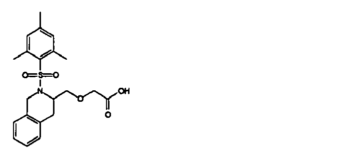 | 1 |
| S18 | 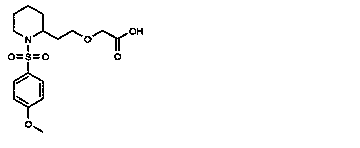 | 1 |
| S19 | 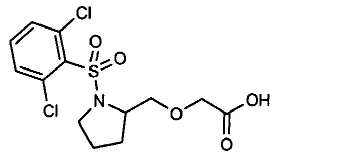 | 1b |
| S20 | 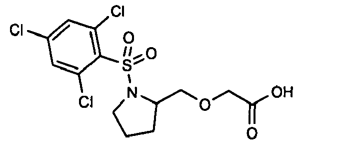 | 1c |
| S21 | 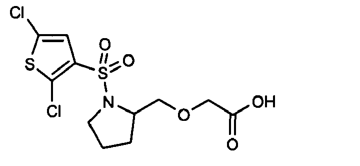 | 1c |
| S22 | 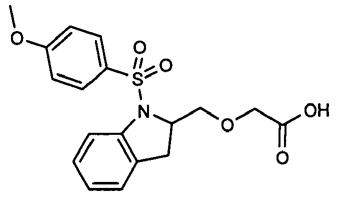 | 2 |
| S23 | 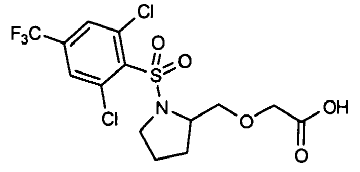 | - |
| S24 | 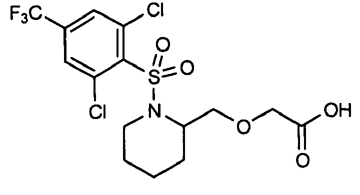 | - |
| S25 | 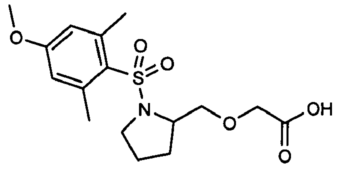 | 1b |
| S26 | 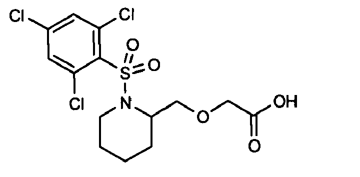 | 1c |
| S27 | 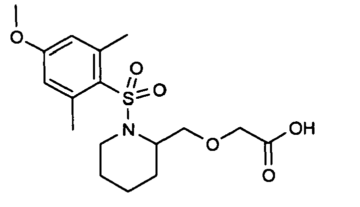 | 3a |
| S28 | 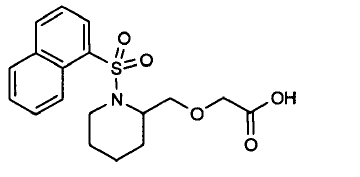 | 1 |
| S29 | 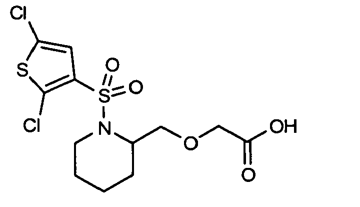 | 3a |
| S30 | 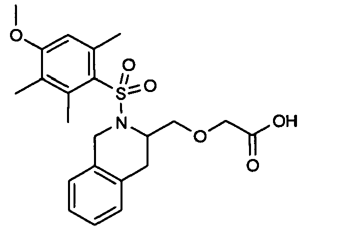 | 1 |
| S31 | 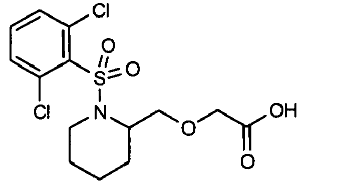 | 3b |
| S32 | 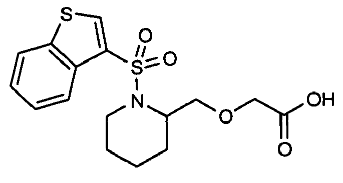 | 3a |
| S33 | 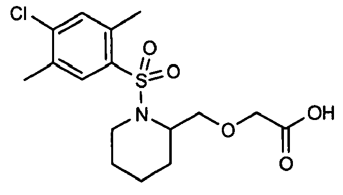 | 3a |
| S34 | 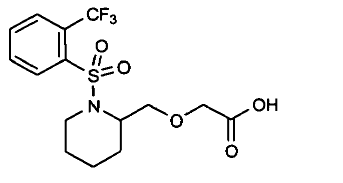 | 3a |
| S35 | 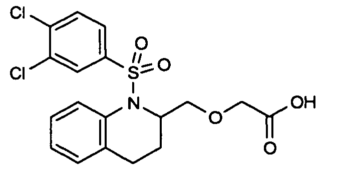 | - |

S18 wurde aus Baustein 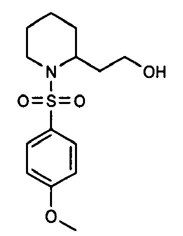 nach Methode 1 hergestellt.

Die eingesetzten Amine waren kommerziell erhältlich oder wurden nach dem Fachmann bekannten Methoden bzw. wie nachfolgend erläutert hergestellt. Die folgenden Aminbausteine wurden für die Synthesen eingesetzt:

### Herstellung der Aminbausteine A1 - A4

### Methode 1

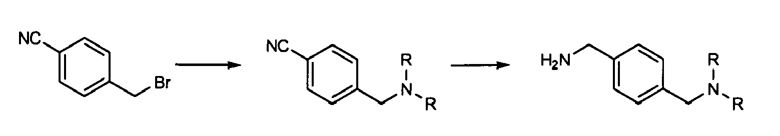

1. 4-Brommethylbenzonitril (500 mg, 2.55 mmol), K₂CO₃ (388 mg, 2.8 mmol), Amin (2.8 mmol) und DMF (6 ml) wurden bei RT für 0.5 - 3 h gerührt und anschließend für 2 - 6 h auf 80 -90°C erwärmt. Die Reaktionsmischung wurde auf RT abgekühlt, Wasser (18 ml) zugegeben und für 0.5 h bei 0 - 5°C gerührt. Die Niederschläge wurden abfiltriert und mit kaltem Wasser (2 x 10ml) gewaschen und unter Vakuum getrocknet. Die Filtrate wurden mit Ethylacetat (3 x 15 ml) extrahiert und über Na₂SO₄ getrocknet. Das organische Lösungsmittel wurde entfernt.
2. Zu einer gerührten Suspension von LAH (4 mmol) in THF (5 ml) gab man tropfenweise eine Lösung der Stufe 1 (1 mmol) gelöst in THF (5 ml) unter Stickstoffatmosphäre. Die Reaktionsmischung wurde bei 25°C gerührt, nach 16-20 h unter Eiskühlung abgekühlt und mit wässriger gesättigter Na₂SO₄-Lösung tropfenweise versetzt. Nach Filtration und waschen des Rückstandes mit Ethylacetat (3x10 ml) wurde das Lösungsmittel weitgehend entfernt und bei 0 - 5°C HCl-Gas eingeleitet. Der Niederschlag wurde abfiltriert und mit Ether gewaschen. Man erhielt die Amin Hydrochloride als Produkte welche ohne weitere Aufarbeitung verwendet wurden.

### Herstellung der Aminbausteine A6, A8

### Methode 2

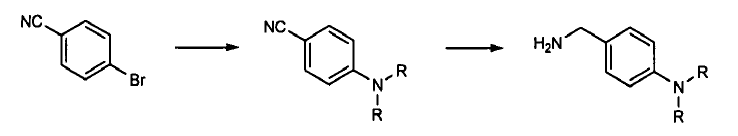

1. Eine Reaktionsmischung von 4-Benzonitril (2 mmol), Amin (3 mmol), K₂CO₃ (4 mmol), Cul (0.2 mmol) und L-Prolin (0.4 mmol) in DMSO (4 ml), wurde bei 80 - 90°C für 40 h unter Rühren erhitzt. Nach Zugabe von Wasser wurde mit Ethylacetat extrahiert. Die organische Phase wurde mit wässriger gesättigter NaCl-Lösung gewaschen und über Na₂SO₄ getrocknet. Nach Filtration und Entfernung des Lösungsmittels wurde der Rückstand über Säulenchromatographie (30% Ethylacetat/CH₂Cl₂) aufgereinigt.
2. Zu einer gerührten Suspension von LAH (4 mmol) in THF (5 ml) gab man tropfenweise eine Lösung der Stufe 1 (1 mmol) gelöst in THF (5 ml) unter Stickstoffatmosphäre. Die Reaktionsmischung wurde unter Rückfluß für 6 h erhitzt, unter Eiskühlung abgekühlt und mit wässriger gesättigter Na₂SO₄-Lösung tropfenweise versetzt. Nach Filtration wurde der Rückstand mit einem Lösungsmittelgemisch (Ethylacetat und 10% Methanol, 3 x 10 ml) gewaschen. Nach Entfernung des Lösungsmittels erhielt man die Amine, welche ohne weitere Aufarbeitung verwendet wurden.

### Herstellung von 4-(Aminomethyl)-N,N-dimethylanilin A5

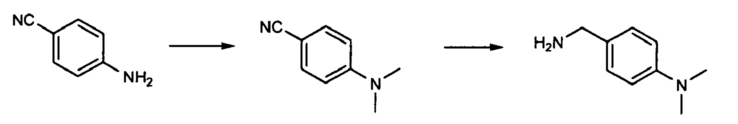

1. Zu einer gerührten Suspension von 4-Aminobenzonitril (0.5 g, 4.23 mmol) in 10%iger wässriger Na₂CO₃-Lösung (18 ml) gab man tropfenweise Me₂SO₄ (1.01 ml, 10.57 mmol) bei 0°C hinzu. Die Reaktionsmischung wurde für 1 h bei 25°C gerührt. Anschließend wurde erneut Me₂SO₄ (1.01 ml, 10.57 mmol) und 10%ige wässrige Na₂CO₃-Lösung (18 ml) hinzugegeben. Nach Zugabe von Wasser wurde mit Ethylacetat extrahiert. Die abgetrennte organische Phase wurde mit wässriger gesättigter NaCl-Lösung gewaschen und über Na₂SO₄ getrocknet. Nach Filtration und Entfernung des Lösungsmittels wurde der Rückstand über Säulenchromatographie (5 % Ethylacetat/Hexan) aufgereinigt.
2. Zu einer gerührten Suspension von LAH (4 mmol) in THF (5 ml) gab man tropfenweise eine Lösung der Stufe 1 (1 mmol) gelöst in THF (5 ml) unter Stickstoffatmosphäre. Die Reaktionsmischung wurde unter Rückfluß für 6 h erhitzt, unter Eiskühlung abgekühlt und mit wässriger gesättigter Na₂SO₄-Lösung tropfenweise versetzt. Nach Filtration wurde der Rückstand mit einem Lösungsmittelgemisch (Ethylacetat und 10% Methanol, 3 x 10 ml) gewaschen. Das Lösungsmittel wurde weitgehend unter Vakuum entfernt und das Amin durch Einleiten von HCl-Gas als Hydrochlorid erhalten.

### Herstellung von (4-Morpholinophenyl)methanamin A7

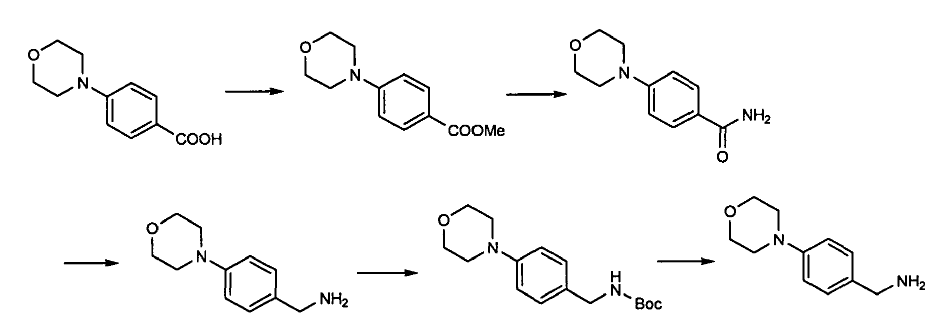

1. Eine Reaktionsmischung von 4-Morpholinbenzoesäure (0.5 g) und methanolischer HCl-Lösung (6 ml, 4%) wurde unter Rühren für 6 h unter Rückfluß erhitzt. Nach Entfernung des Lösungsmittels unter Vakuum wurde der Rückstand mit Wasser (10 ml) versetzt und mit wässriger gesättigter NaHCO₃-Lösung neutralisiert. Die Lösung wurde mit Ethylacetat extrahiert (3 x 20 ml). Die separierte organische Phase wurde über Na₂SO₄ getrocknet. Nach Filtration und Entfernung des Lösungsmittels erhielt man das Produkt, welches ohne weitere Aufarbeitung in die nächste Stufe eingesetzt wurde.
2. Eine Reaktionsmischung von Produkt Stufe 1 (4 g), NH₃ (25 ml) und Methanol (20 ml) wurde im Autoklaven (Druck 50 kg/cm²) für 4 d auf 120°C erhitzt. Nach Entfernung des Lösungsmittels unter Vakuum wurde das Produkt über Säulenchromatographie (50% Ethylacetat/Hexan) aufgereinigt.
3. Zu einer Lösung des Amid aus Stufe 2 (1 g, 4.84 mmol) in THF (15 ml) gab man unter Rühren BH₃ x DMS (1.86 ml, 19.4 mmol) bei 0°C unter Stickstoffatmosphäre hinzu. Die Reaktionsmischung wurde für 18 h unter Rückfluß erhitzt, abgekühlt und mit Methanol versetzt. Das Lösungsmittel wurde unter Vakuum entfernt, der Rückstand in Ethylacetat gelöst und mit wässriger gesättigter NaCl-Lösung gewaschen. Nach Filtration und Trocknung über Na₂SO₄ wurde das Lösungsmittel unter Vakuum entfernt und das Produkt ohne weitere Aufarbeitung in die nächste Stufe eingesetzt.
4. Zu einer Lösung des Amins aus Stufe 3 (0.67 g) gelöst in THF (12 ml) gab man (Boc)₂O (0.82 ml, 3.83 mmol) und rührte für 18 h. Anschließend wurde das Lösungsmittel unter Vakuum entfernt und das Produkt über Säulenchromatographie (10% Ethylacetat/Hexan) aufgereinigt.
5. Zu dem Boc-geschützten Produkt aus Stufe 4 (280 mg) gab man eine Lösung von HCl-Gas in Ethylacetat (4%) und rührte bei RT für 2 h. Das Lösungsmittel wurde unter Vakuum entfernt und der Rückstand wenig in Ethylacetat aufgenommen. Nach Filtration und Trocknung unter Vakuum erhielt man das Produkt als Amin Hydrochlorid.

### Herstellung von N,N-Dimethyl-4-(2-(methylamino)ethyl)cyclohexanamin A9 und N-methyl-2-(4-(Pyrrolidin-1-yl)cyclohexyl)ethanamin A10

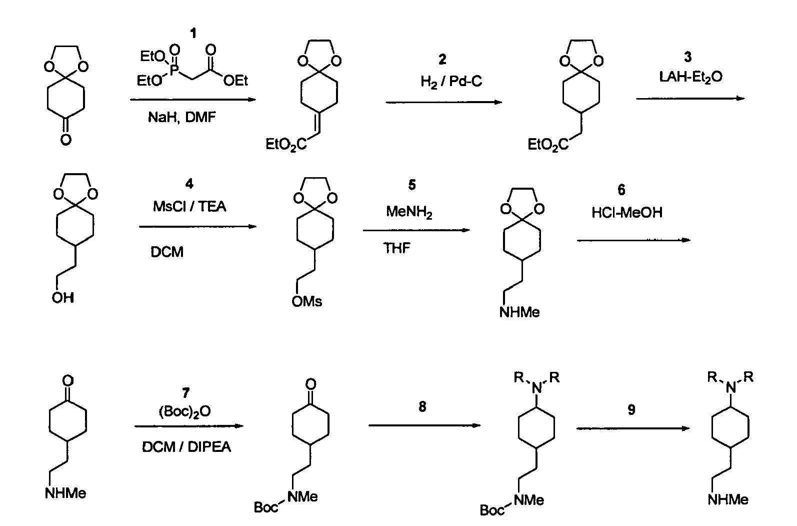

1. Zu einer auf 0°C gekühlten Suspension von NaH (10 mmol) in THF (50 ml) gab man langsam eine Lösung von Triethylphosphonoacetat (11 mmol) in THF (50 ml) und ließ die Reaktionsmischung für 30 min rühren. Bei 0°C wurde anschließend 1,4-Dioxa-spiro[4.5]decan-8-on (10 mmol) in THF (50 ml) tropfenweise zugegeben und für 16 h gerührt. Nach Zugabe von Eis und wässriger gesättigter NaCl-Lösung wurde die wässrige Phase mit Ethylacetat und die organische Phase mit Wasser und wässriger gesättigter NaCl-Lösung gewaschen. Die vereinigten organischen Phasen wurden über Na₂SO₄ getrocknet und das Lösungsmittel nach Filtration unter Vakuum entfernt. Das Produkt wurde über Säulenchromatographie aufgereinigt (20% Ethylacetat/Hexan). Ausbeute: 90%
2. Eine Lösung des Esters (10 mmol) in MeOH (30 ml) wurde unter Argon mit 10% Pd/C (50%) mit Wasserstoff für 16 h hydriert. Nach Filtration über Celite wurde der Rückstand mit MeOH gewaschen und das Lösungsmittel der vereinigten organischen Phasen unter Vakuum entfernt. Das Produkt wurde ohne weitere Aufarbeitung in die nächste Stufe eingesetzt.
3. Zu einer auf 0°C gekühlten Suspension von LaH (10 mmol) in THF (30 ml) gab man langsam eine Lösung von (1,4-Dioxa-spiro[4.5]dec-8-yl)essigsäureethylester (10 mmol) in THF (50 ml) über eine Zeit von 30 min hinzu. Nach vollständiger Reaktion des Esters wurde die Lösung auf 0°C abgekühlt, mit wässriger gesättigter Na₂SO₄-Löung versetzt und über Celite abfiltriert. Das Lösungsmittel wurde unter Vakuum entfernt und das Produkt ohne weitere Aufarbeitung in die nächste Stufe eingesetzt.
4. Zu einer Lösung des Alkohols (10 mmol) in CH₂Cl₂ (50 ml) gab man Methansulfonylchlorid (11 mmol) tropfenweise bei 0°C unter Stickstoff hinzu. Die Reaktionsmischung wurde 2 h weitergerührt und anschließend mit CH₂Cl₂ verdünnt. Nach Extraktion mit wässriger gesättigter NaCl-Lösung wurde über Na₂SO₄ getrocknet. Nach Filtration und Entfernung des Lösungsmittels erhielt man das Rohprodukt mit einer Ausbeute von 80%.
5. Zu einer Lösung des Ms-geschützten Alkohols (5 mmol) in THF (5 ml) gab man eine 2 M Lösung von Methylamin in CH₂Cl₂ (10 ml) und erhitzte die Reaktionslösung für 16 h bei 100°C. Nach Entfernung des Lösungsmittels unter Vakuum wurde das Rohprodukt ohne weitere Aufarbeitung in die nächste Stufe eingesetzt. Ausbeute Rohprodukt: 90%
6. Zu [2-(1,4-Dioxa-spiro[4.5]dec-8-yl)-ethyl]-methylamin (10 mmol) gab man bei 0°C 6 N HCl (20 ml) und rührte für 16 h bei 25°C. Die wässrige Lösung wurde mit Ethylacetat extrahiert und anschließend mit 6 N NaOH auf pH 14 eingestellt. Die wässrige Phase wurde mit CH₂Cl extrahiert und die organische Phase mit Wasser und wässriger gesättigter NaCl-Lösung gewaschen. Die organische Phase wurde über Na₂SO₄ getrocknet und das Lösungsmittel nach Filtration entfernt. Das Rohprodukt wurde ohne weitere Aufarbeitung in die nächste Stufe eingesetzt. Ausbeute Rohprodukt: 80%
7. Zu einer Lösung von 4-(2-Methylamino-ethyl)-cyclohexanon (15 mmol) in CH₂Cl₂ (45 ml) gab man Diisopropylamin (37.5 mmol) und Di-tert.-arylbutyldicarbonat (22.5 mmol) bei 0°C. Die Reaktionsmischung wurde bei 25°C für 16 h gerührt. Nach Zugabe von CH₂Cl₂ wurde mit Wasser und wässriger gesättigter NaCl-Lösung extrahiert. Die abgetrennte organische Phase wurde über Na₂SO₄ getrocknet. Nach Filtration wurde das Lösungsmittel unter Vakuum entfernt und das Produkt über Säulenchromatographie aufgereinigt (5% Methanol/ CH₂Cl₂). Ausbeute: 70%
8. Zu einer Lösung des Ketons (1 äq.) in Methanol (10 ml/mmol) gab man das korrespondierende Amin (1.5 äq.), Natriumcyanoborhydrid (2 äq.) und Essigsäure (2 äq.). Die Reaktionsmischung wurde für 16 h bei 25°C gerührt. Anschließend gab man Eis hinzu und entfernte das Lösungsmittel unter Vakuum. Nach Zugabe von Ethylacetat wurde erst mit wässriger gesättigter Na₂CO₃-Lösung und dann mit wässriger gesättigter NaCl-Lösung extrahiert. Die abgetrennte organische Phase wurde über Na₂SO₄ getrocknet, filtriert und das Lösungsmittel unter Vakuum entfernt. Das Produkt wurde über Säulenchromatographie aufgereinigt (3% Methanol/CH₂Cl₂).
9. Die Boc-geschützte Vorstufe wurde bei 0°C mit 20%iger TFA in CH₂Cl₂ (5 ml/mmol) versetzt und die Reaktionsmischung für 3 h gerührt. Das Lösungsmittel wurde unter Vakuum entfernt und das Amin als TFA Salz in der weiteren Synthese verwendet.

### Aminbausteine:

| **Baustein** | **Struktur** | **Synthesemethode** |
|---|---|---|
| A1 | 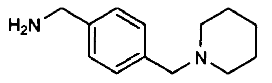 | 1 |
| A2 | 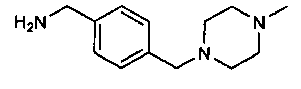 | 1 |
| A3 | 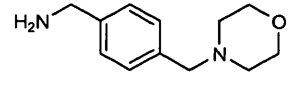 | 1 |
| A4 | 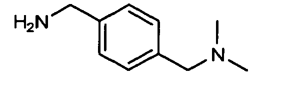 | 1 |
| A5 | 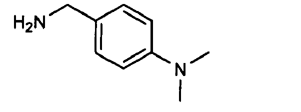 | - |
| A6 | 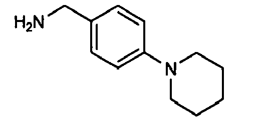 | 2 |
| A7 | 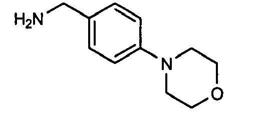 | - |
| A8 | 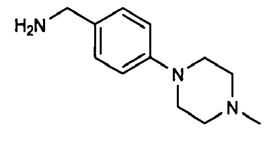 | 2 |
| A9 | 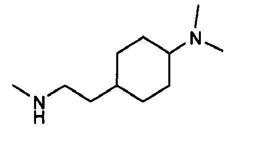 | - |
| A10 | 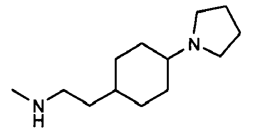 | - |

### Herstellung der Beispielverbindungen durch Amidbildung in der Parallelsynthese:

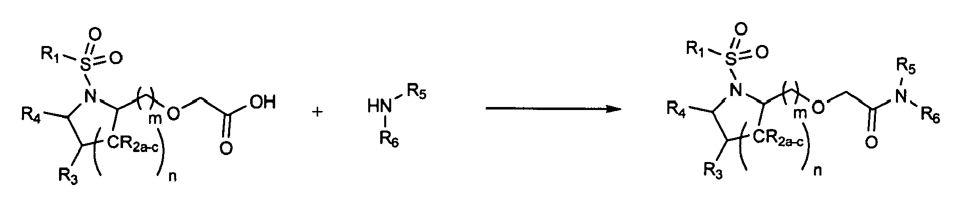

### Parallelsynthese Methode 1

Zu 105 µmol CDI-Lösung (0,105 M in CH₂Cl₂, 1 ml wurde Säure-Lösung (0,05 M in CH₂Cl₂, 2 ml) hinzugegeben und 1 h bei RT geschüttelt. Anschließend wurde bei RT 100 µmol der Amin-Lösung (0,1 M in CH₂Cl₂) zugegeben und weitere 12 h bei RT geschüttelt. Anschließend versetzte man die Reaktionsmischung mit 3 ml Wasser, schüttelte für 15 min und trennte die organische Phase ab. Nach Abdestillation des Lösungsmittels wurden die Rohprodukte mittels LC-MS analysiert und über HPLC aufgereinigt.

### Parallelsynthese Methode 2

Bei RT wurden 100 µmol Säure-Lösung (0,05 M in CH₂Cl₂, 2 ml) vorgelegt und mit 105 µmol CDI-Lösung (0,105 M in CH₂Cl₂, 1 ml) versetzt. Nach 1 h Rührzeit bei RT wurde zu der Reaktionslösung 100 µmol des entsprechenden Amins (0,1 M in CH₂Cl₂) hinzupipettiert. Die Reaktionslösung wurde 16 h bei RT gerührt. Anschließend wurden 3 ml Wasser zugegeben und 30 min. gevortext und durchmischt. Der Rührfisch wurde abfiltriert, und das Gefäß mit 1,5 ml CH₂Cl₂ ausgespült.

Die wässrige Phase wurde abgenommen und verworfen. Die organische Phase wurde mit 3 ml H₂O und 0,5 ml CH₂Cl₂ versetzt, gevortext und 15 min. intensiv durchmischt. Nach dem Zentrifugieren wurde die wässrige Phase abgetrennt und verworfen. Die organische Phase wurde analog ein zweites Mal mit 3 ml gesättigter NaCl-Lösung extrahiert. Anschließend wurde die organische Phase abgenommen, in ein Reagenzglas gegeben und über eine MgSO₄-Kartusche getrocknet. Nach Abdestillation des Lösungsmittels wurden die Rohprodukte mittels LC-MS analysiert und über HPLC aufgereinigt.

### Parallelsynthese Methode 3

Die Säure (50 mg, 1 äq.) wurden mit dem Amin (50-70 mg, 1.2 äq.) in CH₂Cl₂ (3 ml/mmol) unter Verwendung der Kupplungsreagenzien EDCI (1.5 äq.), HOBt (1 äq.) und DIPEA (2 äq.) umgesetzt. Nach Entfernung des Lösungsmittels wurden die Produkte über Säulenchromatographie aufgereinigt.

### Parallelsynthese Methode 4

Die Säuren (50 mg, 1 äq.) in CH₂Cl₂ (3 ml/mmol) wurden mit EDCI (1.5 äq.), HOBt (1 äq.) und DIPEA (1.5 äq.) für 15 min bei 25°C gerührt.

Eine Lösung des Amin-TFA Salzes (1.2 äq.) in CH₂Cl₂ (1 ml/mmol) wurde bei 0°C mit DIPEA (4 äq.) versetzt und zur Lösung der Säure hinzugegeben.

Die Reaktionsmischung wurde für 16 h bei 25°C gerührt und dann mit CH₂Cl₂ verdünnt. Die abgetrennte organische Phase wurde mit wässriger NH₄Cl-Lösung, Na₂CO₃-Lösung, NaCl-Lösung extrahiert und anschließend über Na₂SO₄ getrocknet. Nach Filtration und Entfernung des Lösungsmittels wurden die Produkte über Säulenchromatographie aufgereinigt.

Die folgenden Verbindungen wurden nach einer dieser Methoden für die Parallelsynthese hergestellt.

| **Beispiel** | | **Methode** | **Masse** | **Beispiel** | | **Methode** | **Masse** |
|---|---|---|---|---|---|---|---|
| **1** | | 1 | 543,28 | **353** | | 2 | 529,3 |
| **2** | | 1 | 557,29 | **354** | | 2 | 545,1 |
| **3** | | 1 | 558,29 | **355** | | 2 | 539,1 |
| **4** | | 1 | 601,13 | **356** | | 2 | 579 |
| **5** | | 2 | 568,31 | **357** | | 2 | 593,1 |
| **6** | | 1 | 535,25 | **358** | | 2 | 603 |
| **7** | | 1 | 572,30 | **359** | | 2 | 559,1 |
| **9** | | 2 | 579,17 | **360** | | 2 | 539,1 |
| **10** | | 1 | 587,12 | **361** | | 2 | 531,3 |
| **11** | | 1 | 517,26 | **362** | | 2 | 543,3 |
| **12** | | 1 | 541,30 | **363** | | 2 | 467,3 |
| **13** | | 1 | 602,13 | **364** | | 2 | 543,3 |
| **14** | | 1 | 543,28 | **365** | | 2 | 529,3 |
| **15** | | 1 | 616,14 | **366** | | 2 | 478,2 |
| **16** | | 2 | 601,16 | **367** | | 2 | 545,3 |
| **17** | | 1 | 565,16 | **368** | | 2 | 573,3 |
| **18** | | 1 | 544,27 | **369** | | 2 | 516,2 |
| **19** | | 1 | 491,28 | **370** | | 2 | 497,2 |
| **20** | | 1 | 559,20 | **371** | | 2 | 500,2 |
| **21** | | 1 | 545,26 | **372** | | 2 | 448,2 |
| **22** | | 2 | 546,25 | **373** | | 2 | 543,3 |
| **23** | | 2 | 499,25 | **374** | | 2 | 507,3 |
| **24** | | 1 | 559,27 | **375** | | 2 | 467,3 |
| **25** | | 2 | 556,31 | **376** | | 2 | 618,2 |
| **26** | | 1 | 517,16 | **377** | | 2 | 531,3 |
| **27** | | 2 | 505,30 | **378** | | 2 | 583,2 |
| **28** | | 1 | 546,18 | **379** | | 2 | 455,3 |
| **29** | | 1 | 588,11 | **380** | | 2 | 497,3 |
| **30** | | 1 | 603,11 | **381** | | 2 | 597,2 |
| **31** | | 2 | 527,28 | **382** | | 2 | 607,2 |
| **32** | | 1 | 546,18 | **383** | | 2 | 571,3 |
| **33** | | 2 | 590,29 | **384** | | 2 | 563,2 |
| **34** | | 1 | 549,27 | **385** | | 2 | 543,3 |
| **35** | | 1 | 503,25 | **386** | | 2 | 559,3 |
| **36** | | 1 | 545,19 | **387** | | 2 | 547,3 |
| **37** | | 1 | 561,10 | **388** | | 2 | 529,3 |
| **38** | | 1 | 565,16 | **389** | | 2 | 545,1 |
| **39** | | 1 | 583,13 | **390** | | 2 | 545,1 |
| **40** | | 1 | 558,29 | **391** | | 2 | 533,1 |
| **41** | | 2 | 589,30 | **392** | | 2 | 601,1 |
| **42** | | 1 | 560,20 | **393** | | 2 | 497,1 |
| **43** | | 1 | 626,25 | **394** | | 2 | 505 |
| **44** | | 1 | 569,12 | **395** | | 2 | 532,2 |
| **45** | | 1 | 602,13 | **396** | | 2 | 532,2 |
| **46** | | 1 | 507,14 | **397** | | 2 | 568,3 |
| **47** | | 1 | 565,26 | **398** | | 2 | 543,3 |
| **48** | | 2 | 600,37 | **399** | | 2 | 521,3 |
| **49** | | 1 | 589,10 | **400** | | 2 | 571,3 |
| **50** | | 2 | 577,26 | **401** | | 2 | 572,3 |
| **51** | | 1 | 579,17 | **402** | | 2 | 598,2 |
| **52** | | 1 | 521,29 | **403** | | 2 | 553,2 |
| **53** | | 1 | 555,14 | **404** | | 2 | 593,1 |
| **54** | | 1 | 531,17 | **405** | | 2 | 607,1 |
| **55** | | 1 | 545,26 | **406** | | 2 | 617,1 |
| **56** | | 1 | 525,13 | **407** | | 2 | 573,1 |
| **57** | | 1 | 538,15 | **408** | | 2 | 553,2 |
| **58** | | 2 | 534,25 | **409** | | 2 | 515,3 |
| **59** | | 1 | 543,12 | **410** | | 2 | 492,2 |
| **60** | | 1 | 529,26 | **411** | | 2 | 586,3 |
| **61** | | 1 | 551,25 | **412** | | 2 | 524,3 |
| **62** | | 1 | 532,17 | **413** | | 2 | 495,3 |
| **63** | | 1 | 563,28 | **414** | | 2 | 543,3 |
| **64** | | 1 | 513,27 | **415** | | 2 | 557,3 |
| **65** | | 2 | 581,15 | **416** | | 2 | 478,2 |
| **66** | | 1 | 459,22 | **417** | | 2 | 604,3 |
| **67** | | 2 | 575,28 | **418** | | 2 | 543,3 |
| **68** | | 1 | 545,19 | **419** | | 2 | 543,3 |
| **69** | | 1 | 569,29 | **420** | | 2 | 526,2 |
| **70** | | 1 | 567,17 | **421** | | 2 | 557,3 |
| **71** | | 1 | 573,10 | **422** | | 2 | 563,3 |
| **72** | | 1 | 546,18 | **423** | | 2 | 549,2 |
| **73** | | 1 | 547,09 | **424** | | 2 | 531,3 |
| **74** | | 1 | 587,12 | **425** | | 2 | 476,2 |
| **75** | | 2 | 590,29 | **426** | | 2 | 478,2 |
| **76** | | 1 | 527,28 | **427** | | 2 | 526,2 |
| **77** | | 2 | 529,26 | **428** | | 2 | 543,3 |
| **78** | | 1 | 601,16 | **429** | | 2 | 529,3 |
| **79** | | 1 | 601,16 | **430** | | 2 | 543,3 |
| **80** | | 1 | 542,29 | **431** | | 2 | 544,3 |
| **81** | | 2 | 587,14 | **432** | | 2 | 558,3 |
| **82** | | 1 | 579,17 | **433** | | 2 | 558,3 |
| **83** | | 2 | 575,28 | **434** | | 2 | 558,3 |
| **84** | | 1 | 555,14 | **435** | | 2 | 545,3 |
| **85** | | 1 | 534,18 | **436** | | 2 | 531,2 |
| **86** | | 1 | 543,12 | **437** | | 2 | 545,3 |
| **87** | | 1 | 510,23 | **438** | | 2 | 531,2 |
| **88** | | 1 | 520,17 | **439** | | 2 | 545,3 |
| **89** | | 1 | 511,11 | **440** | | 2 | 591,1 |
| **90** | | 2 | 535,30 | **441** | | 2 | 593,3 |
| **91** | | 2 | 529,26 | **442** | | 2 | 543,1 |
| **194** | | 4 | 563,3 | **443** | | 2 | 517,2 |
| **209** | | 4 | 537,3 | **444** | | 2 | 533,2 |
| **213** | | 4 | 595,2 | **445** | | 2 | 515,3 |
| **216** | | 4 | 621,2 | **446** | | 2 | 572,3 |
| **218** | | 2 | 553,2 | **447** | | 2 | 501,2 |
| **219** | | 1 | 515,3 | **448** | | 2 | 515,3 |
| **220** | | 1 | 598,2 | **449** | | 2 | 515,3 |
| **221** | | 1 | 536,3 | **450** | | 2 | 543,3 |
| **222** | | 2 | 552,3 | **451** | | 2 | 555,1 |
| **223** | | 2 | 589,3 | **452** | | 2 | 506,2 |
| **224** | | 2 | 603,1 | **453** | | 2 | 525,1 |
| **225** | | 2 | 505,3 | **454** | | 2 | 557,1 |
| **226** | | 2 | 499,3 | **455** | | 2 | 539,1 |
| **227** | | 2 | 529,3 | **456** | | 2 | 525,1 |
| **228** | | 2 | 541,3 | **457** | | 2 | 553,2 |
| **229** | | 2 | 527,3 | **458** | | 2 | 539,1 |
| **230** | | 2 | 528,3 | **459** | | 2 | 511,1 |
| **231** | | 2 | 520,3 | **460** | | 2 | 515,3 |
| **232** | | 2 | 534,3 | **461** | | 2 | 501,2 |
| **233** | | 2 | 594,2 | **462** | | 2 | 559,2 |
| **234** | | 2 | 596,3 | **463** | | 2 | 537,2 |
| **235** | | 2 | 513,3 | **464** | | 2 | 537,2 |
| **236** | | 2 | 501,2 | **465** | | 2 | 569,2 |
| **237** | | 2 | 501,2 | **466** | | 2 | 475,2 |
| **238** | | 2 | 501,2 | **467** | | 2 | 453,2 |
| **239** | | 2 | 489,2 | **468** | | 2 | 570,2 |
| **240** | | 2 | 535,3 | **469** | | 2 | 604,2 |
| **241** | | 2 | 557,3 | **470** | | 2 | 589,3 |
| **242** | | 2 | 453,2 | **471** | | 2 | 528,3 |
| **243** | | 2 | 513,2 | **472** | | 2 | 574,2 |
| **244** | | 2 | 511,2 | **473** | | 2 | 543,3 |
| **245** | | 2 | 543,3 | **474** | | 2 | 553,3 |
| **246** | | 2 | 529,3 | **475** | | 2 | 557,3 |
| **247** | | 2 | 501,2 | **476** | | 2 | 453,2 |
| **248** | | 2 | 496,3 | **477** | | 2 | 528,3 |
| **249** | | 2 | 627,3 | **478** | | 2 | 531,1 |
| **250** | | 2 | 483,3 | **479** | | 2 | 577,3 |
| **251** | | 2 | 593,2 | **480** | | 2 | 539,1 |
| **252** | | 2 | 573,3 | **481** | | 2 | 593,1 |
| **253** | | 2 | 619,1 | **482** | | 2 | 499,1 |
| **254** | | 2 | 610,2 | **483** | | 2 | 477,1 |
| **255** | | 2 | 627,2 | **484** | | 2 | 594,1 |
| **256** | | 2 | 604,1 | **485** | | 2 | 528,1 |
| **257** | | 2 | 463,2 | **486** | | 2 | 547,1 |
| **258** | | 2 | 496,2 | **487** | | 2 | 577,1 |
| **259** | | 2 | 440,2 | **488** | | 2 | 552,2 |
| **260** | | 2 | 522,3 | **489** | | 2 | 541,2 |
| **261** | | 2 | 516,3 | **490** | | 2 | 529,3 |
| **262** | | 2 | 542,3 | **491** | | 2 | 531,2 |
| **263** | | 2 | 571,3 | **492** | | 2 | 521,3 |
| **264** | | 2 | 521,2 | **493** | | 2 | 550,2 |
| **265** | | 2 | 589,1 | **494** | | 2 | 485,2 |
| **266** | | 1 | 547,2 | **495** | | 2 | 570,2 |
| **267** | | 2 | 522,3 | **496** | | 2 | 596,2 |
| **268** | | 2 | 535,3 | **497** | | 2 | 511,1 |
| **269** | | 2 | 546,3 | **498** | | 2 | 555,1 |
| **270** | | 2 | 552,3 | **499** | | 2 | 545,2 |
| **271** | | 2 | 487,2 | **500** | | 2 | 544,3 |
| **272** | | 2 | 487,2 | **501** | | 2 | 485,2 |
| **273** | | 2 | 487,2 | **502** | | 2 | 562,2 |
| **274** | | 2 | 502,2 | **503** | | 2 | 561,3 |
| **275** | | 2 | 598,3 | **504** | | 2 | 486,2 |
| **276** | | 2 | 611,3 | **505** | | 2 | 529,3 |
| **277** | | 2 | 605,3 | **506** | | 2 | 502,1 |
| **278** | | 2 | 546,2 | **507** | | 2 | 551,3 |
| **279** | | 2 | 559,2 | **508** | | 2 | 551,3 |
| **280** | | 2 | 570,2 | **509** | | 2 | 583,2 |
| **281** | | 2 | 576,2 | **510** | | 2 | 489,2 |
| **282** | | 2 | 511,1 | **511** | | 2 | 549,2 |
| **283** | | 2 | 526,1 | **512** | | 2 | 543,3 |
| **284** | | 2 | 553,2 | **513** | | 2 | 541,2 |
| **285** | | 2 | 532,2 | **514** | | 2 | 584,2 |
| **286** | | 2 | 545,2 | **515** | | 2 | 567,1 |
| **287** | | 2 | 556,2 | **516** | | 2 | 519,2 |
| **288** | | 2 | 545,3 | **517** | | 2 | 453,2 |
| **289** | | 2 | 533,2 | **518** | | 2 | 493,3 |
| **290** | | 2 | 545,3 | **519** | | 2 | 509,3 |
| **291** | | 2 | 481,3 | **520** | | 2 | 507,3 |
| **292** | | 2 | 568,3 | **521** | | 2 | 572,3 |
| **293** | | 1 | 539,1 | **522** | | 2 | 481,3 |
| **294** | | 2 | 613,3 | **523** | | 2 | 481,3 |
| **295** | | 2 | 497,1 | **524** | | 2 | 536,3 |
| **296** | | 2 | 537,1 | **525** | | 2 | 526,2 |
| **297** | | 2 | 553,1 | **526** | | 2 | 572,2 |
| **298** | | 2 | 551,1 | **527** | | 2 | 536,1 |
| **299** | | 2 | 529,3 | **528** | | 2 | 553,2 |
| **300** | | 2 | 543,3 | **529** | | 2 | 553,2 |
| **301** | | 2 | 530,3 | **530** | | 2 | 555,2 |
| **302** | | 2 | 544,3 | **531** | | 2 | 541,2 |
| **303** | | 2 | 529,3 | **532** | | 2 | 495,3 |
| **304** | | 2 | 559,3 | **533** | | 2 | 549,3 |
| **305** | | 2 | 502,2 | **534** | | 2 | 551,3 |
| **306** | | 2 | 531,2 | **535** | | 2 | 450,2 |
| **307** | | 2 | 519,2 | **536** | | 2 | 512,2 |
| **308** | | 2 | 531,2 | **537** | | 2 | 546,2 |
| **309** | | 2 | 503,2 | **538** | | 2 | 572,2 |
| **310** | | 2 | 529,3 | **539** | | 2 | 518,2 |
| **311** | | 2 | 549,2 | **540** | | 2 | 542,2 |
| **312** | | 2 | 527,2 | **541** | | 2 | 542,2 |
| **313** | | 2 | 553,2 | **542** | | 2 | 530,2 |
| **314** | | 2 | 567,2 | **543** | | 2 | 542,2 |
| **315** | | 2 | 553,2 | **544** | | 2 | 526,2 |
| **316** | | 2 | 526,1 | **545** | | 2 | 512,2 |
| **317** | | 2 | 555,1 | **546** | | 2 | 497,2 |
| **318** | | 2 | 543,1 | **547** | | 2 | 555,3 |
| **319** | | 2 | 555,1 | **548** | | 2 | 566,3 |
| **320** | | 2 | 553,2 | **549** | | 2 | 569,2 |
| **321** | | 2 | 573,1 | **550** | | 2 | 593,1 |
| **322** | | 2 | 539,1 | **551** | | 2 | 594 |
| **323** | | 2 | 553,2 | **552** | | 2 | 493,1 |
| **324** | | 2 | 541,1 | **553** | | 2 | 582,1 |
| **325** | | 2 | 541,1 | **554** | | 2 | 616,1 |
| **326** | | 2 | 539,1 | **555** | | 1 | 530,3 |
| **327** | | 2 | 587,1 | **556** | | 1 | 545,2 |
| **328** | | 2 | 575,1 | **557** | | 1 | 572,3 |
| **329** | | 2 | 573,1 | **558** | | 1 | 531,2 |
| **330** | | 2 | 566,1 | **559** | | 2 | 560,3 |
| **331** | | 2 | 579,2 | **560** | | 2 | 586,3 |
| **332** | | 2 | 590,1 | **561** | | 2 | 540,3 |
| **333** | | 2 | 596,2 | **562** | | 2 | 598,3 |
| **334** | | 2 | 613,1 | **563** | | 2 | 612,3 |
| **335** | | 2 | 546,1 | **564** | | 2 | 534,2 |
| **336** | | 2 | 531,2 | **565** | | 2 | 546,2 |
| **337** | | 2 | 517,2 | **566** | | 2 | 520,2 |
| **338** | | 2 | 511,1 | **567** | | 2 | 532,2 |
| **339** | | 2 | 497,1 | **568** | | 2 | 533,2 |
| **340** | | 2 | 575,3 | **569** | | 2 | 588,2 |
| **341** | | 2 | 566,4 | **570** | | 2 | 600,2 |
| **342** | | 2 | 583,3 | **571** | | 2 | 546,3 |
| **343** | | 2 | 467,3 | **572** | | 2 | 534,3 |
| **344** | | 2 | 497,3 | **573** | | 2 | 548,3 |
| **345** | | 2 | 461,2 | **574** | | 2 | 621,1 |
| **346** | | 2 | 535,2 | **575** | | 3 | 533,1 |
| **347** | | 2 | 529,3 | **576** | | 3 | 489,2 |
| **348** | | 2 | 569,2 | **577** | | 3 | 503,3 |
| **349** | | 2 | 583,2 | **578** | | 3 | 547,1 |
| **350** | | 2 | 593,2 | **579** | | 3 | 531,2 |
| **351** | | 2 | 557,3 | **580** | | 3 | 575,1 |
| **352** | | 2 | 549,2 | **581** | | 3 | 589,1 |

### Beispiel 8: 2-[1-(4-Methoxy-2,6-dimethyl-phenylsulfonyl)-piperidin-2-ylmethoxy]-1-[4-(1-methyl-piperidin-4-yl)-piperazin-1-yl]-ethanon

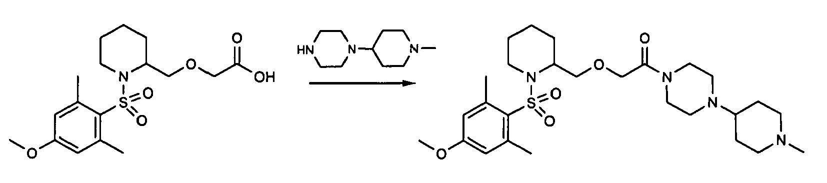

Eine Lösung von 2-((1-(4-Methoxy-2,6-dimethylphenylsulfonyl)piperidin-2-yl)methoxy)essigsäure (250 mg, 0.673 mmol) in Dichlormethan (15 ml) wurde mit N,N'-Carbonyldiimidazol (114 mg, 0.706 mmol) versetzt und 1 h bei Raumtemperatur gerührt. Anschließend wurde eine Lösung von 1-(1-Methylpiperidin-4-yl)piperazin (123 mg, 0.673 mmol) in Dichlormethan (5 ml) zugegeben und das Reaktionsgemisch 15 h bei Raumtemperatur gerührt. Das Reaktionsgemisch wurde danach mit Wasser (20 ml) und gesättigter Natriumchloridlösung (20 ml) extrahiert und die organische Phase mit Magnesiumsulfat getrocknet und i. Vak. eingeengt. Das Rohprodukt wurde durch Flashchromatographie mit Dichlormethan / Methanol (97:3 → 90:10) gereinigt.
Ausbeute: 296 mg (82 %), braunes Harz
¹H-NMR (600MHz, DMSO-d₆): 1.27 (1 H); 1.42 (1 H); 1.55 (4H); 1.69 (2H); 1.79 (1H); 1.89 (2H); 2.18 (3H); 2.40 (4H); 2.53 (6H); 2.83 (2H), 2.95 (1 H); 3.26 (4H); 3.38 (3H); 3.50 (1 H); 3.66 (1 H), 3.80 (4H); 4.05 (2H); 6.79 (2H).

### Darstellung des Hydrochlorids:

### Beispiel 97 :

### 2-((1-(4-Methoxy-2,6-dimethylphenylsulfonyl)piperidin-2-yl)methoxy)-1-(4-(1-methylpiperidin-4-yl)piperazin-1-yl)ethanon Dihydrochlorid

Eine Lösung von 2-((1-(4-Methoxy-2,6-dimethylphenylsulfonyl)piperidin-2-yl)methoxy)essigsäure (600 mg, 1.615 mmol) in Dichlormethan (15 ml) wurde mit *N*,*N*'-Carbonyldiimidazol (272 mg, 1.696 mmol) versetzt und 1 h bei Raumtemperatur gerührt. Anschließend wurde eine Lösung von 1-(1-Methylpiperidin-4-yl)piperazin (293 mg, 1.615 mmol) in Dichlormethan (5 ml) zugegeben und das Reaktionsgemisch 15 h bei Raumtemperatur gerührt. Das Reaktionsgemisch wurde danach mit gesättigter Natriumhydrogencarbonatlösung (20 ml) versetzt und anschließend die wässrige Phase mit Dichlormethan extrahiert (2 x 20 ml). Die vereinigten organischen Phasen wurden mit gesättigter Natriumchloridlösung (20 ml) extrahiert, mit Natriumsulfat getrocknet und i. Vak. eingeengt. Das Rohprodukt wurde durch Flashchromatographie mit Dichlormethan / Methanol (5:1) gereinigt. 2-((1-(4-Methoxy-2,6-dimethylphenylsulfonyl)piperidin-2-yl)methoxy)-1-(4-(1-methylpiperidin-4-yl)piperazin-1-yl)ethanon (590 mg, 1.105 mmol) wurde in Methylethylketon / Ethanol (1:1) (20 ml) [plus einige Tropfen Aceton] gelöst und langsam mit Chlortrimethylsilan (168 µl, 1.326 mmol) versetzt. Anschließend wurde Diethylether (20 ml) hinzugegeben und das Gemisch 1 h bei 0 °C gerührt. Der entstandene Niederschlag wurde abfiltriert, unter Luftausschluß getrocknet und mit Diethylether gewaschen.
Ausbeute: 430 mg (44 %), weißer Feststoff
HPLC-MS, *m*/*z* 537.2 (MH⁺)

### Beispiel 92: 3-((4-(2-((1-(4-Methoxy-2,6-dimethylphenylsulfonyl)piperidin-2-yl)methoxy)acetyl)piperazin-1-yl)methyl)benzonitril hydrochlorid

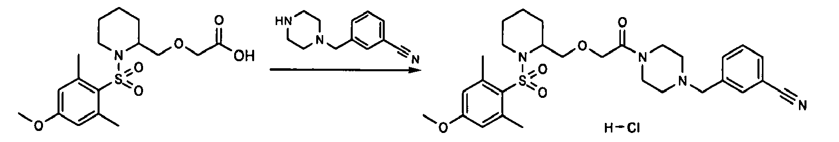

Eine Lösung von 2-((1-(4-Methoxy-2,6-dimethylphenylsulfonyl)piperidin-2-yl)methoxy)essigsäure (150 mg, 0.404 mmol) in Dichlormethan (4 ml) wurde mit *N*,*N*'-Carbonyldiimidazol (68 mg, 0.424 mmol) versetzt und 1 h bei Raumtemperatur gerührt. Anschließend wurde eine Lösung von 3-(Piperazin-1-ylmethyl)benzonitril (81 mg, 0.404 mmol) in Dichlormethan (1 ml) zugegeben und das Reaktionsgemisch 15 h bei Raumtemperatur gerührt. Das Reaktionsgemisch wurde danach mit gesättigter Natriumhydrogencarbonatlösung (5 ml) versetzt und anschließend die wässrige Phase mit Dichlormethan extrahiert (2 x 10 ml). Die vereinigten organischen Phasen wurden mit gesättigter Natriumchloridlösung (10 ml) extrahiert, mit Natriumsulfat getrocknet und i. Vak. eingeengt. Das Rohprodukt wurde durch Flashchromatographie mit Ethylacetat / Hexan (20:1) gereinigt. 3-((4-(2-((1-(4-Methoxy-2,6-dimethylphenylsulfonyl)piperidin-2-yl)methoxy)acetyl)piperazin-1-yl)methyl)benzonitril (100 mg, 0.180 mmol) wurde in Methylethylketon (3 ml) gelöst und langsam mit Chlortrimethylsilan (27 µl, 0.216 mmol) versetzt. Anschließend wurde Diethylether (10 ml) hinzugegeben und das Gemisch 1 h bei 0 °C gerührt. Der entstandene Niederschlag wurde abfiltriert, unter Luftausschluß getrocknet und mit Diethylether gewaschen.
Ausbeute: 100 mg (42 %), weißer Feststoff
¹H-NMR (400 MHz, DMSO-d₆): 1.24 (1 H); 1.54 (4H); 1.79 (1 H); 2.53 (6H); 2.79 (3H); 2.94 (2H); 3.10 (1 H); 3.28 (3H); 3.36 (5H); 3.55 (1 H); 3.69 (1 H); 3.85 (1 H); 4.12 (2H); 4.39 (2H); 6.79 (2H); 7.69 (1 H); 7.95 (2H); 8.10 (1 H); 11.65 (1 H).

### Beispiel 93 3-((4-(2-((1-(4-Methoxy-2,6-dimethylphenylsulfonyl)pyrrolidin-2-yl)methoxy)acetyl)piperazin-1-yl)methyl)benzonitril hydrochlorid

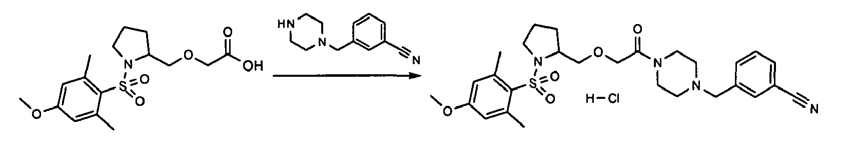

Eine Lösung von 2-((1-(4-Methoxy-2,6-dimethylphenylsulfonyl)pyrrolidin-2-yl)methoxy)essigsäure (150 mg, 0.420 mmol) in Dichlormethan (7 ml) wurde mit *N*,*N*'-Carbonyldiimidazol (71 mg, 0.441 mmol) versetzt und 1 h bei Raumtemperatur gerührt. Anschließend wurde eine Lösung von 3-(Piperazin-1-ylmethyl)benzonitril (84 mg, 0.420 mmol) in Dichlormethan (3 ml) zugegeben und das Reaktionsgemisch 15 h bei Raumtemperatur gerührt. Das Reaktionsgemisch wurde danach mit gesättigter Natriumhydrogencarbonatlösung (10 ml) versetzt und anschließend die wässrige Phase mit Dichlormethan extrahiert (2 x 10 ml). Die vereinigten organischen Phasen wurden mit gesättigter Natriumchloridlösung (10 ml) extrahiert, mit Natriumsulfat getrocknet und i. Vak. eingeengt. Das Rohprodukt wurde durch Flashchromatographie mit Ethylacetat / Hexan / Ammoniaklösung (25 % aq) (100:10:1) gereinigt. 3-((4-(2-((1-(4-Methoxy-2,6-dimethylphenylsulfonyl)pyrrolidin-2-yl)methoxy)acetyl)piperazin-1-yl)methyl)benzonitril (190 mg, 0.351 mmol) wurde in Aceton / Diethylether (1:1; 8 ml) gelöst und langsam mit Chlortrimethylsilan (89 µl, 0.702 mmol) versetzt. Anschließend wurde Diethylether (10 ml) hinzugegeben und das Gemisch 1 h bei 0 °C gerührt. Der entstandene Niederschlag wurde abfiltriert, unter Luftausschluß getrocknet und mit Diethylether gewaschen.
Ausbeute: 200 mg (83 %), weißer Feststoff
HPLC-MS, *m*/*z* 541.2 (MH⁺)

### Beispiel 94: 2-((1-(4-Methoxy-2,6-dimethylphenylsulfonyl)piperidin-2-yl)methoxy)-1-(4-(2-(pyrrolidin-1-yl)ethyl)piperidin-1-yl)ethanon hydrochlorid

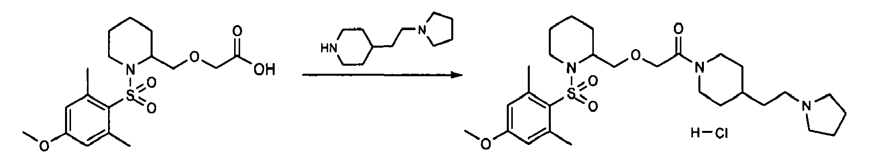

Eine Lösung von 2-((1-(4-Methoxy-2,6-dimethylphenylsulfonyl)piperidin-2-yl)methoxy)essigsäure (150 mg, 0.404 mmol) in Dichlormethan (4 ml) wurde mit *N*,*N*'-Carbonyldiimidazol (68 mg, 0.424 mmol) versetzt und 1 h bei Raumtemperatur gerührt. Anschließend wurde eine Lösung von 4-(2-(Pyrrolidin-1-yl)ethyl)piperidin (73 mg, 0.404 mmol) in Dichlormethan (1 ml) zugegeben und das Reaktionsgemisch 15 h bei Raumtemperatur gerührt. Das Reaktionsgemisch wurde danach mit gesättigter Natriumhydrogencarbonatlösung (5 ml) versetzt und anschließend die wässrige Phase mit Dichlormethan extrahiert (2x10 ml). Die vereinigten organischen Phasen wurden mit gesättigter Natriumchloridlösung (10 ml) extrahiert, mit Natriumsulfat getrocknet und i. Vak. eingeengt. Das Rohprodukt wurde durch Flashchromatographie mit Ethylacetat / Methanol / Ammoniaklösung (25 % aq) (400:100:5) gereinigt. 2-((1-(4-Methoxy-2,6-dimethylphenylsulfonyl)piperidin-2-yl)methoxy)-1-(4-(2-(pyrrolidin-1-yl)ethyl)piperidin-1-yl)ethanon (160 mg, 0.299 mmol) wurde in Methylethylketon (3 ml) gelöst und langsam mit Trimethylchlorsilan (75 µl, 0.358 mmol) versetzt. Anschließend wurde Diethylether (10 ml) hinzugegeben und das Gemisch 1 h bei 0 °C gerührt. Der entstandene Niederschlag wurde abfiltriert, unter Luftausschluß getrocknet und mit Diethylether gewaschen.
Ausbeute: 100 mg (43 %), weißer Feststoff
¹H-NMR (400 MHz, DMSO-d₆): 1.25 (1 H); 1.60 (10H); 1.84 (4H); 1.97 (2H); 2.53 (6H); 2.92 (5H); 3.11 (2H); 3.30 (2H); 3.50 (1 H); 3.66 (3H); 3.80 (5H); 4.03 (2H); 4.29 (1 H); 6.80 (2H); 10.69 (1 H).

### Beispiel 95: 2-((1-(4-Methoxy-2,6-dimethylphenylsulfonyl)pyrrolidin-2-yl)methoxy)-1-(4-(2-(pyrrolidin-1-yl)ethyl)piperidin-1-yl)ethanon

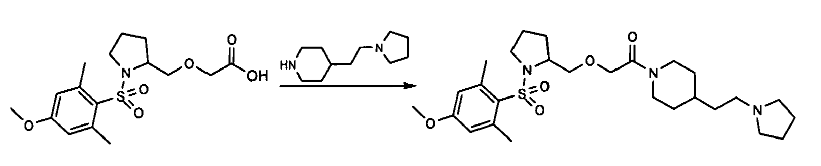

Eine Lösung von 2-((1-(4-methoxy-2,6-dimethylphenylsulfonyl)pyrrolidin-2-yl)methoxy)essigsäure (150 mg, 0.420 mmol) in Dichlormethan (7 ml) wurde mit N,N'-Carbonyldiimidazol (71 mg, 0.441 mmol) versetzt und 1 h bei Raumtemperatur gerührt. Anschließend wurde eine Lösung von 4-(2-(Pyrrolidin-1-yl)ethyl)piperidin (76 mg, 0.420 mmol) in Dichlormethan (3 ml) zugegeben und das Reaktionsgemisch 15 h bei Raumtemperatur gerührt. Das Reaktionsgemisch wurde danach mit gesättigter Natriumhydrogencarbonatlösung (10 ml) versetzt und anschließend die wässrige Phase mit Dichlormethan extrahiert (2 x 10 ml). Die vereinigten organischen Phasen wurden mit gesättigter Natriumchloridlösung (10 ml) extrahiert, mit Natriumsulfat getrocknet und i. Vak. eingeengt. Das Rohprodukt wurde durch Flashchromatographie mit Ethylacetat / Methanol / Ammoniaklösung (25 % aq) (400:100:5) gereinigt.
Ausbeute: 190 mg (87 %), farbloses Öl
HPLC-MS, *m*/*z* 522.3 (MH⁺)

### Beispiel 96: 1-(3,4-Dihydro-2,6-naphthyridin-2(1H)-yl)-2-((1-(4-methoxy-2,6-dimethylphenylsulfonyl)piperidin-2-yl)methoxy)ethanon hydrochlorid

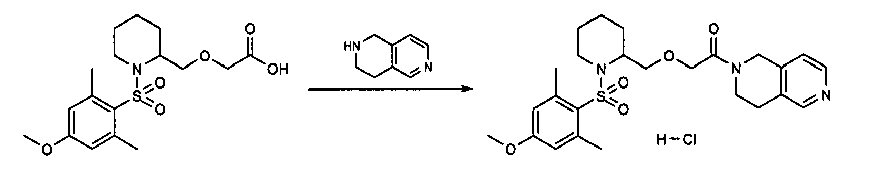

Eine Lösung von 2-((1-(4-Methoxy-2,6-dimethylphenylsulfonyl)piperidin-2-yl)methoxy)essigsäure (150 mg, 0.404 mmol) in Dichlormethan (4 ml) wurde mit *N*,*N*'-Carbonyldiimidazol (68 mg, 0.424 mmol) versetzt und 1 h bei Raumtemperatur gerührt. Anschließend wurde eine Lösung von 1,2,3,4-Tetrahydro-2,6-naphthyridin (54 mg, 0.404 mmol) in Dichlormethan (1 ml) zugegeben und das Reaktionsgemisch 15 h bei Raumtemperatur gerührt. Das Reaktionsgemisch wurde danach mit gesättigter Natriumhydrogencarbonatlösung (5 ml) versetzt und anschließend die wässrige Phase mit Dichlormethan extrahiert (2 x 10 ml). Die vereinigten organischen Phasen wurden mit gesättigter Natriumchloridlösung (10 ml) extrahiert, mit Natriumsulfat getrocknet und i. Vak. eingeengt. Das Rohprodukt wurde durch Flashchromatographie mit Ethylacetat/Methanol (20:1) gereinigt. 1-(3,4-Dihydro-2,6-naphthyridin-2(1H)-yl)-2-((1-(4-methoxy-2,6-dimethylphenylsulfonyl)piperidin-2-yl)methoxy)ethanon (197 mg, 0.404 mmol) wurde in Methylethylketon (5 ml) gelöst und langsam mit Chlortrimethylsilan (61 µl, 0.516 mmol) versetzt. Anschließend wurde Diethylether (10 ml) hinzugegeben und das Gemisch 1 h bei 0 °C gerührt. Der entstandene Niederschlag wurde abfiltriert, unter Luftausschluß getrocknet und mit Diethylether gewaschen.
Ausbeute: 135 mg (64 %), weißer Feststoff
¹H-NMR (400 MHz, DMSO-d₆): 1.25 (1 H); 1.55 (4H); 1.80 (1 H); 2.52 (6H); 2.96 (3H); 3.27 (2H); 3.58 (2H); 3.72 (2H); 3.77 (3H); 3.84 (1 H) 4.20 (2H); 4.85 (2H), 6.77 (2H); 7.85 (1 H); 8.69 (1 H), 8.79 (1 H); (OH verdeckt).

### Umsetzung von 2-((1-(4-Methoxy-2,6-dimethylphenylsulfonyl)piperidin-2-yl)methoxy)essigsäure (Säurebaustein S27) mit Aminen (R⁵R⁶NH):

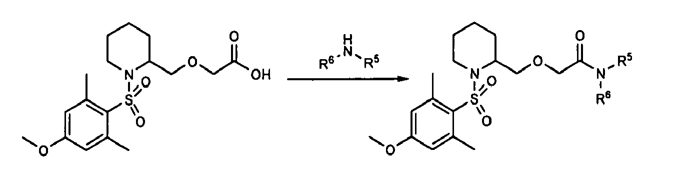

Die in der nachfolgenden Tabelle aufgeführten Beispielverbindungen wurden aus Säurebaustein S27 durch Umsetzung mit den entsprechenden Aminen (R⁵R⁶NH) in enger Anlehnung an die für die Beispiele 8 und 92 - 96 beschriebenen Verfahren hergestellt. Die eingesetzten Amine sind kommerziell verfügbar, können nach dem Fachmann bekannten Methoden hergestellt werden oder wurden nach beschriebenen Verfahren synthetisiert. Anstelle des Lösungsmittels Dichlormethan wurde bei der Synthese folgender Beispielverbindungen das Lösungsmittel N,N-Dimethylformamid verwendet: Beispiel 113 , 143 und 146. Für die Herstellung von Beispielverbindungen, bei denen Amine eingesetzt wurden, die nicht als freie Base vorlagen, sondern als entsprechende Hydrochloride (xHCl), wurde der Reaktion eine entsprechende Menge Triethylamin zugesetzt (äq Et₃N = xHCl). Für das Beispiel 99 erfolgte die Bildung des Hydrochlorides analog zu dem für das Beispiel 97 beschriebene Verfahren. Die Beispiele 106 und 112 wurden nach folgendem allgemeinen Verfahren in die entsprechenden Hydrochloride (xHCl) überführt: Die freien Basen wurden jeweils in einer geringen Menge Methylethylketon gelöst und mit 2 M Chlorwasserstoff Lösung in Diethylether (4 - 5 äq) versetzt. Gegebenenfalls wurde das Gemisch auf 0 °C gekühlt und / oder mit Diethylether versetzt, bevor das Hydrochlorid (xHCl) abfiltriert wurde.

| **Beispiel Nr.** | | **Amin (R⁵R⁶NH)** | **Ausbeute (%)** | **MS, m/z (MH⁺)** |
|---|---|---|---|---|
| 98 | | 2-(Piperidin-4-yl)octahydro-1H-pyrido[1,2-a]pyrazin Trihydrochlorid | 94 | 577,3 |
| 99 | | 2-(Piperidin-4-yl)octahydro-1H-pyrido[1,2-a]pyrazin Trihydrochlorid | 30 | 577,3 |
| 100 | | 2-(Piperidin-4-yl)-1,2,3,4-tetrahydro-2,6-naphthyridin Trihydrochlorid | 82 | 571,3 |
| 101¹ | | tert-Butyl piperazin-1-carboxylat | 89 | 540,3 |
| 103 | | 5-(Piperidin-4-yl)-3-(pyridin-4-yl)-1,2,4-oxadiazol | 89 | 584,3 |
| 106 | | 1-((1-Methylpiperidin-4-yl)methyl)piperazin | 87 | 551,3 |
| 112 | | 1-Methyl-4-(piperidin-4-yl)piperazin | 69 | 537,3 |
| 113 | | 4-(Piperazin-1-yl)thieno[3,2-d]pyrimidin | 78 | 574,2 |
| 143 | | 2-(1-(Pyridin-4-yl)piperidin-4-yl)ethanamin Dihydrochlorid² | 55 | 559,2 |
| 146 | | (4-Methylpiperazin-1-yl)(piperidin-4-yl)methanon Hydrochlorid (xHCl) | 66 | 565,2 |
| 147 | | 1-(Pyridin-4-yl)piperazin | 62 | 517,2 |
| 165 | | 3-(Piperidin-4-yloxy)pyridine Hydrochlorid | 23 | 532,2 |
| 168 | | 7-(Piperazin-1-yl)-4-(pyrrolidin-1-yl)chinazolin Dihydrochlorid (C) | 63 | 637,3 |
| 188 | | 4-(Piperidin-4-ylmethoxy)pyridin Dihydrochlorid | 82 | 546,3 |

| | | | | |
|---|---|---|---|---|
| ¹ Diese Verbindung wurde zusätzlich auch in einer geringeren Ausbeute von 80 % durch Umsetzung in Gegenwart von EDCl / HOBt in Dichlormethan erhalten. ² Das Amin kann in Analogie zu der in WO 2006/071775 beschriebenen Synthese hergestellt werden. | | | | |

### Herstellung der Amine:

### 2-(Piperidin-4-yl)-1,2,3,4-tetrahydro-2,6-naphthyridin Trihydrochlorid

### (eingesetzt in der Synthese der Beispielverbindung 100)

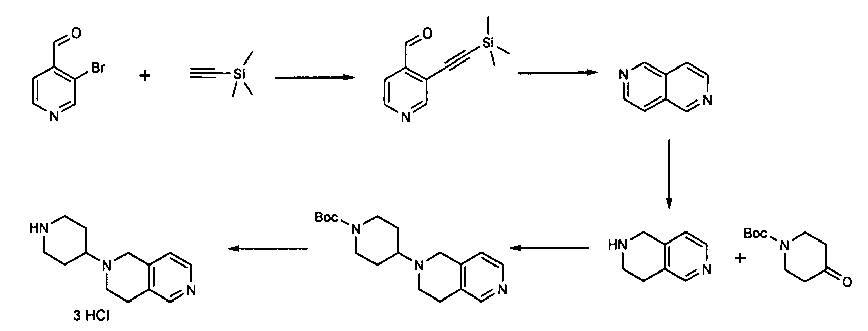

(i) Zu einer Lösung 3-Brom-4-pyridincarboxaldehyd (14,16 g, 76,1 mmol) in trockenem Tetrahydrofuran (140 ml) unter Stickstoff wurden nacheinander Pd(PPh₃)₂Cl₂ (1,07 g, 1,52 mmol), Trimethylsilylacetylen (18,9 ml, 133 mmol), 1,4-Diazabicyclo[2.2.2]octan (DABCO) (17,1 g, 152 mmol) and Kupfer(I) iodid (145 mg, 0,76 mmol) gegeben. Das Reaktionsgemisch wurde 1 h gerührt über Celite abfiltriert, mit Tetrahydrofuran gewaschen und aufkonzentriert. Der Rückstand wurde säulenchromatographisch aufgereinigt (Heptan / Ethylacetat, 9:1 → 9:2).
   Ausbeute: 14,62 g (94 %)
(ii) Eine Lösung 3-((Trimethylsilyl)ethynyl)isonicotinaldehyd (13,22 g, 65,0 mmol) in Ethanol (300 ml) unter Stickstoff wurde bei gleichzeitiger kontinuierlicher Zugabe von Ammoniak 7,5 h refluxiert. Anschließend wurde das Gemisch aufkonzentriert und der Rückstand in Ethylacetat aufgenommen und über Kieselgel filtriert. Das Rohprodukt wurde in heißem Hexan gelöst und 2 x abdekantiert. Das Filtrat wurde aufkonzentriert und aus Hexan / Diisopropylether kristallisiert.
   Ausbeute: 3,29 g (39 %)
(iii) Zu einer Suspension Calciumoxid (758 mg, 13,5 mmol) and 2,6-Naphthyridin (1,60 g, 12,3 mmol) in 2-Methoxyethanol (15 ml) unter Stickstoff wurde Platinum(IV) Oxid (223 mg, 0,984 mmol) hinzugegeben. Das Reaktionsgemisch wurde über Nacht unter einer Wasserstoffatmosphäre gerührt und anschließend über Celite filtriert, mit Ethanol gewaschen, unter Vakuum eingeengt und mit Dichlormethan co-evaporiert. Der Rückstand wurde in Ethylacetat aufgenommen, über einem Microfilter filtriert, mit Ethylacetat gewaschen und wiederum eingeengt. Der Rückstand wurde mit einem zweiten Batch, der analog hergestellt wurde (aus 1,66 g (12,8 mmol) 2,6-Naphthyridin), vereinigt und mit Toluol (2 x) und Dichlormethan (2 x) co-evaporiert. Dann wurde das Rohprodukt über Nacht unter Vakuum getrocknet. Die Aufreinigung erfolgte mittels Säulenchromatographie (Heptan / Dichlormethan / 7 M Ammoniaklösung in Methanol, 10:30:2). Anschließend wurde mit Dichlormethan co-evaporiert.
   Ausbeute: 2,83 g (84 %)
(iv) 1,2,3,4-Tetrahydro-2,6-naphthyridin (1,16 g, 8,65 mmol) und tert-Butyl 4-oxopiperidin-1-carboxylat (1,72 g, 8,65 mmol) wurden in 1,2-Dichlorethan (20,5 ml) gelöst. Zu dieser Lösung wurden bei Raumtemperatur Natriumtriacetoxyborhydrid (2,56 g, 12,10 mmol) und Essigsäure (0,49 ml, 8,65 mmol) hinzugegeben. Das Reaktionsgemisch wurde 15 h bei Raumtemperatur gerührt und anschließend mit gesättigter Natriumhydrogencarbonatlösung (20 ml) versetzt und 30 min gerührt. Die wässrige Phase wurde mit Diethylether (2 x 30 ml) extrahiert und die organische Phase wiederum mit gesättigter Natriumchloridlösung (20 ml) gewaschen. Die organische Phase wurde getrocknet (MgSO₄) und unter Vakuum eingeengt. Das Rohprodukt wurde säulenchromatographisch (Kieselgel) mit Ethylacetat / Hexan / Methanol / Ammoniaklösung (25 % aq) (100:10:10:1) gereinigt.
   Ausbeute: 2,26 g (82 %)
(v) Zu einer Lösung tert-Butyl 4-(3,4-dihydro-2,6-naphthyridin-2(1H)-yl)piperidin-1-carboxylat (2,26 g, 7,12 mmol) in Methanol (10 ml) wurde bei Raumtemperatur Chlorwasserstoff (28,48 ml, 35,60 mmol, 1,25 M Lösung in Methanol) hinzugegeben. Das Reaktionsgemisch wurde 30 min refluxiert. Das Lösungsmittel wurde unter Vakuum entfernt und der Rückstand in ein wenig Ethanol aufgenommen und mit Diethylether versetzt. Anschließend wurde 30 min im Eisbad gekühlt und der entstandene Feststoff abfiltriert und getrocknet.
   Ausbeute: 2,09 g (90 %)

### 2-(Piperidin-4-yl)octahydro-1H-pyrido[1,2-a]pyrazin Trihydrochlorid

### (eingesetzt in der Synthese der Beispiele 98 und 99)

Das Amin wurde analog zu 2-(Piperidin-4-yl)-1,2,3,4-tetrahydro-2,6-naphthyridin Trihydrochlorid aus Octahydro-1H-pyrido[1,2-a]pyrazin und tert-Butyl 4-oxopiperidin-1-carboxylat hergestellt (Stufen iv und v).

### (4-Methylpiperazin-1-yl)(piperidin-4-yl)methanon Hydrochlorid

### (eingesetzt in der Synthese der Beispielverbindung 146)

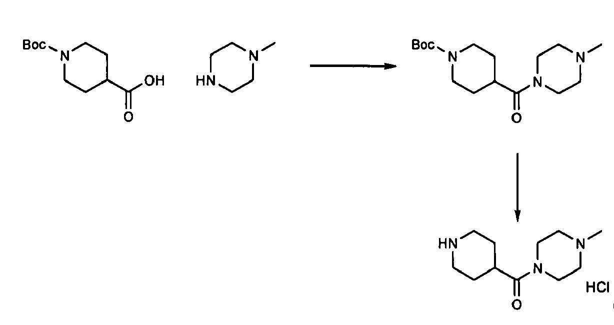

(i) Zu einer Lösung 1-(tert-Butoxycarbonyl)piperidin-4-carbonsäure (5,0 g, 21,82 mmol) in N,N-Dimethylformamid (76,3 ml) wurden 1-Methylpiperazin (2,20 ml, 19,84 mmol) und 4-Methylmopholin (4,37 ml, 39,68 mmol) gegeben. Das Gemisch wurde dann mit Benzotriazol-1-yloxytris(dimethylamino)phosphonium Hexafluorphosphat (11,44 g, 25,79 mmol) versetzt und 15 h bei Raumtemperatur gerührt. Dann wurde unter Vakuum eingeengt. Der Rückstand wurde in Ethylacetat (100 ml) und gesättigter Natriumhydrogencarbonatlösung (100 ml) aufgenommen und die wässrige Phase mit Ethylacetat (2 x 30 ml) extrahiert. Die vereinigten organischen Phasen wurden mit gesättigter Natriumchloridlösung (30 ml) gewaschen, getrocknet (Na₂SO₄) und unter Vakuum eingeengt. Das Rohprodukt wurde säulenchromatographisch (Kieselgel) mit Ethylacetat / Methanol / Ammoniaklösung (25 % aq) (40:10:0,5) aufgereinigt.
   Ausbeute: 5,61 g (83 %)
(ii) Zu tert-Butyl 4-(4-methylpiperazin-1-carbonyl)piperidin-1-carboxylat (4,81 g, 15,46 mmol) wurde bei Raumtemperatur Chlorwasserstoff (49,46 ml, 61,83 mmol, 1,25 M Lösung in Methanol) hinzugegeben und das Reaktionsgemisch 1 h refluxiert. Das Lösungsmittel wurde unter Vakuum entfernt und der Rückstand in ein wenig Ethanol aufgenommen und mit Methylethylketon und Diethylether versetzt und 40 min refluxiert. Anschließend wurde langsam auf Raumtemperatur abgekühlt und dann 30 min im Eisbad gekühlt. Der entstandene Feststoff wurde abfiltriert und getrocknet.
   Ausbeute: 3,83 g (88 %)

### 3-(Piperidin-4-yloxy)pyridin Hydrochlorid

### (eingesetzt in der Synthese der Beispielverbindung 165)

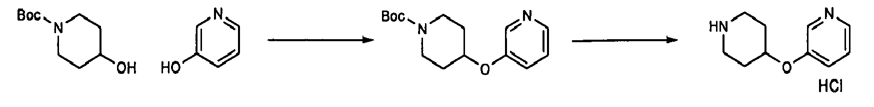

(i) Zu einer Lösung von 3-Pyrolidinol (700 mg, 7,36 mmol) in Tetrahydrofuran (10 ml) wurden bei Raumtemperatur tert-Butyl-4-hydroxypiperidin-1-carboxylat (1,85 g, 9,20 mmol) und Triphenylphosphin (2,41 g, 9,20 mmol) hinzugegeben. Anschließend wurde Diisopropyl-azodicarboxylat (1,79 ml, 125,1 mmol) hinzugetropft und das Gemisch dann 15 h bei 55°C gerührt. Das Lösungsmittel wurde unter Vakuum entfernt und der Rückstand in 1 M Salzsäure (20 ml) aufgenommen und mit Dichlormethan (2x10 ml) extrahiert. Die vereinigten organischen Phasen wurden mit 1 M Salzsäure (20 ml) und Wasser (20 ml) extrahiert. Die wässrigen Phasen wurden vereinigt, mit 1 M Natriumhydroxidlösung auf pH 12 eingestellt und anschließend mit Dichlormethan (4 x 20 ml) extrahiert. Dann wurde die organische Phase mit gesättigter Natriumchloridlösung (20 ml) gewaschen, getrocknet (Na₂SO₄) und unter Vakuum eingeengt. Anschließend wurde das Rohprodukt säulenchromatographisch (Kieselgel) mit Ethylacetat / Hexan (10:1) gereinigt.
   Ausbeute: 410 mg (20 %)
   [analoges Verfahren siehe: J. Chao et al., Tetrahedron Lett., 2007, 48, 791]
(ii) Zu einer Lösung tert-Butyl 4-(pyridin-3-yloxy)piperidin-1-carboxylat (410 mg, 1,473 mmol) in Methanol (2-5 ml) wurde bei Raumtemperatur Chlorwasserstoff (4,71 ml, 5,89 mmol, 1,25 M Lösung in Methanol) hinzugegeben und das Reaktionsgemisch 30 min refluxiert. Das Lösungsmittel wurde unter Vakuum entfernt und der Rückstand in ein wenig Ethanol aufgenommen und mit Diethylether versetzt. Anschließend wurde 30 min im Eisbad gekühlt und der entstandene Feststoff abfiltriert und getrocknet.
   Ausbeute: 270 mg (85 %)

### 4-(Piperidin-4-ylmethoxy)pyridin Dihydrochlorid

### (eingesetzt in der Synthese der Beispielverbindung 188)

Das Amin wurde analog zu 3-(Piperidin-4-yloxy)pyridin Hydrochlorid aus tert-Butyl 4-(hydroxymethyl)piperidin-1-carboxylat und 3-Pyrolidinol hergestellt.

### 7-(Piperazin-1-yl)-4-(pyrrolidin-1-yl)chinazolin Dihydrochlorid (C)

### (eingesetzt in der Synthese der Beispielverbindung 168)

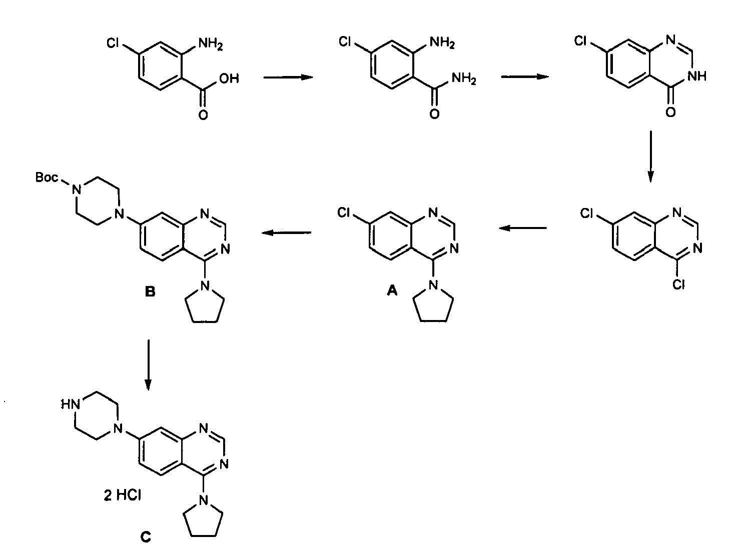

7-Chlor-4-(pyrrolidin-1-yl)chinazolin (A) wurde aus 2-Amino-4-chlorbenzoesäure analog zu folgender literaturbekannter Vorschrift für die Herstellung von Aminochinazolinen hergestellt: H. Hayashi et al., Bioorg. Med. Chem., 2003, 11, 383. [Review zur Synthese von Chinazolinen: P. J. Guiry et al., Tetrahedron, 2005, 61,10153.]

### tert-Butyl 4-(4-(pyrrolidin-1-yl)chinazolin-7-yl)piperazin-1-carboxylat (B)

Zu einem Gemisch aus 7-Chlor-4-(pyrrolidin-1-yl)chinazolin (A) (840 mg, 3,59 mmol) und tert-Butyl piperazin-1-carboxylat (1,0 g, 5,39 mmol) in Toluol (49 ml) wurden unter Stickstoff Kalium tert-butylat (998 mg, 8,99 mmol), 2-Dicyclohexylphosphino-2,4,6-triisopropylbiphenyl (X-Phos) (79 mg, 0,18 mmol) und Tris-(dibenzylidenaceton)-dipalladium [Pd₂(dba)₃] (36 mg, 0,036 mmol) gegeben. Anschließend wurde das Reaktionsgemisch 15 h auf 100 °C erwärmt. Das Reaktionsgemisch wurde auf Raumtemperatur gekühlt und Wasser (25 ml) und Ethylacetat (25 ml) hinzugegeben. Die wässrige Phase wurde mit Ethylacetat (2 x 25 ml) extrahiert und die vereinigten organischen Phasen mit gesättigter Natriumchloridlösung (20 ml) gewaschen. Anschließend wurde die organische Phase getrocknet (MgSO₄) und unter Vakuum eingeengt. Das Rohprodukt wurde säulenchromatographisch (Kieselgel) mit Ethylacetat / Dichlormethan / Ammoniaklösung (25 % aq) (50:10:0,5) und Ethylacetat / Dichlormethan / Methyl-tert-butylether / Ammoniaklösung (25 % aq) (50:10:10:0,7) gereinigt.
Ausbeute: 700 mg (51 %)

### 7-(Piperazin-1-yl)-4-(pyrrolidin-1-yl)chinazolin Dihydrochlorid (C)

tert-Butyl 4-(4-(pyrrolidin-1-yl)chinazolin-7-yl)piperazin-1-carboxylat (B) (130 mg, 0,339 mmol) wurde bei Raumtemperatur in Methanol (2 ml) gelöst und anschließend Chlorwasserstoff (2,71 ml, 3,39 mmol, 1,25 M Lösung in Methanol) hinzugegeben. Das Reaktionsgemisch wurde 1 h refluxiert und dann 15 h bei Raumtemperatur gerührt. Es wurde unter Vakuum eingeengt und der Rückstand in ein wenig Ethanol aufgenommen und erwärmt. Anschließend wurde Diethylether hinzugegeben, im Eisbad gekühlt und schließlich der entstandene Feststoff abfiltriert.
Ausbeute: 103 mg (85 %)

### Umsetzung von 2-((1-(Benzo[b]thiophen-3-ylsulfonyl)piperidin-2-yl)methoxy)essigsäure (Säurebaustein S32) mit 1-(1-Methylpiperidin-4-yl)piperazin:

### Beispiel 137:

### 2-((1-(Benzo[b]thiophen-3-ylsulfonyl)piperidin-2-yl)methoxy)-1-(4-(1-methylpiperidin-4-yl)piperazin-1-yl)ethanon

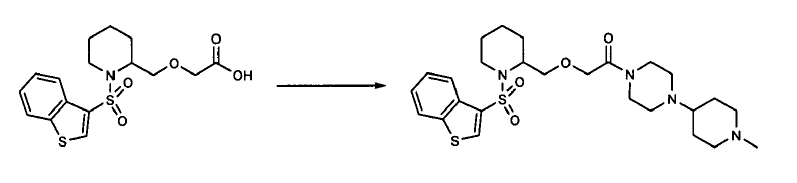

Die Beispielverbindung 137 wurde aus Säurebaustein S32 durch Umsetzung mit 1-(1-Methylpiperidin-4-yl)piperazin in enger Anlehnung an die für die Beispiele 8 und 92 - 96 beschriebenen Verfahren in einer Ausbeute von 79 % hergestellt.
MS, *m*/*z* 535,2 (MH⁺)

### Umsetzung von 2-(2-(1-(4-Methoxy-2,6-dimethylphenylsulfonyl)piperidin-2-yl)ethoxy)essigsäure mit 1-(1-Methylpiperidin-4-yl)piperazin:

### Beispiel 164:

### 2-(2-(1-(4-Methoxy-2,6-dimethylphenylsulfonyl)piperidin-2-yl)ethoxy)-1-(4-(1-methylpiperidin-4-yl)piperazin-1-yl)ethanon

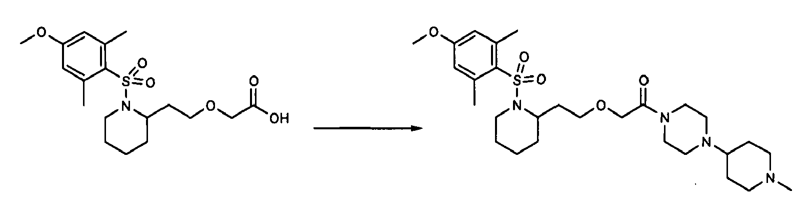

Die Beispielverbindung 164 wurde aus dem entsprechenden Säurebaustein durch Umsetzung mit 1-(1-Methylpiperidin-4-yl)piperazin in enger Anlehnung an die für die Beispiele 8 und 92 - 96 beschriebenen Verfahren in einer Ausbeute von 80 % hergestellt. Der Säurebaustein wurde in Analogie zu dem unter Methode 1 beschriebenen Verfahren für die Herstellung von Säurebausteinen für die Parallelsynthese hergestellt.
MS, *m*/*z* 551,2 (MH⁺)

### Umsetzung von 2-((2-(Naphthalen-2-ylsulfonyl)-1,2,3,4-tetrahydroisochinolin-3-yl)methoxy)essigsäure mit 1-(1-Methylpiperidin-4-yl)piperazin:

### Beispiel 178:

### 1-(4-(1-Methylpiperidin-4-yl)piperazin-1-yl)-2-((2-(naphthalen-2-ylsulfonyl)-1,2,3,4-tetrahydroisochinolin-3-yl)methoxy)ethanon Dihydrochlorid

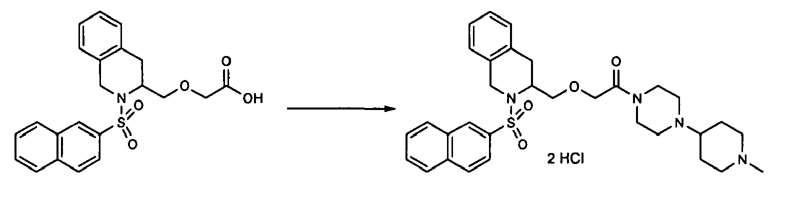

Die Beispielverbindung 178 wurde aus dem entsprechenden Säurebaustein durch Umsetzung mit 1-(1-Methylpiperidin-4-yl)piperazin in enger Anlehnung an die für die Beispiele 8 und 92 - 96 beschriebenen Verfahren in einer Ausbeute von 29 % hergestellt. Der Säurebaustein wurde in Analogie zu dem unter Methode 1 beschriebenen Verfahren für die Herstellung von Säurebausteinen für die Parallelsynthese hergestellt. Die Hydrochloridfällung der freien Base zu Beispiel 178 erfolgte aus einer Methylethylketon / Diethylether Lösung der Base unter Zugabe von 2 M Chlorwasserstoff Lösung in Diethylether.
MS, *m*/*z* 577,2 (MH⁺)

### Umsetzung von 1-(4-(1-Methylpiperidin-4-yl)piperazin-1-yl)-2-(piperidin-2-ylmethoxy)ethanon Trihydrochlorid mit Sulfonylchloriden:

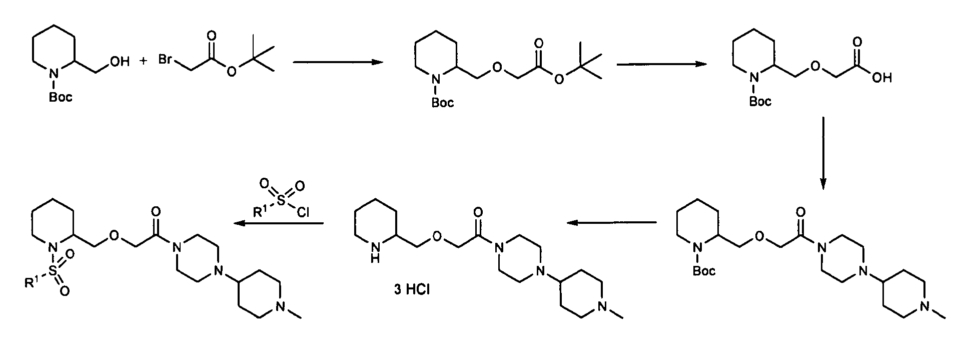

### 1-(4-(1-Methylpiperidin-4-yl)piperazin-1-yl)-2-(piperidin-2-ylmethoxy)ethanon Trihydrochlorid

Stufe (i): Zu einem Gemisch aus Tetra-n-butylammoniumhydrogensulfat (625 mg, 1,859 mmol), wässriger Natriumhydroxidlösung (18,58 g, 464,69 mmol in Wasser (20 ml)) und Toluol (15 ml) wurde bei Raumtemperatur tert-Butyl-2-bromoacetat (4,1 ml, 27,88 mmol) gegeben und anschließend auf 0 °C gekühlt. Dann wurde eine Lösung tert-Butyl-2-(hydroxymethyl)piperidin-1-carboxylat (4,0 g, 18,587 mmol) in Toluol (10 ml) langsam hinzugegeben. Das Reaktionsgemisch wurde auf Raumtemperatur erwärmt und 1 h bei dieser Temperatur gerührt. Die Phasen wurden getrennt und die wässrige Phase mit Diethylether extrahiert (2 x 25 ml). Die vereinigten organischen Phasen wurden mit gesättigter Natriumchloridlösung (20 ml) gewaschen, getrocknet (Na₂SO₄) und unter Vakuum eingeengt. Das Rohprodukt wurde säulenchromatographisch (Kieselgel) mit Hexan / Diethylether (3:1) gereinigt.
Ausbeute: 3,53 g (58 %)

Stufe (ii): tert-Butyl 2-((2-tert-Butoxy-2-oxoethoxy)methyl)piperidin-1-carboxylat (3,53 g, 10,717 mmol) wurde in Tetrahydrofuran (20 ml) gelöst und Natriumhydroxidlösung (1,71g, 42,87 mmol in Wasser (2 ml)) wurde hinzugegeben. Das Reaktionsgemisch wurde 3 h auf 90°C erwärmt und anschließend wieder auf Raumtemperatur gekühlt. Der pH-Wert des Gemisches wurde mit 2 M Salzsäure auf pH 2 eingestellt und es wurde dann mit Ethylacetat (3 x 30 ml) extrahiert. Die vereinigten organischen Phasen wurden getrocknet (Na₂SO₄) und unter Vakuum eingeengt. Das Rohprodukt wurde ohne weitere Aufreinigung in der nächsten Synthesestufe eingesetzt.
Ausbeute: 3,28 g (>99 %)

Stufe (iii): Eine Lösung von 2-((1-(tert-Butoxycarbonyl)piperidin-2-yl)methoxy)essigsäure (3,27 g, 11,965 mmol) in Dichlormethan (15 ml) wurde mit N,N'-Carbonyldiimidazol (2,02 g, 12,563 mmol) versetzt und 1,5 h bei Raumtemperatur gerührt. Anschließend wurde eine Lösung 1-(1-Methylpiperidin-4-yl)piperazin (2,19 g, 11,965 mmol) in Dichlormethan (15 ml) zugegeben und das Reaktionsgemisch 3 d bei Raumtemperatur gerührt. Das Gemisch wurde mit gesättigter Natriumhydrogencarbonatlösung (30 ml) versetzt und anschließend die wässrige Phase mit Dichlormethan (2 x 30 ml) extrahiert. Die vereinigten organischen Phasen wurden mit gesättigter Natriumchloridlösung (30 ml) gewaschen, getrocknet (Na₂SO₄) und unter Vakuum eingeengt. Das Rohprodukt wurde säulenchromatographisch (Kieselgel) mit Ethylacetat / Methanol / Dichlormethan / Ammoniaklösung (25 % aq) (400:100:100:5) gereinigt.
Ausbeute: 4,57 g (87 %)

Stufe (iv): Zu einer Lösung tert-Butyl 2-((2-(4-(1-methylpiperidin-4-yl)piperazin-1-yl)-2-oxoethoxy)methyl)piperidin-1-carboxylat (4,57 g, 10,42 mmol) in einem Gemisch aus Ethylacetat (15 ml) und Diethylether (50 ml) wurde bei Raumtemperatur Chlorwasserstoff (26,0 ml, 52,10 mmol, 2 M Lösung in Diethylether) gegeben. Das Reaktionsgemisch wurde 2 h bei 45 °C gerührt. Dann wurde der entstandene weiße Feststoff abfiltriert und getrocknet.
Ausbeute: 3,59 g (77 %)

### Beispiel 107:

### 2-((1-(Mesitylsulfonyl)piperidin-2-yl)methoxy)-1-(4-(1-methylpiperidin-4-yl)piperazin-1-yl)ethanon

Stufe (v): Zu einer Lösung 1-(4-(1-Methylpiperidin-4-yl)piperazin-1-yl)-2-(piperidin-2-ylmethoxy)ethanon Trihydrochlorid (180 mg, 0,402 mmol) in Tetrahydrofuran (10 ml) wurden bei 0 °C Triethylamin (0,221 ml, 1,608 mmol) und anschließend 2,4,6-Trimethylbenzolsulfonyl chlorid (105 mg, 0,482 mmol) hinzugegeben. Das Reaktionsgemisch wurde langsam auf Raumtemperatur erwärmt, 15 h bei dieser Temperatur gerührt und danach1 h refluxiert. Anschließend wurde das Gemisch mit gesättigter Natriumhydrogencarbonatlösung (5 ml) versetzt und die wässrige Phase mit Ethylacetat (3 x 30 ml) extrahiert. Die vereinigten organischen Phasen wurden getrocknet (Na₂SO₄), unter Vakuum eingeengt und das Rohprodukt anschließend säulenchromatographisch (Kieselgel) mit Ethylacetat / Methanol / Ammoniaklösung (25 % aq) (300:100:5) gereinigt.
Ausbeute: 90 mg (43 %), gelbes Öl
MS, *m*/*z* 520,3 (MH⁺)

### Beispiel 108:

### 2-((1-(2,6-Dichlor-4-(trifluormethyl)phenylsulfonyl)piperidin-2-yl)methoxy)-1-(4-(1-methylpiperidin-4-yl)piperazin-1-yl)ethanon

Stufe (v): 1-(4-(1-Methylpiperidin-4-yl)piperazin-1-yl)-2-(piperidin-2-ylmethoxy)ethanon Trihydrochlorid (150 mg, 0,335 mmol) wurde bei Raumtemperatur in einem Gemisch aus Dichlormethan (5 ml) und Triethylamin (0,208 ml, 1,507 mmol) gelöst und 2,6-Dichlor-4-(trifluormethyl)benzolsulfonyl chlorid (156 mg, 0,503 mmol) in Dichlormethan (5 ml) wurde hinzugegeben. Das Reaktionsgemisch wurde 3 d bei dieser Temperatur gerührt und anschließend mit gesättigter Natriumhydrogencarbonatlösung (5 ml) versetzt. Die wässrige Phase wurde mit Dichlormethan (2 x 20 ml) extrahiert und die vereinigten organischen Phasen getrocknet (Na₂SO₄) und unter Vakuum eingeengt. Das Rohprodukt wurde anschließend säulenchromatographisch (Kieselgel) mit Ethylacetat / Methanol / Ammoniaklösung (25 % aq) (200:100:3) gereinigt.
Ausbeute: 120 mg (58 %), gelbes Öl
MS, *m*/*z* 614,2 (MH⁺)

Die in der nachfolgenden Tabelle aufgeführten Beispielverbindungen wurden aus 1-(4-(1-Methylpiperidin-4-yl)piperazin-1-yl)-2-(piperidin-2-ylmethoxy)ethanon Trihydrochlorid durch Umsetzung mit den entsprechenden Sulfonylchloriden (R¹SO₂Cl) in enger Anlehnung an das für das Beispiel 108 beschriebene Verfahren (Stufe (v)) hergestellt. Der Reaktionsverlauf wurde dünnschichtchromatographisch verfolgt; die Reaktionszeit betrug in den meisten Fällen zwischen 15 h und 3 d. Die eingesetzten Mengen der Reagenzien variierte wie folgt: Sulfonylchlorid (0,9 bis 1,5 äq), Triethylamin (3,5 - 4,5 äq). Die Umsetzungen wurden in einigen Fällen in Tetrahydrofuran als Alternative für Dichlormethan durchgeführt. Die eingesetzten Sulfonylchloride sind kommerziell verfügbar, können nach dem Fachmann bekannten Methoden hergestellt werden oder wurden nach beschriebenen Verfahren synthetisiert. Desweiteren wurden für die Beispiele 141, 155, 156, 158, 159, 169 und 170 die Basen nach folgendem allgemeinen Verfahren in die entsprechenden Dihydrochloride (2 x HCl) überführt: Die freien Basen wurden jeweils in einer geringen Menge Dichlormethan oder Methylethylketon gelöst und mit 2 M Chlorwasserstoff Lösung in Diethylether (4-5 äq) versetzt. In einigen Fällen wurde das Gemisch auf 0 °C gekühlt und / oder mit Diethylether versetzt, bevor das Dihydrochlorid abfiltriert wurde.

| **Beispiel Nr.** | | **Sulfonylchlorid (R¹SO₂Cl)** | **Ausbeute (%)** | **MS, m/z (MH⁺)** |
|---|---|---|---|---|
| 109 | | 2-Chlor-6-methylbenzol-1-sulfonyl chlorid | 85 | 527,2 |
| 110 | | Naphthalen-1-sulfonyl chlorid | 79 | 529,3 |
| 111 | | Naphthalen-2-sulfonyl chlorid | 79 | 529,3 |
| 114 | | 4-Chloro-2,5-dimethylbenzol-1-sulfonyl chlorid | 24 | 541,2 |
| 115 | | 4-Chlor-3-(trifluormethyl)benzol-1-sulfonyl chlorid | 51 | 581,2 |
| 118 | | 2,4,6-Trichlorobenzol-1-sulfonyl chlorid | 46 | 583,1 |
| 119 | | 2,4,6-Triisopropylbenzol-1-sulfonyl chlorid | 54 | 605,4 |
| 120 | | 2,4-Dichlorbenzol-1-sulfonyl chlorid | 60 | 547,2 |
| 128 | | 5-Chlor-1,3-dimethyl-1H-pyrazol-4-sulfonyl chlorid | 39 | 531,3 |
| 129 | | 6-Chlorimidazo[2,1-b]thiazol-5-sulfonyl chlorid | 37 | 559,2 |
| 131 | | 3-(o-Tolyloxy)benzol-1-sulfonyl chlorid | 71 | 585,3 |
| 138 | | 2-Chlor-4-(trifluormethyl)benzol-1-sulfonyl chlorid | 48 | 581,2 |
| 139 | | 2-Chlorbenzol-1-sulfonyl chlorid | 38 | 513,1 |
| 141 | | 2,6-Dichlorbenzol-1-sulfonyl chlorid | 43 | 547,1 |
| 151 | | 5-Chlor-3-methylbenzo[b]thiophen-2-sulfonyl chlorid | 41 | 583,1 |
| 153 | | 2,5-Bis(trifluormethyl)benzol-1-sulfonyl chlorid | 58 | 615,1 |
| 154 | | 7-Chlorbenzo[c][1,2,5]oxadiazol-4-sulfonyl chlorid | 27 | 555,1 |
| 155 | | 4-Methylnaphthalen-1-sulfonyl chlorid | 44 | 543,2 |
| 156 | | 2,4,5-Trichlorobenzol-1-sulfonyl chlorid | 21 | 581,0 |
| 158 | | 5-(Dimethylamino)naphthalen-1-sulfonyl chlorid | 56 | 572,2 |
| 159 | | 2-Methylbenzol-1-sulfonyl chlorid | 55 | 493,2 |
| 169 | | 4-Fluor-2,6-Dimethylbenzol-1-sulfonyl chlorid | 47 | 525,2 |
| 170 | | 2,5-Dichlorthiophen-3-sulfonyl chlorid | 54 | 553,1 |
| 171 | | Benzo[b]thiophen-2-sulfonyl chlorid | 59 | 535,2 |
| 172 | | 2,5-Dimethylthiophen-3-sulfonyl chlorid | 57 | 513,2 |

### Herstellung des Sulfonylchlorides:

### 4-Fluor-2,6-Dimethylbenzol-1-sulfonylchlorid

### (eingesetzt in der Synthese der Beispielverbindung 169)

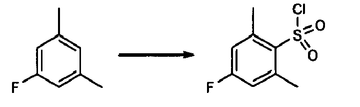

Zu einer Lösung 1-Fluor-3,5-dimethylbenzol (25 g) in Dichlormethan (250 ml) wurde Chlorsulfonsäure (54,3 ml, 4 äq) über 45 min bei 0 °C hinzugetropft. Anschließend wurde das Reaktionsgemisch 1 h bei Raumtemperatur gerührt und der Reaktionsverlauf dünnschichtchromatographisch verfolgt. Das Reaktionsgemisch wurde auf Eis gegeben und die wässrige Phase mit Dichlormethan (3 x 150 ml) extrahiert. Die organische Phase wurde getrocknet (Na₂SO₄) und unter Vakuum eingeengt. Anschließend wurde das Rohprodukt säulenchromatographisch aufgereinigt.
Ausbeute: 19,5 g (44 %), weißer Feststoff

### Umsetzung von 2-((1-(4-Methoxy-2,6-dimethylphenylsulfonyl)piperidin-2-yl)methoxy)-1-(piperazin-1-yl)ethanon Hydrochlorid (Beispiel 102) mit Ketonen und Aldehyden (R^{a}R^{b}C=O):

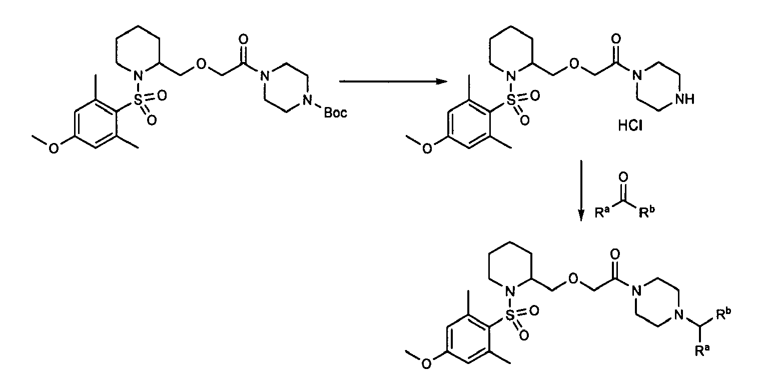

### Beispiel 102:

### 2-((1-(4-Methoxy-2,6-dimethylphenylsulfonyl)piperidin-2-yl)methoxy)-1-(piperazin-1-yl)ethanon Hydrochlorid

Stufe (i): Zu einer Lösung tert-Butyl 4-(2-((1-(4-methoxy-2,6-dimethylphenylsulfonyl)piperidin-2-yl)methoxy)acetyl)piperazin-1-carboxylat (Beispiel 101) (310 mg, 0,574 mmol) in Diethylether (2 - 5 ml) wurde bei Raumtemperatur Chlorwasserstoff (1,15 ml, 2,30 mmol, 2 M Lösung in Diethylether) gegeben. Das Reaktionsgemisch wurde 2 h bei Raumtemperatur gerührt und anschließend 10 min refluxiert. Der entstandene Feststoff wurde abfiltriert und getrocknet.
Ausbeute: 210 mg (77 %), weißer Feststoff

### Umsetzung mit Ketonen:

### Beispiel 104:

### 2-((1-(4-Methoxy-2,6-dimethylphenylsulfonyl)piperidin-2-yl)methoxy)-1-(4-(tetrahydro-2H-pyran-4-yl)piperazin-1-yl)ethanon

Stufe (ii): 2-((1-(4-Methoxy-2,6-dimethylphenylsulfonyl)piperidin-2-yl)methoxy)-1-(piperazin-1-yl)ethanon Hydrochlorid (Beispiel 102) (90 mg, 0,189 mmol) und Dihydro-2H-pyran-4(3H)-on (0,017 ml, 0,189 mmol) wurden in einem Gemisch aus 1,2-Dichlorethan (4 ml) und Triethylamin (0,026 ml, 0,189 mmol) gelöst. Zu dieser Lösung wurden bei Raumtemperatur Natriumtriacetoxyborhydrid (56 mg, 0,265 mmol) und Essigsäure (0,011 ml, 0,189 mmol) gegeben. Das Reaktionsgemisch wurde 1 h bei Raumtemperatur gerührt und anschließend mit gesättigter Natriumhydrogencarbonatlösung (5 ml) versetzt. Die wässrige Phase wurde mit Diethylether (2 x 20 ml) extrahiert und die organische Phase wiederum mit gesättigter Natriumchloridlösung (10 ml) gewaschen. Die organische Phase wurde getrocknet (MgSO₄), unter Vakuum eingeengt und das Rohprodukt säulenchromatographisch (Kieselgel) mit Ethylacetat / Methanol (10:1) gereinigt.
Ausbeute: 90 mg (91 %), gelbes Öl
MS, *m*/*z* 524,3 (MH⁺)

Die in der nachfolgenden Tabelle aufgeführten Beispielverbindungen wurden aus 2-((1-(4-Methoxy-2,6-dimethylphenylsulfonyl)piperidin-2-yl)methoxy)-1-(piperazin-1-yl)ethanon Hydrochlorid (Beispiel 102) durch Umsetzung mit den entsprechenden Ketonen (R^{a}R^{b}C=O) in enger Anlehnung an das für das Beispiel 104 beschriebene Verfahren (Stufe (ii)) hergestellt. Die Umsetzungen wurden dünnschichtchromatographisch verfolgt und hatten Reaktionszeiten von 1 - 15 h. Die eingesetzten Ketone sind kommerziell verfügbar.

| **Beispiel Nr.** | | **Keton (R^{a}R^{b}C=O)** | **Ausbeute (%)** | **MS, m/z (MH⁺)** |
|---|---|---|---|---|
| 105 | | 4-Methylcyclohexanon | 20 | 536,3 |
| 134 | | 4-(Trifluormethyl)cyclohexanon | 19 | 590,3 |

### Umsetzung mit Aldehyden:

### Beispiel 117:

### 1-(4-((1H-Benzo[d]imidazol-2-yl)methyl)piperazin-1-yl)-2-((1-(4-methoxy-2,6-dimethylphenylsulfonyl)piperidin-2-yl)methoxy)ethanon

Stufe (ii): 2-((1-(4-Methoxy-2,6-dimethylphenylsulfonyl)piperidin-2-yl)methoxy)-1-(piperazin-1-yl)ethanon Hydrochlorid (Beispiel 102) (150 mg, 0,315 mmol) wurde in einem Gemisch aus Tetrahydrofuran (3 ml) und Triethylamin (0,052 ml, 0,378 mmol) suspendiert. Zu dieser Suspension wurde bei Raumtemperatur 1 H-Benzo[d]imidazol-2-carbaldehyd (55 mg, 0,378 mmol) gegeben und das resultierende Gemisch 10 min bei Raumtemperatur gerührt. Anschließend wurde Natriumtriacetoxyborhydrid (267 mg, 1,26 mmol) hinzugegeben und weitere 3 d bei Raumtemperatur gerührt. Das Reaktionsgemisch wurde mit gesättigter Natriumhydrogencarbonatlösung (5 ml) versetzt und mit Ethylacetat (4x10 ml) extrahiert. Die organische Phase wurde getrocknet (MgSO₄), unter Vakuum eingeengt und das Rohprodukt säulenchromatographisch (Kieselgel) mit Ethylacetat / Methanol / Ammoniaklösung (25 % aq) (100: 1: 1) gereinigt.
Ausbeute: 60 mg (33 %), gelber Feststoff
MS, *m*/*z* 570,3 (MH⁺)

Die in der nachfolgenden Tabelle aufgeführte Beispielverbindung wurde aus 2-((1-(4-Methoxy-2,6-dimethylphenylsulfonyl)piperidin-2-yl)methoxy)-1-(piperazin-1-yl)ethanon Hydrochlorid (Beispiel 102) durch Umsetzung mit dem entsprechenden Aldehyd (R^{a}R^{b}C=O) in enger Anlehnung an das für das Beispiel 117 beschriebene Verfahren (Stufe (ii)) hergestellt. Der eingesetzte Aldehyd ist kommerziell verfügbar.

| **Beispiel Nr.** | | **Aldehyd (R^{a}R^{b}C=O)** | **Ausbeute (%)** | **MS, m/z (MH⁺)** |
|---|---|---|---|---|
| 166 | | Chinoxalin-6-carbaldehyd | 90 | 582,2 |

### Umsetzung von 2-((1-(4-Methoxy-2,6-dimethylphenylsulfonyl)piperidin-2-yl)methoxy)-1-(piperazin-1-yl)ethanon mit Aldehyden (R^{a}HC=O):

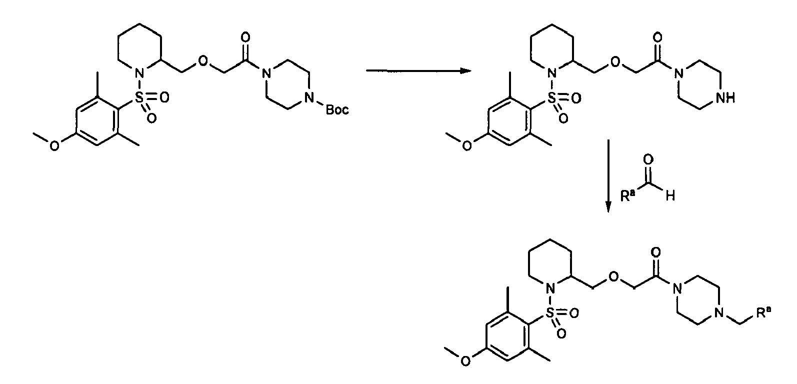

### 2-((1-(4-Methoxy-2,6-dimethylphenylsulfonyl)piperidin-2-yl)methoxy)-1-(piperazin-1-yl)ethanon

Stufe (i): Zu einer Lösung tert-Butyl 4-(2-((1-(4-methoxy-2,6-dimethylphenylsulfonyl)piperidin-2-yl)methoxy)acetyl)piperazin-1-carboxylat (Beispiel 101) (1 äq) in Dichloromethan (10 ml/mmol) wurde bei 0 °C Trifluoressigsäure (13 äq) gegeben. Das Reaktionsgemisch wurde 2 h bei Raumtemperatur gerührt und anschließend unter Vakuum eingeengt. Das Rohprodukt wurde ohne weitere Aufreinigung in der nachfolgenden Synthesestufe eingesetzt.

### Beispiel 197:

### 1-(4-((5-Chlor-2-phenyl-1H-imidazol-4-yl)methyl)piperazin-1-yl)-2-((1-(4-methoxy-2,6-dimethylphenylsulfonyl)piperidin-2-yl)methoxy)ethanon

Stufe (ii): Zu einer Lösung 2-((1-(4-Methoxy-2,6-dimethylphenylsulfonyl)piperidin-2-yl)methoxy)-1-(piperazin-1-yl)ethanon (1 äq) in Dichlormethan (25 ml/mmol) wurden 4-Chlor-2-phenyl-1H-imidazol-5-carbaldehyd (1,5 äq) und Essigsäure (kat.) gegeben. Das Reaktionsgemisch wurde 30 min bei 25°C gerührt, dann mit Natriumtriacetoxyborhydrid (4 äq) versetzt und weitere 16 h bei 25 °C gerührt. Das Gemisch wurde mit Dichlormethan verdünnt und mit gesättigter Bicarbonatlösung und gesättigter Natriumchloridlösung gewaschen. Die organische Phase wurde getrocknet (Na₂SO₄) und unter Vakuum eingeengt. Das Rohprodukt wurde säulenchromatographisch mit 3 % Methanol in Ethylacetat gereinigt.
Ausbeute: 50 %
MS, *m*/*z* 630,2 (MH⁺)

Die in der nachfolgenden Tabelle aufgeführten Beispielverbindungen wurden aus 2-((1-(4-Methoxy-2,6-dimethylphenylsulfonyl)piperidin-2-yl)methoxy)-1-(piperazin-1-yl)ethanon durch Umsetzung mit den entsprechenden Aldehyden (R^{a}HC=O) in enger Anlehnung an das für das Beispiel 197 beschriebene Verfahren (Stufe (ii)) hergestellt. Die eingesetzten Aldehyde sind kommerziell verfügbar, können nach dem Fachmann bekannten Methoden hergestellt werden oder wurden nach beschriebenen Verfahren synthetisiert.

| **Beispiel Nr.** | | **Aldehyd (R^{a}HC=O)** | **Ausbeute (%)** | **MS, m/z (MH⁺)** |
|---|---|---|---|---|
| 198 | | 1,5-Dimethyl-1H-pyrazol-4-carbaldehyd | 40 | 548,2 |
| 199 | | 2-(Dimethylamino)pyrimidin-5-carbaldehyd | 30 | 575,2 |
| 212 | | 2-(1-Methylpiperidin-4-yl)acetaldehyd | 40 | 565,3 |
| 214 | | 2-((4-Fluorphenyl)(methyl)amino)pyrimidin-5-carbaldehyd | 10 | 655,3 |

### Herstellung der Aldehyde:

### 2-((4-Fluorphenyl)(methyl)amino)pyrimidin-5-carbaldehyd

### (eingesetzt in der Synthese der Beispielverbindung 214)

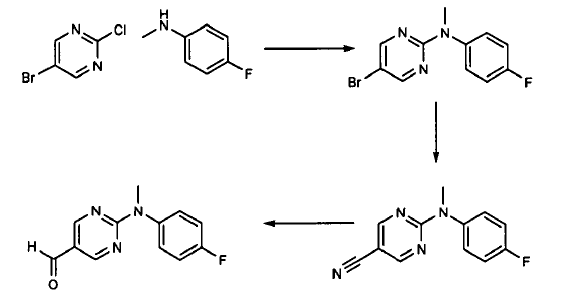

Stufe (i): Zu einer Lösung 5-Brom-2-Chlorpyrimidin (2,5 mmol) in Dimethylsulfoxid (6,5 ml) wurde 4-Fluor-N-methylanilin gegeben, gefolgt von Kaliumcarbonat (5 mmol). Die resultierende Lösung wurde 2 h auf 120°C erwärmt und der Reaktionsverlauf dünnschichtchromatographisch verfolgt. Nach vollständiger Umsetzung wurde das Reaktionsgemisch mit Ethylacetat extrahiert und die organische Phase mit Wasser und gesättigter Natriumchloridlösung gewaschen. Die organische Phase wurde getrocknet (Na₂SO₄), gefiltert und unter Vakuum eingeengt. Das Rohprodukt wurde säulenchromatographisch gereinigt (5 % Ethylacetat in Hexan).
Ausbeute: 35 %

Stufe (ii): Zu einer Lösung von 5-Brom-N-(4-fluorphenyl)-N-methylpyrimidin-2-amin (1 mmol) in Dimethylformamid (3 ml/mmol) wurde Kupfercyanid (2 äq) gegeben. Die resultierende Lösung wurde 18 h auf 100°C erwärmt und der Reaktionsverlauf dünnschichtchromatographisch verfolgt. Nach vollständiger Umsetzung wurde das Reaktionsgemisch mit Ethylacetat extrahiert und die organische Phase mit Wasser und gesättigter Natriumchloridlösung gewaschen. Die organische Phase wurde getrocknet (Na₂SO₄), gefiltert und unter Vakuum eingeengt. Das Rohprodukt wurde säulenchromatographisch gereinigt (20 % Ethylacetat in Hexan).
Ausbeute: 25 %

Stufe (iii): Zu einer kalten Lösung von 2-((4-Fluorphenyl)(methyl)amino)pyrimidin-5-carbonitril (0,5 mmol) in Benzol (8 ml) wurde Diisobutylaluminiumhydrid (1 M solution, 0,75 mmol) gegeben. Die resultierende Lösung wurde 4 h bei 25°C gerührt. Dann wurde das Reaktionsgemisch wieder auf 0 °C gekühlt und 10 %ige Salzsäure (5 ml) hinzugetropft. Anschließend wurde langsam auf 25°C erwärmt und 2 h gerührt. Das Reaktionsgemisch wurde mit gesättigter Bicarbonatlösung neutralisiert und mit Ethylacetat extrahiert. Die organische Phase wurde mit gesättigter Natriumchloridlösung gewaschen, getrocknet (Na₂SO₄), gefiltert und unter Vakuum eingeengt. Das Rohprodukt wurde direkt in der nächsten Synthesestufe eingesetzt.

### 2-(1-Methylpiperidin-4-yl)acetaldehyd

### (eingesetzt in der Synthese der Beispielverbindung 212)

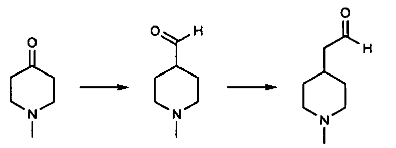

Stufe (i): Zu einer Lösung von Methoxy-methyl-triphenylphosphin (8,84 mmol) in trockenem Tetrahydrofuran (2 ml/mmol) wurde unter Rühren eine Lösung Kalium tert.-Butylat (13,27 mmol) in Tetrahydrofuran (2 ml/mmol) bei 0 °C unter Argon hinzugetropft. Die resultierende Lösung wurde 30 min bei 25 °C gerührt. Dann wurde das Reaktionsgemisch auf 0 °C gekühlt und eine Lösung 1-Methylpiperidin-4-on (4,42 mmol) in trockenem Tetrahydrofuran (2 ml/mmol) hinzugetropft. Das Gemisch wurde 16 h bei 25°C bis zur vollständigen Umsetzung gerührt. Dann wurde auf 0 °C gekühlt, 6 N Salzsäure (22 ml) hinzugetropft und 1 h gerührt. Die wässrige Phase wurde mit Diethylether (10 ml) gewaschen und anschließend mit 5 N Natriumhydroxidlösung basisch gestellt und mit Dichlormethan (4 x 75 ml) extrahiert. Die organische Phase wurde mit gesättigter Natriumchloridlösung gewaschen, getrocknet (Na₂SO₄), gefiltert und unter Vakuum eingeengt. Das Rohprodukt wurde direkt in der nächsten Synthesestufe eingesetzt.
Ausbeute: 95 %

Stufe (ii): Zu einer Lösung von Methoxy-methyl-triphenylphosphin (7,8 mmol) in trockenem Tetrahydrofuran (2 ml/mmol) wurde unter Rühren eine Lösung Kalium tert.-butylat (11,7 mmol) in Tetrahydrofuran (2 ml/mmol) bei 0 °C unter Argon hinzugetropft. Die resultierende Lösung wurde 30 min bei 25 °C gerührt. Dann wurde das Reaktionsgemisch auf 0 °C gekühlt und eine Lösung 1-Methylpiperidin-4-carbaldehyd (3.9 mmol) in trockenem Tetrahydrofuran (2 ml/mmol) wurde hinzugetropft. Das Gemisch wurde 16 h bei 25°C bis zur vollständigen Umsetzung gerührt. Dann wurde auf 0 °C gekühlt, 6 N Salzsäure (22 ml) hinzugetropft und 1 h gerührt. Die wässrige Phase wurde mit Diethylether (10 ml) gewaschen und anschließend mit 5 N Natriumhydroxidlösung basisch gestellt und mit Dichlormethan (4 x 75 ml) extrahiert. Die organische Phase wurde mit gesättigter Natriumchloridlösung gewaschen, getrocknet (Na₂SO₄), gefiltert und unter Vakuum eingeengt. Das Rohprodukt wurde direkt in der nächsten Synthesestufe eingesetzt.
Ausbeute: 95 %

### Umsetzung von 1-(2-((1-(4-Methoxy-2,6-dimethylphenylsulfonyl)piperidin-2-yl)methoxy)acetyl)piperidin-4-on (Beispiel 116) mit Aminen (R^{c}R^{d}NH):

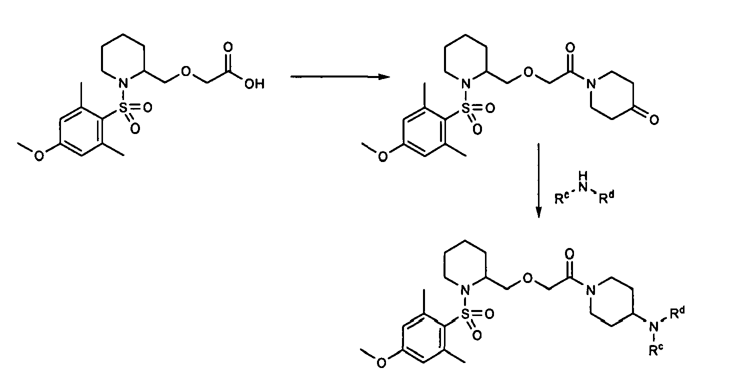

### Beispiel 116:

### 1-(2-((1-(4-Methoxy-2,6-dimethylphenylsulfonyl)piperidin-2-yl)methoxy)acetyl)piperidin-4-on

Stufe (i): Zu einer Lösung 2-((1-(4-Methoxy-2,6-dimethylphenylsulfonyl)pyrrolidin-2-yl)methoxy)essigsäure (Säurebaustein S27) (1,0 g, 2,692 mmol) in N,N-Dimethylformamid (20 ml) wurde 4-Piperidon-monohydrat-hydrochlorid (260 mg, 2,692 mmol), Triethylamin (0,560 ml, 4,038 mmol) und anschließend 4-Methylmopholin (1,62 ml, 14,805 mmol) hinzugegeben. Das Gemisch wurde dann mit Benzotriazol-1-yloxytris(dimethylamino)phosphonium Hexafluorphosphat (1,42 g, 3,230 mmol) versetzt und 3 d bei Raumtemperatur gerührt. Dann wurde unter Vakuum eingeengt und der Rückstand in Ethylacetat (30 ml) und gesättigter Natriumhydrogencarbonatlösung (20 ml) aufgenommen und die wässrige Phase mit Ethylacetat extrahiert (4 x 10 ml). Die vereinigten organischen Phasen wurden mit gesättigter Natriumhydrogencarbonatlösung (20 ml) und gesättigter Natriumchloridlösung (20 ml) gewaschen, getrocknet (MgSO₄) und unter Vakuum eingeengt. Das Rohprodukt wurde säulenchromatographisch (Kieselgel) mit Diethylether / Dichlormethan / Ammoniaklösung (25 % aq) (100:100:2) aufgereinigt.
Ausbeute: 430 mg (35 %)
MS, *m*/*z* 540,3 (MH⁺)

### Beispiel 130:

### 1-(4-Fluor-1,4'-bipiperidin-1'-yl)-2-((1-(4-methoxy-2,6-dimethylphenylsulfonyl)piperidin-2-yl)methoxy)ethanon

Stufe (ii): 1-(2-((1-(4-Methoxy-2,6-dimethylphenylsulfonyl)piperidin-2-yl)methoxy)acetyl)piperidin-4-on (Beispiel 116) (100 mg, 0,221 mmol) und 4-Fluorpiperidin Hydrochlorid (30 mg, 0,221 mmol) wurden in einem Gemisch aus 1,2-Dichlorethan (4 ml) und Triethylamin (0,036 ml, 0,265 mmol) gelöst. Zu diesem Gemisch wurden bei Raumtemperatur Natriumtriacetoxyborhydrid (66 mg, 0,309 mmol) und Essigsäure (0,013 ml, 0,221 mmol) gegeben. Das Reaktionsgemisch wurde 15 h bei Raumtemperatur gerührt und anschließend mit gesättigter Natriumhydrogencarbonatlösung (5 ml) versetzt. Die wässrige Phase wurde mit Diethylether (2 x 20 ml) extrahiert und die organische Phase wiederum mit gesättigter Natriumchloridlösung (10 ml) gewaschen. Die organische Phase wurde getrocknet (MgSO₄) unter Vakuum eingeengt und das Rohprodukt säulenchromatographisch (Kieselgel) mit Ethylacetat / Methanol (20:1) gereinigt.
Ausbeute: 70 mg (59 %)
MS, *m*/*z* 540,3 (MH⁺)

Die in der nachfolgenden Tabelle aufgeführten Beispielverbindungen wurden aus 1-(2-((1-(4-Methoxy-2,6-dimethylphenylsulfonyl)piperidin-2-yl)methoxy)acetyl)piperidin-4-on (Beispiel 116) durch Umsetzung mit den entsprechenden Aminen (R^{c}R^{d}NH) in enger Anlehnung an das für das Beispiel 130 beschriebene Verfahren (Stufe (ii)) hergestellt. Die Umsetzungen wurden dünnschichtchromatographisch verfolgt und hatten Reaktionszeiten von ca. 15 h. In einigen Fällen wurde zusätzliches Natriumtriacetoxyborhydrid nachdosiert. Sofern das Amin nicht als Hydrochlorid (xHCl) vorlag wurde auf die Zugabe von Triethylamin verzichtet. Die eingesetzten Amine sind kommerziell verfügbar, können nach dem Fachmann bekannten Methoden hergestellt werden oder wurden nach beschriebenen Verfahren synthetisiert.

| **Beispiel Nr.** | | **Aminen (R^{c}R^{d}NH)** | **Ausbeute (%)** | **MS, m/z (MH⁺)** |
|---|---|---|---|---|
| 136 | | 2-Morpholino-2-(pyridin-3-yl)ethanamin | 79 | 644,4 |
| 144 | | 5,6,7,8-Tetrahydroimidazo[1,2-a]Pyrazin | 70 | 560,2 |
| 145 | | 5,6,7,8-Tetrahydro-[1,2,4]triazolo[1,5-a]pyrazin | 75 | 561,2 |

### Herstellung der Amine:

### 5,6,7,8-Tetrahydroimidazo[1,2-a]pyrazin

### (eingesetzt in der Synthese der Beispielverbindung 144)

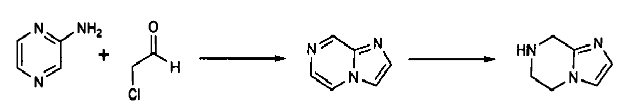

Stufe (i): Ein Gemisch aus 2-Aminopyrazin (25 g, 262,9 mmol) and Chloracetaldehyd (50 % Lösung in Wasser, 50 ml, 394 mmol) wurde in Gegenwart von Natriumhydrogencarbonat (33,1 g, 394 mmol) 2 d auf 100°C erhitzt. Das Reaktionsgemisch wurde auf Raumtemperatur gekühlt und mit gesättigter Kaliumcarbonatlösung (100 ml) versetzt. Anschließend wurde mit Dichlormethan extrahiert, die organische Phase getrocknet (Na₂SO₄) und eingeengt. Die Aufreinigung erfolgte per Säulenchromatographie (Dichlormethan / Methanol 95:5 + 5 % NH₄OH [35 %]).
Ausbeute: 7,6 g (24 %)

Stufe (ii): Imidazo[1,2-a]pyrazin (7,2 g, 60,44 mmol) wurde in 2-Methoxyethanol (100 ml) gelöst. Platinum (IV) Oxid (1,2 g, 5,13 mmol) wurde hinzugegeben und das Gemisch über Nacht bei Raumtemperatur unter einer Wasserstoffatmosphäre von 4 Bar in einem Autoclaven gerührt. Das Reaktionsgemisch wurde mit Stickstoff geflutet, über Celite filtriert, eingeengt und mit Toluol co-evaporiert. Die Aufreinigung erfolgte per Säulenchromatographie (Dichlormethan / 7 N Ammoniak in Methanol 95:5).
Ausbeute: 5,7 g (76 %)

### 5,6,7,8-Tetrahydro-[1,2,4]triazolo[1,5-a]pyrazin

### (eingesetzt in der Synthese der Beispielverbindung 145)

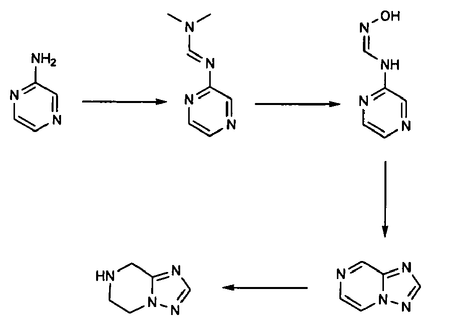

Stufe (i): Zu einer Lösung Pyrazin-2-amin (18,98 g, 200 mmol) in Toluol (110 ml) wurde N,N-Dimethylformamid dimethyl acetal (29,5 ml, 220 mmol) gegeben und das Gemisch 2,25 h refluxiert. Das Reaktionsgemisch wurde auf Raumtemperatur gekühlt, eingeengt und mit Toluol co-evaporiert.
Ausbeute: 32,89 g (100%)

Stufe (ii): Eine Lösung Hydroxylamin Hydrochlorid (17,0 g, 245 mmol) in Methanol (150 ml) wurde zu einer Suspension aus (E)-N,N-Dimethyl-N'-(pyrazin-2-yl)formimidamid (38,27 g, 233 mmol) und Natriumacetat (20,1 g, 245 mmol) in Methanol (450 ml) unter Eiskühlung hinzugetropft. Das Reaktionsgemisch wurde 4 h bei 0 °C gerührt und anschließend auf Raumtemperatur erwärmt und aufkonzentriert. Der Rückstand wurde mit Dichlormethan / 7 M Ammoniaklösung in Methanol (∼9:1) trituriert, der gewünschte Feststoff abgefiltert, und mit Dichlormethan / 7 M Ammoniaklösung in Methanol (∼9:1) gewaschen. Das Filtrat wurde aufkonzentriert, mit Ethanol co-evaporiert und anschließend aus Ethanol kristallisiert. Durch weitere Umkristallisation der gewonnenen Feststoffe aus Ethanol erhielt man schließlich das aufgereinigte Produkt.
Ausbeute: 24,55 g (76 %)

Stufe (iii): Polyphosphorsäure (250 g) wurde zu (Z)-N'-Hydroxy-N-(pyrazin-2-yl)formimidamid (25,07 g, 181 mmol) gegeben und das Reaktionsgemisch anschließend sofort auf 90°C erwärmt. Das Gemisch wurde 4 h gerührt, das heiße Reaktionsgemisch auf Eiswasser gegeben und mit Natriumhydrogencarbonat basisch gestellt. Die wässrige Phase wurde mit Dichlormethan extrahiert (11, 3 x 0,5 l) und die vereinigten organischen Phasen getrocknet (Na₂SO₄) und aufkonzentriert. Das Rohprodukt wurde aus Ethanol in mehreren Chargen kristallisiert.
Ausbeute: 18,10 g (83 %)

Stufe (iv): Platin(IV) oxid (2,75 g, 12.1 mmol) wurde unter Stickstoff zu einer Suspension aus Calciumoxid (9,30 g, 166 mmol) und [1,2,4]Triazolo[1,5-a]pyrazin (18,10 g, 151 mmol) in 2-Methoxyethanol (150 ml) gegeben. Das Reaktionsgemisch wurde 21,5 h unter einer Wasserstoffatmosphäre gerührt, über Celite filtriert, und mit Dichlormethan / Ethanol (9:1) gewaschen. Das Filtrat wurde aufkonzentriert, mit Toluol und Diisopropylether co-evaporiert und anschließend in Ethylacetat gelöst, über Celite filtriert, mit Ethylacetat gewaschen und wiederum aufkonzentriert. Der Rückstand wurde in heißem Diisopropylether gelöst, gefiltert, mit Diisopropylether gewaschen und 7 h unter Vakuum eingeengt.
Ausbeute: 17,12 g (92 %)

### Herstellung von (S)-2-((1-(4-Methoxy-2,6-dimethylphenylsulfonyl)piperidin-2-yl)methoxy)acetamid-Derivaten:

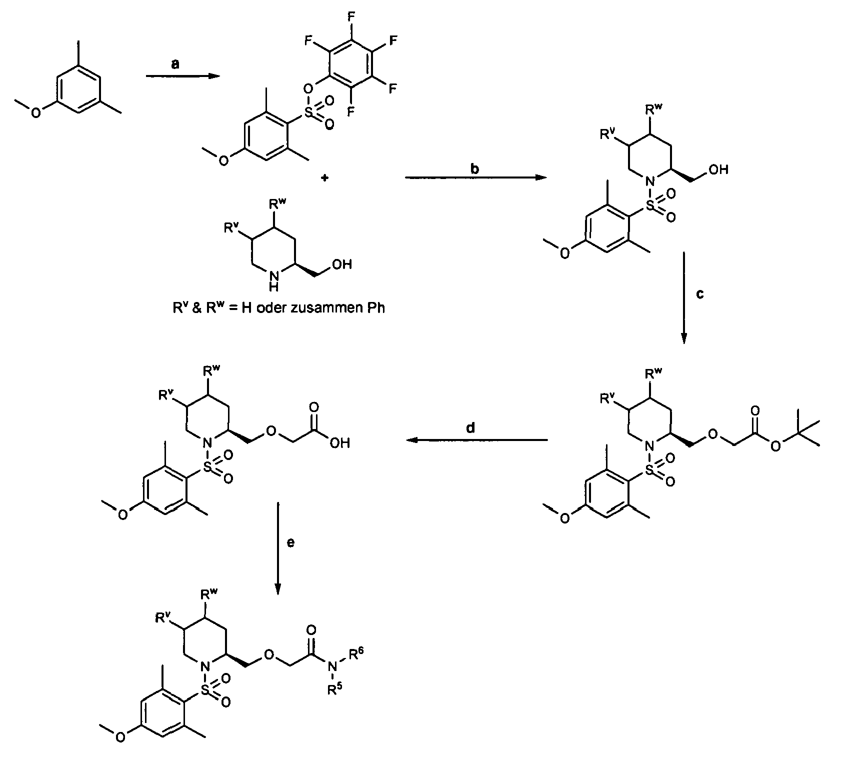

### Beispiel 127:

### (S)-2-((2-(4-Methoxy-2,6-dimethylphenylsulfonyl)-1,2,3,4-tetrahydroisochinolin-3-yl)methoxy)-1-(4-(1-methylpiperidin-4-yl)piperazin-1-yl)ethanon

Stufe (a): Zu einer auf 0 °C gekühlten Lösung 3,5-Dimethylanisol (3,1 g, 22,03 mmol) in Dichlormethan (50 ml) wurde Chlorsulfonsäure (7,3 ml, 110,13 mmol) in Dichlormethan (60 ml) langsam über 10 min hinzugetropft. Das Reaktionsgemisch wurde weitere 10 min gerührt und anschließend langsam in Eiswasser (300 ml) getropft und solange gerührt bis das Eis geschmolzen war. Die Phasen wurden getrennt und die wässrige Phase mit Dichlormethan (50 ml) extrahiert. Die vereinigten organischen Phasen wurden mit gesättigter Natriumchloridlösung (50 ml) gewaschen, getrocknet (Na₂SO₄) und unter Vakuum eingeengt. Eine Lösung Pentafluorphenol (4,1 g, 22,03 mmol) und Triethylamin (6,1 ml, 44,05 mmol) in Dichlormethan (50 ml) wurde 30 min bei Raumtemperatur gerührt. Anschließend wurde eine Lösung des hergestellten Sulfonylchlorides in Dichlormethan (50 ml) langsam hinzugetropft. Das Reaktionsgemisch wurde 1 h bei Raumtemperatur gerührt. Zu dem Gemisch wurde gesättigter Natriumhydrogencarbonatlösung (50 ml) gegeben, die organische Phase mit gesättigter Natriumchloridlösung (50 ml) gewaschen, getrocknet (Na₂SO₄) und unter Vakuum eingeengt. Das Rohprodukt wurde säulenchromatographisch (Kieselgel) mit Hexan / Diethylether / Dichlormethan (20:1:2) aufgereinigt.
Ausbeute: 6,1 g (72 %)
[Das unerwünschte Regioisomer wurde in einer Ausbeute von 14 % isoliert.]

Stufe (b): Zu einer Lösung des Aminoalkohols (S)-(1,2,3,4-Tetrahydroisochinolin-3-yl)methanol (960 mg, 5,89 mmol) in N,N-Dimethylformamid (15 ml) wurden Perfluorophenyl 4-methoxy-2,6-dimethylbenzolsulfonat (1,5 g, 3,92 mmol) und Tetra-n-butylammoniumchlorid (2,18 g, 7,85 mmol) gegeben. Das Reaktionsgemisch wurde 1 h auf 120°C erwärmt. Anschließend wurde unter Vakuum eingeengt, der Rückstand in Ethylacetat (50 ml) aufgenommen und mit 10 % wässriger Ammoniumchloridlösung (20 ml) gewaschen. Die organische Phase wurde getrocknet (Na₂SO₄) und unter Vakuum eingeengt. Das Rohprodukt wurde säulenchromatographisch (Kieselgel) mit Hexan / Diethylether / Dichlormethan (3:2:2) aufgereinigt.
Ausbeute: 1,2 g (85 %)

Stufe (c): Zu einem Gemisch aus Tetra-n-butylammoniumhydrogensulfat (113 mg, 0,332 mmol), wässriger Natriumhydroxidlösung (6,64 g, 165,98 mmol in Wasser (7 ml)) und Toluol (5 ml) wurde bei Raumtemperatur tert-Butyl-2-bromoacetat (1,02 ml, 6,07 mmol) gegeben und anschließend auf 0 °C gekühlt. Dann wurde eine Lösung (S)-(2-(4-Methoxy-2,6-dimethylphenylsulfonyl)-1,2,3,4-tetrahydroisochinolin-3-yl)methanol (1,2 g, 3,32 mmol) in Toluol (5 ml) langsam hinzugegeben. Das Reaktionsgemisch wurde auf Raumtemperatur erwärmt und dann 1 h bei dieser Temperatur gerührt. Die Phasen wurden getrennt und die wässrige Phase mit Diethylether extrahiert (2 x 20 ml). Die vereinigten organischen Phasen wurden mit gesättigter Natriumchloridlösung (20 ml) gewaschen, getrocknet (Na₂SO₄), unter Vakuum eingeengt. Das Rohprodukt wurde ohne weitere Aufreinigung in der nächsten Stufe eingesetzt.
Ausbeute: 1,79 g (>99 %)

Stufe (d): (S)-tert-Butyl 2-((2-(4-methoxy-2,8-dimethylphenylsulfonyl)-1,2,3,4-tetrahydroisochinolin-3-yl)methoxy)acetat (1,58 g, 3,32 mmol) wurde in Tetrahydrofuran (10 ml) gelöst und Natriumhydroxidlösung (531 mg, 13,28 mmol in Wasser (0,5 ml)) hinzugegeben. Das Reaktionsgemisch wurde 2 h refluxiert, anschließend wieder auf Raumtemperatur gekühlt und mit Wasser (20 ml) versetzt. Der pH-Wert der wässrigen Phase wurde mit 2 M Salzsäure auf pH 2 eingestellt und es wurde mit Ethylacetat (3 x 20 ml) extrahiert. Die vereinigten organischen Phasen wurden getrocknet (Na₂SO₄) und unter Vakuum eingeengt. Das Rohprodukt wurde ohne weitere Aufreinigung in der nächsten Stufe eingesetzt.
Ausbeute: 580 mg (42 %)

Stufe (e): Zu einer Lösung (S)-2-((2-(4-Methoxy-2,6-dimethylphenylsulfonyl)-1,2,3,4-tetrahydroisochinolin-3-yl)methoxy)essigsäure (150 mg, 0,358 mmol) in N,N-Dimethylformamid (5 ml) wurden 1-(1-Methylpiperidin-4-yl)piperazin (65 mg, 0,358 mmol) und 4-Methylmopholin (0,117 ml, 1,073 mmol) gegeben. Das Gemisch wurde mit Benzotriazol-1-yloxytris(dimethylamino)phosphonium Hexafluorphosphat (188 mg, 0,429 mmol) versetzt und 15 h bei Raumtemperatur gerührt. Dann wurde unter Vakuum eingeengt, der Rückstand in Ethylacetat (20 ml) und gesättigter Natriumhydrogencarbonatlösung (10 ml) aufgenommen und die wässrige Phase mit Ethylacetat extrahiert (20 ml). Die vereinigten organischen Phasen wurden getrocknet (Na₂SO₄) und unter Vakuum eingeengt. Das Rohprodukt wurde säulenchromatographisch (Kieselgel) mit Diethylether / Dichlormethan / Methanol / Ammoniaklösung (25 % aq) (20:10:10:0,4) aufgereinigt.
Ausbeute: 90 mg (43 %), oranges Öl
MS, *m*/*z* 595,2 (MH⁺)

### Beispiel 185:

### (S)-2-((2-(4-Methoxy-2,6-dimethylphenylsulfonyl)-1,2,3,4-tetrahydroisochinolin-3-yl)methoxy)-1-(4-((1-methylpiperidin-4-yl)methyl)piperazin-1-yl)ethanon

Stufe (e): Eine Lösung (S)-2-((2-(4-Methoxy-2,6-dimethylphenylsulfonyl)-1,2,3,4-tetrahydroisochinolin-3-yl)methoxy)essigsäure (Stufe d, Beispiel 127 ) (325 mg, 0,775 mmol) in Dichlormethan (5 ml) wurde mit N,N'-Carbonyldiimidazol (131 mg, 0,813 mmol) versetzt und 2 h bei 30 °C gerührt. Anschließend wurde bei Raumtemperatur eine Lösung von 1-((1-Methylpiperidin-4-yl)methyl)piperazin (152 mg, 0,775 mmol) in Dichlormethan (5 ml) zugegeben und das Reaktionsgemisch 15 h bei dieser Temperatur gerührt. Das Gemisch wurde mit gesättigter Natriumhydrogencarbonatlösung (10 ml) versetzt und die wässrige Phase mit Dichlormethan (20 ml) extrahiert. Die vereinigten organischen Phasen wurden getrocknet (Na₂SO₄) und unter Vakuum eingeengt. Das Rohprodukt wurde säulenchromatographisch (Kieselgel) mit Ethylacetat / Methanol / Ammoniaklösung (25 % aq) (40:10:0,5) gereinigt.
Ausbeute: 263 mg (57 %)
MS, *m*/*z* 599,3 (MH⁺)

Die in der nachfolgenden Tabelle aufgeführten Beispielverbindungen wurden aus den entsprechenden Edukten in enger Anlehnung an das für das Beispiel 185 beschriebene Verfahren hergestellt. Der jeweilige Reaktionsverlauf wurde dünnschichtchromatographisch verfolgt und hierauf basierend die Reaktionszeiten bei analogen Umsetzungen entsprechend angepasst. Die Reaktionstemperaturen und eingesetzten Mengenäquivalente der Reagenzien können bei analogen Umsetzungen geringfügig abweichen. Die eingesetzten Edukte sind kommerziell verfügbar oder wurden wie beschrieben hergestellt.

| **Beispiel Nr.** | | **Aminoalkohol** | **Amin (R⁵R⁶NH)** | **Ausbeute (%) (über 5 Stufen)** | **MS, m/z (MH⁺)** |
|---|---|---|---|---|---|
| 186³ | | (S)-Piperidin-2-ylmethanol⁴ | 4-(Piperidin-4-yloxy)pyridin Dihydrochlorid⁵ | 14 | 532,2 |
| 187 | | (S)-(1,2,3,4-Tetrahydroisochinol in-3-yl)methanol | 4-(2-(Pyrrolidin-1-yl)ethyl)piperidin | 18 | 584,3 |

| | | | | | |
|---|---|---|---|---|---|
| ³Die Hydrochloridfällung erfolgte aus einer Methylethylketon Lösung der freien Base unter Zugabe von 2 M Chlorwasserstoff Lösung in Diethylether (5 äq). ⁴ Der eingesetzte (S)-Aminoalkohol wurde wie folgt hergestellt: Zu einer auf 0 °C gekühlten Suspension der Carbonsäure (1 äq) in Tetrahydrofuran (4 ml/mmol) wurde Borwasserstoff-Tetrahydrofuran-Komplex (3 äq, 1 M Lösung in Tetrahydrofuran) zugetropft und das Gemisch anschließend 1 h bei Raumtemperatur gerührt. Dann wurde das Reaktionsgemisch 4 h refluxiert und weitere 15 h bei Raumtemperatur gerührt. Zu dem auf 0 °C gekühlten Gemisch wurde 3 M Natriumhydroxidlösung gegeben, dann wurde 6 h refluxiert. Das Gemisch wurde mit Dichlormethan extrahiert (4 x) und die vereinigten organischen Phasen getrocknet (Na₂SO₄) und unter Vakuum eingeengt. Das Rohprodukt wurde ohne Aufreinigung in der nachfolgenden Synthesestufe eingesetzt. ⁵Das Amin R⁵R⁶NH wurde analog zu 3-(Piperidin-4-yloxy)pyridin Hydrochlorid (eingesetzt in der Synthese der Beispielverbindung 165) hergestellt. [analoges Verfahren siehe auch: J. Chao et al., Tetrahedron Lett., 2007, 48, 791] | | | | | |

### Umsetzung von substituierten (S)-2-((2-(Phenylsulfonyl)-1,2,3,4-tetrahydroisochinolin-3-yl)methoxy)essigsäure-Derivaten mit Aminen (R⁵R⁶NH):

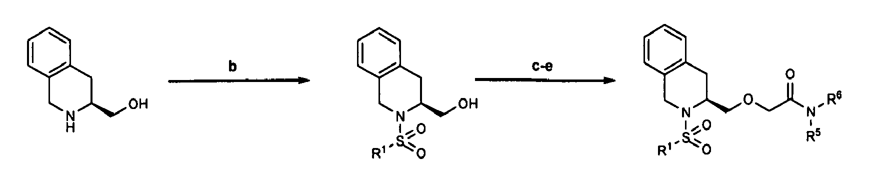

### Beispiel 133:

### (S)-2-((2-(2,4-Dichlorphenylsulfonyl)-1,2,3,4-tetrahydroisochinolin-3-yl)methoxy)-1-(4-(1-methylpiperidin-4-yl)piperazin-1-yl)ethanon

Die Synthese dieser Verbindung wurde größtenteils in Analogie zu der für das Beispiel 185 beschriebenen Synthese hergestellt. Allerdings entfiel Synthesestufe (a) und Synthesestufe (b) wurde wie folgt durchgeführt:
Stufe (b): Zu einer Lösung (S)-(1,2,3,4-Tetrahydroisochinolin-3-yl)methanol (1,0 g, 6,13 mmol) in Dichlormethan (10 ml) wurde Triethylamin (1,27 ml, 9,19 mmol) gegeben und das Gemisch 5 min bei Raumtemperatur gerührt. Anschließend wurde bei 0 °C eine Lösung 2,4-Dichlorbenzol-1-sulfonyl chlorid (1,35 g, 5,51 mmol) in Dichlormethan (10 ml) hinzugetropft. Das Reaktionsgemisch wurde auf Raumtemperatur erwärmt und1 h bei dieser Temperatur gerührt. Dann wurde das Gemisch mit gesättigter Natriumhydrogencarbonatlösung (20 ml) versetzt und die wässrige Phase mit Dichlormethan (30 ml) extrahiert. Die vereinigten organischen Phasen wurden getrocknet (Na₂SO₄), unter Vakuum eingeengt und das Rohprodukt anschließend säulenchromatographisch (Kieselgel) mit Hexan / Diethylether / Dichlormethan (1: 1: 1) gereinigt.
Ausbeute: 1,59 g (70 %)
Die Synthesestufen (c) bis (e) wurden in Analogie zu den für das Beispiel 185 beschriebenen Verfahren durchgeführt. Das Beispiel 133 (klares Öl) wurde auf diese Weise in einer Ausbeute von 85 % über 3 Stufen erhalten.
MS, *m*/*z* 585,3 (MH⁺)

Die in der nachfolgenden Tabelle aufgeführten Beispielverbindungen wurden analog zu Beispiel 133 aus (S)-(1,2,3,4-Tetrahydroisochinolin-3-yl)methanol, dem entsprechenden Sulfonylchlorid (R¹ SO₂Cl) und dem entsprechenden Amin (R⁵R⁶NH) hergestellt.

| **Beispiel Nr.** | | **Sulfonylchlorid (R¹SO₂Cl)** | **Amin (R⁵R⁶NH)** | **Ausbeute (%) (über 4 Stufen)** | **MS, m/z (MH⁺)** |
|---|---|---|---|---|---|
| 142 | | 4-Methoxybenzol-1-sulfonyl chlorid | 1-(1-ethylpiperidin-4-yl)piperazin | 62 | 557,2 |
| 182 | | 4-Methoxybenzol-1-sulfonyl chlorid | 4-(2-(Pyrrolidin-1-yl)ethyl)piperidin | 56 | 556,2 |
| 183 | | 2,4-Dichlorbenzol-1-sulfonyl chlorid | 4-(2-(Pyrrolidin-1-yl)ethyl)pipendin | 20 | 594,1 |
| 184 | | 2,4-Dichlorbenzol-1-sulfonyl chlorid | 1-((1-Methylpiperidin-4-yl)methyl)piperazin | 28 | 609,2 |
| 189⁶ | | 4-Methoxybenzol-1-sulfonyl chlorid | 1-((1-Methylpiperidin-4-yl)methyl)piperazin | 14 | 571,3 |

| | | | | | |
|---|---|---|---|---|---|
| ⁶ Die korrespondierende Base wurde wie folgt in das entsprechende Dihydrochlorid (2 x HCl) überführt: Die freie Base wurde in einer geringen Menge Dichlormethan / Diethylether (1:5) gelöst, mit 2 M Chlorwasserstoff Lösung in Diethylether (3 äq) versetzt und das entstandene Dihydrochlorid abfiltriert. | | | | | |

### Beispiel 135:

### (S)-2-((1-(4-Methoxy-2,6-dimethylphenylsulfonyl)azetidin-2-yl)methoxy)-1-(4-(1-methylpiperidin-4-yl)piperazin-1-yl)ethanon

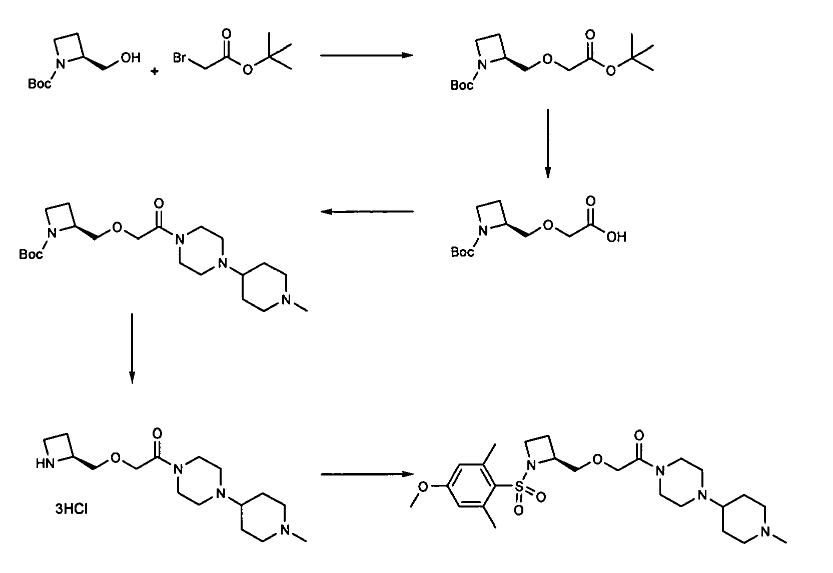

Stufe (i): Zu einem Gemisch aus Tetra-n-butylammoniumhydrogensulfat (122 mg, 0,363 mmol), wässriger Natriumhydroxidlösung (7,27 g, 181,7 mmol in Wasser (7 ml)) und Toluol (5 ml) wurde bei Raumtemperatur tert-Butyl-2-bromoacetat (0,799 ml, 5,45 mmol) gegeben und anschließend auf 0 °C gekühlt. Dann wurde eine Lösung (S)-tert-Butyl 2-(hydroxymethyl)azetidin-1-carboxylat (680 mg, 3,63 mmol) in Toluol (5 ml) langsam hinzugegeben. Das Reaktionsgemisch wurde auf Raumtemperatur erwärmt und 1 h bei dieser Temperatur gerührt. Die Phasen wurden getrennt und die wässrige Phase mit Diethylether extrahiert (2 x 20 ml). Die vereinigten organischen Phasen wurden mit gesättigter Natriumchloridlösung (20 ml) gewaschen, getrocknet (Na₂SO₄) und unter Vakuum eingeengt. Das Rohprodukt wurde säulenchromatographisch (Kieselgel) mit Hexan / Diethylether / Dichlormethan (2: 1: 1) gereinigt.
Ausbeute: 910 mg (83 %)

Stufe (ii): (S)-tert-Butyl 2-((2-tert-butoxy-2-oxoethoxy)methyl)azetidin-1-carboxylat (890 mg, 2,95 mmol) wurde in Tetrahydrofuran (10 ml) gelöst und Natriumhydroxid (708 mg, 17,72 mmol in Wasser (1 ml)) wurde hinzugegeben. Das Reaktionsgemisch wurde 2 h refluxiert, anschließend wieder auf Raumtemperatur gekühlt und Wasser (20 ml) hinzugegeben. Der pH-Wert der wässrigen Phase wurde mit 2 M Salzsäure auf pH 2 eingestellt und es wurde mit Ethylacetat (3 x 20 ml) extrahiert. Die vereinigten organischen Phasen wurden getrocknet (Na₂SO₄) und unter Vakuum eingeengt. Das Rohprodukt wurde ohne weitere Aufreinigung in der nachfolgenden Synthesestufe eingesetzt.
Ausbeute: 700 mg (97 %)

Stufe (iii): Zu einer Lösung (S)-2-((1-(tert-Butoxycarbonyl)azetidin-2-yl)methoxy)essigsäure (650 mg, 2,65 mmol) in N,N-Dimethylformamid (20 ml) wurden 1-(1-Methylpiperidin-4-yl)piperazin (433 mg, 2,39 mmol) und 4-Methylmopholin (0,798 ml, 7,95 mmol) gegeben. Das Gemisch wurde mit Benzotriazol-1-yloxytris(dimethylamino)phosphonium Hexafluorphosphat (1,39 mg, 3,18 mmol) versetzt und 15 h bei Raumtemperatur gerührt. Dann wurde unter Vakuum eingeengt, der Rückstand in Ethylacetat (20 ml) und gesättigter Natriumhydrogencarbonatlösung (10 ml) aufgenommen und die wässrige Phase mit Ethylacetat (2 x 20 ml) extrahiert. Die vereinigten organischen Phasen wurden getrocknet (Na₂SO₄) und unter Vakuum eingeengt. Das Rohprodukt wurde säulenchromatographisch (Kieselgel) mit Diethylether / Dichlormethan / Methanol / Ammoniaklösung (25 % aq) (20:10:10:0,4) aufgereinigt.
Ausbeute: 580 mg (53 %)

Stufe (iv): Zu einer Lösung (S)-tert-Butyl 2-((2-(4-(1-methylpiperidin-4-yl)piperazin-1-yl)-2-oxoethoxy)methyl)azetidin-1-carboxylat (570 mg, 1,39 mmol) in Ethylacetat / Diethylether (2 ml; 2:5) wurde bei Raumtemperatur Chlorwasserstoff (3,47 ml, 6,94 mmol, 2 M Lösung in Diethylether) gegeben. Das Reaktionsgemisch wurde 2 h bei 40 °C gerührt und der entstandene Feststoff abfiltriert und getrocknet.
Ausbeute: 520 mg (89 %)

Stufe (v): Zu einer Lösung (S)-2-(Azetidin-2-ylmethoxy)-1-(4-(1-methylpiperidin-4-yl)piperazin-1-yl)ethanon Trihydrochlorid (100 mg, 0,238 mmol) in Tetrahydrofuran (10 ml) wurden 1,8-Diazabicyclo[5.4.0]-undec-7-en (DBU) (0,15 ml, 0,993 mmol) und Perfluorophenyl 4-methoxy-2,6-dimethylbenzolsulfonat (123 mg, 0,298 mmol) [Synthese siehe Beispiel 127] hinzugegeben. Das Reaktionsgemisch wurde 1 h refluxiert und 3 d bei Raumtemperatur gerührt. Anschließend wurde das Gemisch mit gesättigter Natriumhydrogencarbonatlösung (20 ml) und Ethylacetat (30 ml) versetzt. Die wässrige Phase wurde mit Ethylacetat (2 x 15 ml) extrahiert und die vereinigten organischen Phasen mit gesättigter Natriumchloridlösung (20 ml) gewaschen. Dann wurde die organische Phase getrocknet (Na₂SO₄), unter Vakuum eingeengt und das Rohprodukt anschließend säulenchromatographisch (Kieselgel) mit Diethylether / Dichlormethan / Methanol / Ammoniaklösung (25 % aq) (20:10:10:0,4) gereinigt.
Ausbeute: 40 mg (33 %), gelbes Harz
MS, *m*/*z* 509,3 (MH⁺)

### Umsetzung von Pyrrolidin-2-ylmethanol zu substituierten 2-((1-(Phenylsulfonyl)pyrrolidin-2-yl)methoxylacetamiden:

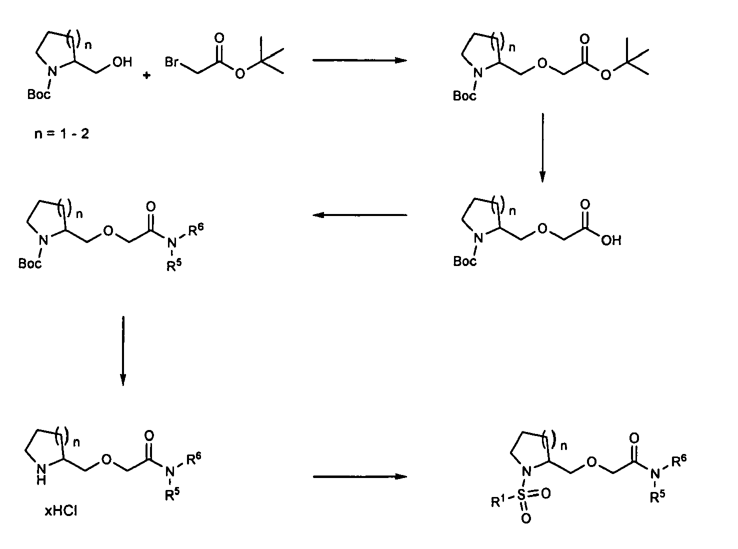

### Beispiel 140:

### (R)-2-((1-(4-Methoxy-2,3,6-trimethylphenylsulfonyl)pyrrolidin-2-yl)methoxy)-1-(4-(1-methylpiperidin-4-yl)piperazin-1-yl)ethanon Dihydrochlorid

Stufe (i): Zu einem Gemisch aus Tetra-n-butylammoniumhydrogensulfat (334 mg, 0,994 mmol), wässriger Natriumhydroxidlösung (9,93 g, 248,43 mmol in Wasser (10ml)) und Toluol (7,5 ml) wurde bei Raumtemperatur tert-Butyl-2-bromoacetat (2,19 ml, 14,91 mmol) gegeben und anschließend auf 0 °C gekühlt. Dann wurde eine Lösung des Aminoalkohols (R)-tert-Butyl 2-(hydroxymethyl)pyrrolidin-1-carboxylat (2,0 g, 9,94 mmol) in Toluol (7,55 ml) langsam hinzugegeben. Das Reaktionsgemisch wurde auf Raumtemperatur erwärmt und dann 2 h bei dieser Temperatur gerührt. Die Phasen wurden getrennt und die wässrige Phase mit Diethylether extrahiert (2 x 30 ml). Die vereinigten organischen Phasen wurden mit gesättigter Natriumchloridlösung (30 ml) gewaschen, getrocknet (Na₂SO₄), unter Vakuum eingeengt. Das Rohprodukt wurde mit Ethylacetat über Kieselgel filtriert und in der nächsten Stufe eingesetzt.
Ausbeute: 3,2 g (>99 %)

Stufe (ii): (R)-tert-Butyl 2-((2-tert-butoxy-2-oxoethoxy)methyl)pyrrolidin-1-carboxylat (3,2 g, 10,15 mmol) wurde in Tetrahydrofuran (25 ml) gelöst und Natriumhydroxidlösung (2,44 g, 60,88 mmol in Wasser (2,5 ml)) wurde hinzugegeben. Das Reaktionsgemisch wurde 2 h refluxiert, anschließend wieder auf Raumtemperatur gekühlt und Wasser (20 ml) hinzugegeben. Der pH-Wert der wässrigen Phase wurde mit 2 M Salzsäure auf pH 2 eingestellt und es wurde mit Ethylacetat (3 x 30 ml) extrahiert. Die vereinigten organischen Phasen wurden getrocknet (Na₂SO₄) und unter Vakuum eingeengt. Das Rohprodukt wurde ohne weitere Aufreinigung in der nachfolgenden Synthesestufe eingesetzt.
Ausbeute: 2,0 g (76 %)

Stufe (iii): Eine Lösung von (R)-2-((1-(tert-Butoxycarbonyl)pyrrolidin-2-yl)methoxy)essigsäure (500 mg, 1,93 mmol) in Dichlormethan (5 ml) wurde mit N,N'-Carbonyldiimidazol (328 mg, 1,93 mmol) versetzt und 30 min bei Raumtemperatur gerührt. Anschließend wurde bei Raumtemperatur eine Lösung von 1-(1-Methylpiperidin-4-yl)piperazin (353 mg, 1,93 mmol) in Dichlormethan (5 ml) zugegeben und das Reaktionsgemisch 15 h bei dieser Temperatur gerührt. Das Gemisch wurde danach mit gesättigter Natriumhydrogencarbonatlösung (10 ml) versetzt und anschließend die wässrige Phase mit Dichlormethan (20 ml) extrahiert. Die vereinigten organischen Phasen wurden getrocknet (Na₂SO₄) und unter Vakuum eingeengt. Das Rohprodukt wurde säulenchromatographisch (Kieselgel) mit Diethylether / Dichlormethan / Methanol / Ammoniaklösung (25 % aq) (20:10:10:0,4) gereinigt.
Ausbeute: 540 mg (66 %)

Stufe (iv): Zu einer Lösung (R)-tert-Butyl 2-((2-(4-(1-methylpiperidin-4-yl)piperazin-1-yl)-2-oxoethoxy)methyl)pyrrolidin-1-carboxylat (530 mg, 1,25 mmol) in Ethylacetat / Diethylether (30 ml; 1:2) wurde bei Raumtemperatur Chlorwasserstoff (6,24 ml, 12,48 mmol, 2 M Lösung in Diethylether) gegeben. Das Reaktionsgemisch wurde 2 h bei 40 °C gerührt und der entstandene Feststoff abfiltriert und getrocknet.
Ausbeute: 440 mg (81 %)

Stufe (v): Zu einer Lösung (R)-1-(4-(1-Methylpiperidin-4-yl)piperazin-1-yl)-2-(pyrrolidin-2-ylmethoxy)ethanon Trihydrochlorid (120 mg, 0,277 mmol) in Dichlormethan (5 ml) wurde Triethylamin (0,172 ml, 1,25 mmol) hinzugegeben. Anschließend wurde bei 0 °C eine Lösung 4-Methoxy-2,3,6-trimethylbenzol-1-sulfonylchlorid¹⁰ (58 mg, 0,249 mmol) in Dichlormethan (5 ml) hinzugetropft. Das Reaktionsgemisch wurde auf Raumtemperatur erwärmt und 3 d bei dieser Temperatur gerührt. Das Gemisch wurde mit gesättigter Natriumhydrogencarbonatlösung (5 ml) versetzt und die wässrige Phase mit Dichlormethan (20 ml) extrahiert. Die vereinigten organischen Phasen wurden getrocknet (Na₂SO₄), unter Vakuum eingeengt und das Rohprodukt anschließend säulenchromatographisch (Kieselgel) mit Diethylether / Dichlormethan / Methanol / Ammoniaklösung (25 % aq) (20:10:10:0,4) gereinigt. Das Rohprodukt wurde in einer geringen Menge Dichlormethan / Diethylether gelöst und langsam mit Chlortrimethylsilan (2,5 äq) versetzt. Der entstandene Niederschlag wurde abfiltriert und getrocknet.
Ausbeute: 80 mg (47 %), weißer Feststoff
MS, *m*/*z* 537,2 (MH⁺)

Die in der nachfolgenden Tabelle aufgeführten Beispielverbindungen wurden aus den entsprechenden Edukten in enger Anlehnung an das für das Beispiel 140 beschriebene Verfahren hergestellt. Der jeweilige Reaktionsverlauf wurde dünnschichtchromatographisch verfolgt und hierauf basierend die Reaktionszeiten bei analogen Umsetzungen entsprechend angepasst. Die Reaktionstemperaturen und eingesetzten Mengenäquivalente der Reagenzien können bei analogen Umsetzungen geringfügig abweichen. Die jeweils eingesetzte Menge Triethylamin in Stufe (v) wurde je nach der Stöchiometrie des eingesetzten Amin Hydrochlorides (x HCl) angepasst. Die eingesetzten Edukte sind kommerziell verfügbar oder wurden wie beschrieben hergestellt.

| **Beispiel Nr.** | | **Amino-alkohol** | **Amin (R⁵R⁶NH)** | **Sulfonylchlorid (R¹SO₂Cl)** | **Ausbeute (%) (über 5 Stufen)** | **MS, m/z (MH⁺)** |
|---|---|---|---|---|---|---|
| 152⁷ | | (R)-tert-Butyl 2-(hydroxymethyl)pyrrolidin-1-carboxylat | 1-(1-Methylpiperidin-4-yl)piperazin | 2-Chloro-6-methylbenz-1-sulfonyl chlorid | 34 | 513,1 |
| 161⁸ | | (S)-tert-Butyl 2-(hydroxymethyl)pyrrolidin-1-carboxylat | 4-(2-(Pyrrolidin-1-yl)ethyl)piperidin | 2-Chloro-6-methylbenz-1-sulfonyl chlorid | 27 | 512,1 |
| 162⁸ | | (S)-tert-Butyl 2-(hydroxymethyl)pyrrolidin-1-carboxylat | 1-(1-Methylpiperidin-4-yl)piperazin | 2-Chloro-6-methylbenz-1-sulfonyl chlorid | 20 | 513,1 |
| 163⁸ | | (S)-tert-Butyl 2-(hydroxymethyl)pyrrolidin-1 -carboxylat | 1-((1-Methylpiperidin-4-yl)methyl)piperazin | 2-Chloro-6-methylbenz-1-sulfonyl chlorid | 11 | 527,1 |
| 167 | | (S)-tert-Butyl 2-(hydroxymethyl)piperidin-1-carboxylat⁹ | 1-(1-Methylpiperidin-4-yl)piperazin | 4-Methoxy-2,6-dimethylbenzol-1-sulfonyl chlorid¹⁰ | 53 | 537,3 |
| 177⁸ | | (R)-tert-Butyl 2-(hydroxymethyl)piperidin-1-carboxylat⁹ | 1-(1-Methylpiperidin-4-yl)piperazin | 4-Methoxy-2,6-dimethylbenzol-1-sulfonyl chlorid¹⁰ | 16 | 537,3 |
| 180 | | (S)-tert-Butyl 2-(hydroxymethyl)pyrrolidin-1-carboxylat | 1-(1-Methylpiperidin-4-yl)piperazin | 4-Methoxy-2,6-dimethylbenzol-1-sulfonyl chlorid¹⁰ | 10 | 523,2 |
| 181 | | (S)-tert-Butyl 2-(hydroxymethyl)pyrrolidin-1-carboxylat | 4-(2-(Pyrrolidin-1-yl)ethyl)piperidin | 4-Methoxy-2,6-dimethylbenzol-1-sulfonyl chlorid 0 | 8 | 537,3 |
| 190⁸ | | (S)-tert-Butyl 2-(hydroxymethyl)pyrrolidin-1-carboxylat | 1-((1-Methylpiperidin-4-yl)methyl)piperazin | 4-Methoxy-2,6-dimethylbenzol-1-sulfonyl chlorid¹⁰ | 20 | 522,3 |

| | | | | | | |
|---|---|---|---|---|---|---|
| ⁷ Die Hydrochloridfällung erfolgte aus einer Dichlormethanlösung der freien Base unter Zugabe von 2 M Chlorwasserstoff Lösung in Diethylether (10 äq). ⁸ Die Hydrochloridfällung erfolgte aus einer Methylethylketon / (optional) Diethylether Lösung der freien Base unter Zugabe von 2 M Chlorwasserstoff Lösung in Diethylether (3 - 5 äq). ⁹ Der eingesetzte Boc geschützte (R)- bzw. (S)-Aminoalkohol wurde wie folgt hergestellt: Zu einer auf 0 °C gekühlten Lösung der Boc geschützten Carbonsäure (1 äq) in Tetrahydrofuran (50 ml/mmol) wurde Borwasserstoff-Tetrahydrofuran-Komplex (1,5 äq, 1 M Lösung in Tetrahydrofuran) zugetropft und das Gemisch anschließend 3 h bei Raumtemperatur gerührt. Zu dem auf 0 °C gekühlten Gemisch wurde Wasser und Kaliumcarbonat gegeben und 30 min gerührt. Dann wurde die wässrige Phase mit Diethylether extrahiert (3 x) und die vereinigten organischen Phasen mit gesättigter Natriumchloridlösung (1 x) gewaschen, anschließend getrocknet (Na₂SO₄) und unter Vakuum eingeengt. Das Rohprodukt wurde säulenchromatographisch (Kieselgel) aufgereinigt. [siehe auch Timothy J. Wilkinson et al.; Org. Lett.; 2000; 155 - 158] ¹⁰ 4-Methoxy-2,6-dimethylbenzol-1-sulfonyl chlorid wurde wie folgt hergestellt: Zu einer auf 0 °C gekühlten Lösung 3,5-Dimethylanisol (1 äq) in Dichlormethan (25 ml/mmol) wurde Chlorsulfonsäure (5 äq) in Dichlormethan (25 ml/mmol) langsam über 10 min hinzugetropft. Das Reaktionsgemisch wurde weitere 10 min gerührt und anschließend langsam in Eiswasser getropft und solange gerührt bis das Eis geschmolzen war. Die Phasen wurden getrennt und die wässrige Phase mit Dichlormethan extrahiert. Die vereinigten organischen Phasen wurden mit gesättigter Natriumchloridlösung gewaschen, getrocknet (Na₂SO₄) und unter Vakuum eingeengt. Das Rohprodukt wurde ohne weitere Aufreinigung in der nachfolgenden Synthesestufe eingesetzt. | | | | | | |

### Umsetzung von 2-((1-(4-Methoxy-2,6-dimethylphenvlsulfonyl)piperidin-2-yl)methoxy)essigsäure (Säurebaustein S27) mit Aminen (R⁵R⁶NH):

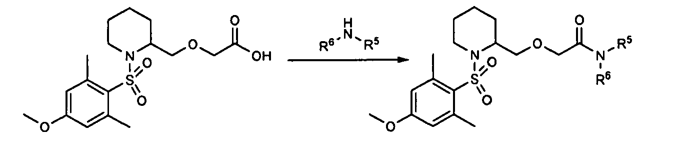

### Beispiel 201:

### 2-((1-(4-Methoxy-2,6-dimethylphenylsulfonyl)piperidin-2-yl)methoxy)-1-(4-(pyridin-4-yl)piperidin-1-yl)ethanon

Zu einer Lösung des Säurebausteines S27 (1 äq) in Dichlormethan (5 ml/mmol) wurde bei 0 °C Diisopropylethylamin (2,5 äq), gefolgt von N-Hydroxybenzotriazol (HOBt) (1 äq) und N-Ethyl-N'-(3-dimethylamino propyl) carbodiimid Hydrochlorid (EDCI) (1,5 äq) hinzugegeben. Das resultierende Reaktionsgemisch wurde 15 min bei 25 °C gerührt. Anschließend wurde auf 0 °C gekühlt und 4-(Piperidin-4-yl)pyridin (1,2 äq) hinzugegeben. Das Gemisch wurde bis zur vollständigen Umsetzung 16 h bei 25°C gerührt. Es wurde mit Dichlormethan (30 ml) verdünnt und mit gesättigter Ammoniumchloridlösung, gesättigter Natriumchloridlösung, gesättigter Natriumhydrogencarbonatlösung und wiederum gesättigter Natriumchloridlösung extrahiert. Die organische Phase wurde getrocknet (Na₂SO₄) und unter Vakuum eingeengt. Das Rohprodukt wurde säulenchromatographisch aufgereinigt (2 % Methanol in Dichloromethan).
Ausbeute: 40 %
MS, *m*/*z* 516,2 (MH⁺)

Die in der nachfolgenden Tabelle aufgeführten Beispielverbindungen wurden aus den entsprechenden Edukten in enger Anlehnung an das für das Beispiel 201 beschriebene Verfahren hergestellt.

2-((1-(4-Methoxy-2,6-dimethylphenylsulfonyl)piperidin-2-yl)methoxy)essigsäure (Säurebaustein S27) wurde in Analogie zu dem für die Beispiele 202 und 210 beschriebenen Verfahren zur Herstellung der entsprechenden Carbonsäure aus dem entsprechenden Aminoalkohol synthetisiert, wobei 4-Methoxy-2,6-dimethylbenzol-1-sulfonyl chlorid analog zu dem für das Beispiel 167 beschriebene Verfahren hergestellt wurde (mit der Ausnahme, dass 2 äq Chlorsulfonsäure eingesetzt wurden).

| **Beispiel Nr.** | | **Amin (R⁵R⁶NH)** | **Ausbeute (%)** | **MS, m/z (MH⁺)** |
|---|---|---|---|---|
| 200 | | 6-Fluor-2-(piperidin-4-yl)-1,2,3,4-tetrahydroisochinolin | 50 | 588,2 |
| 211* | | 2-(Piperidin-4-yl)-1,2,3,4-tetrahydropyrrolo[1,2-a]pyrazin | 11 | 559,3 |
| 217 | | 3-(Piperidin-4-yl)pyridin | 50 | 516,2 |

| | | | | |
|---|---|---|---|---|
| * Die Reaktion wurde in N,N-Dimethylformamid anstelle von Dichlormethan durchgeführt. | | | | |

### Herstellung der Amine:

### 6-Fluor-2-(piperidin-4-yl)-1,2,3,4-tetrahydroisochinolin

### (eingesetzt in der Synthese der Beispielverbindung 200)

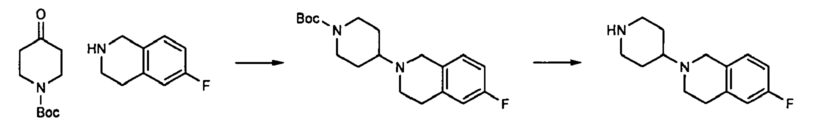

Stufe (i): Zu einer Lösung tert-Butyl 4-oxopiperidin-1-carboxylat (3,12 mmol) and 6-Fluor-1,2,3,4-tetrahydroisochinolin (2,6 mmol) in Methanol (15 ml) wurde unter Stickstoff bei 25 °C eine katalytische Menge Essigsäure, gefolgt von Natriumcyanoborohydrid (2,5 äq) gegeben. Die resultierende Lösung wurde bis zur vollständigen Umsetzung (DC-Kontrolle) 16 h bei dieser Temperatur gerührt. Zu dem Reaktionsgemisch wurde Eis gegeben und anschließend wurde unter Vakuum eingeengt. Der Rückstand wurde in Dichlormethan (100 ml) aufgenommen, mit gesättigter Natriumbicarbonatlösung und mit gesättigter Natriumchloridlösung gewaschen. Die organische Phase wurde getrocknet (Na₂SO₄), filtriert und unter Vakuum eingeengt. Das so erhaltene Rohprodukt wurde direkt in der nachfolgenden Synthesestufe eingesetzt.
Ausbeute: 50 %

Stufe (ii): Zu einer Lösung tert-Butyl 4-(6-fluor-3,4-dihydroisochinolin-2(1 H)-yl)piperidin-1-carboxylat (1 äq) in Dichlormethan (10 ml/mmol) wurde bei 0 °C Trifluoressigsäure (13 äq) gegeben und das Reaktionsgemisch anschließend 2 h bei 25 °C gerührt. Das Lösungsmittel wurde entfernt und das Rohprodukt unter Vakuum getrocknet. Das so erhaltene Amin wurde ohne Aufreinigung weiter umgesetzt.

### 2-(Piperidin-4-yl)-1,2,3,4-tetrahydropyrrolo[1,2-a]pyrazin

### (eingesetzt in der Synthese der Beispielverbindung 211)

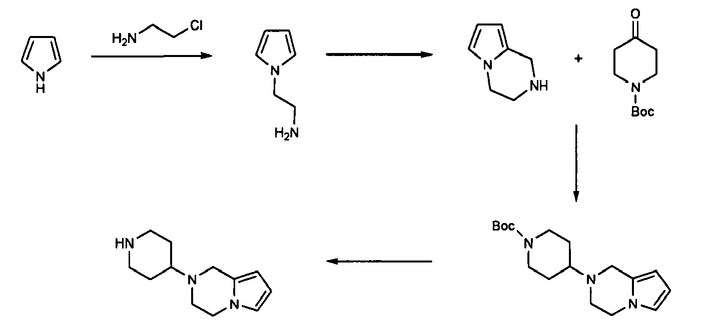

Stufe (i): Zu einer Lösung 1 H-Pyrrol (4 g, 0,06 mol) in Acetonitril (33 ml) wurde Natriumhydroxid (9,4g, 0,23 mol; Pulver) und Tetrabutylammonium hydrogensulfat (0,8 g, 2,36 mmol) hinzugegeben. Das Gemisch wurde 30 min bei 25°C gerührt, dann wurde 2-Chlorethylamin Hydrochlorid (8,2 g, 0,07 mol) hinzugegeben. Das Reaktionsgemisch wurde 24 h refluxiert, der Feststoff abfiltriert und das Filtrat unter Vakuum eingeengt. Das Rohprodukt wurde durch Destillation unter Vakuum aufgereinigt.
Ausbeute: 30 %

Stufe (ii): Zu einer Lösung 1-(2-Aminoethyl)pyrrol (9 mmol) und 37 % wässrige Formaldehydlösung (9 mmol) in Ethanol (20 ml) wurde Trifluoressigsäure (0,5 ml) gegeben und das resultierende Reaktionsgemisch 15 min bei 50 °C gerührt. Dann wurde auf 25 °C abgekühlt und weitere 4 h bei dieser Temperatur gerührt. Das Lösungsmittel wurde unter Vakuum entfernt und der Rückstand in Ethylacetat aufgenommen, mit wässriger Natriumcarbonatlösung basifiziert. Die organische Phase wurde getrocknet (Na₂SO₄) und eingeengt. Das Rohprodukt wurde ohne weitere Aufreinigung in der nächsten Synthesestufe eingesetzt.
Ausbeute: 40 %

Stufe (iii): Zu einer Lösung 4-Oxo-piperidin-1-carbonsäure tert-butyl ester (4,68 mmol) und 1,2,3,4-Tetrahydro-pyrrolo 1,2-a] pyrazin (3,9 mmol) in Methanol (15 ml) wurde unter Stickstoff bei 25 °C eine katalytische Menge Essigsäure, gefolgt von Natriumcyanoborohydrid (2,5 äq) gegeben. Die resultierende Lösung wurde bis zur vollständigen Umsetzung (DC-Kontrolle) 16 h bei dieser Temperatur gerührt. Zu dem Reaktionsgemisch wurde Eis gegeben und anschließend wurde unter Vakuum eingeengt. Der Rückstand wurde in Dichlormethan (100 ml) aufgenommen, mit gesättigter Natriumbicarbonatlösung und mit gesättigter Natriumchloridlösung gewaschen. Die organische Phase wurde getrocknet (Na₂SO₄), filtriert und unter Vakuum eingeengt. Das so erhaltene Rohprodukt wurde direkt in der nachfolgenden Synthesestufe eingesetzt.
Ausbeute: 40 %

Stufe (iv): Eine Lösung tert-Butyl 4-(6-fluor-3,4-dihydroisochinolin-2(1 H)-yl)piperidin-1-carboxylat (1,8 mmol) und Chlorwasserstoff in Ethylacetat (30 ml, gesättigte Lösung) wurde bis zur vollständigen Umsetzung (DC-Kontrolle) 2 h bei Raumtemperatur gerührt. Das Lösungsmittel wurde entfernt und das Rohprodukt unter Vakuum getrocknet. Das so erhaltene Amin wurde ohne weitere Aufreinigung in der nachfolgenden Synthesestufe eingesetzt.

### 3-(Piperidin-4-yl)pyridin

### (eingesetzt in der Synthese der Beispielverbindung 217)

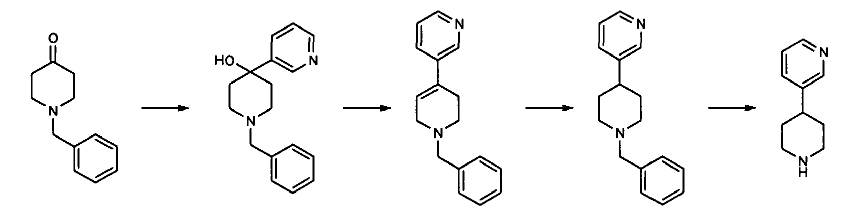

Stufe (i) : Zu einer Lösung von 3-Brompyridin (1 äq) in trockenem Tetrahydrofuran (250 ml) wurde n-Butyllithium (2 äq) bei -78°C über 2 h hinzugetropft. Das Reaktionsgemisch wurde anschließend 1 h bei -78 °C gerührt. N-Benzyl-piperidon (3 g, in 50 ml Tetrahydrofuran) wurde zu dieser Lösung bei -78°C über 30 min langsam hinzugetropft. Das Reaktionsgemisch wurde 1 h bei -78 °C gerührt und der Reaktionsverlauf dünnschichtchromatographisch verfolgt. Nach vollständiger Umsetzung wurde mit Wasser versetzt und mit Ethylacetat (3 x 100 ml) extrahiert. Die vereinigten organischen Phasen wurden getrocknet (Na₂SO₄), filtriert und unter Vakuum eingeengt. Das Rohprodukt wurde säulenchromatographisch aufgereinigt (4 % Methanol in Dichloromethan).
Ausbeute: 20 %

Stufe (ii): Zu einer Lösung 1-Benzyl-4-(pyridin-3-yl)piperidin-4-ol (1 g) in Methanol (10 ml) wurde 80 %ige Schwefelsäure bei 0 °C hinzugetropft. Das Reaktionsgemisch wurde 4 d bei 90°C gehalten und der Reaktionsverlauf dünnschichtchromatographisch verfolgt. Nach vollständiger Umsetzung wurde überschüssiges Methanol entfernt und das Gemisch anschließend durch Zugabe von Natriumhydroxidlösung auf pH 14 eingestellt und mit Dichlormethan (4 x 100 ml) extrahiert. Die organische Phase wurde getrocknet (Na₂SO₄), gefiltert und unter Vakuum eingeengt. Das so erhaltene Rohprodukt wurde direkt in der nachfolgenden Synthesestufe eingesetzt.
Ausbeute: quantitativ

Stufe (iii): Zu einer Lösung 3-(1-Benzyl-1,2,3,6-tetrahydropyridin-4-yl)pyridin (950 mg) in Methanol (10 ml) wurde unter Argon 10% Pd/C (600 mg) gegeben und das resultierende Gemisch 16 h bei 23 °C unter einer Wasserstoffatmosphäre gerührt. Der Reaktionsverlauf wurde per LC-MS Analytik verfolgt und das Gemisch nach vollständiger Umsetzung über Celite filtriert und mit Methanol gewaschen. Das Filtrat wurde unter Vakuum eingeengt und das Rohprodukt direkt in der nachfolgenden Synthesestufe eingesetzt.
Ausbeute: quantitativ

Stufe (iv): Zu einer Lösung 3-(1-Benzylpiperidin-4-yl)pyridin (650 mg) in Methanol (10 ml) wurde unter Argon Pd(OH)₂ (600 mg) gegeben, gefolgt von einer katalytischen Menge Essigsäure. Das resultierende Gemisch wurde 16 h bei 23 °C unter einer Wasserstoffatmosphäre gerührt. Der Reaktionsverlauf wurde per LC-MS Analytik verfolgt und das Gemisch nach vollständiger Umsetzung über Celite filtriert und mit Methanol gewaschen. Das Filtrat wurde unter Vakuum eingeengt und das Rohprodukt direkt in der nachfolgenden Synthesestufe eingesetzt.
Ausbeute: quantitativ

### Beispiel 215:

### 2-((1-(4-Methoxy-2,6-dimethylphenylsulfonyl)piperidin-2-yl)methoxy)-1-(6-(4-methylpiperazin-1-yl)-3,4-dihydroisochinolin-2(1 H)-yl)ethanon

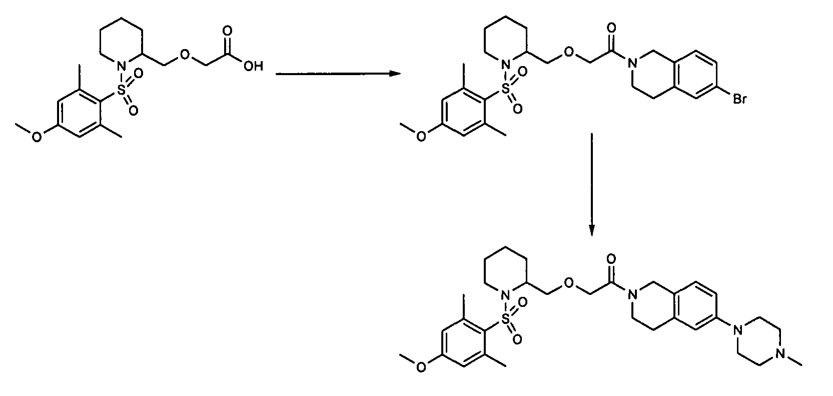

Stufe (i): Zu einer Lösung des Säurebausteines S27 (1,5 g) in Dichlormethan (5 ml/mmol) wurde bei 0 °C Diisopropylethylamin (2,5 äq) hinzugegeben, gefolgt von N-Hydroxybenzotriazol (HOBt) (1 äq) und N-Ethyl-N'-(3-dimethylamino propyl) carbodiimid Hydrochlorid (EDCI) (1.5 äq). Das resultierende Reaktionsgemisch wurde 15 min bei 23 °C gerührt. Anschließend wurde auf 0°C gekühlt und 6-Brom-1,2,3,4-tetrahydro-isochinolin (1.2 äq, gelöst in Dichlormethan) hinzugetropft. Das Reaktionsgemisch wurde bis zur vollständigen Umsetzung 16 h bei 25°C gerührt. Es wurde mit Dichlormethan (100 ml) verdünnt und mit gesättigter Ammoniumchloridlösung, gesättigter Natriumchloridlösung, gesättigter Natriumhydrogencarbonatlösung und wiederum gesättigter Natriumchloridlösung extrahiert. Die organische Phase wurde getrocknet (Na₂SO₂) und unter Vakuum eingeengt. Das Rohprodukt wurde säulenchromatographisch aufgereinigt (5 % Ethylacetat in Dichloromethan).
Ausbeute: 80 %

Stufe (ii): Zu einer Lösung N-Methyl piperazin (3,6 mmol) und 1-(6-Brom-3,4-dihydroisochinolin-2(1H)-yl)-2-((1-(4-methoxy-2,6-dimethylphenylsulfonyl)piperidin-2-yl)methoxy)ethanon (3 mmol) in Dioxan (20 ml) wurde Cäsiumcarbonat (2,5 äq) gegeben und die resultierende Lösung 30 min mit Argon entgast. Dann wurde Xanthphos (2 mmol), gefolgt von Palladium Tris(dibenzylidenaceton)dipalladium (0,15 mmol) unter Argon hinzugegeben und das Gemisch 16 h auf 120 °C erwärmt. Der Reaktionsverlauf wurde dünnschichtchromatographisch verfolgt. Das Reaktionsgemisch wurde filtriert mit Ethylacetat gewaschen und das Filtrat unter Vakuum eingeengt. Das Rohprodukt wurde in Dichlormethan aufgenommen und mit Wasser und gesättigter Natriumchloridlösung gewaschen. Die organische Phase wurde getrocknet (Na₂SO₂) gefiltert und unter Vakuum eingeengt. Das Produkt wurde säulenchromatographisch aufgereinigt (4 % Methanol in Dichloromethan).
Ausbeute: 10 %
MS, m/z 585,3 (MH⁺)

### Herstellung von substituierten 2-((1-(4-Methoxy-2,6-dimethylphenylsulfonyl)-1,2,3,4-tetrahydrochinolin-2-yl)methoxy)essigsäure-Derivaten:

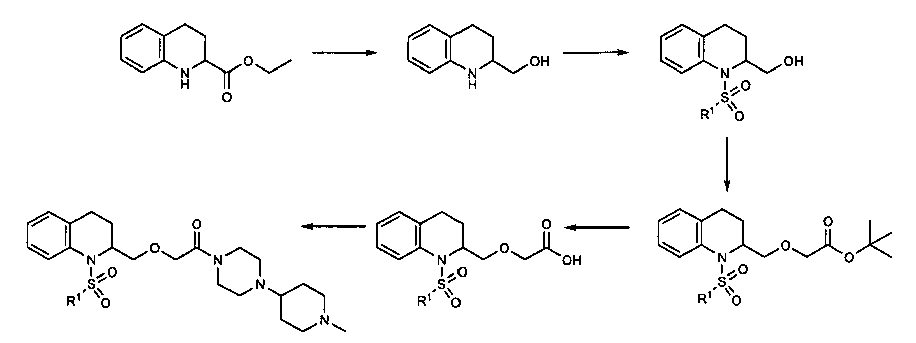

### Beispiel 202:

### 2-((1-(4-Methoxy-2,6-dimethylphenylsulfonyl)-1,2,3,4-tetrahydrochinolin-2-yl)methoxy)-1-(4-(1-methylpiperidin-4-yl)piperazin-1-yl)ethanon

Stufe (i): 1,2,3,4-Tetrahydrochinolin-2-carbonsäure ethyl ester (25 mmol) in Tetrahydrofuran (5 ml/mol) wurde bei 0 °C zu einer Suspension von Lithiumaluminiumhydrid (2 äq) in Tetrahydrofuran (50 ml) hinzugetropft. Das Reaktionsgemisch wurde 1 h bei 25 °C gerührt und dann 4 h refluxiert. Der Reaktionsverlauf wurde dünnschichtchromatographisch verfolgt. Zu dem Reaktionsgemisch wurde gesättigte Natriumsulfatlösung gegeben und das Gemisch anschließend über Celite filtriert und mit Ethylacetat gewaschen. Das Filtrat wurde im Vakuum eingeengt und das Rohprodukt säulenchromatographisch aufgereinigt (3:7 Ethylacetat / Hexan).
Ausbeute: 50 %

Stufe (ii): Zu einer Lösung (1,2,3,4-Tetrahydro-chinolin-2-yl)-methanol (0,67 mmol) in Dichloromethan (5 ml) wurde bei 0 °C Pyridin (5 äq) gegeben, gefolgt von einer katalytischen Menge Dimethylaminopyridin (0,01 äq). Dann wurde 4-Methoxy-2,6-dimethyl-benzolsulfonyl chlorid (1,2 äq) in Dichloromethan (2 ml) hinzugetropft. Das Reaktionsgemisch wurde bis zur vollständigen Umsetzung (DC-Kontrolle) 1 h bei 25 °C gerührt, anschließend mit Dichlormethan (50 ml) verdünnt und mit gesättigter Kupfersulfatlösung (4 x 15 ml) und gesättigter Natriumchloridlösung gewaschen. Die organische Phase wurde getrocknet (Na₂SO₄), filtriert und unter Vakuum eingeengt. Das Rohprodukt wurde säulenchromatographisch aufgereinigt (5 % Ethylacetat in Dichloromethan).
*[4-Methoxy-2,6-dimethylbenzol-1-sulfonylchlorid wurde analog zu dem für das Beispiel 167 beschriebene Verfahren hergestellt (mit der Ausnahme, dass* 2 *äq Chlorsulfonsäure eingesetzt wurden).]*
Ausbeute: 75 %

Stufe (iii): Zu einer kalten Lösung (1-(4-Methoxy-2,6-dimethylphenylsulfonyl)-1,2,3,4-tetrahydrochinolin-2-yl)methanol (0,47 mmol) in Toluol (3 ml) wurden Tetrabutylammonium chlorid (0,33 äq) und 35 %ige Natriumhydroxidlösung (3 ml) bei 0°C hinzugegeben. Zu diesem kalten Reaktionsgemisch wurde tert-Butyl bromoacetat (1,5 äq) bei 0 °C hinzugetropft. Dann wurde das Gemisch bis zur vollständigen Umsetzung (DC-Kontrolle) 90 min bei 25 °C gerührt. Das Gemisch wurde mit Ethylacetat (50 ml) extrahiert und die organische Phase mit Wasser gewaschen bis der pH-Wert neutral war. Anschließend wurde mit gesättigter Natriumchloridlösung extrahiert, die organische Phase getrocknet (Na₂SO₄) und unter Vakuum eingeengt. Das Rohprodukt wurde säulenchromatographisch aufgereinigt (20 % Ethylacetat in Hexan).
Ausbeute: 66 %

Stufe (iv): Zu einer Lösung tert-Butyl 2-((1-(4-methoxy-2,6-dimethylphenylsulfonyl)-1,2,3,4-tetrahydrochinolin-2-yl)methoxy)acetat (1 äq) in Dichlormethan (10 ml/mmol) wurde bei 0 °C Trifluoressigsäure (13 äq) gegeben und das Gemisch 2 h bei 25°C gerührt. Das Reaktionsgemisch wurde anschließend unter Vakuum eingeengt und das Rohprodukt ohne weitere Aufreinigung in der nachfolgenden Synthesestufe eingesetzt.

Stufe (v): Zu einer Lösung 2-((1-(4-Methoxy-2,6-dimethylphenylsulfonyl)-1,2,3,4-tetrahydrochinolin-2-yl)methoxy)essigsäure (1 äq) in Dichlormethan (5 ml/mmol) wurde bei 0 °C Diisopropylethylamin (2,5 äq) hinzugegeben, gefolgt von N-Hydroxybenzotriazol (HOBt) (1 äq) und N-Ethyl-N'-(3-dimethylamino propyl) carbodiimid Hydrochlorid (EDCl) (1,5 äq). Das resultierende Reaktionsgemisch wurde 15 min bei 25 °C gerührt. Anschließend wurde auf 0 °C gekühlt und 1-(1-Methylpiperidin-4-yl)piperazin (1,2 äq) hinzugegeben. Das Reaktionsgemisch wurde bis zur vollständigen Umsetzung 16 h bei 25°C gerührt. Es wurde mit Dichlormethan (30 ml) verdünnt und mit gesättigter Ammoniumchloridlösung, gesättigter Natriumchloridlösung, gesättigter Natriumhydrogencarbonatlösung und wiederum gesättigter Natriumchloridlösung extrahiert. Die organische Phase wurde getrocknet (Na₂SO₄) und unter Vakuum eingeengt. Das Rohprodukt wurde säulenchromatographisch aufgereinigt (2 % Methanol in Dichloromethan).
Ausbeute: 60 %
MS, *m*/*z* 585,3 (MH⁺)

Die in der nachfolgenden Tabelle aufgeführten Beispielverbindungen wurden aus den entsprechenden Sulfonylchloriden in enger Anlehnung an das für das Beispiel 202 beschriebene Verfahren (Stufe (v)) hergestellt.

| **Beispiel Nr.** | | **Sulfonylchlorid (R¹SO₂Cl)** | **Ausbeute (%) (über 5 Stufen)** | **MS, m/z (MH⁺)** |
|---|---|---|---|---|
| 203 | | Naphthalen-2-sulfonyl chlorid | 18 | 577,2 |
| 204 | | 4-Methoxybenzol-1-sulfonyl chlorid | 10 | 557,2 |

### Beispiel 210:

### 2-((1-(6-Methoxynaphthalen-2-ylsulfonyl)piperidin-2-yl)methoxy)-1-(4-(1-methylpiperidin-4-yl)piperazin-1-yl)ethanon

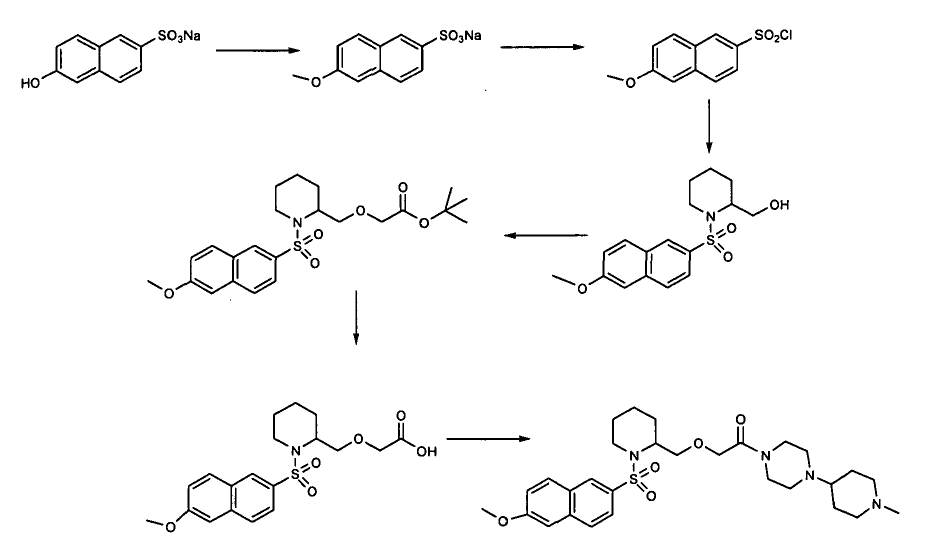

Stufe (i): Zu einer Lösung Natrium 6-hydroxynaphthalen-2-sulfonat (9 mmol) in Wasser (20 ml) wurde Natriumhydroxid (0,7 g) gegeben. Zu dieser Lösung wurde Dimethylsulfat (1,1 äq) über 1 h bei 50-55°C hinzugegeben, gefolgt von Natriumchlorid (3,3 g). Der Feststoff wurde abfiltriert und mit gesättigter Natriumchloridlösung und Toluol gewaschen. Das Rohprodukt wurde anschließend getrocknet.
Ausbeute: 75 %

Stufe (ii): Zu einer Lösung Natrium 6-methoxynaphthalen-2-sulfonat (2 mmol) in trockenem N,N-Dimethylformamid (1 ml) wurde unter Stickstoff bei 0 °C Thionylchlorid (0,25 ml) hinzugegeben. Das Reaktionsgemisch wurde 3 h bei 0 °C gerührt und dann mit Eiswasser (20 ml) versetzt. Der Feststoff wurde abfiltriert und mit kaltem Wasser gewaschen. Dann wurde der Feststoff in Dichlormethan (25 ml) aufgenommen, getrocknet (Na₂SO₄) und unter Vakuum eingeengt.
Ausbeute: 75 %

Stufe (iii): Zu einer kalten Lösung Piperidin-2-ylmethanol (1,5 mmol) in Dichloromethan (10 ml) wurde Triethylamin (2,5 äq) gegeben. Dann wurde eine Lösung 6-Methoxynaphthalen-2-sulfonylchlorid (1,5 mmol) in Dichloromethan (5 ml) hinzugetropft und das Gemisch bis zur vollständigen Umsetzung (DC-Kotrolle) 90 min bei 25 °C gerührt. Das Reaktionsgemisch wurde mit Dichlormethan (100 ml) versetzt und mit Wasser extrahiert. Die organische Phase wurde getrocknet (Na₂SO₄) und unter Vakuum eingeengt. Das Rohprodukt wurde säulenchromatographisch aufgereinigt (5 % Methanol in Dichloromethan).
Ausbeute: 50 %

Stufe (iv): Zu einer kalten Lösung (1-(6-Methoxynaphthalen-2-ylsulfonyl)piperidin-2-yl)methanol (0,75 mmol) in Toluol (4,5 ml) wurden Tetrabutylammonium chlorid (0,33 äq) und 35 %ige Natriumhydroxidlösung (4,5 ml) bei 0 °C hinzugegeben. Zu diesem kalten Reaktionsgemisch wurde tert-Butyl bromoacetat (1,5 äq) bei 0 °C hinzugetropft. Dann wurde das Gemisch bis zur vollständigen Umsetzung (DC-Kontrolle) 90 min bei 25 °C gerührt. Das Gemisch wurde mit Ethylacetat (100 ml) extrahiert und die organische Phase mit Wasser gewaschen bis der pH-Wert neutral war. Anschließend wurde mit gesättigter Natriumchloridlösung extrahiert, die organische Phase getrocknet (Na₂SO₄) und unter Vakuum eingeengt. Das Rohprodukt wurde säulenchromatographisch aufgereinigt (50 % Ethylacetat in Hexan).
Ausbeute: 90 %

Stufe (v): Zu einer Lösung tert-Butyl 2-((1-(6-Methoxynaphthalen-2-ylsulfonyl)piperidin-2-yl)methoxy)acetat (1 äq) in Dichlormethan (10 ml/mmol) wurde bei 0 °C Trifluoressigsäure (13 äq) gegeben und das Gemisch 2 h bei 25 °C gerührt. Das Reaktionsgemisch wurde anschließend unter Vakuum eingeengt und das Rohprodukt ohne weitere Aufreinigung in der nachfolgenden Synthesestufe eingesetzt.

Stufe (vi): Zu einer Lösung 2-((1-(6-Methoxynaphthalen-2-ylsulfonyl)piperidin-2-yl)methoxy)essigsäure (1 äq) in Dichlormethan (5 ml/mmol) wurde bei 0 °C Diisopropylethylamin (2,5 äq) hinzugegeben, gefolgt von N-Hydroxybenzotriazol (HOBt) (1 äq) und N-Ethyl-N'-(3-dimethylamino propyl) carbodiimid Hydrochlorid (EDCl) (1,5 äq). Das resultierende Reaktionsgemisch wurde 15 min bei 25°C gerührt. Anschließend wurde auf 0 °C gekühlt und 1-(1-Methylpiperidin-4-yl)piperazin (1,2 äq) hinzugegeben. Das Reaktionsgemisch wurde bis zur vollständigen Umsetzung 16 h bei 25°C gerührt. Es wurde mit Dichlormethan (100 ml) verdünnt und mit gesättigter Ammoniumchloridlösung, gesättigter Natriumchloridlösung, gesättigter Natriumhydrogencarbonatlösung und wiederum gesättigter Natriumchloridlösung extrahiert. Die organische Phase wurde getrocknet (Na₂SO₄) und unter Vakuum eingeengt. Das Rohprodukt wurde säulenchromatographisch aufgereinigt (2 % Methanol in Dichloromethan).
Ausbeute: 50 %
MS, *m*/*z* 559,3 (MH⁺)

### Beispiele 121 -126 und 148 - -150:

### Reaktionsschema:

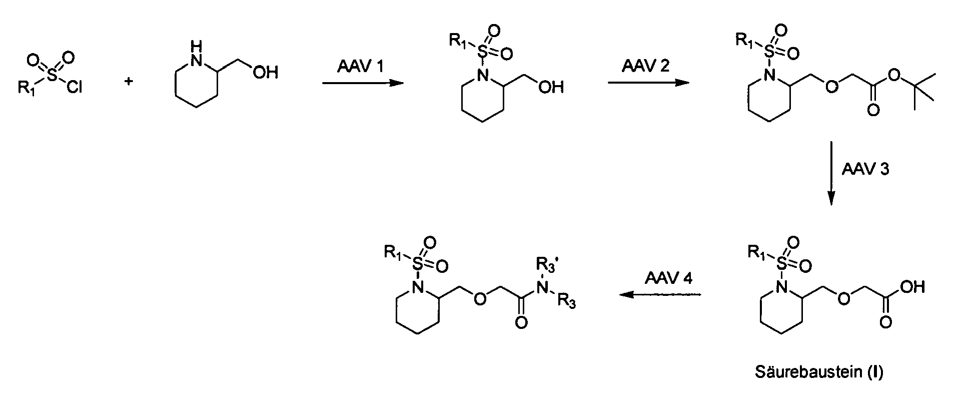

### AAV 1: Sulfonylierung

5 Äquivalente des entsprechenden Aminoalkohols werden in Dichlormethan (ca. 1,5 ml/mmol) gelöst und mit dem entsprechenden, in Dichlormethan (ca. 1,5 ml/mmol) gelösten Sulfonylchlorid (1 Äquivalent) bei Raumtemperatur versetzt. Nach vollständigem Umsatz des Sulfonylchlorids (DC-Kontrolle) wird der Ansatz 3x mit 5 %iger HCl gewaschen. Die organische Phase wird über Natriumsulfat getrocknet und unter Vakuum bis zur Trockne eingeengt.

### AAV 2: Veretherung

Der entsprechende Alkohol (1 Äquivalent) wird in THF (ca. 5,5 ml/mmol) gelöst, auf 0 °C abgekühlt und portionsweise mit Natriumhydrid (1,2 Äquivalente) versetzt. Die Reaktionsmischung wird für 15 min. bei 0 °C gerührt bevor tert.-Butylbromoacetat (2,5 Äquivalente) zugegeben wird. Das Reaktionsgemisch wird für 16 Stunden bei Raumtemperatur gerührt. Zur Aufarbeitung wird der Ansatz mit gesättigter Ammoniumchloridlösung gequencht, die wässrige Phase 2x mit Ethylacetat extrahiert und die vereinigten organischen Phasen mit gesättigter Natriumhydrogencarbonatlösung und gesättigter Natriumchloridlösung gewaschen. Die organische Phase wird über Natriumsulfat getrocknet, unter Vakuum bis zur Trockne eingeengt und der Rückstand säulenchromatographisch aufgereinigt.

### AAV 3: Tertbutylester Spaltung

Der entsprechende Tertbutylester wird in Dichlormethan (ca. 15 ml/mmol) gelöst und bei Raumtemperatur mit Trifluoressigsäure (ca. 3,25 ml/mmol) versetzt. Das Reaktionsgemisch wird bei Raumtemperatur bis zum vollständigen Umsatz des Tertbutylesters (DC-Kontrolle) gerührt und anschließend unter Vakuum bis zur Trockne eingeengt. Der Rückstand wird abschließend 3x mit Toluol versetzt und zur Trockne eingeengt.

### AAV 4: Amin-Kupplung

Die entsprechende Carbonsäure (1 Äquivalent) wir in Dichlormethan (ca. 8 ml/mmol) gelöst, mit 1,1'-Carbonyldiimidazol (ca. 1,05 Äquivalente) versetzt und 1 Stunde bei Raumtemperatur gerührt. Anschließen wird das entsprechende Amin (1 Äquivalent), gelöst in Dichlormethan (ca. 8 ml/mmol) zugegeben und die Reaktionsmischung 16 Stunden bei Raumtemperatur gerührt. Zur Aufarbeitung wird der Ansatz 3x mit gesättigter Ammoniumchloridlösung und 3x mit gesättigter Natriumhydrogencarbonatlösung gewaschen, die organische Phase über Natriumsulfat getrocknet und abschließend unter Vakuum bis zur Trockne eingeengt.

### Säurebausteine (I) (AAV 1 - AAV 3):

### (Ia) 2-((1-(4-Bromphenylsulfonyl)piperidin-2-yl)methoxy)essigsäure

Nach AAV 1 wurde 4-Bromobenzol-1-sulfonyl chlorid (58,7 mmol) mit 2-(Hydroxymethyl)-piperidin in 77% Ausbeute zu (1-(4-Bromphenylsulfonyl)piperidin-2-yl)methanol umgesetzt. Dieses wurde nach AAV2 weiter umgesetzt wobei tert-butyl 2-((1-(4-Bromphenylsulfonyl)piperidin-2-yl)methoxy)acetat in einer Ausbeute von 38% (17,3 mmol) erhalten wurde. Die abschließende Tertbutylester-Spaltung nach AAV 3 führte ohne Verluste zu 2-((1-(4-Bromophenylsulfonyl)piperidin-2-yl)methoxy)essigsäure.

### (Ib) 2-((1-(3-Bromphenylsulfonyl)piperidin-2-yl)methoxy)essigsäure

Nach AAV 1 wurde 3-Brombenzol-1-sulfonyl chlorid (58,7 mmol) mit 2-(Hydroxymethyl)-piperidin in 80% Ausbeute zu (1-(4-Bromophenylsulfonyl)piperidin-2-yl)methanol umgesetzt. Dieses wurde nach AAV2 weiter umgesetzt wobei tert-Butyl 2-((1-(3-Bromphenylsulfonyl)piperidin-2-yl)methoxy)acetat in eine Ausbeute von 31% (14,9 mmol) erhalten wurde. Die abschließende Tertbutylester-Spaltung nach AAV 3 führte ohne Verluste zu 2-((1-(3-Bromphenylsulfonyl)piperidin-2-yl)methoxy)essigsäure.

### (Ic) (1-(4-Brom-2,6-dimethylphenylsulfonyl)piperidin-2-yl)methanol

*4-Brom-2,6-dimethylbenzol-1-sulfonyl chlorid:* Zu einer auf -5 °C abgekühlten Mischung aus 200 ml 48%iger HBr und 50 g 3,5-Dimethylanilin wird eine Lösung aus 42 g Natriumnitrit in 150 ml Wasser gegeben. Die Reaktionsmischung wird 1 Stunde zwischen -5 °C und 0 °C gerührt. Abschließend wurde diese Reaktionsmischung langsam zu einer auf 80°C erhitzten Mischung aus 88,3 g Kupfer(I)bromid und 150 ml 48% iger HBr gegeben. Das Reaktionsgemisch wird für 2 Stunden bei 80°C gerührt und mittels DC Kontrolle beobachtet (Slilica; Hexan). Nach Beendigung der Reaktion wird das Produkt durch Wasserdampfdestillation erhalten und säulenchromatographisch (Silica; Hexan) aufgereinigt. 1-Brom-3,5-dimethylbenzol wurde so in einer Ausbeute von 60% (30g) erhalten.

Das soeben erhaltene Bromid, gelöst in 150 ml Dichlormethan, wurde innerhalb von 20 Minuten, tropfenweise bei 0 °C zu 90 ml Chlorsulfonsäure gegeben. Nach Beendigung der Zugabe wird 1 Stunde bei Raumtemperatur gerührt. Der Fortschritt der Reaktion wird mittels DC kontrolliert (Hexan). Nach Beendigung der Reaktion wird das Reaktionsgemisch auf Eis geschüttet und 3x mit Dichlormethan (je 200 ml) extrahiert. Die vereinigten organischen Phasen werden über Na₂SO₄ getrocknet, unter Vakuum konzentriert und säulenchromatographisch aufgereinigt (Silika; Hexan). Das gewünschte 4-Brom-2,6-dimethylbenzol-1-sulfonyl chlorid wurde in einer Ausbeute von 65% (29,8 g) erhalten.

*(1-(4-Brom-2,6-dimethylphenylsulfony*/*)piperidin-2-y*/*)methanol:* Zu einer Lösung von (1-(4-Brom-2,6-dimethylphenylsulfonyl)piperidin-2-yl)methanol (22,5 g) in Dichlormethan (290 ml) wird Kaliumcarbonat gegeben und dar Gemisch auf 0 °C abgekühlt. Eine Suspension des Hydrochlorids von Piperidin-2-ylmethanol (39 g, kommerziell erhältlich, CAS No.: 3433372) in Dichlormethan (300 ml) wurde innerhalb von 45 Minuten bei 0 °C zu der Sulfonylchlorid Suspension gegeben. Nach Beendigung der Zugabe wurde das Reaktionsgemisch für 12 Stunden bei Raumtemperatur gerührt. Der Fortschritt der Reaktion wurde dünnschichtchromatographisch überwacht (Silika; 10% EtOAc/Heptan). Nach Beendigung der Reaktion wurde abfiltriert, mit Wasser gewaschen und über Na₂SO₄ getrocknet. Das Lösungsmittel wurde unter Vakuum entfernt, und das Rohprodukt säulenchromatographisch aufgereinigt (Silika; 10% EtOAc/Hexan). (1-(4-Brom-2,6-dimethylphenylsulfonyl)piperidin-2-yl)methanol wurde in einer Ausbeute von 61,2% (30 g) erhalten.

*tert-Butyl 2-((1-(4-Brom-2,6-dimethylphenylsulfonyl)piperidin-2-yl)methoxy)acetat:* Zu einer Lösung von 18 g (1-(4-Brom-2,6-dimethylphenylsulfonyl)piperidin-2-yl)methanol in 180 ml Toluol wurden 180 ml 50%ige Natriumhydroxid -Lösung und 180 ml tert-Butylbromacetat gegeben und für 10 Minuten gerührt. Anschließend wurden 1,815 g Tetra-n-butylammoniumhydrogensulfat zugegeben und für 45 Minuten bei Raumtemperatur weitergerührt. Der Fortschritt der Reaktion dünnschichtchromatographisch überwacht (20% EtOAc/Hexan). Nach Beendigung der Reaktion wurden 400 ml Ethylacetat zugegeben und die Phasen getrennt. Die organische Phase wurde mit gesättigter Natriumchloridlösung gewaschen, über Natriumsulfat getrocknet und unter Vakuum konzentriert. Das Rohprodukt wurde Säulenchromatographisch aufgereinigt (Silika; 5% EtoAc/Hexan). tert-Butyl 2-((1-(4-brom-2,6-dimethylphenylsulfonyl)piperidin-2-yl)methoxy)acetat wurde in einer Ausbeute von 78,2% (18 g) erhalten.

Die Tertbutylester-Spaltung von 40g 2-((1-(4-Brom-2,6-dimethylphenylsulfonyl)piperidin-2-yl)methoxy)acetat nach AAV 3 führte in einer Ausbeute von 76% zu 2-((1-(4-Brom-2,6-dimethylphenylsulfonyl)piperidin-2-yl)methoxy)essigsäure.

### Beispiel Nr. 121:

### 2-((1-(3-Bromphenylsulfonyl)piperidin-2-yl)methoxy)-1-(4-(1-methylpiperidin-4-yl)piperazin-1-yl)ethanon

Säurebaustein (**Ib**) (6,4 mmol) wurde nach der AAV4 mit 1-(1-Methyl-4-piperidinyl)piperazin umgesetzt. Das gewünschte Produkt wurde in einer Ausbeute von 57% erhalten. HPLC-MS, m/z 557,2 (MH⁺)

### Beispiel Nr. 122:

### 2-((1-(3-Brom phenylsulfonyl)piperidin-2-yl)methoxy)-1-(4-(pyridin-4-yl)piperazin-1-yl)ethanon

Säurebaustein (**Ib**) (4 mmol) wurde nach der AAV4 mit 1-(4-Pyridyl)piperazin umgesetzt. Das gewünschte Produkt wurde in einer Ausbeute von 97% erhalten. HPLC-MS, m/z 537,1 (MH⁺)

### Beispiel Nr. 123:

### 2-((1-(4-Bromphenylsulfonyl)piperidin-2-yl)methoxy)-1-(4-(1-methylpiperidin-4-yl)piperazin-1-yl)ethanon

Säurebaustein **(Ia)** (7,6 mmol) wurde nach der AAV4 mit 1-(1-Methyl-4-piperidinyl)piperazin umgesetzt. Das gewünschte Produkt wurde in einer Ausbeute von 73% erhalten. HPLC-MS, m/z 557,2 (MH⁺)

### Beispiel Nr. 124:

### 2-((1-(3-Bromphenylsulfonyl)piperidin-2-yl)methoxy)-1-(4-(2-(pyrrolidin-1-yl)ethyl)piperidin-1-yl)ethanon

Säurebaustein **(Ib)** (5 mmol) wurde nach der AAV4 mit 4-(2-Pyrrolidinoethyl)piperidin umgesetzt. Das gewünschte Produkt wurde in einer Ausbeute von 69% erhalten. HPLC-MS, m/z 556,2 (MH⁺)

### Beispiel Nr.125:

### 2-((1-(4-Bromphenylsulfonyl)piperidi n-2-yl)methoxy)-1-(4-(2-(pyrrolidin-1-yl)ethyl)piperidin-1-yl)ethanon

Säurebaustein (**Ia**) (7,6 mmol) wurde nach der AAV4 mit 4-(2-Pyrrolidinoethyl)piperidin umgesetzt. Das gewünschte Produkt wurde in einer Ausbeute von 71% erhalten. HPLC-MS, m/z 556,2 (MH⁺)

### Beispiel Nr. 126:

### 2-((1-(4-Bromphenylsulfonyl)piperidin-2-yl)methoxy)-1-(4-(pyridin-4-yl)piperazin-1-yl)ethanon

Säurebaustein **(Ia)** (5 mmol) wurde nach der AAV4 mit 1-(4-Pyridyl)piperazin umgesetzt. Das gewünschte Produkt wurde in einer Ausbeute von 70% erhalten. HPLC-MS, m/z 537,1 (MH⁺)

### Beispiel Nr. 148:

### 2-((1-(4-Brom-2,6-dimethylphenylsulfonyl)piperidin-2-yl)methoxy)-1-(4-(1-methylpiperidin-4-yl)piperazin-1-yl)ethanon

Säurebaustein (**Ic**) (7,1 mmol) wurde nach der AAV4 mit 1-(1-Methyl-4-piperidinyl)piperazin umgesetzt. Das gewünschte Produkt wurde in einer Ausbeute von 70% erhalten. HPLC-MS, m/z 585,1 (MH⁺)

### Beispiel Nr. 149:

### 2-((1-(4-Brom-2,6-dimethylphenylsulfonyl)piperidin-2-yl)methoxy)-1-(4-(2-(pyrrolidin-1-yl)ethyl)piperidin-1-yl)ethanon

Säurebaustein (**Ic**) (7,1 mmol) wurde nach der AAV4 mit 4-(2-Pyrrolidinoethyl)piperidin umgesetzt. Das gewünschte Produkt wurde in einer Ausbeute von 70% erhalten. HPLC-MS, m/z 584,0 (MH⁺)

### Beispiel Nr. 150:

### 2-((1-(4-Brom-2,6-dimethylphenylsulfonyl)piperidin-2-yl)methoxy)-1-(4-(pyridin-4-yl)piperazin-1-yl)ethanon

Säurebaustein (**Ic**) (7,1 mmol) wurde nach der AAV4 mit 1-(4-Pyridyl)piperazin umgesetzt. Das gewünschte Produkt wurde in einer Ausbeute von 79% erhalten. HPLC-MS, m/z 565,0 (MH⁺)

### Herstellung der Sulfonylchloride:

### Herstellung von 2-Methylnaphthalin-1-sulfonylchlorid:

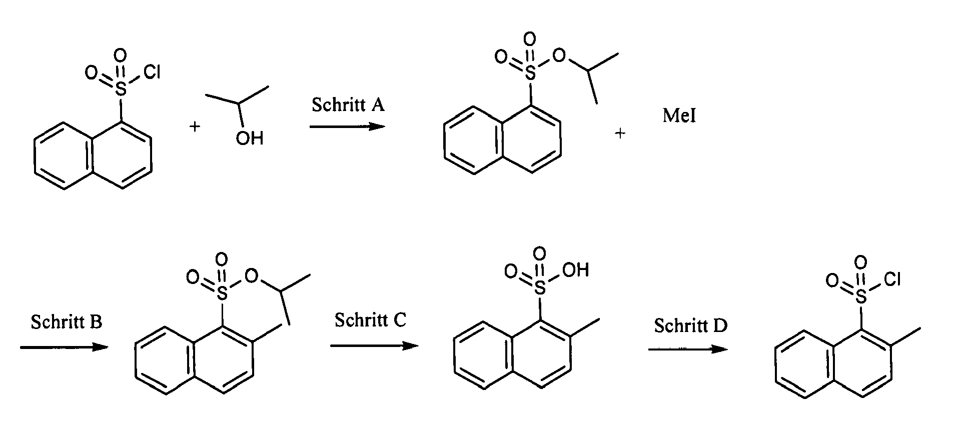

### Schritt A:

Zu einer Mischung von 2-Propanol (7.3 ml) und Pyridin (25 ml) wurde bei -5°C kommerziell erhältliches 1-Naphthylsulfonylchlorid (21.5 g) portionsweise zugegeben. Die Reaktionsmischung wurde bei 0°C für 15 h gerührt. Zur Aufarbeitung wurde bei dieser Temperatur mit DCM (75 ml) und 1 M HCl (75 ml) versetzt und die organische Phase abgetrennt. Die wässrigen Phase wurde mit DCM (3x75 ml) extrahiert und die vereinigten organischen Phasen wurden mit 1 M HCl (2 x 50 ml) und ges. NaCl-Lsg. (50 ml) gewaschen. Nach Trocknen über MgSO₄ wurde komplett eingeengt und man erhielt das gewünschte Produkt in der erforderlichen Reinheit. 22.8 g , 96%.

### Schritt B:

Die Titelverbindung aus Schritt A (18.6 g) wurde in THF (190 ml) gelöst und die Mischung unter Schutzgasatmosphäre auf -78°C gekühlt. Dann wurde 1.6 M n-BuLi in n-Hexan (51 ml) so langsam zugegeben, dass die Temperatur nicht über -70°C steigt. Die Reaktionsmischung wurde bei -70°C für 2 h gerührt und anschließend mit Mel (9.7 ml) versetzt. Man lässt die Reaktionsmischung auf 0°C erwärmen und für 3 h bei dieser Temperatur gerührt. Zur Aufarbeitung wurde bei dieser Temperatur mit ges. NH₄Cl (80 ml) versetzt und anschließend mit EtOAc (500 ml) verdünnt. Die organische Phase wurde abgetrennt und die wässrigen Phase wurde mit EtOAc (2 x 120 ml) extrahiert und die vereinigten organischen Phasen wurden mit Wasser (50 ml) und ges. NaCl-Lsg. (50 ml) gewaschen. Nach Trocknen über MgSO₄ wurde komplett eingeengt und der Rückstand durch Säulenchromatographie an Kieselgel (Hexan/EtOAc) gereinigt. Man erhält das gewünschte Produkt. 12.4 g , 63%.

### Schritt C:

Die Titelverbindung aus Schritt B (0.8 g) wurde in 4M HCl (31 ml) suspendiert und die Mischung für 1-2 h auf 110°C erhitzt. Anschließend wurde über 15 h auf Raumtemperatur abgekühlt, komplett eingeengt und der Rückstand nach zweimaliger Koevaporation mit DCM (2 x 30 ml) im Hochvakuum getrocknet. Das so erhaltene Produkt wurde ohne weitere Aufreinigung in den nächsten Schritt eingesetzt. 0.47 g , 70%.

### Schritt D:

Die Titelverbindung aus Schritt C (0.46 g) wurde in Toluol (2.2 ml) suspendiert und die resultierende Mischung mit SOCl₂ (0.75 ml) und DMF (0.010 ml) versetzt. Anschließend wurde für 1 h auf 90°C erhitzt bis eine Lösung vorlag. Die Lösung wurde komplett eingeengt und der Rückstand im Hochvakuum getrocknet. Das so erhaltene Produkt wurde ohne weitere Aufreinigung in den nächsten Schritt eingesetzt.

### Herstellung von 2-Chlornaphthalin-1-sulfonsäure:

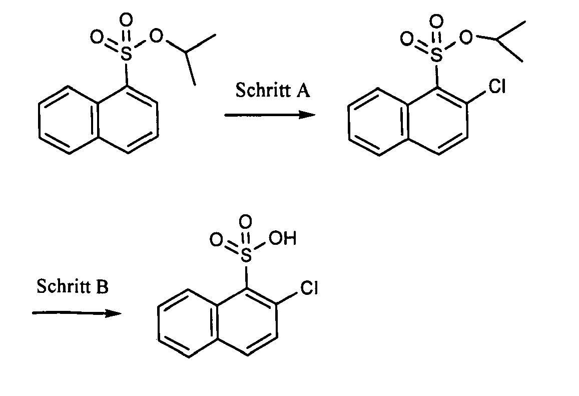

### Schritt A:

Die Titelverbindung aus der Herstellung von 2-Methylnaphthalin-1-sulfonylchlorid, Schritt A (20 g) wurde in THF (213 ml) gelöst und die Mischung unter Schutzgasatmosphäre auf -78°C gekühlt. Dann wurde 1.6 M n-BuLi in n-Hexan (55 ml) so langsam zugegeben, dass die Temperatur nicht über -70°C steigt. Die Reaktionsmischung wurde bei -70°C für 2 h gerührt und anschließend mit Hexachlorethan (21 g) versetzt. Man lässt die Reaktionsmischung auf 0°C erwärmen und für 15 h bei dieser Temperatur gerührt. Zur Aufarbeitung wurde bei dieser Temperatur mit ges. NH₄Cl (100 ml) versetzt und anschließend mit EtOAc (350 ml) verdünnt. Die organische Phase wurde abgetrennt und die wässrigen Phase wurde mit EtOAc (2 x 120 ml) extrahiert und die vereinigten organischen Phasen wurden mit Wasser (50 ml) und ges. NaCl-Lsg. (50 ml) gewaschen. Nach Trocknen über MgSO₄ wurde komplett eingeengt und der Rückstand durch Säulenchromatographie an Kieselgel (Hexan/EtOAc) gereinigt. Man erhält das gewünschte Produkt. 19.8 g , 87%.

### Schritt B:

Die Titelverbindung aus Schritt A (3 g) wurde in EtOH (15.4 ml) gelöst und mit TFA (0.04 ml) versetzt. Die resultierende Lösung wurde für 5 h refluxiert. Nach dem Abkühlen wurde eingeengt und der Rückstand wird in DCM (20 ml) aufgelöst und mit Wasser (10 ml) gewaschen. Nach Trocknen über MgSO₄ wurde komplett eingeengt. Das erhaltene Produkt wurde ohne weitere Aufreinigung in den nächsten Schritt eingesetzt. 2.2 g , 87%.

### Herstellung von 4-Methoxynaphthalin-1-sulfonsäure:

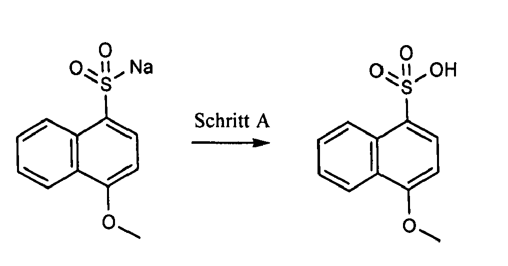

### Schritt A:

Zu einer Mischung von Wasser (5.2 ml) und konz. HCl (22 ml) wurde bei RT kommerziell erhältliches Natrium-4-methoxynaphthyl-1-sulfonat (2.5 g) zugegeben. Die Mischung wurde mehrfach mit MeOH/EtOAc 1:15 (insgesamt 400 ml) extrahiert und die vereinigten organischen Phasen mit ges. NaCl-Lsg. (50 ml) gewaschen. Nach Trocknen über MgSO₄ wurde komplett eingeengt. Das erhaltene Produkt wurde ohne weitere Aufreinigung in den nächsten Schritt eingesetzt. 1.2 g , 52%.

In enger Anlehnung an das für das 2-Methylnaphthalin-1-sulfonylchlorid beschriebene Verfahren, mit der Ausnahme, dass die in der nachfolgenden Tabelle genannten Sulfonsäuren verwendet wurden, erfolgte die Darstellung der folgenden Beispiele.

| Sulfonsäure | Sulfonylchlorid |
|---|---|
| 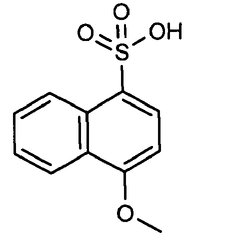 | 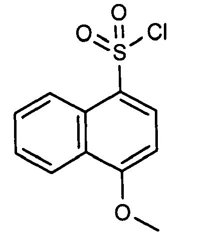 |
| 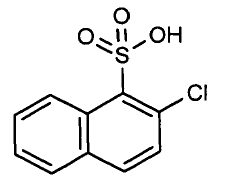 | 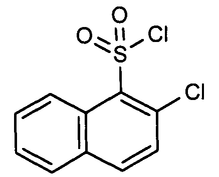 |

### Beispiel 179:

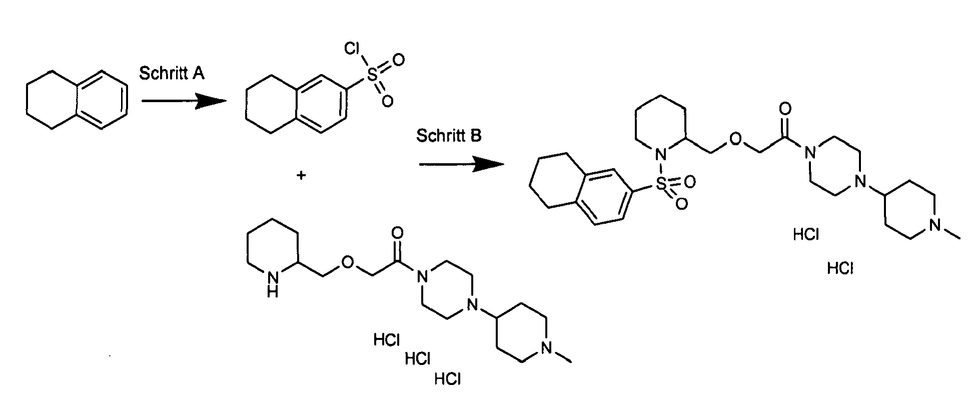

### Schritt A:

Kommerziell erhältliches 1,2,3,4-Tetrahydronaphthalin (50 ml)wurde in Chloroform (110 ml) vorgelegt. Bei -5 bis -10°C wurde unter Rühren innerhalb von 30 min Chlorsulfonsäure (73 ml) zugetropft. Die Kühlung wurde entfernt und die Lösung innerhalb 1 h auf RT erwärmt. Die Reaktionslösung wurde auf Eis gegeben und die organische Phase abgetrennt. Die wässrige Phase wird mit extrahiert DCM/MeOH 9 : 1 (3 x150 ml) und die vereinigten organischen Phasen werden mit Eiswasser (2x 50 ml) gewaschen. Nach Trocknen über MgSO₄ wurde komplett eingeengt und der Rückstand durch Säulenchromatographie an Kieselgel (Hexan/EtOAc) gereinigt. Man erhält das gewünschte Produkt. 8.4 g , 10%.

### Schritt B:

Die Titelverbindung aus **Beispiel 107**, Schritt **4** (1.0 g) wurde in DCM (19 ml) vorlegt, auf 0°C gekühlt und Et₃N (1.8 ml) zugeben. Dann wurde bei dieser Temperatur die Titelverbindung aus Schritt **A** (0.5g) rasch zugeben. Es wurde bei RT gerührt bis eine komplette Umsetzung per DC-Kontrolle angezeigt wurde. Zur Aufarbeitung wurde mit NH₄Cl-Lsg. (10 ml) und Wasser (10 ml) versetzt und die organische Phase abgetrennt. Die wässrigen Phase wurde mit DCM (3x15 ml) extrahiert und die vereinigten organischen Phasen wurden mit Wasser (10 ml) und ges. NaCl-Lsg. (10 ml) gewaschen. Nach Trocknen über MgSO₄ wurde komplett eingeengt und der Rückstand durch Säulenchromatographie an Kieselgel (DCM/ MeOH/NH₃) gereinigt. Man erhielt 638 mg der Base, die aus Aceton (10 ml) / Ether (10 ml) durch Zugabe von H2O (21 µl) und TMSCI (302 µl) gefällt wurde. 598 mg, 46%, 533 (MH⁺).

In enger Anlehnung an das für das Beispiel 179 beschriebene Verfahren, mit der Ausnahme, dass die in der nachfolgenden Tabelle genannten Sulfonylchloride und Amine verwendet wurden, wurden die Beispiele 157, 160, 173 - 176 und 191 dargestellt. Die Darstellung der nicht kommerziell erhältlichen Sulfonylchloride erfolgte wie oben beschrieben.

| Nr. | Sulfonylchlorid | Amin | Ausb. (%) | MS (MH+) |
|---|---|---|---|---|
| 157 | 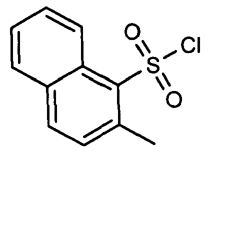 | 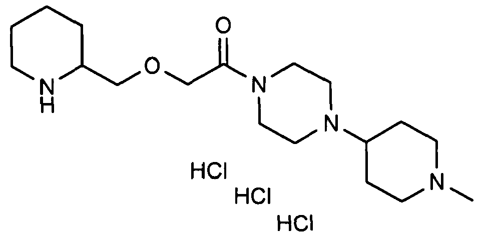 | 8 | 543 |
| 191 | 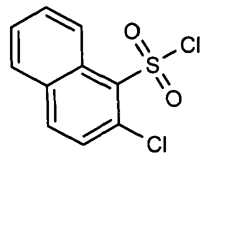 | 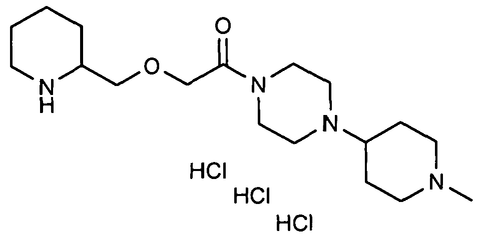 | 27 | 563 |
| 174 | 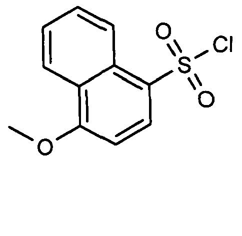 | 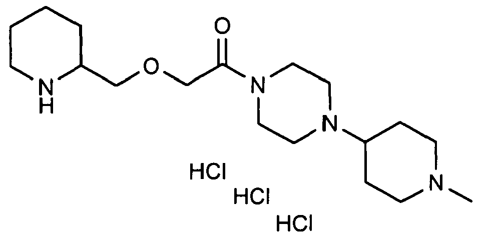 | 30 | 559 |
| 176 | 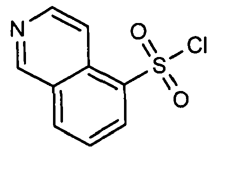 | 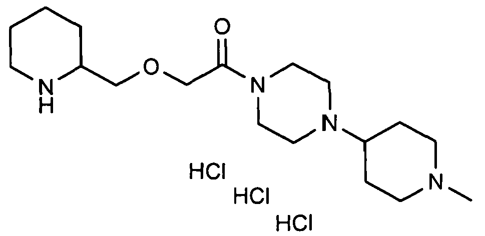 | 57 | 530 |
| 175 | 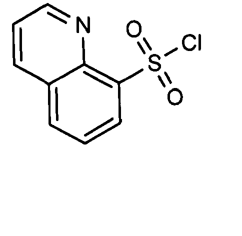 | 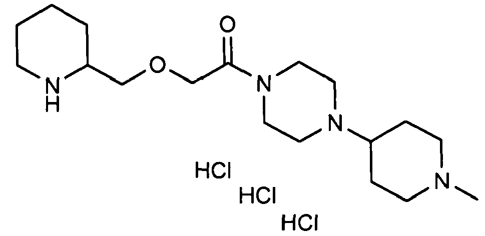 | 3 | 530 |
| 173 | 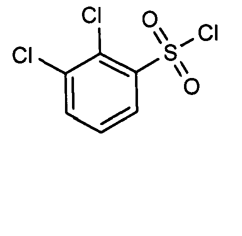 | 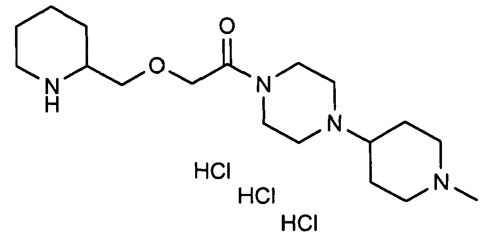 | 61 | 547 |
| 160 | 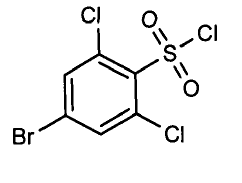 | 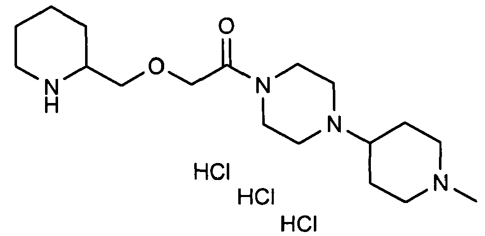 | 69 | 627 |

### Beispiel 192: Herstellung von 2-((1-(4-Methoxy-2,6-dimethylphenylsulfonyl)pyrrolidin-2-yl)methoxy)-1-(4-(1-methylpiperidin-4-yl)piperazin-1-yl)ethanon

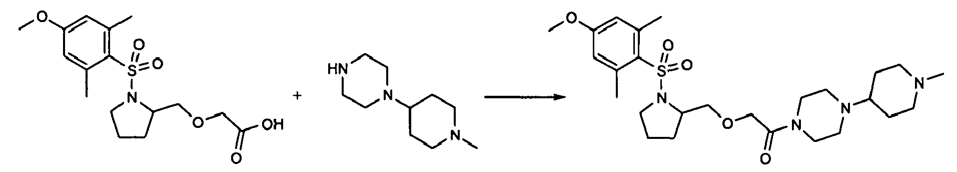

Eine Suspension der Säure (500 mg, 1.4 mmol), Amin (256 mg, 1.4 mmol) und HOAt (19 mg, 0.14 mmol) in CH₂Cl₂ (10 ml) wurde auf 0°C gekühlt. Nach Zugabe von EDCI (296 mg, 1.54 mmol) wurde die Reaktionsmischung für 30 min bei 0°C und über Nacht bei RT unter Stickstoffatmosphäre gerührt. Nach Zugabe von CH₂Cl₂ (20 ml) extrahierte man die organische Phase mit wässriger gesättigter NaHCO₃-Lösung (30 ml) und anschließend die abgetrennte wässrige Phase mit CH₂Cl₂ (15 ml). Die vereinigten organischen Phasen wurden über Na₂SO₄ getrocknet. Nach Filtration und Entfernung des Lösungsmittels unter Vakuum wurde das Produkt über Säulenchromatographie (Silica, CH₂Cl₂/(7 M NH₃ in Methanol) 4/6-93/7) aufgereinigt. Ausbeute: 331 mg, 45%

### Beispiel 208: Herstellung von 2-((1-(4-Methoxy-2,6-dimethylphenylsulfonyl)piperidin-2-yl)methoxy)-1-(3-(pyridin-3-ylmethyl)pyrrolidin-1-yl)ethanon

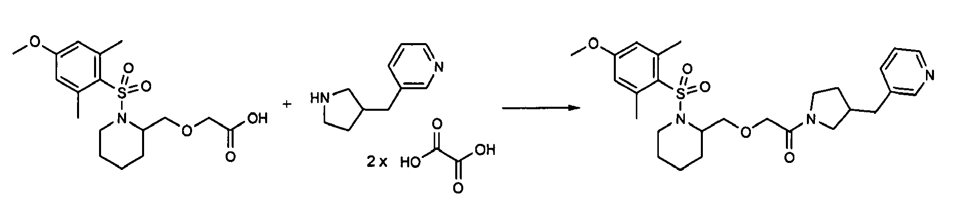

Zu einer Lösung der Säure (520 mg, 1.4 mmol), 3-(Pyrrolidin-3-ylmethyl)pyridin Dioxalat (527 mg, 1.54 mmol), HOAt (28.6 mg, 0.21 mmol)und DIPEA (0.98 ml, 5.6 mmol) in CH₂Cl₂ (10 ml) gab man EDCI (402 mg, 2.1 mmol) und rührte bei RT über Nacht. Nach Entfernung des Lösungsmittels unter Vakuum wurde das Produkt über Säulenchromatographie (Silica, CH₂Cl₂/(7 M NH₃ in Methanol), 99/1 - 96/4) aufgereinigt. Ausbeute: 378 mg, 52%

### Beispiel 206: Herstellung von 2-((1-(4-Methoxy-2,6-dimethylphenylsulfonyl)piperidin-2-yl)methoxy)-1-(1H-pyrrolo[3,4-c]pyridin-2(3H)-yl)ethanon

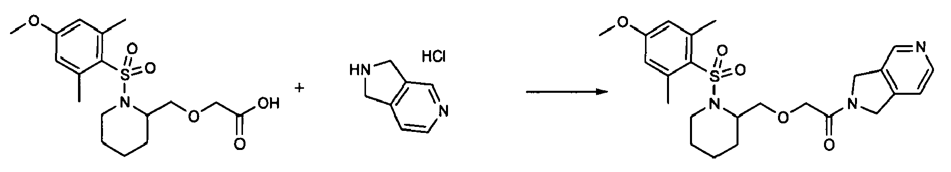

Zu einer Lösung der Säure (627 mg, 1.69 mmol), 2,3-Dihydro-1 H-pyrrolo[3,4-c]pyridin Hydrochlorid (291 mg, 1.86 mmol), HOAt (34.5 mg, 0.25 mmol) und DIPEA (0.89 ml, 5.07 mmol) in CH₂Cl₂ (10 ml) gab man EDCI (486 mg, 2.53 mmol) und rührte bei RT über Nacht. Nach Entfernung des Lösungsmittels unter Vakuum wurde das Produkt über Säulenchromatographie (Silica, CH₂Cl₂/(7 M NH₃ in Methanol), 99/1 - 97/3) aufgereinigt. Ausbeute: 481 mg, 60%

### Beispiel 207: Herstellung von 2-((1-(4-Methoxy-2,6-dimethylphenylsulfonyl)piperidin-2-yl)methoxy)-1-(2-(pyridin-3-yl)morpholino)ethanon

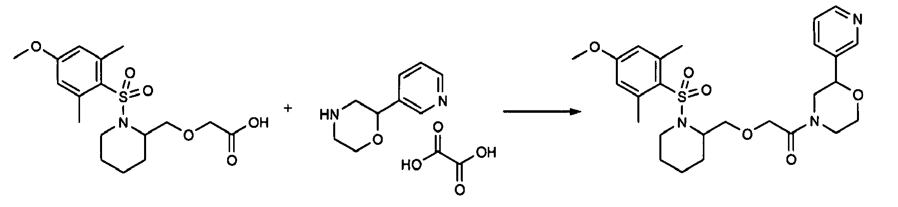

Zu einer Lösung der Säure (642 mg, 1.73 mmol), 2-(Pyridin-3-yl)morpholin Oxalat (483 mg, 1.90 mmol), HOAt (35.3 mg, 0.26 mmol)und DIPEA (0.91 ml, 5.18 mmol) in CH₂Cl₂ (10 ml) gab man EDCI (497 mg, 2.59 mmol) und rührte bei RT über Nacht. Nach Entfernung des Lösungsmittels unter Vakuum wurde das Produkt über Säulenchromatographie (Silica, CH₂Cl₂/(7 M NH₃ in Methanol), 99/1 - 96/4) aufgereinigt. Ausbeute: 453 mg, 51 %

### Beispiel 205: Herstellung von 2-((1-(4-Methoxy-2,6-dimethylphenylsulfonyl)piperidin-2-yl)methoxy)-N-(2-(pyrazin-2-yloxy)benzyl)acetamid

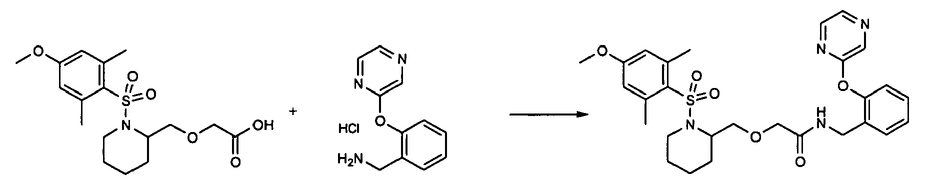

Zu einer Lösung der Säure (749 mg, 2.02 mmol), (2-(Pyrazin-2-yloxy)phenyl)methanamin Hydrochlorid (527 mg, 2.22 mmol), HOAt (41.2 mg, 0.30 mmol)und DIPEA (1.06 ml, 6.05 mmol) in CH₂Cl₂ (10 ml) gab man EDCI (580 mg, 3.02 mmol) und rührte bei RT über Nacht. Nach Entfernung des Lösungsmittels unter Vakuum wurde das Produkt über Säulenchromatographie (Silica, CH₂Cl₂/(7 M NH₃ in Methanol), 99/1 - 97/3) aufgereinigt. Ausbeute: 977 mg, 87%

### Beispiel 196: Herstellung von 2-((1-(4-Methoxy-2,6-dimethylphenylsulfonyl)piperidin-2-yl)methoxy)-N-(4-(pyrazin-2-yloxy)benzyl)acetamid

### Herstellung von 2-((1-(4-Methoxy-2,6-dimethylphenylsulfonyl)piperidin-2-yl)methoxy)acetylchlorid

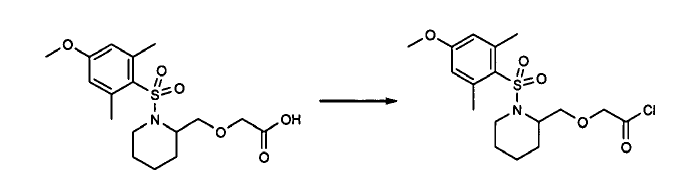

Zu einer Lösung der Säure (5,3 g, 14.3 mmol) in CH₂Cl₂ (100 ml) gab man Oxalylchlorid (3.68 ml, 42.8 mmol) und eine katalytische Menge DMF und rührte bei RT über Nacht. Nach Entfernung des Lösungsmittels unter Vakuum wurde der Rückstand mit CH₂Cl₂ (3 x 50 ml) co-evaporiert. Ausbeute: 5.38 g, 96%

### Herstellung von 2-((1-(4-Methoxy-2,6-dimethylphenylsulfonyl)piperidin-2-yl)methoxy)-N-(4-(pyrazin-2-yloxy)benzyl)acetamid

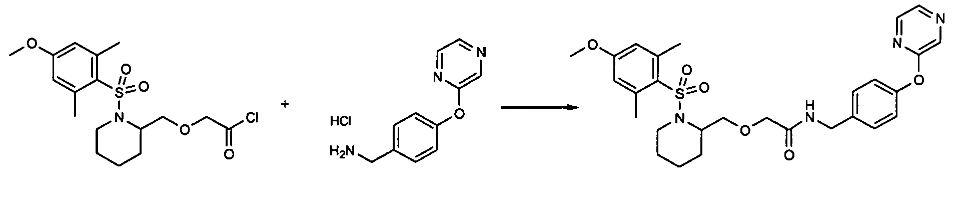

Zu einer Lösung des Säurechlorids (600 mg, 1.54 mmol) und Et₃N (535 µl, 3.85 mmol) in CH₂Cl₂ (10 ml) gab man 4-(Pyrazin-2-yloxy)-benzylamin Hydrochlorid (439 mg, 1.85 mmol) hinzu. Man rührte die Reaktionsmischung für 4 h bei RT. Anschließend wurde das Lösungsmittel unter Vakuum entfernt. Das Produkt wurde über Säulenchromatographie (Silica, CH₂Cl₂/(7 M NH₃ in Methanol), 95/5) aufgereinigt. Ausbeute: 468 mg, 55%

### Beispiel 195: Herstellung von 2-((1-(4-Methoxy-2,6-dimethylphenylsulfonyl)piperidin-2-yl)methoxy)-N-(3-(pyrazin-2-yloxy)benzyl)acetamid

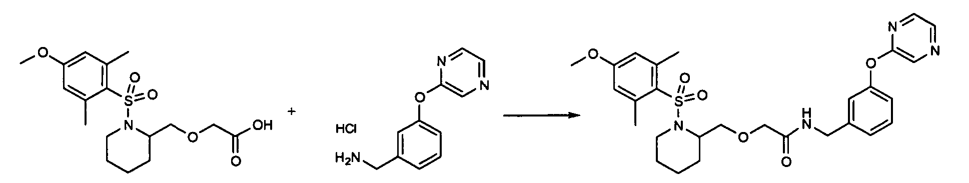

Zu einer Lösung der Säure (500 mg, 1.35 mmol), (3-(Pyrazin-2-yloxy)phenyl)methanamin Hydrochlorid (320 mg, 1.35 mmol), HOAt (27.5 mg, 0.20 mmol) und DIPEA (705 µl, 4.1 mmol) in CH₂Cl₂ (10 ml) gab man bei 0°C EDCI (387 mg, 2.0 mmol) und rührte bei 0°C für 30 min und über Nacht bei RT. Nach Entfernung des Lösungsmittels unter Vakuum wurde das Produkt über Säulenchromatographie (Silica, CH₂Cl₂/(7 M NH₃ in Methanol), 99/1 - 95/5) aufgereinigt. Ausbeute: 412 mg, 55%

### Beispiel 193: Herstellung von N-(4-(4,5-Dihydro-1H-imidazol-2-yl)benzyl)-2-((1-(4-methoxy-2,6-dimethylphenylsulfonyl)piperidin-2-yl)methoxy)-N-methylacetamid Hydrochlorid

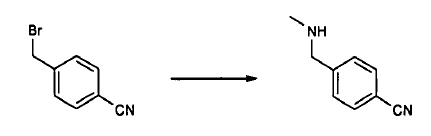

Zu einer Lösung von 4-(Brommethyl)benzonitril (51.3 g, 262 mmol) in EtOH (500 ml) gab man Methylamin (350 ml einer 40%igen wässrigen Lösung, 4.06 mol). Nach 2 h wurde das Lösungsmittel unter Vakuum entfernt und CH₂Cl₂ (500 ml) und wässrige gesättigte NaHCO₃-Löung (400 ml) hinzugegeben. Die abgetrennte organische Phase wurde mit wässriger gesättigter NaCl-Lösung (250 ml) extrahiert, über Na₂SO₄ und das Lösungsmittel nach Filtration unter Vakuum entfernt. Der Rückstand wurde in 1 M HCl in Et₂O (300 ml) aufgenommen und für 30 min gerührt, abfiltriert und mit Et₂O gewaschen. Der Rückstand wurde in H₂O (500 ml) aufgenommen, mit wässriger 6 M NaOH basisch gestellt und mit CH₂Cl₂ (500 ml) extrahiert. Die organische Phase wurde über Na₂SO₄ getrocknet, abfiltriert und das Lösungsmittel Vakuum entfernt. Ausbeute: 31.17 g, 81%

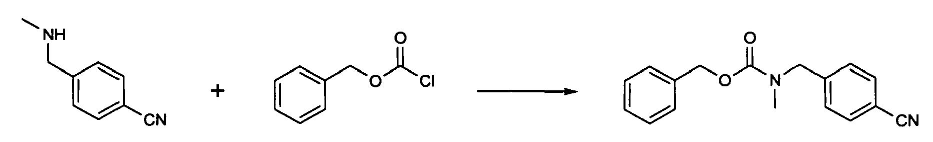

Zu einer Lösung des Amins (31.17 g, 213 mmol) in CH₂Cl₂ (150 ml) wurde Et₃N (35.96 ml, 256 mmol) hinzugegeben. Bei einer Temperatur von 0°C gab anschließend Benzylchloroformat (36.37 ml, 256 mmol) in CH₂Cl₂ (50 ml) tropfenweise hinzu. Die Reaktionsmischung wurde über Nacht bei RT gerührt und mit wässriger 0.1 M HCl (150 ml) und H₂O (150 ml) gewaschen, über Na₂SO₄ getrocknet, abfiltriert und das Lösungsmittel unter Vakuum entfernt.

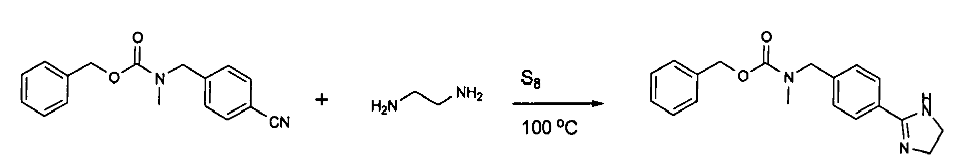

Zu einer Reaktionsmischung des Carbamats (62.1 g, max. 213 mmol) und Ethylenediamin (192 ml, 2.87 mol) gab man Schwefel (3.41 g, 107 mmol) und rührte für 2 h bei 100°C. Nach Abkühlung auf RT wurde H₂O (250 ml) hinzugegeben und extrahierte mit Ethylacetat (2 x 250 ml). Die vereinigten organischen Phasen wurden mit H₂O (250 ml) extrahiert, über Na₂SO₄ getrocknet und das Lösungsmittel nach Filtration unter Vakuum entfernt. Ausbeute 67.6 g, 98%

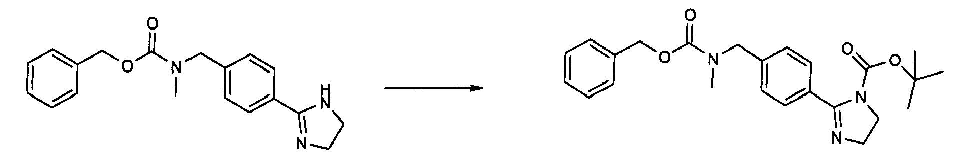

Zu einer Lösung des Imidazolins (67.6 g, 209 mmol) und DMAP (28.1 g, 230 mmol) in CH₂Cl₂ (500 ml), gab man tropfenweise eine Lösung von (Boc)₂O (50.2 g, 230 mmol) in CH₂Cl₂ (500 ml) und rührte bei RT über Nacht. Anschließend wurde 0.5 M HCl (300 ml) und H₂O (300 ml) hinzugegeben. Die abgetrennte organische Phase wurde über Na₂SO₄ getrocknet. Nach Filtration wurde das Lösungsmittel unter Vakuum entfernt und das Produkt über Säulenchromatographie (Silica, CH₂Cl₂/MeOH, 98:2) aufgereinigt. Ausbeute: 50.7 g, 57%

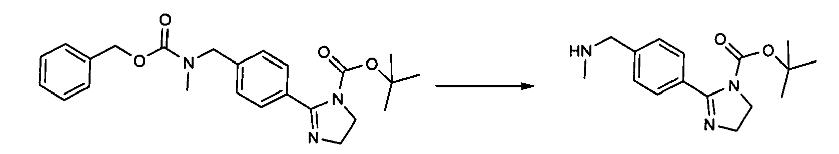

Das Boc-geschützte Imidazolin (3.03 g, 7.15 mmol) wurde in absolutem EtOH (60 ml) gelöst und unter Stickstoff für 10 min mit Pd/C (10%, 381 mg, 0.36 mmol) und Wasserstoff hydriert. Nach Rühren bei RT für 2 h wurde über Kieselgur abfiltriert und mit EtOH gewaschen. Nach Entfernung des Lösungsmittels unter Vakuum wurde das Produkt ohne weitereAufarbeitung in die nächste Stufe eingesetzt.

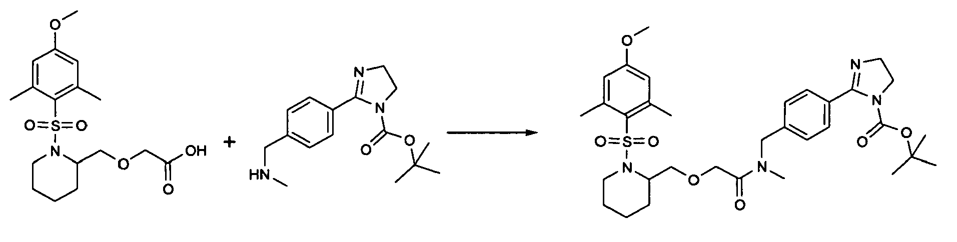

Eine Reaktionsmischung der Carbonsäure (683 mg, 1.84 mmol), Amin (2.10 g, 1.93 mmol), DIPEA (608 µl, 3.68 mmol) and HOAt (38 mg, 0.28 mmol) in CH₂Cl₂ (100 ml) wurde auf 0 °C abgekühlt. Anschließend gab man EDCI (388 mg, 2.02 mmol) zu und rührte bei RT über Nacht. Nach Zugabe von wässriger gesättigter NaCl-Lösung (25 ml) wurde die abgetrennte wässrige Phase mit CH₂Cl₂ (25 ml) extrahiert. Die vereinigten organischen Phasen wurden über Na₂SO₄ getrocknet und das Lösungsmittel nach Filtration unter Vakuum entfernt. Die Aufreinigung erfolgte über Säulenchromatographie (Silica, CH₂Cl₂/MeOH, 95:5). Ausbeute: 750 mg, 63%

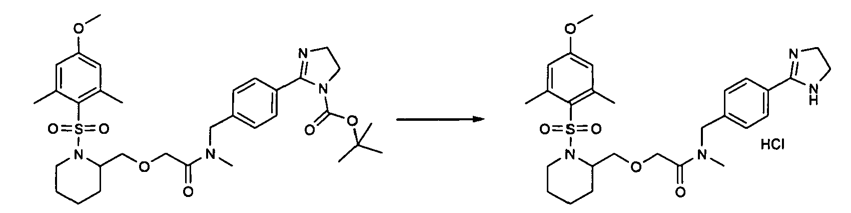

Zu einer Lösung des Boc-geschützten Derivates (750 mg, 1.16 mmol) in CH₂Cl₂ (10 ml) gab man TFA (4.3 ml, 58 mmol) und rührte für 4 h bei RT. Das Lösungsmittel wurde unter Vakuum entfernt, der Rückstand in CH₂Cl₂ (20 ml) gelöst und mit wässriger gesättigter NaHCO₃-Lösung(25 ml) gewaschen. Die abgetrennte organische Phase wurde über Na₂SO₄ getrocknet, abfiltriert und das Lösungsmittel unter Vakuum entfernt. Das Produkt wurde über Säulenchromatographie (S, CH₂Cl₂/MeOH/Et₃N, 95:5:2) aufgereinigt. Das freie Amin wurde für 1 h in 1 M HCl Et₂O (10 ml) gerührt und das Lösungsmittel unter Vakuum entfernt. Man erhielt das Produkt als Hydrochlorid mit einer Ausbeute von 641 mg, 95%.

### Pharmakologische Untersuchungen

### Funktionelle Untersuchung am Bradykinin Rezeptor 1 (B1R)

Die agonistische bzw. antagonistische Wirkung von Substanzen kann am Bradykinin Rezeptor 1 (B1R) der Spezies Mensch und Ratte mit folgendem Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Es werden Chinese Hamster Ovary Zellen (CHO K1 Zellen) verwendet, die stabil mit dem humanen B1 R-Gen (hB1R-Zellen, Euroscreen s.a., Gosselies, Belgien) bzw. dem B1 R-Gen der Ratte (rB1R-Zellen, Axxam, Mailand, Italien) transfiziert sind. Für funktionelle Untersuchungen werden diese Zellen auf schwarze 96-Loch-Platten mit klarem Boden (BD Biosciences, Heidelberg, Deutschland) in einer Dichte von 20.000 - 25.000 Zellen/Loch ausplattiert. Über Nacht werden die Zellen bei 37°C und 5 % CO₂ in Kulturmedium (hB1R-Zellen: Nutrient Mixture Ham's F12, Gibco Invitrogen GmbH, Karlsruhe, Deutschland; rB1R-Zellen: D-MEM/F12, Gibco Invitrogen GmbH, Karlsruhe, Deutschland) mit 10 Vol-% FBS (Fetal bovine serum, Gibco Invitrogen GmbH, Karlsruhe, Deutschland) inkubiert. Am folgenden Tag werden die Zellen mit 2,13 µM Fluo-4 (Molecular Probes Europe BV, Leiden Niederlande) in HBSS-Puffer (Hank's buffered saline solution, Gibco Invitrogen GmbH, Karlsruhe, Deutschland) mit 2,5 M Probenecid (Sigma-Aldrich, Taufkirchen, Deutschland) und 10 mM HEPES (Sigma-Aldrich, Taufkirchen, Deutschland) für 60 min bei 37 °C beladen. Anschließend werden die Platten 2 x mit HBSS-Puffer gewaschen und mit HBSS-Puffer versetzt, der zusätzlich 0,1 % BSA (bovines Serumalbumin; Sigma-Aldrich, Taufkirchen, Deutschland), 5,6 mM Glucose und 0,05 % Gelatine (Merck KGaA, Darmstadt, Deutschland) enthält. Nach einer weiteren Inkubation von 20 Minuten bei Raumtemperatur werden die Platten zur Ca²⁺-Messung im FLIPR eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden Testsubstanzen (10 µM) auf die Zellen pipettiert und der Ca²⁺-Einstrom mit der Kontrolle (hB1 R: Lys-Des-Arg⁹-Bradykinin 0,5 nM; rB1 R: Des-Arg⁹-Bradykinin 100 nM) verglichen. Daraus ergibt sich die Angabe in % Aktivierung bezogen auf das Ca²⁺-Signal nach Zugabe von Lys-Des-Arg⁹-Bradykinin (0,5 nM), bzw. Des-Arg⁹-Bradykinin (100 nM).
Nach 10 Minuten Inkubation werden 0,5 nM Lys-Des-Arg⁹-Bradykinin (hB1 R) bzw. 100 nM Des-Arg⁹-Bradykinin (rB1 R) appliziert und ebenfalls der Einstrom von Ca²⁺ ermittelt.
Antagonisten führen zu einer Unterdrückung des Ca²⁺-Einstroms. Es werden % Inhibition im Vergleich zu der maximal erreichbaren Inhibition berechnet. Die Verbindungen zeigen eine gute Wirksamkeit am humanen und am Ratten-Rezeptor.

| **Beispiel** | **B1R Antagonismus, human [10 µM] % Inhibition** | **B1R Antagonismus, Ratte [10 µM] % Inhibition** | | **Beispiel** | **B1R Antagonismus, human [10 µM] % Inhibition** | **B1R Antagonismus, Ratte [10 µM] % Inhibition** |
|---|---|---|---|---|---|---|
| 1 | 104,68 | 106,8 | | 15 | 96 | 92,47 |
| 2 | 104,12 | 87,41 | | 16 | 95,89 | 25,53 |
| 3 | 103,84 | 108,97 | | 17 | 95,53 | 96,94 |
| 4 | 103,63 | 106,31 | | 18 | 95,33 | 109,83 |
| 5 | 102,96 | 102,66 | | 19 | 95,03 | 98,89 |
| 6 | 102,21 | 94,88 | | 20 | 94,86 | 86,42 |
| 7 | 101,85 | 99,28 | | 21 | 93,47 | 107,92 |
| 8 | 101,66 | 106,6 | | 22 | 92,64 | 21,4 |
| 9 | 101,53 | 85,33 | | 23 | 92,35 | 85,35 |
| 10 | 100 | 91,84 | | 24 | 92,25 | 106,89 |
| 11 | 99,62 | 105,97 | | 25 | 91,03 | 80,59 |
| 12 | 99,1 | 74,91 | | 26 | 86,78 | 4,56 |
| 13 | 97,67 | 112,52 | | 27 | 85,41 | 97,69 |
| 14 | 96,97 | 104,22 | | 28 | 84,73 | 0 |
| 29 | 84,37 | 109,8 | | 78 | 45,73 | 35,22 |
| 30 | 83,97 | 108,46 | | 79 | 45,47 | 54,49 |
| 31 | 83,75 | 94,69 | | 80 | 45,26 | 22,47 |
| 32 | 83,26 | 63,28 | | 81 | 44,9 | 34,14 |
| 33 | 82,71 | 47,69 | | 82 | 44,86 | 4,42 |
| 34 | 79,9 | 39,48 | | 83 | 43,94 | 30,77 |
| 35 | 79,7 | 99 | | 84 | 43,73 | 0 |
| 36 | 79,43 | 45,71 | | 85 | 43,69 | 40,84 |
| 37 | 78,23 | 102,66 | | 86 | 43,22 | 0 |
| 38 | 74,95 | 65,36 | | 87 | 42,69 | 44,38 |
| 39 | 73,21 | 0 | | 88 | 42,06 | 7,33 |
| 40 | 72,7 | 88,65 | | 89 | 41,34 | 0 |
| 41 | 72,61 | 16,38 | | 90 | 49,96 | 99,55 |
| 42 | 70,57 | 57, 85 | | 91 | 48,78 | |
| 43 | 69,88 | 24,16 | | 92 | 103,49 | 101,34 |
| 44 | 69,62 | 0 | | 93 | 105,57 | 99,12 |
| 45 | 69,07 | 92,41 | | 94 | 105,3 | 103,1 |
| 46 | 68,25 | 0 | | 95 | 106,86 | 100,63 |
| 47 | 68,07 | 48,84 | | 96 | 104,91 | 91,35 |
| 48 | 66,59 | 30,49 | | 97 | 98,05 | 98,2 |
| 49 | 65,88 | 92,34 | | 98 | 104,09 | 101,37 |
| 50 | 65,71 | 50,31 | | 99 | 103,76 | 100,08 |
| 51 | 65,06 | 87,91 | | 100 | 103,71 | 101,69 |
| 52 | 65,01 | 92,83 | | 101 | 100,27 | 100,47 |
| 53 | 64,83 | 0 | | 102 | 103,94 | 99,95 |
| 54 | 63,8 | 0 | | 103 | 105,15 | 100,39 |
| 55 | 63,23 | 88,78 | | 104 | 105,3 | 100,57 |
| 56 | 62,66 | 0 | | 105 | 105,66 | 100,37 |
| 57 | 62,39 | 0 | | 106 | 102,6 | 102,64 |
| 58 | 62,23 | 10,18 | | 107 | 102,05 | 102,39 |
| 59 | 61,48 | 0 | | 108 | 102,41 | 102,31 |
| 60 | 59,89 | 108,64 | | 109 | 102,82 | 101,69 |
| 61 | 59,7 | 30,54 | | 110 | 102,5 | 101,77 |
| 62 | 59,58 | 33,97 | | 111 | 91,86 | 101,4 |
| 63 | 58,57 | 26,41 | | 112 | 102,43 | 101,88 |
| 64 | 57,92 | 25,48 | | 113 | 99,23 | 97,07 |
| 65 | 55,41 | 93,17 | | 114 | 102,24 | 102,56 |
| 66 | 55,38 | 38,64 | | 115 | 102,47 | 58,09 |
| 67 | 55,03 | 80,49 | | 116 | 74,25 | 100,94 |
| 68 | 54,11 | 0 | | 117 | 100,65 | 100,53 |
| 69 | 53,64 | 82,89 | | 118 | 103,54 | 102,07 |
| 70 | 53,27 | 42,16 | | 119 | 95,94 | 61,85 |
| 71 | 53,19 | 95,22 | | 120 | 103,28 | 102,56 |
| 72 | 52,58 | 13,25 | | 121 | 70,47 | 52,72 |
| 73 | 51,02 | 105,54 | | 122 | 12,11 | 13,04 |
| 74 | 50,42 | 102,6 | | 123 | 43,88 | 79,94 |
| 75 | 50,14 | 9,11 | | 124 | 76,28 | 63,16 |
| 76 | 48,94 | 40,3 | | 125 | 50,56 | 95,36 |
| 77 | 47,95 | 104,71 | | 126 | 46,36 | 81,06 |
| 127 | 102,31 | 93,69 | | 177 | 103,86 | 100,43 |
| 128 | 90,8 | 94,47 | | 178 | 101,6 | 99,82 |
| 129 | 0,03 | 31,23 | | 179 | 104,26 | 100,13 |
| 130 | 103,2 | 96,3 | | 180 | 104 | 98,6 |
| 131 | 82,09 | 95,37 | | 181 | 105,03 | 98,54 |
| 133 | 103,01 | 98,08 | | 182 | 91,1 | 99,72 |
| 134 | 102,44 | 98,25 | | 183 | 100,16 | 96,92 |
| 135 | 102,68 | 98,31 | | 184 | 104,83 | 99 |
| 136 | 103,97 | 98,94 | | 185 | 105,43 | 97,93 |
| 137 | 102,25 | 98,75 | | 186 | 105,87 | 97,13 |
| 138 | 103,55 | 98,45 | | 187 | 100,41 | 101,8 |
| 139 | 99,91 | 98,83 | | 188 | 101,92 | 102,25 |
| 140 | 100,08 | 80,7 | | 189 | 70,72 | 86,37 |
| 141 | 100,51 | 100,7 | | 190 | 101,56 | 101,94 |
| 142 | 89,91 | 62,99 | | 191 | - | - |
| 143 | 99,91 | 99,3 | | 192 | 105,69 | 100,29 |
| 144 | 100,57 | 98,16 | | 193 | 103,95 | 98,28 |
| 145 | 99,4 | 98,64 | | 194 | 103,08 | 100,27 |
| 146 | 97,26 | 99,64 | | 195 | 85,11 | 98,81 |
| 147 | 99,95 | 99,96 | | 196 | 100,47 | 98,8 |
| 148 | 100,09 | 99,97 | | 197 | 103,6 | 100 |
| 149 | 99,41 | 100,05 | | 198 | 105,4 | 99,75 |
| 150 | 98,31 | 99,06 | | 199 | 104,01 | 100,83 |
| 151 | 106,94 | 103,01 | | 200 | 102,44 | 98,73 |
| 152 | 100,06 | 90,83 | | 201 | 103,19 | 98,19 |
| 153 | 81,64 | 40,58 | | 202 | 103,84 | 99,79 |
| 154 | 83,21 | 84,36 | | 203 | 103,19 | 98,78 |
| 155 | 107,43 | 89,64 | | 204 | 104,58 | 98,98 |
| 156 | 108,3 | 102,71 | | 205 | 49,98 | 99,71 |
| 157 | 98,2 | 99,97 | | 206 | 53,94 | 99,49 |
| 158 | 98,49 | 99,3 | | 207 | 101,93 | 100,16 |
| 159 | 99,17 | 100,6 | | 208 | 104,84 | 100,76 |
| 160 | 98,97 | 99,04 | | 209 | 103,97 | 99,93 |
| 161 | 98,53 | 99,25 | | 210 | 106,17 | 96,03 |
| 162 | 99,53 | 99,51 | | 211 | 106,08 | 100,06 |
| 163 | 95,05 | 100,8 | | 212 | 106,74 | 100,18 |
| 164 | 99,63 | 100,63 | | 213 | 59,72 | 94,53 |
| 165 | 97,13 | 96,78 | | 214 | 101,16 | 101,91 |
| 166 | 99,44 | 99,28 | | 215 | 99,94 | 100,96 |
| 167 | 101,53 | 100,86 | | 216 | -10,12 | 4,48 |
| 168 | 93,83 | 98,38 | | 217 | - | - |
| 169 | 100,44 | 101,1 | | 218 | 100,43 | |
| 170 | 101,02 | 97,85 | | 219 | 5,04 | 96,23 |
| 171 | 101,89 | 82,78 | | 220 | 15,27 | 86,34 |
| 172 | 101,45 | 85,19 | | 221 | 38,04 | 89,58 |
| 173 | 100,61 | 100,68 | | 222 | 7,55 | 113,93 |
| 174 | 101,35 | 97,95 | | 223 | 26,23 | 86,37 |
| 175 | 103,24 | 86,31 | | 224 | 72,06 | 52,77 |
| 176 | -24,81 | 36,52 | | 225 | 104,47 | 84,08 |
| 226 | 76,38 | 96,87 | | 275 | 101,73 | 93,06 |
| 227 | 26,87 | 94,52 | | 276 | 98,92 | 94,16 |
| 228 | 88,56 | 99,82 | | 277 | 85,55 | 81,59 |
| 229 | 101,09 | 100,53 | | 278 | 103,71 | 96,94 |
| 230 | 95,42 | 62,33 | | 279 | 103,74 | 95,39 |
| 231 | 102,03 | 83,34 | | 280 | 103,9 | 92,04 |
| 232 | 102,37 | 90,03 | | 281 | 103,69 | 92,37 |
| 233 | 101,96 | 86,74 | | 282 | 102,79 | 85,95 |
| 234 | 51,96 | 103,02 | | 283 | 103,46 | 92,9 |
| 235 | 102,3 | 83,46 | | 284 | 102,93 | 93,55 |
| 236 | 95,86 | 99,58 | | 285 | 103,77 | 90,97 |
| 237 | 99,72 | 99,45 | | 286 | 103,72 | 97,93 |
| 238 | 99,93 | 99,35 | | 287 | 101,02 | 89,25 |
| 239 | 94,7 | 99,7 | | 288 | 101,83 | 93,34 |
| 240 | 78,48 | 97,3 | | 289 | 102,52 | 95,5 |
| 241 | 103,76 | 99,26 | | 290 | 104,09 | 92,91 |
| 242 | 102,04 | 87,99 | | 291 | 104,28 | 92,99 |
| 243 | 24,76 | 80,63 | | 292 | 105,83 | 99,22 |
| 244 | 70,16 | 97,28 | | 293 | 90,51 | -6,96 |
| 245 | 69,86 | 80,5 | | 294 | 105,91 | 100 |
| 246 | 56,38 | 72,1 | | 295 | 96,67 | 103,18 |
| 247 | 70,78 | 96,75 | | 296 | 105,85 | 99,28 |
| 248 | 95,6 | 89,86 | | 297 | 105,76 | 102,53 |
| 249 | 59,22 | 92,36 | | 298 | 105,09 | 103,58 |
| 250 | 96,85 | 90,55 | | 299 | 106,32 | 102,79 |
| 251 | 45,81 | 98,51 | | 300 | 105,73 | 103,1 |
| 252 | 101,72 | 97,94 | | 301 | 106,36 | 100,99 |
| 253 | 59,31 | 101,11 | | 302 | 100,69 | 102,41 |
| 254 | 105,08 | 97,8 | | 303 | 106,27 | 100,4 |
| 255 | 104,91 | 100,81 | | 304 | 71,12 | 101,52 |
| 256 | 50,23 | 91,02 | | 305 | 76,41 | 95,47 |
| 257 | 34,31 | 99,94 | | 306 | 103,75 | 99,16 |
| 258 | 75,25 | 99,75 | | 307 | 105,47 | 98,38 |
| 259 | 66,06 | 99,3 | | 308 | 104,85 | 100,95 |
| 260 | 101,6 | 60,62 | | 309 | 52,02 | 101,11 |
| 261 | 78,92 | 98,87 | | 310 | 106,11 | 102,07 |
| 262 | 81,81 | 99,65 | | 311 | 94,88 | 103,15 |
| 263 | 100,01 | 97,85 | | 312 | 98,52 | 98,68 |
| 264 | 103,07 | 31,71 | | 313 | 103,46 | 102,58 |
| 265 | 99,95 | 51,3 | | 314 | 105,18 | 103,91 |
| 266 | 102,46 | 2,12 | | 315 | 105,13 | 100,59 |
| 267 | 103,79 | 95,16 | | 316 | 82,66 | 98,17 |
| 268 | 103,88 | 101,64 | | 317 | 92,19 | 100,22 |
| 269 | 103,6 | 99 | | 318 | 101,58 | 101,55 |
| 270 | 103,77 | 93,42 | | 319 | 104,47 | 100,08 |
| 271 | 59,08 | 85,9 | | 320 | 105,66 | 101,93 |
| 272 | 97,14 | 84,61 | | 321 | 41,19 | 100,13 |
| 273 | 96,05 | 84,87 | | 322 | 101,81 | 98,72 |
| 274 | 103,75 | 98,08 | | 323 | 104,72 | 88,06 |
| 324 | 83,97 | 98,44 | | 373 | 104,41 | 98,85 |
| 325 | 101,91 | 100,88 | | 374 | 80,78 | 101,66 |
| 326 | 103,59 | 101,04 | | 375 | 98,74 | 99,85 |
| 327 | 104,27 | 100,52 | | 376 | 67,07 | 99,02 |
| 328 | 103,86 | 101,85 | | 377 | 87,21 | 99,56 |
| 329 | 104,89 | 98,16 | | 378 | 63,42 | 98,75 |
| 330 | 104,39 | 100,44 | | 379 | 67,92 | 101,75 |
| 331 | 105,94 | 100,52 | | 380 | 91,51 | 100,9 |
| 332 | 105,29 | 100,79 | | 381 | 77,88 | 100,15 |
| 333 | 107,07 | 100,76 | | 382 | 103,44 | 101,03 |
| 334 | 106,56 | 100,58 | | 383 | 40,17 | 101,54 |
| 335 | 106,11 | 99,82 | | 384 | 104,56 | 98,56 |
| 336 | 103,22 | 104,8 | | 385 | 101,05 | 99,45 |
| 337 | 103,65 | 104,4 | | 386 | 100,74 | 97,86 |
| 338 | 92,57 | 92,22 | | 387 | 90,98 | 100,56 |
| 339 | 77,54 | 41,63 | | 388 | 22,88 | 100,66 |
| 340 | 104,92 | 100,73 | | 389 | 100,51 | 100,49 |
| 341 | 104,72 | 102,33 | | 390 | 102,87 | 99,78 |
| 342 | 104,5 | 100,29 | | 391 | 97,78 | 101,54 |
| 343 | 79,97 | 100,23 | | 392 | 100,93 | 99,09 |
| 344 | 48,77 | 88,4 | | 393 | 103,77 | 101,49 |
| 345 | 22,26 | 95,94 | | 394 | 31,48 | 99,08 |
| 346 | 95.54 | 100,91 | | 395 | 12,63 | 100,41 |
| 347 | 56,56 | 102,91 | | 396 | 24,69 | 80,61 |
| 348 | 87,09 | 102,94 | | 397 | 97,25 | 101,46 |
| 349 | 100,78 | 102,8 | | 398 | 59,84 | 98 |
| 350 | 103,91 | 103,72 | | 399 | 93,05 | 102,16 |
| 351 | 44,01 | 103,99 | | 400 | 62,01 | 102,17 |
| 352 | 103,57 | 103,63 | | 401 | 90,84 | 95,62 |
| 353 | 102,72 | 102,17 | | 402 | 47,09 | 101,25 |
| 354 | 62,46 | 102,22 | | 403 | 100,68 | 100,41 |
| 355 | 83,39 | 101,52 | | 404 | 55,06 | 98,55 |
| 356 | 43,04 | 103,33 | | 405 | 98,59 | 100,41 |
| 357 | 79,38 | 104,35 | | 406 | 102,96 | 100,21 |
| 358 | 101,48 | 102,53 | | 407 | 102,44 | 99,02 |
| 359 | 98,56 | 102,84 | | 408 | 102,22 | 99,79 |
| 360 | 96,66 | 103,03 | | 409 | 28,09 | 81,39 |
| 361 | 93,42 | 100,74 | | 410 | 102,43 | 102,85 |
| 362 | 103,34 | 101,91 | | 411 | 101,07 | 100,19 |
| 363 | 90,7 | 101,09 | | 412 | 101,44 | 102,89 |
| 364 | 104,69 | 101,44 | | 413 | 99,93 | 102,79 |
| 365 | 55,61 | 99,85 | | 414 | 100,97 | 100,12 |
| 366 | 14,76 | 99,03 | | 415 | 99,23 | 97,52 |
| 367 | 20,1 | 102,67 | | 416 | 50,22 | 100,96 |
| 368 | 62,06 | 100,94 | | 417 | 96,16 | 103,38 |
| 369 | 82,34 | 100,93 | | 418 | 101,28 | 103,53 |
| 370 | 18,72 | 98,83 | | 419 | 99,98 | 101,17 |
| 371 | 0,38 | 100,72 | | 420 | 100,33 | 92,05 |
| 372 | -2,76 | 83,81 | | 421 | 97,3 | 93,36 |
| 422 | 48,04 | 98,61 | | 471 | 102,93 | 98,79 |
| 423 | 58,71 | 80,13 | | 472 | 92,06 | 101,17 |
| 424 | 35,11 | 97,61 | | 473 | 94,59 | 99,49 |
| 425 | -0,97 | 98,84 | | 474 | 96,7 | 96,3 |
| 426 | -4,95 | 94,38 | | 475 | 90,42 | 101,75 |
| 427 | 101,54 | 98,83 | | 476 | 90,06 | 100,14 |
| 428 | 23,84 | 101,75 | | 477 | 94,46 | 98,77 |
| 429 | 14,33 | 99,48 | | 478 | 36,16 | 90,24 |
| 430 | 38,56 | 100,57 | | 479 | 32,3 | 99,07 |
| 431 | 28,42 | 96,18 | | 480 | 100,76 | 102,62 |
| 432 | 94,03 | 100,3 | | 481 | 72,64 | 103,71 |
| 433 | 103,47 | 99,97 | | 482 | 97,92 | 102,46 |
| 434 | 103,69 | 101,47 | | 483 | 102,72 | 104,43 |
| 435 | 6,23 | 99,56 | | 484 | 75,57 | 100,57 |
| 436 | 39,2 | 101,6 | | 485 | 65,25 | 103,41 |
| 437 | 36,08 | 101,67 | | 486 | 70,44 | 101,22 |
| 438 | 22,12 | 99,98 | | 487 | 100,93 | 99,13 |
| 439 | 48,25 | 100,94 | | 488 | 100,99 | 101,64 |
| 440 | 98,07 | 95,62 | | 489 | 99,28 | 99,96 |
| 441 | 103,96 | 98,36 | | 490 | 93,52 | 103,22 |
| 442 | 101,8 | 98,56 | | 491 | 96,35 | 97,94 |
| 443 | 103,85 | 99,33 | | 492 | 102,54 | 103,95 |
| 444 | 92,79 | 98,58 | | 493 | 40,64 | 99,47 |
| 445 | 102,23 | 98,61 | | 494 | 26,16 | 73,41 |
| 446 | 95,03 | 99,75 | | 495 | 30,7 | 95,72 |
| 447 | 66,5 | 94,21 | | 496 | 68,19 | 103,02 |
| 448 | 85,75 | 99,78 | | 497 | 66,04 | 101,25 |
| 449 | 90,75 | 99,72 | | 498 | 94,25 | 96,7 |
| 450 | 87,36 | 99,66 | | 499 | 103,35 | 100,58 |
| 451 | 80,68 | 99,23 | | 500 | 41,81 | 93,22 |
| 452 | 104,82 | 95,19 | | 501 | 65,06 | 89,7 |
| 453 | 40,51 | 74,44 | | 502 | 96,74 | 103,44 |
| 454 | 101,91 | 100,32 | | 503 | 103,07 | 97,92 |
| 455 | 102,81 | 99,34 | | 504 | 24,18 | 90,61 |
| 456 | 100,44 | 100,76 | | 505 | 74,02 | 100,82 |
| 457 | 102 | 99,65 | | 506 | 98,27 | 98,91 |
| 458 | 102,65 | 100,43 | | 507 | -10,08 | 102,4 |
| 459 | 57,14 | 102,04 | | 508 | 31,93 | 102,55 |
| 460 | 96,59 | 100,78 | | 509 | 92,72 | 102,13 |
| 461 | 100,64 | 99,1 | | 510 | 51,42 | 101,9 |
| 462 | 101,3 | 98,25 | | 511 | 86,96 | 102,91 |
| 463 | 97,39 | 100,72 | | 512 | 93,03 | 102,26 |
| 464 | 56,56 | 101,94 | | 513 | 73,53 | 101,16 |
| 465 | 101,95 | 98,82 | | 514 | 85,25 | 99,02 |
| 466 | 92,52 | 99,72 | | 515 | 30,9 | 100,15 |
| 467 | 101,34 | 100,88 | | 516 | 101,34 | 99,39 |
| 468 | 84,49 | 101,4 | | 517 | 98,85 | 101,92 |
| 469 | 92,34 | 101,61 | | 518 | 102,58 | 99,69 |
| 470 | 92,67 | 98,07 | | 519 | 81,88 | 102,71 |
| 520 | 102,91 | 102,07 | | 553 | 54,09 | 100,52 |
| 521 | 85,17 | 99,34 | | 554 | 25,2 | 101,97 |
| 522 | 54,17 | 98,7 | | 555 | 62,48 | 102,09 |
| 523 | 66,38 | 101,41 | | 556 | 12,13 | 85,58 |
| 524 | 101,62 | 100,66 | | 557 | 29,73 | 87,56 |
| 525 | 15,41 | 102,31 | | 558 | -3,53 | 102,69 |
| 526 | 73,35 | 101,65 | | 559 | 99,91 | 100,17 |
| 527 | 97,16 | 97,33 | | 560 | 101,72 | 99,35 |
| 528 | 88,22 | 101,57 | | 561 | 102,13 | 101,17 |
| 529 | 105,85 | 101,47 | | 562 | 102,46 | 100,03 |
| 530 | 93,08 | 101,41 | | 563 | 101,86 | 100,42 |
| 531 | 92,59 | 96,17 | | 564 | 104,58 | 102,33 |
| 532 | 96,4 | 107,74 | | 565 | 104,82 | 102,52 |
| 533 | 105,76 | 107,71 | | 566 | 103,44 | 102,51 |
| 534 | 104,14 | 107,17 | | 567 | 104,25 | 102,19 |
| 535 | 48,36 | 107,33 | | 568 | 38,49 | 81,34 |
| 536 | 63,12 | 107,33 | | 569 | 90,04 | 92,89 |
| 537 | 12,53 | 98,72 | | 570 | 97,65 | 98,06 |
| 538 | -11,76 | 105,77 | | 571 | 103,97 | 100,34 |
| 539 | 38,81 | 107,39 | | 572 | - | 98,98 |
| 540 | 7,17 | 100,18 | | 573 | - | 101,21 |
| 541 | 103,13 | 107,61 | | 574 | 89,99 | - |
| 542 | 49,24 | 107,2 | | 575 | 23,44 | 96,83 |
| 543 | 21,14 | 104,54 | | 576 | 1,85 | 107,17 |
| 544 | 11,72 | 106,99 | | 577 | 17,34 | 107,05 |
| 545 | 39,81 | 106,41 | | 578 | 8,96 | 87,13 |
| 546 | 7,44 | 104,05 | | 579 | 39,4 | 107,07 |
| 547 | 77,44 | 99,97 | | 580 | 39,09 | 88,5 |
| 548 | 24,68 | 95,44 | | 581 | 27,54 | 76,31 |
| 549 | 75,84 | 99,05 | | | | |
| 550 | 57,78 | 101,12 | | | | |
| 551 | 52,7 | 101,87 | | | | |
| 552 | 46,78 | 101,41 | | | | |

### Hemmung der Bradykinin Rezeptor1 (B1 R)-vermittelten Bildung von IL-6 bei Fibroblasten durch erfindungsgemäße Substanzen als B1 R-Antagonisten

Die pro-entzündlichen Zytokine TNFα (oder IL-1) führen bei verschiedenen Zelltypen wie z.B. Fibroblasten zu einer Aktivierung, welche u. a. eine verstärkte Expression des B1 R bewirkt. Eine anschließende Stimulation dieser Zellen mit einem B1 R-Agonisten resultiert in der Bildung weiterer pro-entzündlicher Zytokine wie z.B. IL-6. Hierdurch wird die Chronifizierung von Entzündungen gefördert. Eine Behandlung mit einem B1R-Antagonisten sollte dazu führen, die B1R-Agonist-induzierte IL-6 Bildung zu hemmen. Beispielhaft wurde einer der im FLIPR-Assay identifizierten B1R-Antagonisten daraufhin untersucht.

### Methoden:

Die humane Fibroblasten Zelllinie IMR-90 (ATCC, CCL-186) wurde in Kulturmedium (Nutrient mixture Ham's F12, Gibco Invitrogen GmbH, Karlsruhe Deutschland mit 10% FBS, Fetal bovine serum, Gibco, Invitrogen GmbH, Karlsruhe Deutschland; 0,1 mM Non-Essential Amino Acids, Gibco, 11140-035, 1 mM Sodium Pyruvate, Euro Clone, ECM0542D; 1,5g/l Sodium bicarbonate, Euro Clone, ECM0980D) auf 80 cm2 Flaschen (Nunc;178905) passagiert (Ratio:1:2 to 1:6; Mediumerneuerung alle 3-4 Tage) und für Experimente mit 1x10⁵ Zellen pro Loch auf 96-Loch-Platten (Greiner bio-one; No.655180) ausplattiert. Über Nacht werden die Zellen bei 37°C und 5 % CO₂ inkubiert.

Zur Aktivierung mit TNFα (human, recombinant, expressed in E.coli, SIGMA-ALDRICH T6674, 10 ng/ml) alleine oder TNFα kombiniert mit dem humanem B1R-Agonisten Lys-Des-Arg⁹-Bradykinin (Lys-Des-Arg⁹-BK) wurden die IMR-90 Fibroblasten über Nacht bei 37°C und 5% CO₂ in Kulturmedium inkubiert. Die Lys-Des-Arg⁹-BK Endkonzentrationen betrugen 1000nM bzw. 10nM. Manche IMR-90 Stimulationsansätze enthielten zusätzlich den B1R-Antagonisten Beispiel 8 in einer Endkonzentration von 10µM. 24 Stunden nach Aktivierung wurde von allen unterschiedlichen Stimulationsansätzen jeweils 150 µl Kulturmedium entnommen und auf eine neue Platte übertragen und eingefroren. Nach dem Auftauen der Überstände erfolgte die Bestimmung des Gehalts an IL-6 mittels eines kommerziell erhältlichen IL-6 Elisa.

### Elisa-Assay (Durchführung, modifiziert nach Angaben des Herstellers):

Elisa-Kit: Firma: BIOSOURCE; CytoSets^{™}, Art.-Nr.: CHC 1264
Material: 96 well Mikroplatten: NUNC Brand Systems, Art.-Nr.: 442404A
Durchführung:
Coating: Auftrag primärer Antikörperlösung: 50 µL / well
Inkubation: abgedeckte Platte über Nacht bei RT, anschließend Platte ausklopfen
Blocking: Auftrag Blocking-Puffer: 300 µL/well; Platte 2 Std. bei RT inkubieren
Waschen: 3 x mit 300 µL Wasch-Lösung / well
Standard; Proben; sekundärer Antikörper: Auftrag Standard, Proben je 50 µL / well
sofortige Zugabe der sekundären Antikörperlösung: Auftrag: 25 µL / well
Inkubation: abgedeckte Platte 2 Std. bei RT unter Schütteln
Waschen: 3 x mit 300 µL Wasch-Lösung / well
Streptavidin: Auftrag Streptavidinlösung: 100 µL / well
Inkubation: abgedeckte Platte 30 Min. bei RT
Waschen: 3 x mit 300 µL Wasch-Lösung / well
Substrat: Auftrag Substratlösung: 100 µL / well
Inkubation: Platte ca. 20 Min. bei RT im Dunkeln unter Schütteln (abhängig von der Farbreaktion)
Abstoppen: Zugabe 1,8 N H₂SO₄: 50 µL / well
Messen (innerhalb von 30 Min.):
ELISA-Reader: Fa. Mikrotek; MPP 4008
Auswertesoftware: Fa. Mikrotek; MikroWin 3.0
Messfilter: 450 nm
Referenzfilter: 620 nm

Die oben beschriebenen Verfahren wurden mit Beispielverbindung 8, , durchgeführt. Die Ergebnisse sind in der folgenden Tabelle dargestellt:

| | Produktion von IL-6 (pg/ml) durch IMR-90 Fibroblasten | | |
|---|---|---|---|
| | TNFα alleine | TNFα + Lys-Des-Arg⁹-BK (1000nM) | TNFα + Lys-Des-Arg⁹-BK (10nM) |
| ohne Antagonist | 149 pg/ml | 2155 pg/ml | 2462 pg/ml |
| Beispiel 8 | 136 pg/ml | 483 pg/ml | 119 pg/ml |

IMR-90 Fibroblasten stimuliert mit TNFα oder Lys-Des-Arg⁹-BK alleine produzieren nur geringe Mengen an IL-6 (149pg/ml bzw. 34pg/ml). Die Stimulation mit TNFα in Kombination mit Lys-Des-Arg⁹-BK resultiert in einer circa 10- bis 20-fach gesteigerten IL-6 Synthese. Diese Aktivierung der IL-6 Bildung wird durch den B1R-Antagonisten Beispiel 8 dosisabhängig gehemmt. Die Wirkung der relativ niedrigen Dosis des Agonisten Lys-Des-Arg⁹-BK (10nM) wird nahezu komplett aufgehoben wohingegen eine relativ hohe Dosis von Lys-Des-Arg⁹-BK (1000nM) noch partiell inhibiert wird. Die inhibitorische Wirkung von Beispiel 8 ist B1R-spezifisch, da der aktivierende Effekt von TNFα alleine nicht beeinträchtigt ist.

### Formalintest Maus:

Der Formalin Test (Dubuisson, D. and Dennis, S.G., 1977, Pain, 4, 161 - 174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustern erfasst wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

### Formalintest Maus:

Formalin wird mit dem Volumen von 20 µl und einer Konzentration von 1 % subkutan in die dorsale Seite der rechten Hinterpfote jedes Tieres appliziert. Die spezifischen Verhaltensänderungen, wie Anheben, Schütteln oder Lecken der Pfote (score 3, Dubuisson & Dennis, 1977), werden im Beobachtungszeitraum von 21 bis 24 min nach Formalininjektion beobachtet und registriert. Das Verhalten der Tiere nach Substanzgabe (n = 10 pro Substanzdosierung) wurde mit einer Kontrollgruppe, welcher Vehikel appliziert wurde (n=10) verglichen.

Basierend auf der Quantifizierung des Schmerzverhaltens wurde die Substanzwirkung im Formalin-Test als Änderung gegenüber der Kontrolle in Prozent ermittelt. Die Berechnungen der ED₅₀ (ED₅₀ = mittlere effektive Dosis) erfolgten mittels Regressionsanalyse nach der Methode von Litchfield and Wilcoxon (Litchfield, J.T., Wilcoxon, J.J., 1949, J. Pharmacol. Exp. Ther. 96, 99 - 113). Abhängig von der Applikationsart der erfindungsgemäßen Verbindungen wurde der Applikationszeitpunkt vor der Formalin-Injektion gewählt (intravenös: 5 min).

Die ED₅₀ -Werte einiger Beispiele sind in nachfolgender Tabelle wiedergegeben:

| Beispiel | Applikationsart | ED₅₀ -Wert [mg/kg] |
|---|---|---|
| 8 | i.v. | 12,8 |
| 97 | i.v. | 13,3 |
| 98 | i.v. | 13,6 |
| 193 | i.v. | 2,59 |

### Parenterale Lösung eines erfindungsgemäßen substituierten Sulfonamid-Derivats

38 g eines der erfindungsgemäßen substituierten Sulfonamid-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

In einem weiteren Aspekt betrifft die vorliegenden Erfindung ein Verfahren zur Herstellung der erfindungsgemäßen substituierten Sulfonamid-Derivate gemäß dem nachfolgend gezeigten Reaktonsschema, 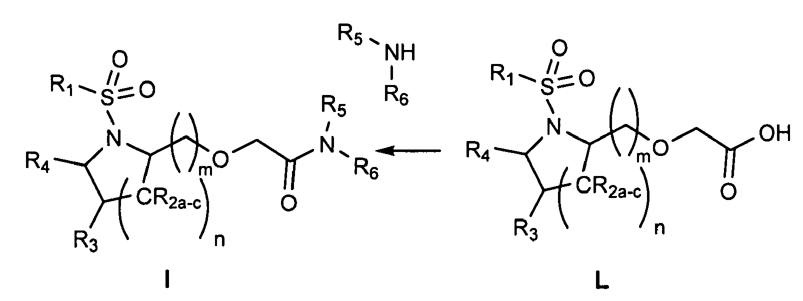 wobei Carbonsäuren der allgemeinen Formel L in einer Amidbildung mit primären oder sekundären Aminen der allgemeinen Formel M, in Gegenwart wasserentziehender Mittel wie Natrium- oder Magnesiumsulfat, Phosphoroxid oder Reagenzien wie beispielsweise CDI, DCC, TBTU, EDCI, oder in Gegenwart von HOAt oder HOBt und einer organischen Base, beispielsweise DIPEA oder Pyridin, in einem organischen Lösungsmittel, beispielsweise THF, Dichlormethan, Diethylether, Dioxan, DMF oder Acetonitril umgesetzt werden.

In einem weiteren Aspekt betrifft die vorliegende Erfindung Arzneimittel enthaltend wenigstens ein erfindungsgemäßes, substituiertes Sulfonamid-Derivat, gegebenenfalls enthaltend geeignete Zusatz- und/oder Hilfsstoffe und/oder gegebenenfalls weiterer Wirkstoffe.

## Patentansprüche

1. Substituierte Sulfonamid-Derivate der allgemeinen Formel I 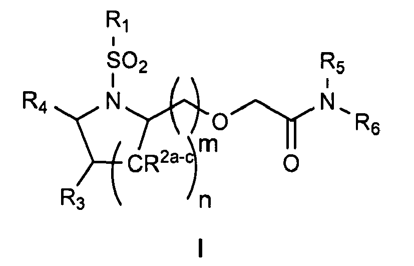 worin
n für 0, 1, 2 oder 3 steht;
m für 1 oder 2 steht;
R¹ für Aryl oder Heteroaryl steht;
R^{2a-c}, R³ und R⁴ für H stehen oder mit einem benachbarten Rest R^{2a-c}, R³ oder R⁴ einen fünf- oder sechsgliedrigen Ring bilden, der gesättigt oder ungesättigt und einfach oder mehrfach substituiert sein kann und Heteroatome aus der Gruppe N oder O enthalten kann,
R⁵ und R⁶ zusammen einen 4-8-gliedrigen Ring bilden, der gesättigt oder ungesättigt, aber nicht aromatisch sein kann, wobei der 4-8-gliedrige Ring mit einem aromatischen, gesättigten oder ungesättigten 4-10-gliedrigen Ring kondensiert sein kann, und der 4-8-gliedrige Ring und/oder der ankondensierte 4-1 0-gliedrige Ring mit einem basischen Rest substituiert oder kondensiert ist und mit einem weiteren basischen Rest oder Resten aus der Gruppe C₁-₆-Alkyl, C₁-₃-Alkyloxy, C₃₋₈-Cycloalkyl, =O, Aralkyl oder Aryl substituiert sein kann;
oder R⁵ und R⁶ zusammen einen 4-8-gliedrigen Ring bilden, der ein weiteres Heteroatom aus der Gruppe N oder O enthält und mit einem basischen oder nicht basischen Rest substituiert sein kann und in dem Fall, dass der basische oder nicht basische Substituent nicht über das weitere Heteroatom N an den 4-8-gliedrigen Ring gebunden ist, dieses weitere Heteroatom N zusätzlich mit einer C₁-₆-Alkylgruppe, einer Aryl- oder Aralkyl-Gruppe substituiert sein kann;
oder
R⁵ H, C₁-₆-Alkyl, C₃₋₈-Cycloalkyl, Aryl oder Aralkyl bedeutet und
R⁶ Aryl oder C₃-₈-Cycloalkyl; oder einen über eine C₁-₃-Alkylkette verknüpften Arylrest oder C₃-₈-Cycloalkyl bedeutet, wobei der Arylrest und der C₃-₈-Cycloalkyl-Ring mit einem 5-10-gliedrigen, ggf ein oder mehrere Heteroatome umfassenden, gesättigten oder ungesättigten Ring kondensiert sein können, und der Aryl- oder C₃₋₈-Cycloalkyl-Ring jeweils mit einem basischen Rest substituiert ist oder gegebenenfalls die Substitution mit dem basischen Rest an der verbrückenden C₁-₃-Alkylkette erfolgt,
oder R⁵ H, C₁-₆-Alkyl, C₃₋₈-Cycloalkyl, Aryl oder Aralkyl bedeutet und R⁶ C₄₋₈-Heterocyclyl; oder einen über eine C₁-₄-Alkylkette verknüpften C₄₋₈-Heterocyclylrest bedeutet, wobei der Heterocyclyl-Ring an einem oder zwei Ringgliedern mit einem basischen oder nicht basischen Rest substituiert sein kann,
oder R⁵ und R⁶ unabhängig voneinander für H, Aralkyl oder einen verzweigten oder unverzweigten, gegebenenfalls einfach oder mehrfach substituierten C₁₋₁₀-Alkylrest steht, der ein bis drei Stickstoffatome enthält, wobei R⁵ und R⁶ nicht beide H bedeuten,
oder
R⁵ für H, C₁-₆-Alkyl, C₃₋₈-Cycloalkyl, Aralkly oder Aryl steht und R⁶ für einen ggf. über eine C₁-₄-Alkylgruppe verknüpften basischen Heteroarylrest, der einfach oder mehrfach substituiert sein kann, steht, wobei die C₁-₄-Alkylkette mit einen basischen Rest substituiert sein kann;
wobei, soweit nichts anderes angegeben ist, die oben genannten Reste C₁₋₆-Alkyl, C₁-₃-Alkoxy, Aralkyl, Aryl und Heteroaryl unsubstituiert, einfach oder mehrfach substituiert sein können und der Rest C₃₋₈-Cycloalkyl unsubstituiert oder an einem oder mehreren Ringgliedern einfach substituiert sein kann,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

2. Substituierte Sulfonamid-Derivate gemäß Anspruch 1, worin
n für 0, 1, 2 oder 3 steht;
m für 1 oder 2 steht;
R¹ für Aryl oder Heteroaryl, unsubstituiert oder einfach oder mehrfach substituiert, steht;
R ^{2a-c}, R³ und R⁴ für H stehen oder mit einem benachbarten Rest R^{2a-c}, R³ oder R⁴ einen fünf- oder sechsgliedrigen Ring bilden, der gesättigt oder ungesättigt und einfach oder mehrfach substituiert sein kann und Heteroatome aus der Gruppe N oder O enthalten kann,
R⁵ und R⁶ zusammen einen 4-8-gliedrigen Ring bilden, der gesättigt oder ungesättigt, aber nicht aromatisch sein kann, wobei der 4-8-gliedrige Ring mit einem aromatischen, gesättigten oder ungesättigten 4-10-gliedrigen Ring kondensiert sein kann, und der 4-8-gliedrige Ring und/oder der ankondensierte 4-1 0-gliedrige Ring mit einem basischen Rest substituiert oder kondensiert ist und mit einem weiteren basischen Rest oder Resten aus der Gruppe C₁-₆-Alkyl, C₁-₃-Alkyloxy, C₃₋₈-Cycloalkyl, =O, Aralkyl oder Aryl substituiert sein kann;
oder R⁵ und R⁶ zusammen einen 4-8-gliedrigen Ring bilden, der ein weiteres Heteroatom aus der Gruppe N oder O enthält und mit einem basischen oder nicht basischen Rest substituiert sein kann und in dem Fall, dass der basische oder nicht basische Substituent nicht über das weitere Heteroatom N an den 4-8-gliedrigen Ring gebunden ist, dieses weitere Heteroatom N zusätzlich mit einer C₁₋₆-Alkylgruppe, einer Aryl- oder Aralkyl-Gruppe substituiert sein kann;
oder
R⁵ H, C₁₋₆-Alkyl, C₃₋₈-Cycloalkyl, Aryl oder Aralkyl bedeutet und
R⁶ Aryl oder C₃₋₈-Cycloalkyl; oder einen über eine C₁₋₃-Alkylkette verknüpften Arylrest oder C₃₋₈-Cycloalkyl bedeutet, wobei der Arylrest und der C₃-₈-Cycloalkyl-Ring mit einem 5-10-gliedrigen, ggf ein oder mehrere Heteroatome umfassenden, gesättigten oder ungesättigten Ring kondensiert sein können, und der Aryl- oder C₃₋₈-Cycloalkyl-Ring jeweils mit einem basischen Rest substituiert ist oder gegebenenfalls die Substitution mit dem basischen Rest an der verbrückenden C₁-₃-Alkylkette erfolgt,
oder R⁵ H, C₁-₆-Alkyl, C₃₋₈-Cycloalkyl, Aryl oder Aralkyl bedeutet und R⁶ C₄₋₈-Heterocyclyl; oder einen über eine C₁-₄-Alkylkette verknüpften C₄₋₈-Heterocyclylrest bedeutet, wobei der Heterocyclyl-Ring an einem oder zwei Ringgliedern mit einem basischen oder nicht basischen Rest substituiert sein kann,
oder R⁵ und R⁶ unabhängig voneinander für H, Aralkyl oder einen verzweigten oder unverzweigten, gegebenenfalls einfach oder mehrfach substituierten C₁₋₁₀-Alkylrest steht, der ein bis drei Stickstoffatome enthält, wobei R⁵ und R⁶ nicht beide H bedeuten,
oder
R⁵ für H, C₁₋₆-Alkyl, C₃₋₈-Cycloalkyl, Aralkly oder Aryl steht und R⁶ für einen ggf. über eine C₁-₄-Alkylgruppe verknüpften basischen Heteroarylrest, der einfach oder mehrfach substituiert sein kann, steht, wobei die C₁₋₄-Alkylkette mit einen basischen Rest substituiert sein kann;
wobei, soweit nichts anderes angegeben ist, die oben genannten Reste C₁₋₆-Alkyl, C₁-₃-Alkoxy, Aralkyl, Aryl und Heteroaryl unsubstituiert, einfach oder mehrfach substituiert sein können und der Rest C₃₋₈-Cycloalkyl unsubstituiert oder an einem oder mehreren Ringgliedern einfach substituiert sein kann,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.
worin
ein substituierter Aryl- oder Heteroarylrest einfach oder mehrfach mit F, Cl, Br, I, CN, NH₂, NH-C₁₋₆-Alkyl, NH-C₁₋₆-Alkyl-OH, N(C₁₋₆Alkyl)₂, N(C₁₋₆-Alkyl-OH)₂, NHAryl; N(Aryl)₂, N(C₁₋₆-Alkyl)Aryl, NO₂, SH, S-C₁₋₆-Alkyl, OH, O-C₁₋₆-Alkyl, O-C₁₋₆Alkyl-OH,
C(=O)C₁₋₆-Alkyl, CO₂H, CH₂SO₂-Phenyl CO₂-C₁₋₆-Alkyl, OCF₃, CF₃, 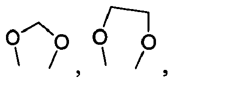 C₁₋₆-Alkyl, Pyrolidinyl, Imidazolyl, Piperidinyl, Morpholinyl, Benzyloxy, Phenoxy, Phenyl, Pyridyl, Alkylaryl, insbesondere Benzyl, Thienyl oder Furyl substituiert ist,
ein substituierter Cycloalkylrest oder Alkylrest mit F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl substituiert ist,
ein basischer Rest für einen zumindest ein Stickstoffatom als Heteroatom enthaltenen, gegebenenfalls anellierten Heterocyclus steht, wobei der Heterocyclus gegebenenfalls an einem oder mehreren Ringgliedern einfach mit C₁₋₆-Alkyl, F, Cl, Br, J, -CN, NH₂, NH(C₁₋₆-Alkyl), N(C₁₋₆-Alkyl)₂, -NH(Aryl), -N(C₁₋₃-Alkyl)(Aryl), wobei diese Arylreste einfach oder mehrfach mit F, Cl, Br, CF₃, CN, OH oder OMe substituiert sein können, O-C₁₋₆-Alkyl, Heterocyclyl-, oder OH substituiert sein kann; N(C₁₋₆-Alkyl)₂, NHC₁₋₆-Alkyl, einem mit N(C₁-₆-Alkyl)₂ substituierten Arylrest, einem mit einem 5-7-gliedrigen, zumindest ein N-Heteroatom enthaltenden Heterocyclyl substituierten Aryl- oder Heteroarylrest;
wobei alle vorgenannten basischen Reste über eine verbrückende -O-, -NH-, -NH[(-CH₂)ₚ-]-Gruppe, -N(C₁₋₃-Alkyl)[-(CH₂)ₚ-]-Gruppe, -O-[(CH₂)ₚ-]-Gruppe, -O-[-(CH₂)ₚ-O-]-Gruppe, mit jeweils p = 1, 2 oder 3, oder C₁₋₃-Alkylgruppe mit der Struktur der allgemeinen Formel I verknüpft sein können, und wobei die verbrückende -(CH₂)ₚ-Gruppen oder die C₁₋₃-Alkylkette gegebenenfalls mit =O, F, Cl, Br, J, -CN, Phenyl oder Pyridinyl substituiert sind und die übrigen Reste ihrerseits mit C₁₋₆-Alkyl substituiert sein können;
C₁₋₆-AlkylN(C₁₋₆-Alkyl)₂ oder C₁₋₆-AlkylNH(C₁₋₆-Alkyl) steht und für den Fall, dass der basische Rest an den aus R⁵ und R⁶ gebildeten Heterocyclus kondensiert ist, für einen 6-gliedrigen, gesättigten, ungesättigten oder aromatischen, mindestens ein N-Heteroatom enthaltenden Heterocyclus steht, ein nicht basischer Rest für -CN, C₁₋₆-Alkyl, gegebenenfalls substituiert mit Methoxy oder C₁₋₃-Alkyloxy; oder für Aryl, Heteroaryl, einen 3-7-gliedrigen, zumindest ein Sauerstoff- oder Schwefelatom enthaltenden Heterocyclus (Pyran, Thiophen), jeweils unsubstituiert oder einfach oder mehrfach substituiert, oder unsubstituiertes oder an einem oder mehreren Ringgliedern einfach substituiertes C₃₋₈-Cycloalkyl, steht, die über eine -O-, eine -O-[(CH₂)_{q}-]-Gruppe, -O-[-(CH₂)_{q}-O-]-Gruppe oder eine verbrückende C₁₋₃-Alkylgruppe mit der Struktur der allgemeinen Formel I verknüpft sein können, wobei die -(CH₂)_{q}-Kette bzw. die Alkylkette jeweils mit =O substituiert sein kann und q = 1, 2 oder 3 gilt; und wobei die Substituenten am Aryl, Heteroaryl, 3-7-gliedrigen Heterocyclus und C₃₋₈-Cycloalkyl ausgewählt sind aus F, Cl, Br, I, CN, NO₂, Aralkyl, SH, S-C₁₋₆-Alkyl, OH, O-C₁₋₆-Alkyl, O-C₁₋₆Alkyl-OH, C(=O)C₁₋₆-Alkyl, CO₂H, CH₂SO₂-Phenyl, CO₂-C₁₋₆-Alkyl, OCF₃, CF₃, 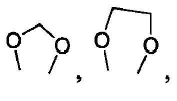 C₁₋₆-Alkyl
im Zusammenhang mit R⁶ C₄₋₈-Heterocyclyl für einen gesättigten oder ungesättigten, 4-8-gliedrigen cyclischen Rest, der 1, 2, 3, 4 oder 5 gleiche oder verschiedene Heteroatome im Ringsystem aufweisen kann, steht, wobei die Heteroatome vorzugsweise ausgesucht sind aus der Gruppe N, O und S und wobei sowohl die Bindung des Heterocycylrestes an die allgemeine Grundstruktur gemäß der Formel I als auch die gegebenenfalls vorliegende Substitution mit den basischen oder nicht basischen Gruppen an jedem beliebigen Ringglied vorliegen kann;
im Zusammenhang mit R⁶ ein basischer Heteroaryl-Rest für einen 5-10 gliedrigen, kondensierten oder nicht-kondensierten Heteroatom-haltigen Rest steht, der zumindest ein Stickstoffatom als Heteroatom enthält und wobei sowohl die Bindung des Heteroarylrestes an die allgemeine Grundstruktur gemäß der Formel I bzw. die Bindung des Heteroarylrestes an die verbrückende C₁₋₄-Alkylgruppe als auch die gegebenenfalls vorhandene Substitution an jedem beliebigen Ringglied des Heteroarylrestes vorliegen kann;
im Zusammenhang mit R⁶ ein an eine Arylgruppe ankondensierter 5-10 gliedriger, aromatischer oder ungesättigter, gegebenenfalls ein oder mehrere Heteroatome enthaltender Ring ausgewählt ist aus der Gruppe: 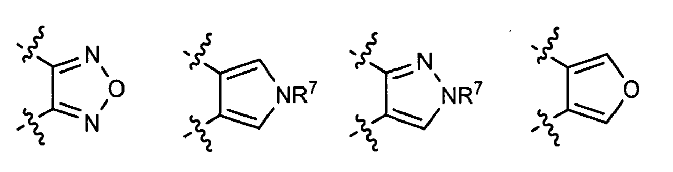 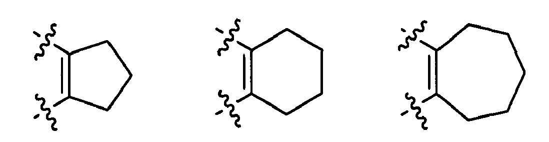
wobei R⁷ für H oder C₁₋₆-Alkyl steht,
im Zusammenhang mit R⁶ ein an einen C₃₋₈-Cycloalkyl-Ring ankondensierter 5-10-gliedriger, gesättigter oder ungesättigter, gegebenenfalls ein oder mehrere Heteroatome enthaltender Ring ausgewählt ist aus der Gruppe: Phenyl, Pyridinyl, Cyclopentan, Cyclohexan oder Cycloheptan,
und
ein an den durch R⁵ und R⁶ gebildeten 4-8 gliedrigen Ring ankondensierter, aromatischer, ungesättigter oder gesättigter 4-10 gliedriger Ring ausgesucht ist aus der Gruppe bestehend aus: C₄₋₁₀-Cycloalkan, C₄₋₁₀-Cycloalken oder C₆₋₁₀-Aromaten und 6-gliedrige Heteroaromaten.

3. Substituierte Sulfonamid-Derivate Gemäß Anspruch 1 oder 2, worin R¹ für Phenyl, Naphthyl, Indolyl, Benzofuranyl, Benzothiophenyl, Benzooxazoly, Benzooxadiazolyl, Pyrrolyl, Furanyl, Thiophenyl, Pyridinyl, Pyridazinyl, Pyrimidinyl, Pyrazinyl, Imidazothiazolyl, Carbazolyl, Dibenzofuranyl und Dibenzothiophenyl, vorzugsweise, Phenyl, Naphthyl, Benzothiophenyl, Benzooxadiazolyl,Thiophenyl, Pyridinyl, Imidazothiazolyl, und Dibenzofuranyl, insbesondere vorzugsweise für Phenyl, Naphthyl, und Benzothiophenyl steht, unsubstituiert oder einfach oder mehrfach substituiert, vorzugsweise mit C₁₋₃-Alkyloxy, C₁₋₆-Alkyl, Br, Cl, F, I, CF₃, OCF₃, OH, SH, Aryl oder Heteroaryl, jeweils unsubstituiert oder einfach oder mehrfach substituiert.

4. Substituierte Sulfonamid-Derivate gemäß einem der Ansprüche 1 oder 2, worin R¹ für Phenyl oder Naphthyl steht, gegebenenfalls einfach oder mehrfach substituiert mit Methyl, Methoxy, CF₃-, Cl, Br, und/oder F steht.

5. Substituierte Sulfonamid-Derivate gemäß Anspruch 1 oder 2, worin R^{2a-c}, R³ und R⁴ für H stehen oder mit einem benachbarten Rest R^{2a-c} , R³ oder R⁴ einen aromatischen Ring, vorzugsweise eine Benzengruppe bilden, der gegebenenfalls einfach oder mehrfach substituiert ist, vorzugsweise mit Methyl, Methoxy, CF₃-, Cl, Br und/oder F.

6. Substituierte Sulfonamid-Derivate, gemäß Anspruch 1 oder 2, worin in der (CR^{2a-c})ₙ-Gruppe n = 1 oder 2, vorzugsweise 2 steht.

7. Substituierte Sulfonamid-Derivate, gemäß Anspruch 1 oder 2, worin m = 1 gilt.

8. Substituierte Sulfonamid-Derivate gemäß einem der Ansprüche 1 oder 2, worin
a) die Gruppe NR⁵R⁶ in der allgemeinen Formel I eine ringförmige Gruppe gemäß den Formeln a1, a2 oder a3 bildet, 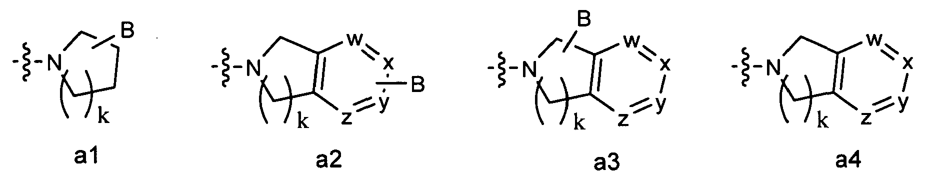 wobei
k = 0,1,2, oder 3, vorzugsweise 1 oder 2,
w, x, y und z unabhängig voneinander CH oder N darstellen, unter der Bedingung, dass höchstens zwei der Gruppen w, x, y und z gleichzeitig N darstellen und dass in der ringförmigen Gruppe gemäß der Formel a4 mindestens eine Gruppe aus w, x, y und z N darstellt, und
B einen basischen Rest darstellt, ausgesucht aus der Gruppe bestehend aus: -NR⁸R⁹ , wobei R⁸ und R⁹ unabhängig voneinander H oder C₁-₆-Alkyl darstellen können, und einem Rest mit der allgemeinen Formel aa1 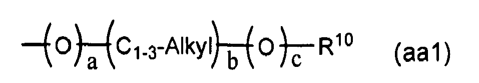
wobei a, b und c unabhängig voneinander 0 oder 1 sein können, unter der Bedingung, dass wenn b gleich 0 ist, a und c nicht gleichzeitig 1 ist;
das verbrückende C1-3-Alkyl einfach mit =O substituiert sein kann;
und R¹⁰ eine 4-10-gliedrige, aromatische, ungesättigte oder gesättigte, mono- oder polycyclische Heterocyclylgruppe darstellt, die 1, 2, 3 oder 4 N-Heteroatome enthalten und gegebenenfalls O und/oder S als weitere Heteroatome enthalten kann und wobei die Heterocyclylgruppe unsubstituiert oder an einem oder mehreren Ringgliedern einfach substituiert ist;
oder R¹⁰ eine mit zumindest einer -NR¹¹R¹²-Gruppe oder einem 5- oder 6-gliedrigen, monocyclischen N-haltigen aromatischen, gesättigten oder ungesättigten, 1 oder 2 N-Heteroatome enthaltenden Heterocyclus substituierte Arylgruppe darstellt, wobei R¹¹ und R¹² unabhängig voneinander H oder C₁₋₆-Alkyl darstellen, und die Arylgruppe gegebenenfalls noch weitere Substituenten tragen kann;
oder R¹⁰ eine Gruppe der allgemeinen Formel aa2 darstellt 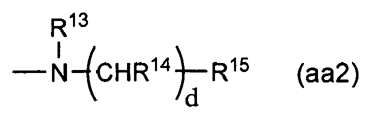 wobei
d 1, 2, oder 3 ist,
R¹³ H oder C₁₋₃-Alkyl sein kann, unabhängig für jedes Kettenglied d R¹⁴ H, oder eine gegebenenfalls substituierte Aryl- oder N-Heteroarylgruppe, vorzugsweise Phenyl, Naphthyl oder Pyrdinyl, sein kann, wobei innerhalb der durch d definierten Alkylkette R¹⁴ nur einmal kein H sein kann, und R¹⁵ eine 5-7-gliedrige, gesättigte oder ungesättigte, gegebenenfalls an einem oder mehreren Ringgliedern einfach substituierte Heterocycylgruppe ist, die 1 oder 2 N-Heteroatome enthält und als weitere Heteroatome O oder S enthalten kann, vorzugsweise Morpholinyl, Piperidinyl oder 4-Methylpiperazinyl;
oder
b) die Gruppe NR⁵R⁶ in der allgemeinen Formel I eine ringförmige Gruppe gemäß einer der allgemeinen Formeln b1 oder b2 darstellt: 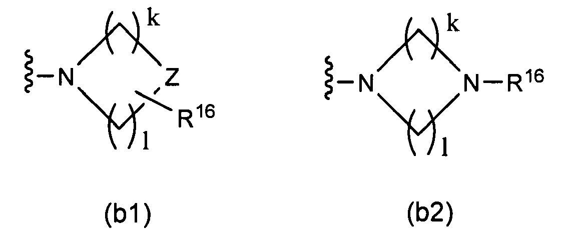 wobei
k = 1 oder 2, vorzugsweise 2,
l = 1, 2, oder 3, vorzugsweise 2,
Z NR¹⁷ oder O sein kann und R¹⁷ H oder C₁₋₆-Alkyl ist,
und R¹⁶ H oder eine Gruppe der allgemeinen Formel bb1 darstellt: 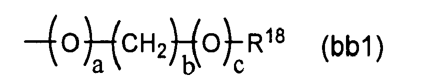 wobei
a und c unabhängig voneinander 0 oder 1 sind,
b = 0, 1, 2, oder 3, mit der Maßgabe, dass wenn b = 0 ist, dann sind a und c nicht gleichzeitig 1, und wobei in der durch b definierten Alkylkette ein CH₂-Kettenglied durch C(=O) ausgetauscht sein kann,
R¹⁸ ausgesucht ist aus der Gruppe bestehend aus unsubstituiertem, einfach oder mehrfach substituiertem Aryl oder Heteroaryl, wobei das Heteroaryl zumindest ein N-Heteroatom, vorzugsweise 1, 2 oder 3 N-Heteroatome, enthält, und O und S als weitere Heteroatome enthalten kann; gesättigtes oder ungesättigtes 5-7-gliedriges Heterocycyl, wobei das Heterocyclyl zumindest ein Heteroatom ausgesucht aus der Gruppe N,O und S enthält, vorzugsweise 1 oder 2 N-Heteroatome, und gegebenenfalls einfach oder an mehreren Ringgliedern einfach substituiert sein kann;
C₁-₆-Alkyl, gegebenenfalls einfach oder mehrfach substituiert; C₃₋₈-Cycloalkyl, gegebenenfalls einfach oder an mehreren Ringgliedern einfach substituiert;
und außer für die Fälle, dass i) c = 1 oder ii) b = c = 0 und a = 1 ist, R¹⁸ auch ausgesucht sein kann aus der Gruppe bestehen aus -CN, NR¹⁹R²⁰, wobei R¹⁹ und R²⁰ unabhängig voneinander H oder C₁₋₆-Alkyl sein können aber nicht gleichzeitig für H stehen;
oder
c) in der Gruppe NR⁵R⁶ aus der allgemeinen Formel I
R⁵ ausgesucht ist aus der Gruppe bestehend aus H und C₁₋₆-Alkyl, gegebenenfalls einfach oder mehrfach substituiert, und
R⁶ ausgesucht ist aus Gruppen der allgemeinen Formel c1 und c2 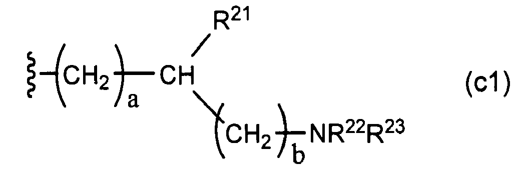 wobei
a=0,1,oder2;
b = 0, 1, 2, oder 3;
R²¹ eine unsubstituierte oder substituierte Arylgruppe ist, vorzugsweise Phenyl, oder Naphthyl, beide substituiert oder unsubstituiert,
R²² und R²³ unabhängig voneinander -H oder C₁₋₆-Alkyl darstellen oder die -NR²²R²³-Gruppe zusammen einen zumindest ein N-Heteroatom enthaltenden 5-, 6-, oder 7-gliedrigen gesättigten oder ungesättigten Heterocyclus darstellen, der gegebenenfalls einfach oder an mehreren Ringgliedern einfach substituiert sein kann und ; 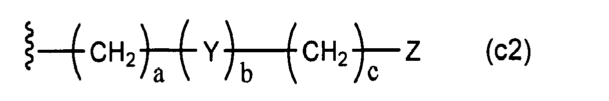 wobei
a = 0, 1, 2 oder 3;
b = 0 oder 1, mit der Maßgabe, dass wenn b = 0 auch c = 0 gilt;
c = 0, 1, 2, oder 3
Y für Aryl oder Heteroaryl (vorzugsweise N-haltig), gegebenenfalls substituiert; C₃₋₈-Cycloalkyl, vorzugsweise C₅₋₆-Cycloalkyl, steht;
und
Z eine gesättigte, ungesättigte oder aromatische, gegebenenfalls substituierte Heterocyclylgruppe darstellt, die zumindest ein N-Heteroatom enthält und als weitere Heteroatome O und/oder S enthalten kann; oder Z eine NR²⁴R²⁵-Gruppe darstellt, wobei R²⁴ und R²⁵ unabhängig voneinander H, C₁₋₆-Alkyl oder -C(=O)-(CH₂)_{d}-NR²⁶R²⁷, mit d = 1 oder 2, R²⁶ und R²⁷ unabhängig voneinander H oder C₁₋₆-Alkyl, oder die -NR²⁶R²⁷ einen 5- , 6- oder 7-gliedrigen, vorzugsweise gesättigten, Heterocyclus bilden;
oder
d) in der Gruppe NR⁵R⁶ aus der allgemeinen Formel I
R⁵ ausgesucht ist aus der Gruppe bestehend aus H, C₁₋₆-Alkyl, gegebenenfalls einfach oder mehrfach substituiert, C₃₋₈-Cycloalkyl, gegebenenfalls an einem oder mehreren Ringgliedern einfach substituiert, und
R⁶ ausgesucht ist aus Gruppen der allgemeinen Formel d1 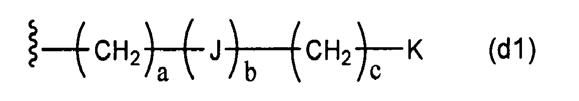 wobei
a = 0, 1, 2, 3 oder 4;
b = 0 oder 1, mit der Maßgabe, dass wenn b = 0 auch c = 0 gilt;
c = 0, 1, 2 oder 3;
J einen zumindest ein N-Heteroatom enthaltenden, vorzugsweise gesättigten oder ungesättigten 4-7-gliedrigen Heterocyclus darstellt, der gegebenenfalls an einem oder mehreren Ringgliedern einfach substituiert sein kann, und
K H oder eine gegebenenfalls substituierte Aryl- oder Heteroarylgruppe darstellt;
oder
e) in der Gruppe NR⁵R⁶ aus der allgemeinen Formel I :
R⁵ und R⁶ unabhängig voneinander ausgewählt sind aus der Gruppe bestehend aus H; C₁₋₆-Alkyl; Aralkyl; und -(CH₂)ᵣ-NR²⁸R²⁹, mit r = 1-6 und wobei R²⁸ und R²⁹ unabhängig voneinander ausgesucht sind aus der Gruppe bestehend aus H und C_{I}-₃-Alkyl;
oder
f) in der Gruppe NR⁵R⁶ aus der allgemeinen Formel I :
R⁵ ausgesucht ist aus der Gruppe bestehend aus H, C₁₋₆-Alkyl, Aryl und Aralkyl, und
R⁶ eine Gruppe vom Typ -(C₁₋₄-Alkyl)ₛ-X ist, mit s = 0 oder 1 und wobei X eine mindestens ein N-Heteroatom enthaltende Heteroarylgruppe ist, die gegebenenfalls einfach oder mehrfach substituiert ist und wobei in der C₁₋₄-Alkylgruppe ein H-Atom durch eine 5- oder 6-gliedrige, gesättigte, zumindest ein N-Heteroatom enthaltende Heterocyclylgruppe, die neben N noch O und/oder S als Heteroatom enthalten kann, ersetzt sein kann.

9. Substituierte Sulfonamid-Derivate gemäß Anspruch 8, wobei
a) die durch die NR⁵R⁶-Gruppe gebildete ringförmige Gruppe ausgesucht ist aus der Gruppe bestehend aus 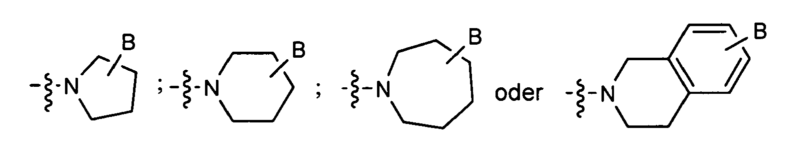 vorzugsweise in 4-Position mit dem basischen Rest B substituiertes Piperidin, und der basische Rest ausgesucht ist aus der Gruppe umfassend: -N(C₁₋₆-Alkyl), vorzugsweise -N(CH₃)₂; einen Rest mit der allgemeinen Formel (aa1), wobei R¹⁰ ausgesucht ist aus der Gruppe umfassend: 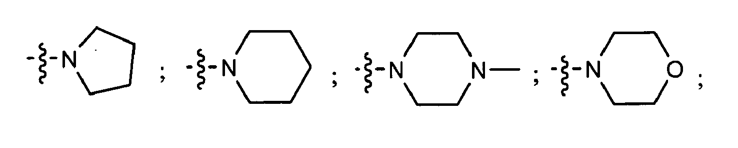 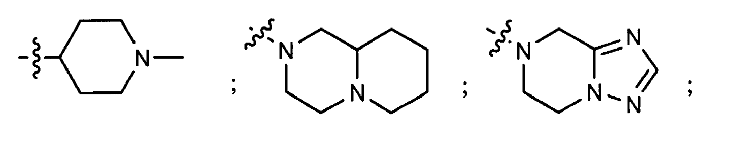 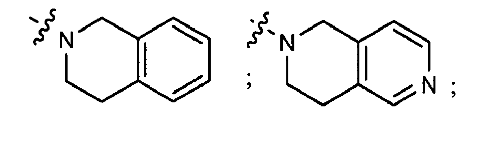 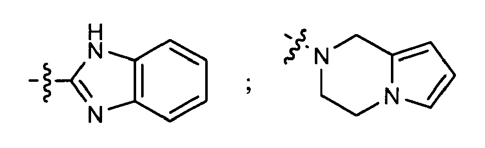
wobei diese Reste unsubstituiert oder an einem oder mehreren Ringgliedern einfach substituiert sein können, vorzugsweise mit C₁₋₆-Alkyl, insbesondere Methyl oder Ethyl, F, Cl, oder Br;
oder R¹⁰ 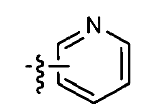
darstellt, das unsubstituiert, einfach oder mehrfach substituiert sein kann, vorzugsweise mit C₁₋₆-Alkyl, insbesondere Methyl oder Ethyl, F, Cl, Br, C₁₋₆-Alkoxy, insbesondere Methoxy, oder Phenyl; oder
R¹⁰ für Phenyl steht, das substituiert ist mit -N(C₁₋₃-Alkyl)₂, vorzugsweise Dimethylamino oder Diethylamino, oder
für Phenyl steht, das substituiert ist mit Pyrrolidinyl, Imidazolidinyl, Dihydroimidazoly, ,wobei die Verknüpfung an jedem der Ringglieder des Phenyls und des Substituenten erfolgen kann,
oder
R¹⁰ für eine Gruppe der allgemeinen Formel (aa2) steht, d = 1 oder 2 ist, R¹³ H oder Methyl bedeutet, R¹⁴ für Phenyl oder Pyridinyl steht, und R¹⁵ für Morpholinyl oder 4-Methylpiperazinyl steht;
oder
b) die durch die NR⁵R⁶-Gruppe gebildete ringförmige Gruppe ausgesucht ist aus den Gruppe bestehend aus 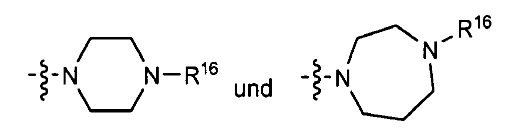 , wobei R¹⁶ eine Gruppe der allgemeinen Formel bb1 darstellt und in der Formel bb1 (i) a = 0, b = 1, 2 oder 3 und c = 0 oder (ii) a = 0, b = 1, 2, oder 3 und c = 0 gilt; und R¹⁸ ausgesucht ist aus der Gruppe bestehend aus 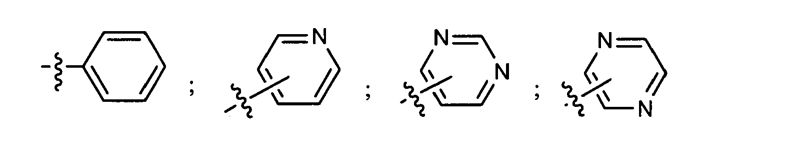 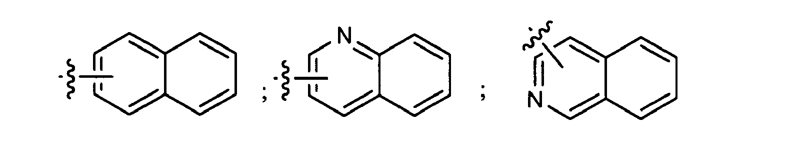 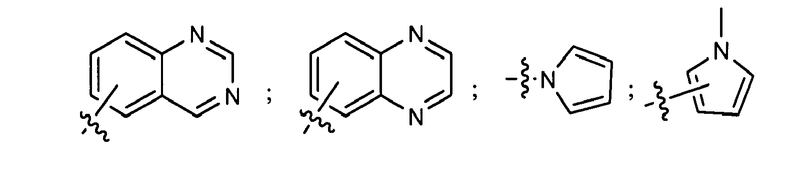 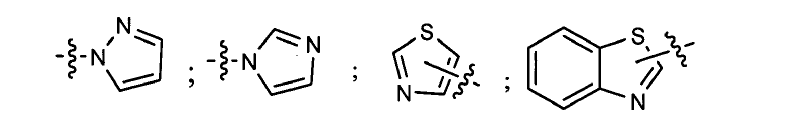 wobei diese Gruppen unsubstituiert, einfach oder mehrfach substituiert sein können, vorzugsweise mit C₁₋₃-Alkyl, insbesondere Methyl und/oder Ethyl; C₁₋₃-Alkoxy, insbesondere Methoxy; F, Cl, Br, I; -CN; CF₃; N(C₁₋₃-Alkyl)₂, NH(C₁₋₃-Alkyl), N(C₁₋₃-Alkyl)(Aryl), insbesondere N(C₁₋₃-Alkyl)(Phenyl oder Phenethyl), wobei Phenyl bzw. Phenethyl einfach oder mehrfach substituiert sein kann; Benzyl oder 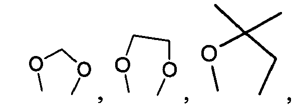 , wobei alle diese Substituenten ebenfalls einfach oder mehrfach substituiert sein können, vorzugsweise mit F, Cl, Br, -CN, -CF₃, C₁₋₃-Alkyl; Pyrrolidinyl, Piperidinyl, 4-Methylpiperindiyl oder Morpholinyl;
oder R¹⁸ ein Heterocyclyl darstellt, das ausgesucht ist aus der Gruppe bestehend aus: 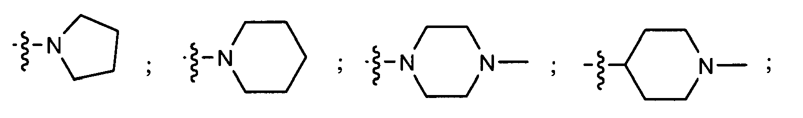 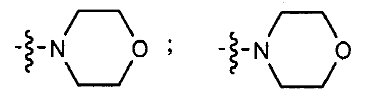 wobei diese Heterocyclylgruppen an einem oder mehreren Ringgliedern einfach substituiert sein können, oder
R¹⁸ Cyclopentyl, Cyclohexyl, gegebenenfalls an einem oder mehreren Ringgliedern einfach substituiert, oder C₁₋₃-Alkyl, gegebenenfalls einfach oder mehrfach substituiert, darstellt; oder
c) in der Gruppe NR⁵R⁶ aus der allgemeinen Formel I
R⁵ ausgesucht ist aus der Gruppe bestehend aus H und Methyl, Ethyl, Propyl und Isopropyl, gegebenenfalls einfach oder mehrfach substituiert, und
R⁶ eine Gruppe gemäß der allgemeinen Formel c1 darstellt, worin R²¹ eine Phenyl oder Naphthylgruppe ist, die einfach oder mehrfach substituiert sein kann, und vorzugsweise unsubstituiert oder mit F, Cl, Br, I, CN, C₁₋₃-Alkyl, vorzugsweise Methyl oder Ethyl ist,
R²² und R²³ unabhängig voneinander Methyl, Ethyl oder Propyl oder Isopropyl bedeuten oder die NR²²R²³-Gruppe zusammen einen N-Heterocyclus darstellt, der ausgesucht ist aus der Gruppe bestehend aus: 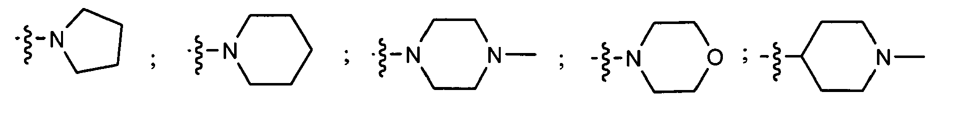
wobei diese N-Heterocyclen unsubstituiert oder an einem oder mehreren Ringgliedern einfach substituiert sein können,
oder
R⁶ eine Gruppe der allgemeinen Formel c2 darstellt, worin Y Phenyl, Naphthyl, Benzooxadiazol, Cyclopentyl, Cyclohexyl, oder Cycloheptyl darstellt, alle gegebenenfalls an einem oder mehreren Ringgliedern einfach substituiert, und
Z ausgesucht ist aus der Gruppe bestehend aus:
N(C₁₋₃-Alkyl)₂; 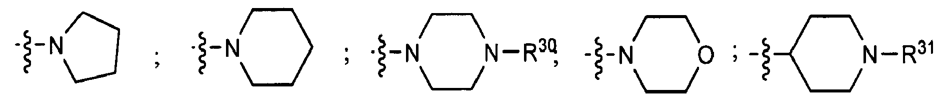 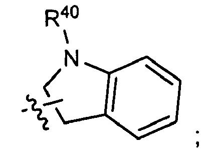
; wobei diese Reste an einem oder mehreren Ringglieder einfach substituiert sein können sein, und R³⁰, R³¹ und R₄₀ H, Methyl, Ethyl, Propyl, oder Isopropyl, gegebenenfalls einfach oder mehrfach substituiert, darstellen können oder Z ausgesucht ist aus der Gruppe bestehend aus: 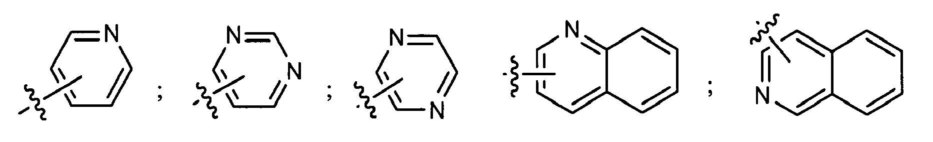 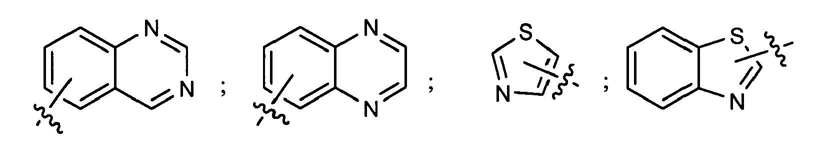 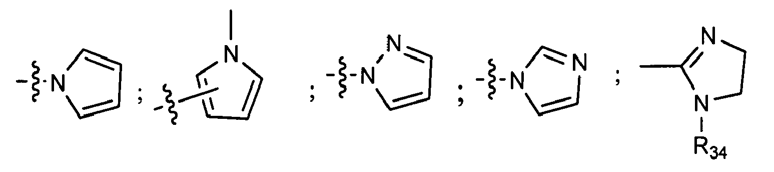 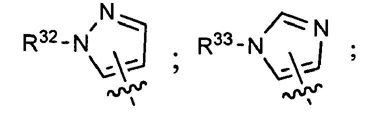
wobei R³², R³³ und R³⁴ ausgesucht sind aus H, Methyl, Ethyl, Propyl und Isopropyl, gegebenenfalls einfach oder mehrfach substituiert, und die N-Heterocyclen gegebenenfalls an einem oder mehreren Ringgliedern einfach substituiert sein können;
oder Z eine NR²⁴R²⁵-Gruppe ist, worin R²⁴ und R²⁵ unabhängig voneinander Methyl, Ethyl, Propyl oder Isopropyl darstellen oder Z eine -C(=O)-(CH₂)_{d}-NR²⁶R²⁷-Gruppe darstellt, wobei d 1 oder 2 ist und NR²⁶R²⁷ zusammen einen Heterocyclus, ausgesucht aus 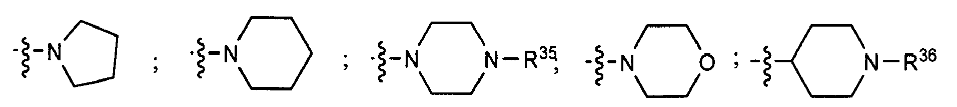 wobei die Ringglieder dieser Reste an einem oder mehreren Ringgliedern einfach substituiert sein können, und R³⁵ und R³⁶ Methyl, Ethyl, Propyl, oder Isopropyl, gegebenenfalls einfach oder mehrfach substituiert, darstellen können,
oder
d) in der Gruppe NR⁵R⁶ aus der allgemeinen Formel I
R⁵ ausgesucht ist aus der Gruppe bestehend aus H und Methyl, Ethyl, Propyl und Isopropyl, gegebenenfalls einfach oder mehrfach substituiert, und
R⁶ eine Gruppe gemäß der allgemeinen Formel d1 darstellt,
wobei J ausgesucht ist aus der Gruppe: 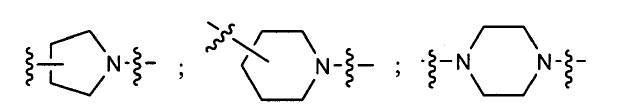
und K ausgesucht ist aus der Gruppe bestehend aus H, Phenyl, Naphthyl, Pyridinyl, alle gegebenenfalls einfach oder mehrfach substituiert,
oder
e) in der Gruppe NR⁵R⁶ aus der allgemeinen Formel I:
R⁵ ausgesucht ist aus der Gruppe bestehend aus H, C₁₋₃-Alkyl, -(CH₂)ᵣ-NR²⁸R²⁹, mit r = 1-3 und wobei R²⁸ und R²⁹ C₁₋₃-Alkyl sein können, und
R⁶ -(CH₂)ᵣ-NR²⁸R²⁹, mit r = 1-3 und wobei R²⁸ und R²⁹ C₁₋₃-Alkyl sein können, oder
R6 eine mit einer N(C₁₋₃-Alkyl)(Aryl)-gruppe substituierte C₁₋₃-Alkylgruppe darstellt,
die Arylgruppe substituiert sein kann und Aryl vorzugsweise für Phenyl oder Naphthyl steht,
oder
f) in der Gruppe NR⁵R⁶ aus der allgemeinen Formel I:
R⁵ H, Methyl, Ethyl, Propyl , Isopropyl, Phenyl oder Benzyl darstellt, und
R⁶ eine Gruppe vom Typ (C₁₋₃-Alkyl)ₛ-X darstellt, mit s = 0 oder 1 wobei die C₁-₃-Alkylgruppe mit einer Pyrrolidinyl, Piperidinyl, Piperazinyl oder Morpholinylgruppe substituiert sein kann und X ausgesucht ist aus der Gruppe bestehend aus: 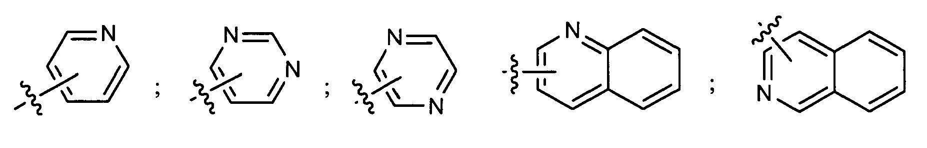 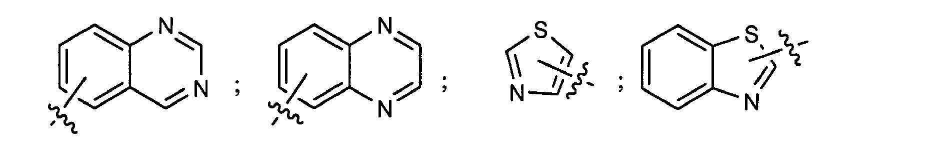 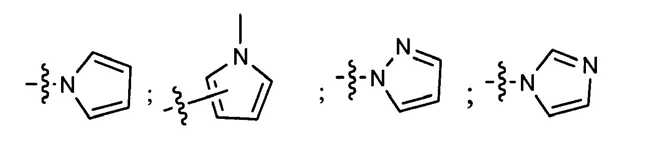 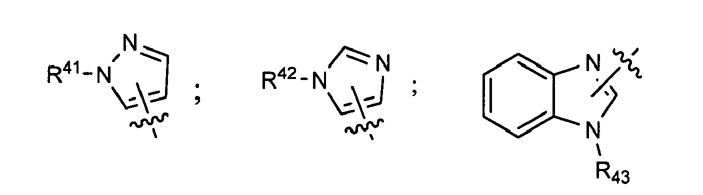
wobei R⁴¹, R⁴² und R⁴³ ausgesucht sind aus H, Methyl, Ethyl, Propyl und Isopropyl, gegebenenfalls einfach oder mehrfach substituiert, und die N-Heteroaryle gegebenenfalls einfach oder mehrfach substituiert sein können;

10. Substituierte Sulfonamid-Derivate gemäß Anspruch 8, wobei:
a) die durch die NR⁵R⁶-Gruppe gebildete ringförmige Gruppe 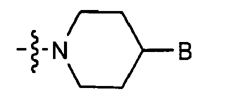 ist und der basische Rest B eine Gruppe mit der allgemeinen Formel (aa1) ist; wobei, i) a = b = c = 0, ii) a = c = 0 und b = 1, iii) a = b = 0 und c = 1 oder iv) a = b = 1 und c = 0; gilt; oder der basische Rest B eine Gruppe mit der allgemeinen Formel (aa2) ist, wobei d =1 oder 2, vorzugsweise, 2 gilt und R¹⁴, Pyridinyl, vorzugsweise 3-Pyridinyl, ist, und R¹⁵ Morpholinyl ist.
oder
b) die durch die NR⁵R⁶-Gruppe gebildete ringförmige Gruppe 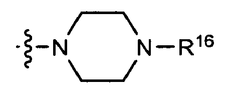 ist und R¹⁶ eine Gruppe der allgemeinen Formel bb1 darstellt, worin a = c = 0 und b = 0, 1 oder 2 gilt, und R¹⁸ ausgesucht ist aus 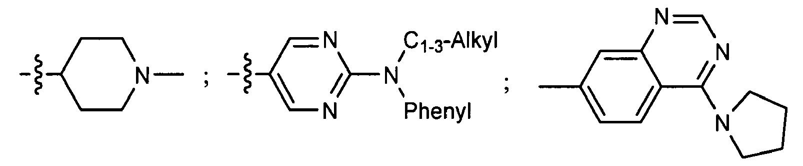 worin die Phenylgruppe substituiert sein kann, vorzugsweise einfach substituiert mit F, Cl, oder Br, insbesondere F, vorzugsweise in 4-Position;
oder
c) in der Gruppe NR⁵R⁶ aus der allgemeinen Formel I :
R⁵ ausgesucht ist aus der Gruppe bestehend aus H, Methyl, Ethyl, Propyl und Isopropyl, vorzugsweise Methyl, und
R⁶ eine Gruppe gemäß der allgemeinen Formel c2 darstellt, worin a = 1 oder 2, b = 1 und c = 0 gilt, Y Phenyl oder Cyclohexyl darstellt und Z Pyrrolidinyl oder
Dihydroimidazolyl darstellt.

11. Substituierte Sulfonamid-Derivate gemäß Anspruch 1 oder 2, worin
n für 0, 1, 2 oder 3 steht;
m für 1 oder 2 steht;
R¹ für Aryl oder Heteroaryl, unsubstituiert oder einfach oder mehrfach substituiert, steht;
R^{2a-c}, R³ und R⁴ für H stehen oder mit einem benachbarten Rest R^{2a-c}, R³ oder R⁴ einen fünf- oder sechsgliedrigen Ring bilden, der gesättigt oder ungesättigt und einfach oder mehrfach substituiert sein kann und Heteroatome aus der Gruppe N oder O enthalten kann,
R⁵ und R⁶ zusammen einen 4-8-gliedrigen Ring bilden, der gesättigt oder ungesättigt, aber nicht aromatisch sein kann, mit einem basischen Rest substituiert oder kondensiert ist und mit einem weiteren basischen Rest oder Resten aus der Gruppe C₁-₆-Alkyl, C₁₋₃-Alkyloxy, C₃₋₈-Cycloalkyl oder gegebenenfalls substituiertem Phenyl substituiert sein kann;
oder R⁵ und R⁶ zusammen einen 4-8-gliedrigen Ring bilden, der ein weiteres Heteroatom aus der Gruppe N oder O enthält und mit einem basischen oder nicht basischen Rest substituiert sein kann,
oder R⁵ H oder C₁₋₅-Alkyl bedeutet und R⁶ Aryl oder C₃₋₈-Cycloalkyl; oder einen über eine C₁-₃-Alkylkette verknüpften Arylrest bedeutet, wobei der Aryl- oder C₃₋₈-Cycloalkyl-Ring jeweils mit einem basischen Rest substituiert ist oder gegebenenfalls die basische Substitution an der verbrückenden C₁-₃-Alkylkette erfolgt,
oder R⁵ H oder C₁₋₅-Alkyl bedeutet und R⁶ C₄₋₈-Heterocyclyl; oder einen über eine C₁₋₃-Alkylkette verknüpften C₄₋₈-Heterocyclylrest bedeutet, wobei der Heterocyclyl-Ring mit einem basischen Rest oder nicht basischen Rest substituiert ist,
oder
R⁵ und R⁶ unabhängig voneinander für H oder einen verzweigten oder unverzweigten C₁₋₁₀-Alkylrest steht, der ein bis drei Stickstoffatome enthält, wobei R⁵ und R⁶ nicht beide H bedeuten,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

12. Substituierte Sulfonamid-Derivate gemäß Anspruch 11 worin R⁵ und R⁶ zusammen einen 4-8-gliedrigen Ring bilden, der gesättigt oder ungesättigt, aber nicht aromatisch sein kann, und mit einem basischen Rest substituiert sein kann
oder
worin R⁵ und R⁶ zusammen einen 4-8-gliedrigen Ring bilden, der mindestens ein weiteres Heteroatom aus der Gruppe N oder O enthält und mit einem basischen Rest oder einem nicht basischen Rest aus der Gruppe Aryl, Heteroaryl, jeweils unsubstituiert oder einfach oder mehrfach substituiert mit F, Cl, Br, I, CF₃, OCH₃, C₁₋₆-Alkyl, C₃₋₈-Cycloalkyl, unsubstituiert oder einfach oder mehrfach substituiert mit F, Cl, Br, I, CF₃, OCH₃, C₁₋₆-Alkyl; die über eine C₁₋₃-Alkylkette mit der Struktur der allgemeinen Formel I verknüpft sein können, wobei die Alkylkette mit =O substituiert sein kann; C₁₋₆-Alkyl oder C₁₋₃-Alkyloxy;
substituiert sein kann
oder
worin R⁵ H bedeutet und R⁶ Aryl oder C₃₋₈-Cycloalkyl; oder einen über eine C₁₋₃-Alkylkette verknüpften Arylrest bedeutet, wobei der Aryl- oder C₃₋₈-Cycloalkyl-Ring jeweils mit mindestens einem basischen Rest substituiert ist
oder
worin R⁵ H oder C₁₋₅-Alkyl bedeutet und R⁶ C₄₋₈-Heterocyclyl, wobei der Heterocyclyl-Rest über ein C-Atom mit der Struktur der allgemeinen Formel I verknüpft ist; oder einen über eine C₁₋₃-Alkylkette verknüpften C₄₋₈-Heterocyclylrest bedeutet, wobei der Heterocyclyl-Ring substituiert ist mit einem basischen Rest oder einem nicht basischen Rest aus der Gruppe Aryl, Heteroaryl, jeweils unsubstituiert oder einfach oder mehrfach substituiert mit F, Cl, Br, I, CF₃, OCH₃, C₁₋₆-Alkyl, C₃₋₈-Cycloalkyl, unsubstituiert oder einfach oder mehrfach substituiert mit F, Cl, Br, l, CF₃, OCH₃, C1-₆-Alkyl; die über eine C₁₋₃-Alkylkette mit der Struktur der allgemeinen Formel I verknüpft sein können, wobei die Alkylkette mit =O substituiert sein kann; C₁₋₆-Alkyl oder C₁₋₃-Alkyloxy;
substituiert ist,
wobei in allen Fällen der basische Rest ausgesucht ist aus der Gruppe 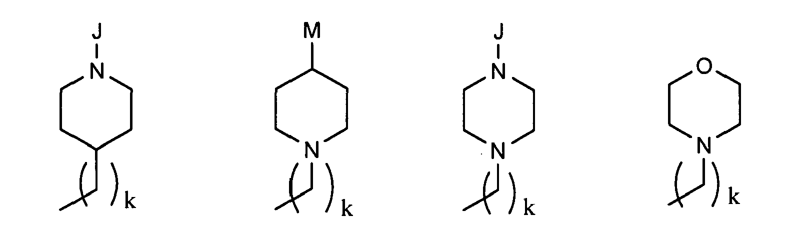 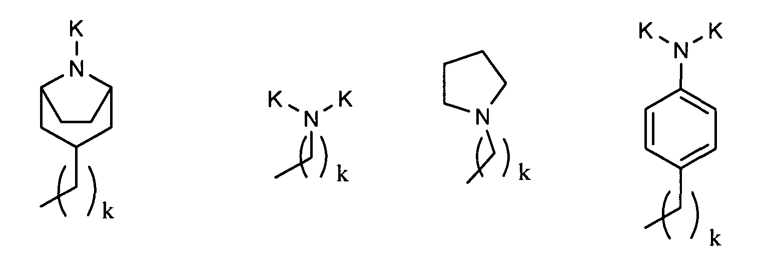 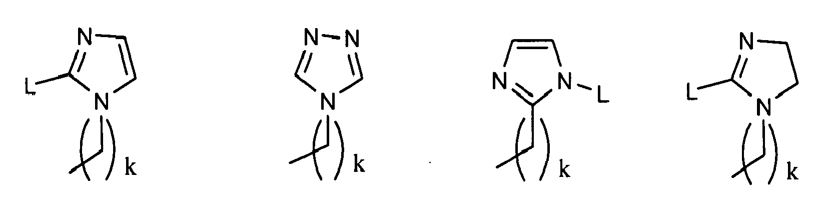 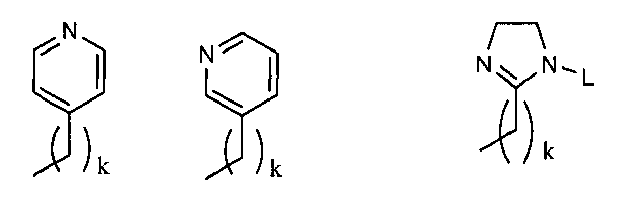 wobei
k für 0, 1 oder 2 steht
L für H oder C₁₋₆-Alkyl steht
K für C₁₋₆-Alkyl steht
M für C₁₋₆-Alkyl oder N(CH₃)₂ steht
J für 2-, 3- oder 4-Pyridyl, Phenyl, Piperidyl oder C₁₋₆-Alkyl steht.

13. Substituierte Sulfonamid-Derivate gemäß Anspruch 7, worin die Gruppe 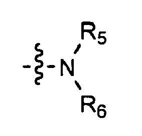 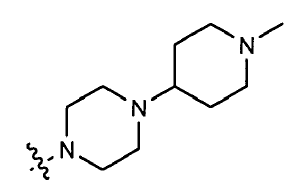 bedeutet.

14. Substituierte Sulfonamid-Derivate gemäß einem der Ansprüche 1 oder 2, wobei das Sulfonamid Derivat ausgesucht ist aus der Gruppe
1 2-[1-(4-Methoxy-2,6-dimethyl-phenylsulfonyl)-pyrrolidin-2-ylmethoxy]-N-(4-piperidin-1-ylmethyl-benzyl)-acetamid
2 2-[1-(4-Methoxy-2,6-dimethyl-phenylsulfonyl)-piperidin-2-ylmethoxy]-N-(4-piperidin-1-ylmethyl-benzyl)-acetamid
3 2-[1-(4-Methoxy-2,6-dimethyl-phenylsulfonyl)-piperidin-2-ylmethoxy]-N-[4-(4-methyl-piperazin-1-yl)-benzyl]-acetamid
4 N-(4-Piperidin-1-ylmethyl-benzyl)-2-[1-(2,4,6-trichlor-phenylsulfonyl)-piperidin-2-ylmethoxy]-acetamid
5 1-[4-(1-Methyl-piperidin-4-yl)-piperazin-1-yl]-2-[2-(2,4,6-trimethyl-phenylsulfonyl)-1,2,3,4-tetrahydro-isochinolin-3-ylmethoxy]-ethanon
6 1-(4-Benzyl-[1,4]diazepan-1-yl)-2-[1-(naphthyl-1-sulfonyl)-piperidin-2-ylmethoxy]-ethanon
7 2-[1-(4-Methoxy-2,6-dimethyl-phenylsulfonyl)-piperidin-2-ylmethoxy]-N-[4-(4-methyl-piperazin-1-ylmethyl)-benzyl]-acetamid
8 2-[1-(4-Methoxy-2,6-dimethyl-phenylsulfonyl)-piperidin-2-ylmethoxy]-1-[4-(1-methyl-piperidin-4-yl)-piperazin-1-yl]-ethanon
9 1-(1,4'-bipiperidin-1'-yl)-2-((2-(2,4-dichlorphenylsulfonyl)-1,2,3,4-tetrahydroisochinolin-3-yl)methoxy)ethanon
10 N-(4-Piperidin-1-ylmethyl-benzyl)-2-[1-(2,4,6-trichlor-phenylsulfonyl)-pyrrolidin-2-ylmethoxy]-acetamid
11 N-(4-Dimethylaminomethyl-benzyl)-2-[1-(4-methoxy-2,6-dimethyl-phenylsulfonyl)-piperidin-2-ylmethoxy]-acetamid
12 N-(1-Benzyl-piperidin-3-ylmethyl)-2-[1-(2,4,6-trimethyl-phenylsulfonyl)-piperidin-2-ylmethoxy]-acetamid
13 N-[4-(4-Methyl-piperazin-1-yl)-benzyl]-2-[1-(2,4,6-trichlor-phenylsulfonyl)-piperidin-2-ylmethoxy]-acetamid
14 2-[1-(4-Methoxy-2,6-dimethyl-phenylsulfonyl)-piperidin-2-ylmethoxy]-N-(4-piperidin-1-yl-benzyl)-acetamid
15 N-[4-(4-Methyl-piperazin-1-ylmethyl)-benzyl]-2-[1-(2,4,6-trichlor-phenylsulfonyl)-piperidin-2-ylmethoxy]-acetamid
16 N-(1-Benzyl-piperidin-3-ylmethyl)-2-[1-(3,4-dichlor-phenylsulfonyl)-2,3-dihydro-1H-indol-2-ylmethoxy]-acetamid
17 2-((2-(2,4-dichlorphenylsulfonyl)-1,2,3,4-tetrahydroisochinolin-3-yl)methoxy)-1-(4-(pyrrolidin-1-yl)piperidin-1-yl)ethanon
18 2-[1-(4-Methoxy-2,6-dimethyl-phenylsulfonyl)-pyrrolidin-2-ylmethoxy]-N-[4-(4-methyl-piperazin-1-yl)-benzyl]-acetamid
19 2-((1-(mesitylsulfonyl)piperidin-2-yl)methoxy)-1-(4-(pyrrolidin-1-yl)piperidin-1-yl)ethanon
20 2-((1-(3,4-dichlorphenylsulfonyl)piperidin-2-yl)methoxy)-1-(4-(2-(piperidin-1-yl)ethyl)piperidin-1-yl)ethanon
21 2-[1-(4-Methoxy-2,6-dimethyl-phenylsulfonyl)-piperidin-2-ylmethoxy]-N-(4-morpholin-4-yl-benzyl)-acetamid
22 1-[4-(1-Methyl-piperidin-4-yl)-piperazin-1-yl]-2-[1-(3-trifluormethyl-phenylsulfonyl)-piperidin-2-ylmethoxy]-ethanon
23 2-((1-(naphthalen-1-ylsulfonyl)piperidin-2-yl)methoxy)-1-(4-(pyrrolidin-1-yl)piperidin-1-yl)ethanon
24 2-[1-(4-Methoxy-2,6-dimethyl-phenylsulfonyl)-piperidin-2-ylmethoxy]-N-(4-morpholin-4-ylmethyl-benzyl)-acetamid
25 1-[4-(3-Dimethylamino-propyl)-piperazin-1-yl]-2-[2-(2,4,6-trimethyl-phenylsulfonyl)-1,2,3,4-tetrahydro-isochinolin-3-ylmethoxy]-ethanon
26 2-[1-(3,4-Dichlor-phenylsulfonyl)-piperidin-2-ylmethoxy]-1-(4-pyrrolidin-1-yl-piperidin-1-yl)-ethanon
27 1-(2-Piperidin-1-ylmethyl-pyrrolidin-1-yl)-2-[1-(2,4,6-trimethyl-phenylsulfonyl)-piperidin-2-ylmethoxy]-ethanon
28 2-[1-(3,4-Dichlor-phenylsulfonyl)-piperidin-2-ylmethoxy]-1-[4-(4-methyl-piperazin-1-yl)-piperidin-1-yl]-ethanon
29 N-[4-(4-Methyl-piperazin-1-yl)-benzyl]-2-[1-(2,4,6-trichlor-phenylsulfonyl)-pyrrolidin-2-ylmethoxy]-acetamid
30 N-(4-Morpholin-4-ylmethyl-benzyl)-2-[1-(2,4,6-trichlor-phenylsulfonyl)-piperidin-2-ylmethoxy]-acetamid
31 1-(4-Benzyl-[1,4]diazepan-1-yl)-2-[1-(2,4,6-trimethyl-phenylsulfonyl)-piperidin-2-ylmethoxy]-ethanon
32 2-[1-(3,4-Dichlor-phenylsulfonyl)-piperidin-2-ylmethoxy]-1-[4-(1-methyl-piperidin-4-yl)-piperazin-1-yl]-ethanon
33 N-[3-(4-Methyl-piperazin-1-yl)-benzyl]-2-[2-(2,4,6-trimethyl-phenylsulfonyl)-1,2,3,4- tetrahydro-isochinolin-3-ylmethoxy]-acetamid
34 N-(1-Benzyl-piperidin-3-ylmethyl)-2-[1-(naphthyl-1-sulfonyl)-piperidin-2-ylmethoxy]-acetamid
35 N-(4-Dimethylaminomethyl-benzyl)-2-[1-(4-methoxy-2,6-dimethyl-phenylsulfonyl)-pyrrolidin-2-ylmethoxy]-acetamid
36 2-((1-(3,4-dichlorphenylsulfonyl)pyrrolidin-2-yl)methoxy)-1-(4-(2-(piperidin-1-yl)ethyl)piperidin-1-yl)ethanon
37 N-(4-Dimethylaminomethyl-benzyl)-2-[1-(2,4,6-trichlor-phenylsulfonyl)-piperidin-2-ylmethoxy]-acetamid
38 2-((2-(3,4-dichlorphenylsulfonyl)-1,2,3,4-tetrahydroisochinolin-3-yl)methoxy)-1-(4-(pyrrolidin-1-yl)piperidin-1-yl)ethanon
39 1-(4-Benzo[1,3]dioxol-5-ylmethyl-piperazin-1-yl)-2-[1-(3,4-dichlor-phenylsulfonyl)-piperidin-2-ylmethoxy]-ethanon
40 2-[1-(4-Methoxy-2,6-dimethyl-phenylsulfonyl)-pyrrolidin-2-ylmethoxy]-N-[4-(4-methyl-piperazin-1-ylmethyl)-benzyl]-acetamid
41 N-(1 -Benzyl-piperidin-3-ylmethyl)-2-[2-(2,4,6-trimethyl-phenylsulfonyl)-1,2,3,4-tetrahydro-isochinolin-3-ylmethoxy]-acetamid
42 2-((1-(3,4-dichlorphenylsulfonyl)piperidin-2-yl)methoxy)-1-(4-(2-(piperidin-1-yl)ethyl)piperazin-1-yl)ethanon
43 2-[2-(3,4-Dichlor-phenylsulfonyl)-1,2,3,4-tetrahydro-isochinolin-3-ylmethoxy]-N,N-bis-(2-diethylamino-ethyl)-acetamid
44 1-(4-Benzo[1,3]dioxol-5-ylmethyl-piperazin-1-yl)-2-[1-(3,4-dichlor-phenylsulfonyl)-pyrrolidin-2-ylmethoxy]-ethanon
45 N-[4-(4-Methyl-piperazin-1-ylmethyl)-benzyl]-2-[1-(2,4,6-trichlor-phenylsulfonyl)-pyrrolidin-2-ylmethoxy]-acetamid
46 2-[1-(3,4-Dichlor-phenylsulfonyl)-piperidin-2-ylmethoxy]-1-[4-(2-methoxy-ethyl)-piperazin-1-yl]-ethanon
47 N-Methyl-N-(2-morpholin-4-yl-1-phenyl-ethyl)-2-[1-(naphthyl-1-sulfonyl)-piperidin-2-ylmethoxy]-acetamid
48 N,N-Bis-(2-diethylamino-ethyl)-2-[2-(2,4,6-trimethyl-phenylsulfonyl)-1,2,3,4-tetrahydro-isochinolin-3-ylmethoxy]-acetamid
49 N-(4-Morpholin-4-ylmethyl-benzyl)-2-[1-(2,4,6-trichlor-phenylsulfonyl)-pyrrolidin-2-ylmethoxy]-acetamid
50 N-(3-Morpholin-4-yl-benzyl)-2-[2-(2,4,6-trimethyl-phenylsulfonyl)-1,2,3,4-tetrahydro-isochinolin-3-ylmethoxy]-acetamid
51 2-[2-(2,4-Dichlor-phenylsulfonyl)-1,2,3,4-tetrahydro-isochinolin-3-ylmethoxy]-1-(2-piperidin-1-ylmethyl-pyrrolidin-1-yl)-ethanon
52 2-((1-(4-methoxyphenylsulfonyl)piperidin-2-yl)methoxy)-1-(4-(2-(piperidin-1-yl)ethyl)piperidin-1-yl)ethanon
53 2-[1-(3,4-Dichlor-phenylsulfonyl)-piperidin-2-ylmethoxy]-1-[4-(3-methoxy-phenyl)-piperazin-1-yl]-ethanon
54 1-(4-Cyclohexylmethyl-piperazin-1-yl)-2-[1-(3,4-dichlor-phenylsulfonyl)-pyrrolidin-2-ylmethoxy]-ethanon
55 2-[1-(4-Methoxy-2,6-dimethyl-phenylsulfonyl)-pyrrolidin-2-ylmethoxy]-N-(4-morpholin-4-ylmethyl-benzyl)-acetamid
56 2-[1-(3,4-Dichlor-phenylsulfonyl)-piperidin-2-ylmethoxy]-1-(4-phenyl-piperazin-1-yl)-ethanon
57 1-(4-Benzyl-piperidin-1-yl)-2-[1-(3,4-dichlor-phenylsulfonyl)-piperidin-2-ylmethoxy]-ethanon
58 1-[4-(3-Dimethylamino-propyl)-piperazin-1-yl]-2-[1-(3-trifluormethyl-phenylsulfonyl)-piperidin-2-ylmethoxy]-ethanon
59 2-[1-(3,4-Dichlor-phenylsulfonyl)-piperidin-2-ylmethoxy]-1-[4-(4-fluor-phenyl)-piperazin-1-yl]-ethanon
60 2-[1-(4-Methoxy-2,6-dimethyl-phenylsulfonyl)-pyrrolidin-2-ylmethoxy]-N-(4-piperidin-1-yl-benzyl)-acetamid
61 N-(4-Morpholin-4-ylmethyl-benzyl)-2-[1-(naphthyl-1-sulfonyl)-piperidin-2-ylmethoxy]-acetamid
62 2-[1-(3,4-Dichlor-phenylsulfonyl)-pyrrolidin-2-ylmethoxy]-1-[4-(1-methyl-piperidin-4-yl)-piperazin-1-yl]-ethanon
63 1-[2-(4-Dimethylamino-phenyl)-azepan-1-yl]-2-[1-(naphthyl-1-sulfonyl)-piperidin-2-ylmethoxy]-ethanon
64 2-[1-(naphthyl-1-sulfonyl)-piperidin-2-ylmethoxy]-1-(2-piperidin-1-ylmethyl-pyrrolidin-1-yl)-ethanon
65 2-((2-(2,4-dichlorphenylsulfonyl)-1,2,3,4-tetrahydroisochinolin-3-yl)methoxy)-1-(4-morpholinopiperidin-1-yl)ethanon
66 1-(3-Dimethylamino-pyrrolidin-1-yl)-2-[1-(naphthyl-1-sulfonyl)-piperidin-2-ylmethoxy]-ethanon
67 1-(4-Benzyl-[1,4]diazepan-1-yl)-2-[2-(2,4,6-trimethyl-phenylsulfonyl)-1,2,3,4-tetrahydro-isochinolin-3-ylmethoxy]-ethanon
68 1-(4-Cyclohexylmethyl-piperazin-1-yl)-2-[1-(3,4-dichlor-phenylsulfonyl)-piperidin-2-ylmethoxy]-ethanon
69 2-((2-(4-methoxyphenylsulfonyl)-1,2,3,4-tetrahydroisochinolin-3-yl)methoxy)-1-(4-(2-(piperidin-1-yl)ethyl)piperidin-1-yl)ethanon
70 N-(1-Benzyl-piperidin-3-ylmethyl)-2-[1-(3,4-dichlor-phenylsulfonyl)-piperidin-2-ylmethoxy]-acetamid
71 N-(4-Piperidin-1-yl-benzyl)-2-[1-(2,4,6-trichlor-phenylsulfonyl)-pyrrolidin-2-ylmethoxy]-acetamid
72 2-((1-(3,4-dichlorphenylsulfonyl)pyrrolidin-2-yl)methoxy)-1-(4-(2-(piperidin-1-yl)ethyl)piperazin-1-yl)ethanon
73 N-(4-Dimethylaminomethyl-benzyl)-2-[1-(2,4,6-trichlor-phenylsulfonyl)-pyrrolidin-2-ylmethoxy]-acetamid
74 N-(4-Piperidin-1-yl-benzyl)-2-[1-(2,4,6-trichlor-phenylsulfonyl)-piperidin-2-ylmethoxy]-acetamid
75 N-[2-(4-Methyl-piperazin-1-yl)-benzyl]-2-[2-(2,4,6-trimethyl-phenylsulfonyl)-1,2,3,4-tetrahydro-isochinolin-3-ylmethoxy]-acetamid
76 N-(3-Piperidin-1-ylmethyl-phenyl)-2-[1-(2,4,6-trimethyl-phenylsulfonyl)-piperidin-2-ylmethoxy]-acetamid
77 1-[4-(3-Methoxy-phenyl)-piperazin-1-yl]-2-[1-(2,4,6-trimethyl-phenylsulfonyl)-piperidin-2-ylmethoxy]-ethanon
78 N-(1-Benzyl-pyrrolidin-3-yl)-2-[2-(2,4-dichlor-phenylsulfonyl)-1,2,3,4-tetrahydro- isochinolin-3-ylmethoxy]-N-methyl-acetamid
79 1-(4-Benzyl-[1,4]diazepan-1-yl)-2-[2-(2,4-dichlor-phenylsulfonyl)-1,2,3,4-tetrahydro-isochinolin-3-ylmethoxy]-ethanon
80 N-[2-(4-Methyl-piperazin-1-yl)-benzyl]-2-[1-(2,4,6-trimethyl-phenylsulfonyl)-piperidin-2-ylmethoxy]-acetamid
81 2-[1-(3,4-Dichlor-phenylsulfonyl)-2,3-dihydro-1 H-indol-2-ylmethoxy]-N-(4-pyrrolidin-1-ylmethyl-benzyl)-acetamid
82 2-[2-(3,4-Dichlor-phenylsulfonyl)-1,2,3,4-tetrahydro-isochinolin-3-ylmethoxy]-1-(2-piperidin-1-ylmethyl-pyrrolidin-1-yl)-ethanon
83 N-(4-Pyrrolidin-1-ylmethyl-benzyl)-2-[2-(2,4,6-trimethyl-phenylsulfonyl)-1,2,3,4-tetrahydro-isochinolin-3-ylmethoxy]-acetamid
84 2-[1-(3,4-Dichlor-phenylsulfonyl)-piperidin-2-ylmethoxy]-1-[4-(4-methoxy-phenyl)-piperazin-1-yl]-ethanon
85 2-[1-(3,4-Dichlor-phenylsulfonyl)-piperidin-2-ylmethoxy]-1-[4-(3-dimethylamino-propyl)-piperazin-1-yl]-ethanon
86 2-[1-(3,4-Dichlor-phenylsulfonyl)-piperidin-2-ylmethoxy]-1-[4-(2-fluor-phenyl)-piperazin-1-yl]-ethanon
87 N-[4-(2-Methyl-imidazol-1-yl)-phenyl]-2-[1-(2,4,6-trimethyl-phenylsulfonyl)-piperidin-2-ylmethoxy]-acetamid
88 2-[1-(3,4-Dichlor-phenylsulfonyl)-pyrrolidin-2-ylmethoxy]-1-[4-(3-dimethylamino-propyl)-piperazin-1-yl]-ethanon
89 2-[1-(3,4-Dichlor-phenylsulfonyl)-pyrrolidin-2-ylmethoxy]-1-(4-phenyl-piperazin-1-yl)-ethanon
90 2-(2-(1-(4-Methoxyphenylsulfonyl)piperidin-2-yl)ethoxy)-1-(4-(2-(piperidin-1-yl)ethyl)piperidin-1-yl)ethanon
91 2-(2-(1-(4-methoxyphenylsulfonyl)piperidin-2-yl)ethoxy)-1-(4-phenethylpiperazin-1-yl)ethanon
92 3-((4-(2-((1-(4-Methoxy-2,6-dimethylphenylsulfonyl)piperidin-2-yl)methoxy)acetyl)piperazin-1-yl)methyl)benzonitril hydrochlorid
93 3-((4-(2-((1-(4-Methoxy-2,6-dimethylphenylsulfonyl)pyrrolidin-2-yl)methoxy)acetyl)piperazin-1-yl)methyl)benzonitril hydrochlorid
94 2-((1-(4-Methoxy-2,6-dimethylphenylsulfonyl)piperidin-2-yl)methoxy)-1-(4-(2-(pyrrolidin-1-yl)ethyl)piperidin-1-yl)ethanon hydrochlorid
95 2-((1-(4-Methoxy-2,6-dimethylphenylsulfonyl)pyrrolidin-2-yl)methoxy)-1-(4-(2-(pyrrolidin-1-yl)ethyl)piperidin-1-yl)ethanon
96 1-(3,4-Dihydro-2,6-naphthyridin-2(1 H)-yl)-2-((1-(4-methoxy-2,6-dimethylphenylsulfonyl)piperidin-2-yl)methoxy)ethanon hydrochlorid
97 2-((1-(4-methoxy-2,6-dimethylphenylsulfonyl)piperidin-2-yl)methoxy)-1-(4-(1-methylpiperidin-4-yl)piperazin-1-yl)ethanon dihydrochlorid
98 1-(4-(Dihydro-1H-pyrido[1,2-a]pyrazin-2(6H,7H,8H,9H,9aH)-yl)piperidin-1-yl)-2-((1-(4-methoxy-2,6-dimethylphenylsulfonyl)piperidin-2-yl)methoxy)ethanon
99 1-(4-(Dihydro-1H-pyrido[1,2-a]pyrazin-2(6H,7H,8H,9H,9aH)-yl)piperidin-1-yl)-2-((1-(4-methoxy-2,6-dimethylphenylsulfonyl)piperidin-2-yl)methoxy)ethanon Dihydrochlorid
100 1-(4-(3,4-Dihydro-2,6-naphthyridin-2(1H)-yl)piperidin-1-yl)-2-((1-(4-methoxy-2,6- dimethylphenylsulfonyl)piperidin-2-yl)methoxy)ethanon
101 tert-Butyl 4-(2-((1-(4-methoxy-2,6-dimethylphenylsulfonyl)piperidin-2-yl)methoxy)acetyl)piperazin-1-carboxylat
102 2-((1-(4-Methoxy-2,6-dimethylphenylsulfonyl)piperidin-2-yl)methoxy)-1-(piperazin-1-yl)ethanon hydrochlorid
103 2-((1-(4-Methoxy-2,6-dimethylphenylsulfonyl)piperidin-2-yl)methoxy)-1-(4-(3-(pyridin-4-yl)-1,2,4-oxadiazol-5-yl)piperidin-1-yl)ethanon
104 2-((1-(4-Methoxy-2,6-dimethylphenylsulfonyl)piperidin-2-yl)methoxy)-1-(4-(tetrahydro-2H-pyran-4-yl)piperazin-1-yl)ethanon
105 2-((1-(4-Methoxy-2,6-dimethylphenylsulfonyl)piperidin-2-yl)methoxy)-1-(4-(4-methylcyclohexyl)piperazin-1-yl)ethanon
106 2-((1-(4-Methoxy-2,6-dimethylphenylsulfonyl)piperidin-2-yl)methoxy)-1-(4-((1-methylpiperidin-4-yl)methyl)piperazin-1-yl)ethanon dihydrochlorid
107 2-((1-(Mesitylsulfonyl)piperidin-2-yl)methoxy)-1-(4-(1-methylpiperidin-4-yl)piperazin-1-yl)ethanon
108 2-((1-(2,6-Dichlor-4-(trifluormethyl)phenylsulfonyl)piperidin-2-yl)methoxy)-1-(4-(1-methylpiperidin-4-yl)piperazin-1-yl)ethanon
109 2-((1-(2-Chlor-6-methylphenylsulfonyl)piperidin-2-yl)methoxy)-1-(4-(1-methylpiperidin-4-yl)piperazin-1-yl)ethanon
110 1-(4-(1-Methylpiperidin-4-yl)piperazin-1-yl)-2-((1-(naphthalen-1-ylsulfonyl)piperidin-2-yl)methoxy)ethanon
111 1-(4-(1-Methylpiperidin-4-yl)piperazin-1-yl)-2-((1-(naphthalen-2-ylsulfonyl)piperidin-2-yl)methoxy)ethanon
112 2-((1-(4-Methoxy-2,6-dimethylphenylsulfonyl)piperidin-2-yl)methoxy)-1-(4-(4-methylpiperazin-1-yl)piperidin-1-yl)ethanon hydrochlorid
113 2-((1-(4-Methoxy-2,6-dimethylphenylsulfonyl)piperidin-2-yl)methoxy)-1-(4-(thieno[3,2-d]pyrimidin-4-yl)piperazin-1-yl)ethanon
114 2-((1-(4-Chlor-2,5-dimethylphenylsulfonyl)piperidin-2-yl)methoxy)-1-(4-(1-methylpiperidin-4-yl)piperazin-1-yl)ethanon dihydrochlorid
115 2-((1-(4-Chlor-3-(trifluormethyl)phenylsulfonyl)piperidin-2-yl)methoxy)-1-(4-(1-methylpiperidin-4-yl)piperazin-1-yl)ethanon
116 1-(2-((1-(4-Methoxy-2,6-dimethylphenylsulfonyl)piperidin-2-yl)methoxy)acetyl)piperidin-4-on
117 1-(4-((1H-Benzo[d]imidazol-2-yl)methyl)piperazin-1-yl)-2-((1-(4-methoxy-2,6-dimethylphenylsulfonyl)piperidin-2-yl)methoxy)ethanon
118 1-(4-(1-Methylpiperidin-4-yl)piperazin-1-yl)-2-((1-(2,4,6-trichlorphenylsulfonyl)piperidin-2-yl)methoxy)ethanon
119 1-(4-(1-Methylpiperidin-4-yl)piperazin-1-yl)-2-((1-(2,4,6-triisopropylphenylsulfonyl)piperidin-2-yl)methoxy)ethanon
120 2-((1-(2,4-Dichlorphenylsulfonyl)piperidin-2-yl)methoxy)-1-(4-(1-methylpiperidin-4-yl)piperazin-1-yl)ethanon
121 2-((1-(3-Bromphenylsulfonyl)piperidin-2-yl)methoxy)-1-(4-(1-methylpiperidin-4-yl)piperazin-1-yl)ethanon
122 2-((1-(3-Bromphenylsulfonyl)piperidin-2-yl)methoxy)-1-(4-(pyridin-4-yl)piperazin-1-yl)ethanon
123 2-((1-(4-Bromphenylsulfonyl)piperidin-2-yl)methoxy)-1-(4-(1-methylpiperidin-4-yl)piperazin-1-yl)ethanon
124 2-((1-(3-Bromphenylsulfonyl)piperidin-2-yl)methoxy)-1-(4-(2-(pyrrolidin-1-yl)ethyl)piperidin-1-yl)ethanon
125 2-((1-(4-Bromphenylsulfonyl)piperidin-2-yl)methoxy)-1-(4-(2-(pyrrolidin-1-yl)ethyl)piperidin-1-yl)ethanon
126 2-((1-(4-Bromphenylsulfonyl)piperidin-2-yl)methoxy)-1-(4-(pyridin-4-yl)piperazin-1-yl)ethanon
127 (S)-2-((2-(4-Methoxy-2,6-dimethylphenylsulfonyl)-1,2,3,4-tetrahydroisochinolin-3-yl)methoxy)-1-(4-(1-methylpiperidin-4-yl)piperazin-1-yl)ethanon
128 2-((1-(5-Chlor-1,3-dimethyl-1H-pyrazol-4-ylsulfonyl)piperidin-2-yl)methoxy)-1-(4-(1-methylpiperidin-4-yl)piperazin-1-yl)ethanon
129 2-((1-(6-Chlorimidazo[2,1-b]thiazol-5-ylsulfonyl)piperidin-2-yl)methoxy)-1-(4-(1-methylpiperidin-4-yl)piperazin-1-yl)ethanon
130 1-(4-Fluor-1,4'-bipiperidin-1'-yl)-2-((1-(4-methoxy-2,6-dimethylphenylsulfonyl)piperidin-2-yl)methoxy)ethanon
131 1-(4-(1-Methylpiperidin-4-yl)piperazin-1-yl)-2-((1-(3-(o-tolyloxy)phenylsulfonyl)piperidin-2-yl)methoxy)ethanon
133 (S)-2-((2-(2,4-Dichlorphenylsulfonyl)-1,2,3,4-tetrahydroisochinolin-3-yl)methoxy)-1-(4-(1-methylpiperidin-4-yl)piperazin-1-yl)ethanon
134 2-((1-(4-Methoxy-2,6-dimethylphenylsulfonyl)piperidin-2-yl)methoxy)-1-(4-(4-(trifluormethyl)cyclohexyl)piperazin-1-yl)ethanon
135 (S)-2-((1-(4-Methoxy-2,6-dimethylphenylsulfonyl)azetidin-2-yl)methoxy)-1-(4-(1-methylpiperidin-4-yl)piperazin-1-yl)ethanon
136 2-((1-(4-Methoxy-2,6-dimethylphenylsulfonyl)piperidin-2-yl)methoxy)-1-(4-(2-morpholin-2-(pyridin-3-yl)ethylamino)piperidin-1-yl)ethanon
137 2-((1-(Benzo[b]thiophen-3-ylsulfonyl)piperidin-2-yl)methoxy)-1-(4-(1-methylpiperidin-4-yl)piperazin-1-yl)ethanon
138 2-((1-(2-Chlor-4-(trifluormethyl)phenylsulfonyl)piperidin-2-yl)methoxy)-1-(4-(1-methylpiperidin-4-yl)piperazin-1-yl)ethanon
139 2-((1-(2-Chlorphenylsulfonyl)piperidin-2-yl)methoxy)-1-(4-(1-methylpiperidin-4-yl)piperazin-1-yl)ethanon dihydrochlorid
140 (R)-2-((1-(4-Methoxy-2,3,6-trimethylphenylsulfonyl)pyrrolidin-2-yl)methoxy)-1-(4-(1-methylpiperidin-4-yl)piperazin-1-yl)ethanon dihydrochlorid
141 2-((1-(2,6-Dichlorphenylsulfonyl)piperidin-2-yl)methoxy)-1-(4-(1-methylpiperidin-4-yl)piperazin-1-yl)ethanon dihydrochlorid
142 (S)-2-((2-(4-Methoxyphenylsulfonyl)-1,2,3,4-tetrahydroisochinolin-3-yl)methoxy)-1-(4-(1-methylpiperidin-4-yl)piperazin-1-yl)ethanon
143 2-((1-(4-Methoxy-2,6-dimethylphenylsulfonyl)piperidin-2-yl)methoxy)-N-(2-(1-(pyridin-4-yl)piperidin-4-yl)ethyl)acetamid
144 1-(4-(5,6-Dihydroimidazo[1,2-a]pyrazin-7(8H)-yl)piperidin-1-yl)-2-((1-(4-methoxy-2,6-dimethylphenylsulfonyl)piperidin-2-yl)methoxy)ethanon
145 1-(4-(5,6-Dihydro-[1,2,4]triazolo[1,5-a]pyrazin-7(8H)-yl)piperidin-1-yl)-2-((1-(4-methoxy-2,6-dimethylphenylsulfonyl)piperidin-2-yl)methoxy)ethanon
146 2-((1-(4-Methoxy-2,6-dimethylphenylsulfonyl)piperidin-2-yl)methoxy)-1-(4-(4- methylpiperazin-1-carbonyl)piperidin-1-yl)ethanon
147 2-((1-(4-Methoxy-2,6-dimethylphenylsulfonyl)piperidin-2-yl)methoxy)-1-(4-(pyridin-4-yl)piperazin-1-yl)ethanon
148 2-((1-(4-Brom-2,6-dimethylphenylsulfonyl)piperidin-2-yl)methoxy)-1-(4-(1-methylpiperidin-4-yl)piperazin-1-yl)ethanon
149 2-((1-(4-Brom-2,6-dimethylphenylsulfonyl)piperidin-2-yl)methoxy)-1-(4-(2-(pyrrolidin-1-yl)ethyl)piperidin-1-yl)ethanon
150 2-((1-(4-Brom-2,6-dimethylphenylsulfonyl)piperidin-2-yl)methoxy)-1-(4-(pyridin-4-yl)piperazin-1-yl)ethanon
151 2-((1-(5-Chlor-3-methylbenzo[b]thiophen-2-ylsulfonyl)piperidin-2-yl)methoxy)-1-(4-(1-methylpiperidin-4-yl)piperazin-1-yl)ethanon dihydrochlorid
152 (R)-2-((1-(2-Chlor-6-methylphenylsulfonyl)pyrrolidin-2-yl)methoxy)-1-(4-(1-methylpiperidin-4-yl)piperazin-1-yl)ethanon dihydrochlorid
153 2-((1-(2,5-Bis(trifluormethyl)phenylsulfonyl)piperidin-2-yl)methoxy)-1-(4-(1-methylpiperidin-4-yl)piperazin-1-yl)ethanon
154 2-((1-(7-Chlorbenzo[c][1,2,5]oxadiazol-4-ylsulfonyl)piperidin-2-yl)methoxy)-1-(4-(1-methylpiperidin-4-yl)piperazin-1-yl)ethanon
155 2-((1-(4-Methylnaphthalen-1-ylsulfonyl)piperidin-2-yl)methoxy)-1-(4-(1-methylpiperidin-4-yl)piperazin-1-yl)ethanon dihydrochlorid
156 1-(4-(1-Methylpiperidin-4-yl)piperazin-1-yl)-2-((1-(2,4,5-trichlorphenylsulfonyl)piperidin-2-yl)methoxy)ethanon dihydrochlorid
157 2-((1-(2-Methylnaphthalen-1-ylsulfonyl)piperidin-2-yl)methoxy)-1-(4-(1-methylpiperidin-4-yl)piperazin-1-yl)ethanon dihydrochlorid
158 2-((1-(5-(Dimethylamino)naphthalen-1-ylsulfonyl)piperidin-2-yl)methoxy)-1-(4-(1-methylpiperidin-4-yl)piperazin-1-yl)ethanon dihydrochlorid
159 1-(4-(1-Methylpiperidin-4-yl)piperazin-1-yl)-2-((1-(o-tolylsulfonyl)piperidin-2-yl)methoxy)ethanon dihydrochlorid
160 2-((1-(4-Brom-2,6-dichlorphenylsulfonyl)piperidin-2-yl)methoxy)-1-(4-(1-methylpiperidin-4-yl)piperazin-1-yl)ethanon dihydrochlorid
161 (S)-2-((1-(2-Chlor-6-methylphenylsulfonyl)pyrrolidin-2-yl)methoxy)-1-(4-(2-(pyrrolidin-1-yl)ethyl)piperidin-1-yl)ethanon hydrochlorid
162 (S)-2-((1-(2-Chlor-6-methylphenylsulfonyl)pyrrolidin-2-yl)methoxy)-1-(4-(1-methylpiperidin-4-yl)piperazin-1-yl)ethanon dihydrochlorid
163 (S)-2-((1-(2-Chlor-6-methylphenylsulfonyl)pyrrolidin-2-yl)methoxy)-1-(4-((1-methylpiperidin-4-yl)methyl)piperazin-1-yl)ethanon dihydrochlorid
164 2-(2-(1-(4-Methoxy-2,6-dimethylphenylsulfonyl)piperidin-2-yl)ethoxy)-1-(4-(1-methylpiperidin-4-yl)piperazin-1-yl)ethanon
165 2-((1-(4-Methoxy-2,6-dimethylphenylsulfonyl)piperidin-2-yl)methoxy)-1-(4-(pyridin-3-yloxy)piperidin-1-yl)ethanon
166 2-((1-(4-Methoxy-2,6-dimethylphenylsulfonyl)piperidin-2-yl)methoxy)-1-(4-(quinoxalin-6-ylmethyl)piperazin-1-yl)ethanon
167 (S)-2-((1-(4-Methoxy-2,6-dimethylphenylsulfonyl)piperidin-2-yl)methoxy)-1-(4-(1-methylpiperidin-4-yl)piperazin-1-yl)ethanon
168 2-((1-(4-Methoxy-2,6-dimethylphenylsulfonyl)piperidin-2-yl)methoxy)-1-(4-(4-(pyrrolidin-1-yl)quinazolin-7-yl)piperazin-1-yl)ethanon
169 2-((1-(4-Fluor-2,6-dimethylphenylsulfonyl)piperidin-2-yl)methoxy)-1-(4-(1-methylpiperidin-4-yl)piperazin-1-yl)ethanon dihydrochlorid
170 2-((1-(2,5-Dichlorthiophen-3-ylsulfonyl)piperidin-2-yl)methoxy)-1-(4-(1-methylpiperidin-4-yl)piperazin-1-yl)ethanon dihydrochlorid
171 2-((1-(Benzo[b]thiophen-2-ylsulfonyl)piperidin-2-yl)methoxy)-1-(4-(1-methylpiperidin-4-yl)piperazin-1-yl)ethanon
172 2-((1-(2,5-Dimethylthiophen-3-ylsulfonyl)piperidin-2-yl)methoxy)-1-(4-(1-methylpiperidin-4-yl)piperazin-1-yl)ethanon
173 2-((1-(2,3-Dichlorphenylsulfonyl)piperidin-2-yl)methoxy)-1-(4-(1-methylpiperidin-4-yl)piperazin-1-yl)ethanon dihydrochlorid
174 2-((1-(4-Methoxynaphthalen-1-ylsulfonyl)piperidin-2-yl)methoxy)-1-(4-(1-methylpiperidin-4-yl)piperazin-1-yl)ethanon dihydrochlorid
175 1-(4-(1-Methylpiperidin-4-yl)piperazin-1-yl)-2-((1-(chinolin-8-ylsulfonyl)piperidin-2-yl)methoxy)ethanon dihydrochlorid
176 2-((1-(Isochinolin-5-ylsulfonyl)piperidin-2-yl)methoxy)-1-(4-(1-methylpiperidin-4-yl)piperazin-1-yl)ethanon dihydrochlorid
177 (R)-2-((1-(4-Methoxy-2,6-dimethylphenylsulfonyl)piperidin-2-yl)methoxy)-1-(4-(1-methylpiperidin-4-yl)piperazin-1-yl)ethanon dihydrochlorid
178 1-(4-(1-Methylpiperidin-4-yl)piperazin-1-yl)-2-((2-(naphthalen-2-ylsulfonyl)-1,2,3,4-tetrahydroisochinolin-3-yl)methoxy)ethanon dihydrochlorid
179 1-(4-(1-Methylpiperidin-4-yl)piperazin-1-yl)-2-((1-(5,6,7,8-tetrahydronaphthalen-2-ylsulfonyl)piperidin-2-yl)methoxy)ethanon dihydrochlorid
180 (S)-2-((1-(4-Methoxy-2,6-dimethylphenylsulfonyl)pyrrolidin-2-yl)methoxy)-1-(4-(1-methylpiperidin-4-yl)piperazin-1-yl)ethanon
181 (S)-2-((1-(4-Methoxy-2,6-dimethylphenylsulfonyl)pyrrolidin-2-yl)methoxy)-1-(4-((1-methylpiperidin-4-yl)methyl)piperazin-1-yl)ethanon
182 (S)-2-((2-(4-Methoxyphenylsulfonyl)-1,2,3,4-tetrahydroisochinolin-3-yl)methoxy)-1-(4-(2-(pyrrolidin-1-yl)ethyl)piperidin-1-yl)ethanon
183 (S)-2-((2-(2,4-Dichlorphenylsulfonyl)-1,2,3,4-tetrahydroisochinolin-3-yl)methoxy)-1-(4-(2-(pyrrolidin-1-yl)ethyl)piperidin-1-yl)ethanon
184 (S)-2-((2-(2,4-Dichlorphenylsulfonyl)-1,2,3,4-tetrahydroisochinolin-3-yl)methoxy)-1-(4-((1-methylpiperidin-4-yl)methyl)piperazin-1-yl)ethanon
185 (S)-2-((2-(4-Methoxy-2,6-dimethylphenylsulfonyl)-1,2,3,4-tetrahydroisochinolin-3-yl)methoxy)-1-(4-((1-methylpiperidin-4-yl)methyl)piperazin-1-yl)ethanon
186 (S)-2-((1-(4-Methoxy-2,6-dimethylphenylsulfonyl)piperidin-2-yl)methoxy)-1-(4-(pyridin-4-yloxy)piperidin-1-yl)ethanon hydrochlorid
187 (S)-2-((2-(4-Methoxy-2,6-dimethylphenylsulfonyl)-1,2,3,4-tetrahydroisochinolin-3-yl)methoxy)-1-(4-(2-(pyrrolidin-1-yl)ethyl)piperidin-1-yl)ethanon
188 2-((1-(4-Methoxy-2,6-dimethylphenylsulfonyl)piperidin-2-yl)methoxy)-1-(4-((pyridin-4-yloxy)methyl)piperidin-1-yl)ethanon
189 (S)-2-((2-(4-Methoxyphenylsulfonyl)-1,2,3,4-tetrahydroisochinolin-3-yl)methoxy)-1-(4-((1-methylpiperidin-4-yl)methyl)piperazin-1-yl)ethanon dihydrochlorid
190 (S)-2-((1-(4-Methoxy-2,6-dimethylphenylsulfonyl)pyrrolidin-2-yl)methoxy)-1-(4-(2-(pyrrolidin-1-yl)ethyl)piperidin-1-yl)ethanon hydrochlorid
191 2-((1-(2-Chlornaphthalen-1-ylsulfonyl)piperidin-2-yl)methoxy)-1-(4-(1-methylpiperidin- 4-yl)piperazin-1-yl)ethanon dihydrochlorid
192 2-((1-(4-Methoxy-2,6-dimethylphenylsulfonyl)pyrrolidin-2-yl)methoxy)-1-(4-(1-methylpiperidin-4-yl)piperazin-1-yl)ethanon
193 N-(4-(4,5-Dihydro-1 H-imidazol-2-yl)benzyl)-2-((1-(4-methoxy-2,6-dimethylphenylsulfonyl)piperidin-2-yl)methoxy)-N-methylacetamid Hydrochlorid
194 2-((1-(4-Methoxy-2,6-dimethylphenylsulfonyl)piperidin-2-yl)methoxy)-N-methyl-N-(2-(4-(pyrrolidin-1-yl)cyclohexyl)ethyl)acetamid
195 2-((1-(4-Methoxy-2,6-dimethylphenylsulfonyl)piperidin-2-yl)methoxy)-N-(3-(pyrazin-2-yloxy)benzyl)acetamid
196 2-((1-(4-Methoxy-2,6-dimethylphenylsulfonyl)piperidin-2-yl)methoxy)-N-(4-(pyrazin-2-yloxy)benzyl)acetamid
197 1-(4-((5-Chlor-2-phenyl-1H-imidazol-4-yl)methyl)piperazin-1-yl)-2-((1-(4-methoxy-2,6-dimethylphenylsulfonyl)piperidin-2-yl)methoxy)ethanon
198 1-(4-((1,5-Dimethyl-1H-pyrazol-4-yl)methyl)piperazin-1-yl)-2-((1-(4-methoxy-2,6-dimethylphenylsulfonyl)piperidin-2-yl)methoxy)ethanon
199 1-(4-((2-(Dimethylamino)pyrimidin-5-yl)methyl)piperazin-1-yl)-2-((1-(4-methoxy-2,6-dimethylphenylsulfonyl)piperidin-2-yl)methoxy)ethanon
200 1-(4-(6-Fluor-3,4-dihydroisochinolin-2(1 H)-yl)piperidin-1-yl)-2-((1-(4-methoxy-2,6-dimethylphenylsulfonyl)piperidin-2-yl)methoxy)ethanon
201 2-((1-(4-Methoxy-2,6-dimethylphenylsulfonyl)piperidin-2-yl)methoxy)-1-(4-(pyridin-4-yl)piperidin-1-yl)ethanon
202 2-((1-(4-Methoxy-2,6-dimethylphenylsulfonyl)-1,2,3,4-tetrahydrochinolin-2-yl)methoxy)-1-(4-(1-methylpiperidin-4-yl)piperazin-1-yl)ethanon
203 1-(4-(1-Methylpiperidin-4-yl)piperazin-1-yl)-2-((1-(naphthalen-2-ylsulfonyl)-1,2,3,4-tetrahydrochinolin-2-yl)methoxy)ethanon
204 2-((1-(4-Methoxyphenylsulfonyl)-1,2,3,4-tetrahydrochinolin-2-yl)methoxy)-1-(4-(1-methylpiperidin-4-yl)piperazin-1-yl)ethanon
205 2-((1-(4-Methoxy-2,6-dimethylphenylsulfonyl)piperidin-2-yl)methoxy)-N-(2-(pyrazin-2-yloxy)benzyl)acetamid
206 2-((1-(4-Methoxy-2,6-dimethylphenylsulfonyl)piperidin-2-yl)methoxy)-1-(1 H-pyrrol[3,4-c]pyridin-2(3H)-yl)ethanon
207 2-((1-(4-Methoxy-2,6-dimethylphenylsulfonyl)piperidin-2-yl)methoxy)-1-(2-(pyridin-3-yl)morpholin)ethanon
208 2-((1-(4-Methoxy-2,6-dimethylphenylsulfonyl)piperidin-2-yl)methoxy)-1-(3-(pyridin-3-ylmethyl)pyrrolidin-1-yl)ethanon
209 N-(2-(4-(Dimethylamino)cyclohexyl)ethyl)-2-((1-(4-methoxy-2,6-dimethylphenylsulfonyl)piperidin-2-yl)methoxy)-N-methylacetamid
210 2-((1-(6-Methoxynaphthalen-2-ylsulfonyl)piperidin-2-yl)methoxy)-1-(4-(1-methylpiperidin-4-yl)piperazin-1-yl)ethanon
211 1-(4-(3,4-Dihydropyrrolo[1,2-a]pyrazin-2(1H)-yl)piperidin-1-yl)-2-((1-(4-methoxy-2,6-dimethylphenylsulfonyl)piperidin-2-yl)methoxy)ethanon
212 2-((1-(4-Methoxy-2,6-dimethylphenylsulfonyl)piperidin-2-yl)methoxy)-1-(4-(2-(1-methylpiperidin-4-yl)ethyl)piperazin-1-yl)ethanon
213 2-((1-(3,4-Dichlorphenylsulfonyl)-1,2,3,4-tetrahydroquinolin-2-yl)methoxy)-N-(2-(4-(dimethylamino)cyclohexyl)ethyl)-N-methylacetamid
214 1-(4-((2-((4-Fluorphenyl)(methyl)amino)pyrimidin-5-yl)methyl)piperazin-1-yl)-2-((1-(4-methoxy-2,6-dimethylphenylsulfonyl)piperidin-2-yl)methoxy)ethanon
215 2-((1-(4-Methoxy-2,6-dimethylphenylsulfonyl)piperidin-2-yl)methoxy)-1-(6-(4-methylpiperazin-1-yl)-3,4-dihydroisochinolin-2(1H)-yl)ethanon
216 2-((1-(3,4-Dichlorphenylsulfonyl)-1,2,3,4-tetrahydroquinolin-2-yl)methoxy)-N-methyl-N-(2-(4-(pyrrolidin-1-yl)cyclohexyl)ethyl)acetamid
217 2-((1-(4-Methoxy-2,6-dimethylphenylsulfonyl)piperidin-2-yl)methoxy)-1-(4-(pyridin-3-yl)piperidin-1-yl)ethanon
218 N-((S)-1-Benzylpyrrolidin-3-yl)-N-methyl-2-((1-(2-(trifluormethyl)phenylsulfonyl)piperidin-2-yl)methoxy)acetamid
219 2-((1-(4-Methoxyphenylsulfonyl)piperidin-2-yl)methoxy)-1-(4-(3-methylbenzyl)piperazin-1-yl)ethanon
220 2-((1-(4-Methoxyphenylsulfonyl)piperidin-2-yl)methoxy)-1-(4-(4-phenethylthiazol-2-yl)piperazin-1-yl)ethanon
221 2-(2-(1-(4-Methoxyphenylsulfonyl)piperidin-2-yl)ethoxy)-1-(4-(2-(piperidin-1-yl)ethyl)piperazin-1-yl)ethanon
222 2-((1-(Mesitylsulfonyl)piperidin-2-yl)methoxy)-1-(4-(5-methyl-1H-benzo[d]imidazol-2-yl)piperidin-1-yl)ethanon
223 1-(2-((4,6-Dimethylpyridin-2-yl)methyl)piperidin-1-yl)-2-((2-(mesitylsulfonyl)-1,2,3,4-tetrahydroisoquinolin-3-yl)methoxy)ethanon
224 1-(2-(5-Brompyridin-3-yl)piperidin-1-yl)-2-((1-(3-(trifluormethyl)phenylsulfonyl)piperidin-2-yl)methoxy)ethanon
225 1-(1,4'-Bipiperidin-1'-yl)-2-((1-(mesitylsulfonyl)piperidin-2-yl)methoxy)ethanon
226 2-((1-(Mesitylsulfonyl)piperidin-2-yl)methoxy)-1-(2-(pyridin-2-ylmethyl)pyrrolidin-1-yl)ethanon
227 2-((1-(Mesitylsulfonyl)piperidin-2-yl)methoxy)-1-(2-(6-methoxypyridin-3-yl)piperidin-1-yl)ethanon
228 1-(2-((5-Ethylpyridin-2-yl)methyl)piperidin-1-yl)-2-((1-(mesitylsulfonyl)piperidin-2-yl)methoxy)ethanon
229 2-((1-(Mesitylsulfonyl)piperidin-2-yl)methoxy)-1-(2-((3-methylpyridin-2-yl)methyl)piperidin-1-yl)ethanon
230 1-(4-(4-Methylpiperazin-1-yl)piperidin-1-yl)-2-((1-(naphthen-1-ylsulfonyl)piperidin-2-yl)methoxy)ethanon
231 2-((1-(Mesitylsulfonyl)piperidin-2-yl)methoxy)-1-(4-(4-methylpiperazin-1-yl)piperidin-1-yl)ethanon
232 2-((1-(Mesitylsulfonyl)piperidin-2-yl)methoxy)-1-(4-((1-methylpiperidin-4-yl)methyl)piperazin-1-yl)ethanon
233 2-((2-(2,4-Dichlorphenylsulfonyl)-1,2,3,4-tetrahydroisoquinolin-3-yl)methoxy)-1-(4-(4-methylpiperazin-1-yl)piperidin-1-yl)ethanon
234 1-(4-(4-Benzylthiazol-2-yl)piperazin-1-yl)-2-((1-(mesitylsulfonyl)piperidin-2-yl)methoxy)ethanon
235 1-(1,4'-Bipiperidin-1'-yl)-2-((1-(naphthen-1-ylsulfonyl)piperidin-2-yl)methoxy)ethanon 2-((1-(4-Methoxy-2,6-dimethylphenylsulfonyl)piperidin-2-yl)methoxy)-1-(3-(pyridin-2-
236 yl)pyrrolidin-1-yl)ethanon
237 2-((1-(4-Methoxy-2,6-dimethylphenylsulfonyl)piperidin-2-yl)methoxy)-1-(3-(pyridin-3-yl)pyrrolidin-1-yl)ethanon
238 2-((1-(4-Methoxy-2,6-dimethylphenylsulfonyl)piperidin-2-yl)methoxy)-1-(3-(pyridin-4-yl)pyrrolidin-1-yl)ethanon
239 2-((1-(4-Methoxy-2,6-dimethylphenylsulfonyl)piperidin-2-yl)methoxy)-N-methyl-N-(2-(pyridin-4-yl)ethyl)acetamid
240 1-(2-((4,6-Dimethylpyridin-2-yl)methyl)pyrrolidin-1-yl)-2-((1-(naphthen-1-ylsulfonyl)piperidin-2-yl)methoxy)ethanon
241 2-((1-(4-Methoxy-2,6-dimethylphenylsulfonyl)piperidin-2-yl)methoxy)-N-methyl-N-(1-phenyl-2-(pyrrolidin-1-yl)ethyl)acetamid
242 2-((1-(4-Methoxy-2,6-dimethylphenylsulfonyl)piperidin-2-yl)methoxy)-1-(4-methylpiperazin-1-yl)ethanon
243 N-(2-(1H-Indol-3-yl)ethyl)-2-((1-(4-methoxy-2,6-dimethylphenylsulfonyl)piperidin-2-yl)methoxy)acetamid
244 2-((1-(4-Methoxy-2,6-dimethylphenylsulfonyl)piperidin-2-yl)methoxy)-N-(2-methylquinolin-4-yl)acetamid
245 1-(4-(2-Ethoxyethyl)piperazin-1-yl)-2-((2-(mesitylsulfonyl)-1,2,3,4-tetrahydroisoquinolin-3-yl)methoxy)ethanon
246 2-((2-(Mesitylsulfonyl)-1,2,3,4-tetrahydroisoquinolin-3-yl)methoxy)-1-(4-(2-methoxyethyl)piperazin-1-yl)ethanon
247 2-((1-(4-Methoxy-2,6-dimethylphenylsulfonyl)piperidin-2-yl)methoxy)-1-(2-(pyridin-2-yl)pyrrolidin-1-yl)ethanon
248 2-((1-(4-Methoxy-2,6-dimethylphenylsulfonyl)piperidin-2-yl)methoxy)-N-(2-(4-methylpiperazin-1-yl)ethyl)acetamid
249 2-((1-(4-Methoxy-2,6-dimethylphenylsulfonyl)piperidin-2-yl)methoxy)-N-(2-morpholin-2-(4-(trifluormethyl)phenyl)ethyl)acetamid
250 N-(5-(Dimethylamino)pentyl)-2-((1-(4-methoxy-2,6-dimethylphenylsulfonyl)piperidin-2-yl)methoxy)acetamid
251 N-(2-(2-Chlorphenyl)-2-morpholinoethyl)-2-((1-(4-methoxy-2,6-dimethylphenylsulfonyl)piperidin-2-yl)methoxy)acetamid
252 2-((1-(4-Methoxy-2,6-dimethylphenylsulfonyl)piperidin-2-yl)methoxy)-N-(2-(4-methoxyphenyl)-2-(pyrrolidin-1-yl)ethyl)acetamid
253 1-(4-(3,5-Dimethoxyphenyl)piperazin-1-yl)-2-((1-(2,4,6-trichlorphenylsulfonyl)piperidin-2-yl)methoxy)ethanon
254 1-(4-(2-(Diisopropylamino)ethyl)piperazin-1-yl)-2-((1-(2,4,6-trichlorphenylsulfonyl)piperidin-2-yl)methoxy)ethanon
255 N-(1-Benzylpiperidin-4-yl)-N-cyclopropyl-2-((1-(2,4,6-trichlorphenylsulfonyl)piperidin-2-yl)methoxy)acetamid
256 N-(2-Morpholino-2-(pyridin-3-yl)ethyl)-2-((1-(2,4,6-trichlorphenylsulfonyl)piperidin-2-yl)methoxy)acetamid
257 N-(1-Ethyl-1 H-pyrazol-5-yl)-2-((1-(4-methoxy-2,6-dimethylphenylsulfonyl)piperidin-2-yl)methoxy)acetamid
258 N-(Isoquinolin-5-yl)-2-((1-(4-methoxy-2,6-dimethylphenylsulfonyl)piperidin-2-yl)methoxy)acetamid
259 N-(2-(Dimethylamino)ethyl)-2-((1-(4-methoxy-2,6-dimethylphenylsulfonyl)piperidin-2- yl)methoxy)acetamid
260 N-(3-(2,6-Dimethylpiperidin-1-yl)propyl)-2-((1-(4-methoxy-2,6-dimethylphenylsulfonyl)piperidin-2-yl)methoxy)acetamid
261 N-(2-(Dimethylamino)-1-phenylethyl)-2-((1-(4-methoxy-2,6-dimethylphenylsulfonyl)piperidin-2-yl)methoxy)acetamid
262 2-((1-(4-Methoxy-2,6-dimethylphenylsulfonyl)piperidin-2-yl)methoxy)-N-(1-phenyl-2-(pyrrolidin-1-yl)ethyl)acetamid
263 2-((1-(4-Methoxy-2,6-dimethylphenylsulfonyl)piperidin-2-yl)methoxy)-N-(2-(4-methylpiperazin-1-yl)-1-phenylethyl)acetamid
264 N-(3-Morpholinophenyl)-2-((2-(phenylsulfonyl)-1,2,3,4-tetrahydroisoquinolin-3-yl)methoxy)acetamid
265 2-((2-(2,4-Dichlorphenylsulfonyl)-1,2,3,4-tetrahydroisoquinolin-3-yl)methoxy)-N-(3-morpholinophenyl)acetamid
266 2-((1-(3,4-Dichlorphenylsulfonyl)piperidin-2-yl)methoxy)-1-(2-(piperidin-1-ylmethyl)morpholino)ethanon
267 2-((1-(4-Methoxy-2,6-dimethylphenylsulfonyl)pyrrolidin-2-yl)methoxy)-1-(4-(4-methylpiperazin-1-yl)piperidin-1-yl)ethanon
268 2-((1-(4-Methoxy-2,6-dimethylphenylsulfonyl)pyrrolidin-2-yl)methoxy)-1-(4-(2-(piperidin-1-yl)ethyl)piperidin-1-yl)ethanon
269 1-(4-(2-(2,5-Dimethyl-1 H-pyrrol-1-yl)ethyl)piperazin-1-yl)-2-((1-(4-methoxy-2,6-dimethylphenylsulfonyl)pyrrolidin-2-yl)methoxy)ethanon
270 1-(4-(2-(Diisopropylamino)ethyl)piperazin-1-yl)-2-((1-(4-methoxy-2,6-dimethylphenylsulfonyl)pyrrolidin-2-yl)methoxy)ethanon
271 2-((1-(4-Methoxy-2,6-dimethylphenylsulfonyl)pyrrolidin-2-yl)methoxy)-1-(2-(pyridin-2-yl)pyrrolidin-1-yl)ethanon
272 2-((1-(4-Methoxy-2,6-dimethylphenylsulfonyl)pyrrolidin-2-yl)methoxy)-1-(3-(pyridin-3-yl)pyrrolidin-1-yl)ethanon
273 2-((1-(4-Methoxy-2,6-dimethylphenylsulfonyl)pyrrolidin-2-yl)methoxy)-1-(3-(pyridin-4-yl)pyrrolidin-1-yl)ethanon
274 2-((1-(4-Methoxy-2,6-dimethylphenylsulfonyl)pyrrolidin-2-yl)methoxy)-1-(4-(pyridin-4-yl)piperazin-1-yl)ethanon
275 2-((2-(4-Methoxy-2,3,6-trimethylphenylsulfonyl)-1,2,3,4-tetrahydroisoquinolin-3-yl)methoxy)-1-(4-(4-methylpiperazin-1-yl)piperidin-1-yl)ethanon
276 2-((2-(4-Methoxy-2,3,6-trimethylphenylsulfonyl)-1,2,3,4-tetrahydroisoquinolin-3-yl)methoxy)-1-(4-(2-(piperidin-1-yl)ethyl)piperidin-1-yl)ethanon
277 2-((2-(4-Methoxy-2,3,6-trimethylphenylsulfonyl)-1,2,3,4-tetrahydroisoquinolin-3-yl)methoxy)-1-(2-((6-methylpyridin-2-yl)methyl)piperidin-1-yl)ethanon
278 2-((1-(2,6-Dichlorphenylsulfonyl)piperidin-2-yl)methoxy)-1-(4-(4-methylpiperazin-1-yl)piperidin-1-yl)ethanon
279 2-((1-(2,6-Dichlorphenylsulfonyl)piperidin-2-yl)methoxy)-1-(4-(2-(piperidin-1-yl)ethyl)piperidin-1-yl)ethanon
280 2-((1-(2,6-Dichlorphenylsulfonyl)piperidin-2-yl)methoxy)-1-(4-(2-(2,5-dimethyl-1 H-pyrrol-1-yl)ethyl)piperazin-1-yl)ethanon
281 2-((1-(2,6-Dichlorphenylsulfonyl)piperidin-2-yl)methoxy)-1-(4-(2-(diisopropylamino)ethyl)piperazin-1-yl)ethanon
282 2-((1-(2,6-Dichlorphenylsulfonyl)piperidin-2-yl)methoxy)-1-(3-(pyridin-4-yl)pyrrolidin-1-yl)ethanon
283 2-((1-(2,6-Dichlorphenylsulfonyl)piperidin-2-yl)methoxy)-1-(4-(pyridin-4-yl)piperazin-1-yl)ethanon
284 2-((1-(2,6-Dichlorphenylsulfonyl)piperidin-2-yl)methoxy)-1-(2-((6-methylpyridin-2-yl)methyl)piperidin-1-yl)ethanon
285 2-((1-(2,6-Dichlorphenylsulfonyl)pyrrolidin-2-yl)methoxy)-1-(4-(4-methylpiperazin-1-yl)piperidin-1-yl)ethanon
286 2-((1-(2,6-Dichlorphenylsulfonyl)pyrrolidin-2-yl)methoxy)-1-(4-(2-(piperidin-1-yl)ethyl)piperidin-1-yl)ethanon
287 2-((1-(2,6-Dichlorphenylsulfonyl)pyrrolidin-2-yl)methoxy)-1-(4-(2-(2,5-dimethyl-1H-pyrrol-1-yl)ethyl)piperazin-1-yl)ethanon
288 2-((1-(4-Methoxy-2,6-dimethylphenylsulfonyl)piperidin-2-yl)methoxy)-1-(4-(3-methoxyphenyl)piperazin-1-yl)ethanon
289 1-(4-(4-Fluorphenyl)piperazin-1-yl)-2-((1-(4-methoxy-2,6-dimethylphenylsulfonyl)piperidin-2-yl)methoxy)ethanon
290 2-((1-(4-Methoxy-2,6-dimethylphenylsulfonyl)piperidin-2-yl)methoxy)-1-(4-(4-methoxyphenyl)piperazin-1-yl)ethanon
291 1-(4-Isopropylpiperazin-1-yl)-2-((1-(4-methoxy-2,6-dimethylphenylsulfonyl)piperidin-2-yl)methoxy)ethanon
292 2-((2-(Mesitylsulfonyl)-1,2,3,4-tetrahydroisoquinolin-3-yl)methoxy)-1-(4-(4-methylpiperazin-1-yl)piperidin-1-yl)ethanon
293 2-((1-(3,4-Dichlorphenylsulfonyl)piperidin-2-yl)methoxy)-N-(3-(pyrrolidin-1-ylmethyl)phenyl)acetamid
294 N-(4-(Dimethylamino)butyl)-2-((1-(2,4,6-trichlorphenylsulfonyl)pyrrolidin-2-yl)methoxy)acetamid
295 1-(4-Ethylpiperazin-1-yl)-2-((1-(2,4,6-trichlorphenylsulfonyl)pyrrolidin-2-yl)methoxy)ethanon
296 1-(4-(Pyrrolidin-1-yl)piperidin-1-yl)-2-((1-(2,4,6-trichlorphenylsulfonyl)pyrrolidin-2-yl)methoxy)ethanon
297 1-(4-Morpholinopiperidin-1-yl)-2-((1-(2,4,6-trichlorphenylsulfonyl)pyrrolidin-2-yl)methoxy)ethanon
298 1-(1,4'-Bipiperidin-1'-yl)-2-((1-(2,4,6-trichlorphenylsulfonyl)pyrrolidin-2-yl)methoxy)ethanon
299 2-((1-(4-Methoxy-2,6-dimethylphenylsulfonyl)pyrrolidin-2-yl)methoxy)-N-(4-(pyrrolidin-1-ylmethyl)benzyl)acetamid
300 N-((1-Benzylpiperidin-3-yl)methyl)-2-((1-(4-methoxy-2,6-dimethylphenylsulfonyl)pyrrolidin-2-yl)methoxy)acetamid
301 2-((1-(4-Methoxy-2,6-dimethylphenylsulfonyl)pyrrolidin-2-yl)methoxy)-N-(4-(4-methylpiperazin-1-yl)phenyl)acetamid
302 2-((1-(4-Methoxy-2,6-dimethylphenylsulfonyl)pyrrolidin-2-yl)methoxy)-N-(4-(4-methylpiperazin-1-yl)benzyl)acetamid
303 N-((S)-1-Benzylpyrrolidin-3-yl)-2-((1-(4-methoxy-2,6-dimethylphenylsulfonyl)pyrrolidin-2-yl)methoxy)-N-methylacetamid
304 2-((1-(4-Methoxy-2,6-dimethylphenylsulfonyl)pyrrolidin-2-yl)methoxy)-N-methyl-N-(2- morpholino-1-phenylethyl)acetamid
305 2-((1-(4-Methoxy-2,6-dimethylphenylsulfonyl)pyrrolidin-2-yl)methoxy)-1-(4-(pyridin-2-yl)piperazin-1-yl)ethanon
306 2-((1-(4-Methoxy-2,6-dimethylphenylsulfonyl)pyrrolidin-2-yl)methoxy)-1-(4-(3-methoxyphenyl)piperazin-1-yl)ethanon
307 1-(4-(4-Fluorphenyl)piperazin-1-yl)-2-((1-(4-methoxy-2,6-dimethylphenylsulfonyl)pyrrolidin-2-yl)methoxy)ethanon
308 2-((1-(4-Methoxy-2,6-dimethylphenylsulfonyl)pyrrolidin-2-yl)methoxy)-1-(4-(4-methoxyphenyl)piperazin-1-yl)ethanon
309 2-((1-(4-Methoxy-2,6-dimethylphenylsulfonyl)pyrrolidin-2-yl)methoxy)-1-(4-(pyrimidin-2-yl)piperazin-1-yl)ethanon
310 2-((1-(4-Mhoxy-2,6-dimethylphenylsulfonyl)pyrrolidin-2-yl)methoxy)-1-(4-phenethylpiperazin-1-yl)ethanon
311 1-(4-(5-Chlor-2-methylphenyl)piperazin-1-yl)-2-((1-(4-methoxy-2,6-dimethylphenylsulfonyl)pyrrolidin-2-yl)methoxy)ethanon
312 2-(4-(2-((1-(4-Methoxy-2,6-dimethylphenylsulfonyl)pyrrolidin-2-yl)methoxy)acetyl)piperazin-1-yl)nicotinonitrile
313 2-((1-(2,6-Dichlorphenylsulfonyl)piperidin-2-yl)methoxy)-N-(4-(pyrrolidin-1-ylmethyl)benzyl)acetamid
314 N-((1-Benzylpiperidin-3-yl)methyl)-2-((1-(2,6-dichlorphenylsulfonyl)piperidin-2-yl)methoxy)acetamid
315 N-((S)-1-Benzylpyrrolidin-3-yl)-2-((1-(2,6-dichlorphenylsulfonyl)piperidin-2-yl)methoxy)-N-methylacetamid
316 2-((1-(2,6-Dichlorphenylsulfonyl)piperidin-2-yl)methoxy)-1-(4-(pyridin-2-yl)piperazin-1-yl)ethanon
317 2-((1-(2,6-Dichlorphenylsulfonyl)piperidin-2-yl)methoxy)-1-(4-(3-methoxyphenyl)piperazin-1-yl)ethanon
318 2-((1-(2,6-Dichlorphenylsulfonyl)piperidin-2-yl)methoxy)-1-(4-(4-fluorphenyl)piperazin-1-yl)ethanon
319 2-((1-(2,6-Dichlorphenylsulfonyl)piperidin-2-yl)methoxy)-1-(4-(4-methoxyphenyl)piperazin-1-yl)ethanon
320 2-((1-(2,6-Dichlorphenylsulfonyl)piperidin-2-yl)methoxy)-1-(4-phenethylpiperazin-1-yl)ethanon
321 1-(4-(5-Chlor-2-methylphenyl)piperazin-1-yl)-2-((1-(2,6-dichlorphenylsulfonyl)piperidin-2-yl)methoxy)ethanon
322 2-((1-(2,6-Dichlorphenylsulfonyl)pyrrolidin-2-yl)methoxy)-N-(4-(pyrrolidin-1-ylmethyl)benzyl)acetamid
323 N-((1-Benzylpiperidin-3-yl)methyl)-2-((1-(2,6-dichlorphenylsulfonyl)pyrrolidin-2-yl)methoxy)acetamid
324 2-((1-(2,6-Dichlorphenylsulfonyl)pyrrolidin-2-yl)methoxy)-1-(4-(3-methoxyphenyl)piperazin-1-yl)ethanon
325 2-((1-(2,6-Dichlorphenylsulfonyl)pyrrolidin-2-yl)methoxy)-1-(4-(4-methoxyphenyl)piperazin-1-yl)ethanon
326 2-((1-(2,6-Dichlorphenylsulfonyl)pyrrolidin-2-yl)methoxy)-1-(4-phenethylpiperazin-1-yl)ethanon
327 N-((1-Benzylpiperidin-3-yl)methyl)-2-((1-(2,4,6-trichlorphenylsulfonyl)pyrrolidin-2-yl)methoxy)acetamid
328 1-(4-(4-Methoxyphenyl)piperazin-1-yl)-2-((1-(2,4,6-trichlorphenylsulfonyl)pyrrolidin-2-yl)methoxy)ethanon
329 1-(4-Phenethylpiperazin-1-yl)-2-((1-(2,4,6-trichlorphenylsulfonyl)pyrrolidin-2-yl)methoxy)ethanon
330 1-(4-(4-Methylpiperazin-1-yl)piperidin-1-yl)-2-((1-(2,4,6-trichlorphenylsulfonyl)pyrrolidin-2-yl)methoxy)ethanon
331 1-(4-(2-(Piperidin-1-yl)ethyl)piperidin-1-yl)-2-((1-(2,4,6-trichlorphenylsulfonyl)pyrrolidin-2-yl)methoxy)ethanon
332 1-(4-(2-(2,5-Dimethyl-1H-pyrrol-1-yl)ethyl)piperazin-1-yl)-2-((1-(2,4,6-trichlorphenylsulfonyl)pyrrolidin-2-yl)methoxy)ethanon
333 1-(4-(2-(Diisopropylamino)ethyl)piperazin-1-yl)-2-((1-(2,4,6-trichlorphenylsulfonyl)pyrrolidin-2-yl)methoxy)ethanon
334 N-(1-Benzylpiperidin-4-yl)-N-cyclopropyl-2-((1-(2,4,6-trichlorphenylsulfonyl)pyrrolidin-2-yl)methoxy)acetamid
335 1-(4-(Pyridin-4-yl)piperazin-1-yl)-2-((1-(2,4,6-trichlorphenylsulfonyl)pyrrolidin-2-yl)methoxy)ethanon
336 1-(1,4'-Bipiperidin-1'-yl)-2-((1-(2,6-dichlorphenylsulfonyl)piperidin-2-yl)methoxy)ethanon
337 1-(1,4'-Bipiperidin-1'-yl)-2-((1-(2,6-dichlorphenylsulfonyl)pyrrolidin-2-yl)methoxy)ethanon
338 N-(2-(Piperidin-1-yl)ethyl)-2-((1-(2,4,6-trichlorphenylsulfonyl)pyrrolidin-2-yl)methoxy)acetamid
339 N-(2-(Pyrrolidin-1-yl)ethyl)-2-((1-(2,4,6-trichlorphenylsulfonyl)pyrrolidin-2-yl)methoxy)acetamid
340 1-(4-(3,5-Dimethoxyphenyl)piperazin-1-yl)-2-((1-(4-methoxy-2,6-dimethylphenylsulfonyl)piperidin-2-yl)methoxy)ethanon
341 1-(4-(2-(Diisopropylamino)ethyl)piperazin-1-yl)-2-((1-(4-methoxy-2,6-dimethylphenylsulfonyl)piperidin-2-yl)methoxy)ethanon
342 N-(1-Benzylpiperidin-4-yl)-N-cyclopropyl-2-((1-(4-methoxy-2,6-dimethylphenylsulfonyl)piperidin-2-yl)methoxy)acetamid
343 2-((1-(4-Methoxy-2,6-dimethylphenylsulfonyl)piperidin-2-yl)methoxy)-N-(2-(pyrrolidin-1-yl)ethyl)acetamid
344 2-((1-(4-Methoxy-2,6-dimethylphenylsulfonyl)piperidin-2-yl)methoxy)-N-(3-morpholinopropyl)acetamid
345 2-((1-(4-Methoxy-2,6-dimethylphenylsulfonyl)piperidin-2-yl)methoxy)-N-(pyridin-3-ylmethyl)acetamid
346 1-(4-(3-Chlorphenyl)piperazin-1-yl)-2-((1-(4-methoxy-2,6-dimethylphenylsulfonyl)pyrrolidin-2-yl)methoxy)ethanon
347 1-(4-(3,4-Dimethylphenyl)piperazin-1-yl)-2-((1-(4-methoxy-2,6-dimethylphenylsulfonyl)pyrrolidin-2-yl)methoxy)ethanon
348 1-(4-(3,4-Dichlorphenyl)piperazin-1-yl)-2-((1-(4-methoxy-2,6-dimethylphenylsulfonyl)pyrrolidin-2-yl)methoxy)ethanon
349 1-(4-(3,4-Dichlorbenzyl)piperazin-1-yl)-2-((1-(4-methoxy-2,6-dimethylphenylsulfonyl)pyrrolidin-2-yl)methoxy)ethanon
350 1-(4-(4-Brombenzyl)piperazin-1-yl)-2-((1-(4-methoxy-2,6-dimethylphenylsulfonyl)pyrrolidin-2-yl)methoxy)ethanon
351 2-((1-(4-Methoxy-2,6-dimethylphenylsulfonyl)pyrrolidin-2-yl)methoxy)-1-(4-(2,4,6-trimethylbenzyl)piperazin-1-yl)ethanon
352 1-(4-(4-Chlorbenzyl)piperazin-1-yl)-2-((1-(4-methoxy-2,6-dimethylphenylsulfonyl)pyrrolidin-2-yl)methoxy)ethanon
353 2-((1-(4-Methoxy-2,6-dimethylphenylsulfonyl)pyrrolidin-2-yl)methoxy)-1-(4-(4-methylbenzyl)piperazin-1-yl)ethanon
354 1-(4-(3-Chlorphenyl)piperazin-1-yl)-2-((1-(2,6-dichlorphenylsulfonyl)pyrrolidin-2-yl)methoxy)ethanon
355 2-((1-(2,6-Dichlorphenylsulfonyl)pyrrolidin-2-yl)methoxy)-1-(4-(3,4-dimethylphenyl)piperazin-1-yl)ethanon
356 1-(4-(3,4-Dichlorphenyl)piperazin-1-yl)-2-((1-(2,6-dichlorphenylsulfonyl)pyrrolidin-2-yl)methoxy)ethanon
357 1-(4-(3,4-Dichlorbenzyl)piperazin-1-yl)-2-((1-(2,6-dichlorphenylsulfonyl)pyrrolidin-2-yl)methoxy)ethanon
358 1-(4-(4-Brombenzyl)piperazin-1-yl)-2-((1-(2,6-dichlorphenylsulfonyl)pyrrolidin-2-yl)methoxy)ethanon
359 1-(4-(4-Chlorbenzyl)piperazin-1-yl)-2-((1-(2,6-dichlorphenylsulfonyl)pyrrolidin-2-yl)methoxy)ethanon
360 2-((1-(2,6-Dichlorphenylsulfonyl)pyrrolidin-2-yl)methoxy)-1-(4-(4-methylbenzyl)piperazin-1-yl)ethanon
361 N-Benzyl-N-(2-(dimethylamino)ethyl)-2-((1-(4-methoxy-2,6-dimethylphenylsulfonyl)piperidin-2-yl)methoxy)acetamid
362 1-(4-Benzyl-1,4-diazepan-1-yl)-2-((1-(4-methoxy-2,6-dimethylphenylsulfonyl)piperidin-2-yl)methoxy)ethanon
363 1-((R)-3-(Dimethylamino)pyrrolidin-1-yl)-2-((1-(4-methoxy-2,6-dimethylphenylsulfonyl)piperidin-2-yl)methoxy)ethanon
364 N-((S)-1-Benzylpyrrolidin-3-yl)-2-((1-(4-methoxy-2,6-dimethylphenylsulfonyl)piperidin-2-yl)methoxy)-N-methylacetamid
365 N-(1-Benzylpyrrolidin-3-yl)-2-((1-(4-methoxy-2,6-dimethylphenylsulfonyl)piperidin-2-yl)methoxy)acetamid
366 N-(3-(1H-Imidazol-1-yl)propyl)-2-((1-(4-methoxy-2,6-dimethylphenylsulfonyl)piperidin-2-yl)methoxy)acetamid
367 N-(4-(Dimethylamino)benzyl)-N-isopropyl-2-((1-(4-methoxy-2,6-dimethylphenylsulfonyl)piperidin-2-yl)methoxy)acetamid
368 2-((1-(4-Methoxy-2,6-dimethylphenylsulfonyl)piperidin-2-yl)methoxy)-N-methyl-N-(2-morpholino-1-phenylethyl)acetamid
369 2-((1-(4-Methoxy-2,6-dimethylphenylsulfonyl)piperidin-2-yl)methoxy)-1-(4-(pyridin-2-yl)piperazin-1-yl)ethanon
370 N-(Isoquinolin-1-yl)-2-((1-(4-methoxy-2,6-dimethylphenylsulfonyl)piperidin-2-yl)methoxy)acetamid
371 2-((1-(4-Methoxy-2,6-dimethylphenylsulfonyl)piperidin-2-yl)methoxy)-N-(1-methyl-1 H-benzo[d]imidazol-2-yl)acetamid
372 2-((1-(4-Methoxy-2,6-dimethylphenylsulfonyl)piperidin-2-yl)methoxy)-N-(pyrimidin-4-yl)acetamid
373 2-((1-(4-Methoxy-2,6-dimethylphenylsulfonyl)piperidin-2-yl)methoxy)-1-(4-phenethylpiperazin-1-yl)ethanon
374 2-((1-(4-Methoxy-2,6-dimethylphenylsulfonyl)piperidin-2-yl)methoxy)-1-((S)-2-(pyrrolidin-1-ylmethyl)pyrrolidin-1-yl)ethanon
375 2-((1-(4-Methoxy-2,6-dimethylphenylsulfonyl)piperidin-2-yl)methoxy)-1-(4-methyl-1,4-diazepan-1-yl)ethanon
376 1-(4-(3-Chlor-5-(trifluormethyl)pyridin-2-yl)piperazin-1-yl)-2-((1-(4-methoxy-2,6-dimethylphenylsulfonyl)piperidin-2-yl)methoxy)ethanon
377 N-Benzyl-N-(2-(dimethylamino)ethyl)-2-((1-(4-methoxy-2,6-dimethylphenylsulfonyl)piperidin-2-yl)methoxy)acetamid
378 1-(4-(3,4-Dichlorphenyl)piperazin-1-yl)-2-((1-(4-methoxy-2,6-dimethylphenylsulfonyl)piperidin-2-yl)methoxy)ethanon
379 N-(2-(Dimethylamino)ethyl)-2-((1-(4-methoxy-2,6-dimethylphenylsulfonyl)piperidin-2-yl)methoxy)-N-methylacetamid
380 N-(2-(Diethylamino)ethyl)-N-ethyl-2-((1-(4-methoxy-2,6-dimethylphenylsulfonyl)piperidin-2-yl)methoxy)acetamid
381 1-(4-(3,4-Dichlorbenzyl)piperazin-1-yl)-2-((1-(4-methoxy-2,6-dimethylphenylsulfonyl)piperidin-2-yl)methoxy)ethanon
382 1-(4-(4-Brombenzyl)piperazin-1-yl)-2-((1-(4-methoxy-2,6-dimethylphenylsulfonyl)piperidin-2-yl)methoxy)ethanon
383 2-((1-(4-Methoxy-2,6-dimethylphenylsulfonyl)piperidin-2-yl)methoxy)-1-(4-(2,4,6-trimethylbenzyl)piperazin-1-yl)ethanon
384 1-(4-(4-Chlorbenzyl)piperazin-1-yl)-2-((1-(4-methoxy-2,6-dimethylphenylsulfonyl)piperidin-2-yl)methoxy)ethanon
385 2-((1-(4-Methoxy-2,6-dimethylphenylsulfonyl)piperidin-2-yl)methoxy)-1-(4-(4-methylbenzyl)piperazin-1-yl)ethanon
386 2-((1-(4-Methoxy-2,6-dimethylphenylsulfonyl)piperidin-2-yl)methoxy)-1-(4-(4-methoxybenzyl)piperazin-1-yl)ethanon
387 1-(4-(2-Fluorbenzyl)piperazin-1-yl)-2-((1-(4-methoxy-2,6-dimethylphenylsulfonyl)piperidin-2-yl)methoxy)ethanon
388 2-((1-(4-Methoxy-2,6-dimethylphenylsulfonyl)piperidin-2-yl)methoxy)-1-(4-methyl-2-phenylpiperazin-1-yl)ethanon
389 1-(3-(Pyridin-3-yl)pyrrolidin-1-yl)-2-((1-(2,4,6-trichlorphenylsulfonyl)piperidin-2-yl)methoxy)ethanon
390 1-(3-(Pyridin-4-yl)pyrrolidin-1-yl)-2-((1-(2,4,6-trichlorphenylsulfonyl)piperidin-2-yl)methoxy)ethanon
391 N-Methyl-N-(2-(pyridin-4-yl)ethyl)-2-((1-(2,4,6-trichlorphenylsulfonyl)piperidin-2-yl)methoxy)acetamid
392 N-Methyl-N-(1-phenyl-2-(pyrrolidin-1-yl)ethyl)-2-((1-(2,4,6-trichlorphenylsulfonyl)piperidin-2-yl)methoxy)acetamid
393 1-(4-Methylpiperazin-1-yl)-2-((1-(2,4,6-trichlorphenylsulfonyl)piperidin-2-yl)methoxy)ethanon
394 N-(Pyridin-4-ylmethyl)-2-((1-(2,4,6-trichlorphenylsulfonyl)piperidin-2- yl)methoxy)acetamid
395 2-((1-(4-Methoxy-2,6-dimethylphenylsulfonyl)piperidin-2-yl)methoxy)-N-(2-morpholinopyridin-3-yl)acetamid
396 2-((1-(4-Methoxy-2,6-dimethylphenylsulfonyl)piperidin-2-yl)methoxy)-N-(6-morpholinopyridin-3-yl)acetamid
397 2-((1-(4-Methoxy-2,6-dimethylphenylsulfonyl)piperidin-2-yl)methoxy)-1-(4-(5-methyl-1 H-benzo[d]imidazol-2-yl)piperidin-1-yl)ethanon
398 1-(2-(4-(Dimethylamino)phenyl)pyrrolidin-1-yl)-2-((1-(4-methoxy-2,6-dimethylphenylsulfonyl)piperidin-2-yl)methoxy)ethanon
399 2-((1-(4-Methoxy-2,6-dimethylphenylsulfonyl)piperidin-2-yl)methoxy)-1-(2-(piperidin-1-ylmethyl)pyrrolidin-1-yl)ethanon
400 1-(2-(4-(Dimethylamino)phenyl)azepan-1-yl)-2-((1-(4-methoxy-2,6-dimethylphenylsulfonyl)piperidin-2-yl)methoxy)ethanon
401 N-(2-(4-Benzylpiperazin-1-yl)ethyl)-2-((1-(4-methoxy-2,6-dimethylphenylsulfonyl)piperidin-2-yl)methoxy)acetamid
402 2-((1-(4-Methoxy-2,6-dimethylphenylsulfonyl)piperidin-2-yl)methoxy)-1-(4-(4-phenylthiazol-2-yl)piperazin-1-yl)ethanon
403 2-((1-(2,6-Dichlorphenylsulfonyl)piperidin-2-yl)methoxy)-1-(4-(3,4-dimethylphenyl)piperazin-1-yl)ethanon
404 1-(4-(3,4-Dichlorphenyl)piperazin-1-yl)-2-((1-(2,6-dichlorphenylsulfonyl)piperidin-2-yl)methoxy)ethanon
405 1-(4-(3,4-Dichlorbenzyl)piperazin-1-yl)-2-((1-(2,6-dichlorphenylsulfonyl)piperidin-2-yl)methoxy)ethanon
406 1-(4-(4-Brombenzyl)piperazin-1-yl)-2-((1-(2,6-dichlorphenylsulfonyl)piperidin-2-yl)methoxy)ethanon
407 1-(4-(4-Chlorbenzyl)piperazin-1-yl)-2-((1-(2,6-dichlorphenylsulfonyl)piperidin-2-yl)methoxy)ethanon
408 2-((1-(2,6-Dichlorphenylsulfonyl)piperidin-2-yl)methoxy)-1-(4-(4-methylbenzyl)piperazin-1-yl)ethanon
409 2-((1-(4-Methoxy-2,6-dimethylphenylsulfonyl)piperidin-2-yl)methoxy)-N-(4-(pyrrolidin-1-yl)phenyl)acetamid
410 3-(4-(2-((1-(4-Methoxy-2,6-dimethylphenylsulfonyl)piperidin-2-yl)methoxy)acetyl)piperazin-1-yl)propanenitrile
411 N-(3-(4-Menzylpiperazin-1-yl)propyl)-2-((1-(4-methoxy-2,6-dimethylphenylsulfonyl)piperidin-2-yl)methoxy)acetamid
412 N-(3-(4-Ethylpiperazin-1-yl)propyl)-2-((1-(4-methoxy-2,6-dimethylphenylsulfonyl)piperidin-2-yl)methoxy)acetamid
413 2-((1-(4-Methoxy-2,6-dimethylphenylsulfonyl)piperidin-2-yl)methoxy)-N-(3-(piperidin-1-yl)propyl)acetamid
414 N-((1-Benzylpyrrolidin-3-yl)methyl)-2-((1-(4-methoxy-2,6-dimethylphenylsulfonyl)piperidin-2-yl)methoxy)acetamid
415 N-((1-Benzylpiperidin-3-yl)methyl)-2-((1-(4-methoxy-2,6-dimethylphenylsulfonyl)piperidin-2-yl)methoxy)acetamid
416 2-((1-(4-Methoxy-2,6-dimethylphenylsulfonyl)piperidin-2-yl)methoxy)-N-(2-(5-methyl-1 H-pyrazol-1-yl)ethyl)acetamid
417 2-((1-(4-Methoxy-2,6-dimethylphenylsulfonyl)piperidin-2-yl)methoxy)-N-(4-(2-(1-methyl-1 H-benzo[d]imidazol-2-yl)ethyl)phenyl)acetamid
418 2-((1-(4-Methoxy-2,6-dimethylphenylsulfonyl)piperidin-2-yl)methoxy)-N-(3-(piperidin-1-ylmethyl)phenyl)acetamid
419 2-((1-(4-Methoxy-2,6-dimethylphenylsulfonyl)piperidin-2-yl)methoxy)-N-(4-(piperidin-1-ylmethyl)phenyl)acetamid
420 N-(4-((1H-Imidazol-1-yl)methyl)phenyl)-2-((1-(4-methoxy-2,6-dimethylphenylsulfonyl)piperidin-2-yl)methoxy)acetamid
421 2-((1-(4-Methoxy-2,6-dimethylphenylsulfonyl)piperidin-2-yl)methoxy)-N-(4-((4-methylpiperidin-1-yl)methyl)phenyl)acetamid
422 N-((1-Benzylpiperidin-3-yl)methyl)-2-((1-(4-methoxyphenylsulfonyl)indolin-2-yl)methoxy)acetamid
423 2-((1-(4-Methoxyphenylsulfonyl)indolin-2-yl)methoxy)-N-(3-(piperidin-1-ylmethyl)phenyl)acetamid
424 N-(3-(Ethyl(phenyl)amino)propyl)-2-((1-(4-methoxy-2,6-dimethylphenylsulfonyl)piperidin-2-yl)methoxy)acetamid
425 2-((1-(4-Methoxy-2,6-dimethylphenylsulfonyl)piperidin-2-yl)methoxy)-N-(2-(pyrazin-2-yl)ethyl)acetamid
426 N-(1-(1H-pyrazol-1-yl)propan-2-yl)-2-((1-(4-methoxy-2,6-dimethylphenylsulfonyl)piperidin-2-yl)methoxy)acetamid
427 2-((1-(4-Methoxy-2,6-dimethylphenylsulfonyl)piperidin-2-yl)methoxy)-N-(4-(2-methyl-1H-imidazol-1-yl)phenyl)acetamid
428 2-((1-(4-Methoxy-2,6-dimethylphenylsulfonyl)piperidin-2-yl)methoxy)-N-(2-(piperidin-1-yl)benzyl)acetamid
429 2-((1-(4-Methoxy-2,6-dimethylphenylsulfonyl)piperidin-2-yl)methoxy)-N-(3-(piperidin-1-yl)phenyl)acetamid
430 2-((1-(4-Methoxy-2,6-dimethylphenylsulfonyl)piperidin-2-yl)methoxy)-N-(3-(piperidin-1-yl)benzyl)acetamid
431 2-((1-(4-Methoxy-2,6-dimethylphenylsulfonyl)piperidin-2-yl)methoxy)-N-(2-(4-methylpiperazin-1-yl)phenyl)acetamid
432 2-((1-(4-Methoxy-2,6-dimethylphenylsulfonyl)piperidin-2-yl)methoxy)-N-(2-(4-methylpiperazin-1-yl)benzyl)acetamid
433 2-((1-(4-Methoxy-2,6-dimethylphenylsulfonyl)piperidin-2-yl)methoxy)-N-(3-(4-methylpiperazin-1-yl)benzyl)acetamid
434 2-((1-(4-Methoxy-2,6-dimethylphenylsulfonyl)piperidin-2-yl)methoxy)-N-(4-(4-methylpiperazin-1-yl)benzyl)acetamid
435 2-((1-(4-Methoxy-2,6-dimethylphenylsulfonyl)piperidin-2-yl)methoxy)-N-(2-morpholinobenzyl)acetamid
436 2-((1-(4-Methoxy-2,6-dimethylphenylsulfonyl)piperidin-2-yl)methoxy)-N-(3-morpholinophenyl)acetamid
437 2-((1-(4-Methoxy-2,6-dimethylphenylsulfonyl)piperidin-2-yl)methoxy)-N-(3-morpholinobenzyl)acetamid
438 2-((1-(4-Methoxy-2,6-dimethylphenylsulfonyl)piperidin-2-yl)methoxy)-N-(4-morpholinophenyl)acetamid
439 2-((1-(4-Methoxy-2,6-dimethylphenylsulfonyl)piperidin-2-yl)methoxy)-N-(4- morpholinobenzyl)acetamid
440 2-((1-(2,6-Dichlorphenylsulfonyl)piperidin-2-yl)methoxy)-N-(2-(piperidin-1-yl)ethyl)acetamid
441 2-((1-(2,6-Dichlorphenylsulfonyl)piperidin-2-yl)methoxy)-N-(4-(dimethylamino)butyl)acetamid
442 2-((1-(2,6-Dichlorphenylsulfonyl)piperidin-2-yl)methoxy)-1-(4-(2 fluorphenyl)piperazin-1-yl)ethanon
443 2-((1-(2,6-Dichlorphenylsulfonyl)piperidin-2-yl)methoxy)-1-(4-(pyrrolidin-1-yl)piperidin-1-yl)ethanon
444 1-(4-(2-Fluorbenzyl)piperazin-1-yl)-2-((1-(4-methoxy-2,6-dimethylphenylsulfonyl)pyrrolidin-2-yl)methoxy)ethanon
445 2-((1-(4-Methoxy-2,6-dimethylphenylsulfonyl)pyrrolidin-2-yl)methoxy)-1-(4-methyl-2-phenylpiperazin-1-yl)ethanon
446 2-((1-(4-Methoxy-2,6-dimethylphenylsulfonyl)pyrrolidin-2-yl)methoxy)-N-methyl-N-(2-(4-methylpiperazin-1-yl)-1-phenylethyl)acetamid
447 2-((1-(4-Methoxy-2,6-dimethylphenylsulfonyl)pyrrolidin-2-yl)methoxy)-1-(2-(pyridin-2-ylmethyl)pyrrolidin-1-yl)ethanon
448 2-((1-(4-Methoxy-2,6-dimethylphenylsulfonyl)pyrrolidin-2-yl)methoxy)-1-(2-((6-methylpyridin-2-yl)methyl)pyrrolidin-1-yl)ethanon
449 2-((1-(4-Methoxy-2,6-dimethylphenylsulfonyl)pyrrolidin-2-yl)methoxy)-1-(2-(pyridin-2-ylmethyl)piperidin-1-yl)ethanon
450 2-((1-(4-Methoxy-2,6-dimethylphenylsulfonyl)pyrrolidin-2-yl)methoxy)-N-methyl-N-(1-phenyl-2-(pyrrolidin-1-yl)ethyl)acetamid
451 2-((1-(2,6-Dichlorphenylsulfonyl)pyrrolidin-2-yl)methoxy)-1-(4-(4-methoxybenzyl)piperazin-1-yl)ethanon
452 2-((1-(2,6-Dichlorphenylsulfonyl)pyrrolidin-2-yl)methoxy)-1-(4-(2-(dimethylamino)ethyl)piperazin-1-yl)ethanon
453 2-((1-(2,6-Dichlorphenylsulfonyl)pyrrolidin-2-yl)methoxy)-1-(2-(pyridin-4-ylmethyl)piperidin-1-yl)ethanon
454 2-((1-(2,6-Dichlorphenylsulfonyl)piperidin-2-yl)methoxy)-1-(4-(2-fluorbenzyl)piperazin-1-yl)ethanon
455 2-((1-(2,6-Dichlorphenylsulfonyl)piperidin-2-yl)methoxy)-1-(4-methyl-2-phenylpiperazin-1-yl)ethanon
456 2-((1-(2,6-Dichlorphenylsulfonyl)piperidin-2-yl)methoxy)-1-(2-(pyridin-2-ylmethyl)pyrrolidin-1-yl)ethanon
457 2-((1-(2,6-Dichlorphenylsulfonyl)piperidin-2-yl)methoxy)-1-(2-((4,6-dimethylpyridin-2-yl)methyl)pyrrolidin-1-yl)ethanon
458 2-((1-(2,6-Dichlorphenylsulfonyl)piperidin-2-yl)methoxy)-1-(2-((6-methylpyridin-2-yl)methyl)pyrrolidin-1-yl)ethanon
459 N-(2-(Pyrrolidin-1-yl)ethyl)-2-((1-(2,4,6-trichlorphenylsulfonyl)piperidin-2-yl)methoxy)acetamid
460 1-(4-Benzylpiperazin-1-yl)-2-((1-(4-methoxy-2,6-dimethylphenylsulfonyl)pyrrolidin-2-yl)methoxy)ethanon
461 2-((1-(4-Methoxy-2,6-dimethylphenylsulfonyl)pyrrolidin-2-yl)methoxy)-1-(4-phenylpiperazin-1-yl)ethanon
462 1-(4-(Benzo[d][1,3]dioxol-5-ylmethyl)piperazin-1-yl)-2-((1-(4-methoxy-2,6-dimethylphenylsulfonyl)pyrrolidin-2-yl)methoxy)ethanon
463 N-Benzyl-2-((1-(4-methoxy-2,6-dimethylphenylsulfonyl)pyrrolidin-2-yl)methoxy)-N-(4-methylpyridin-2-yl)acetamid
464 N-Benzyl-2-((1-(4-methoxy-2,6-dimethylphenylsulfonyl)pyrrolidin-2-yl)methoxy)-N-(6-methylpyridin-2-yl)acetamid
465 2-((1-(4-Methoxy-2,6-dimethylphenylsulfonyl)pyrrolidin-2-yl)methoxy)-1-(4-(3-(trifluormethyl)phenyl)piperazin-1-yl)ethanon
466 N-Ethyl-2-((1-(4-methoxy-2,6-dimethylphenylsulfonyl)pyrrolidin-2-yl)methoxy)-N-(pyridin-4-ylmethyl)acetamid
467 2-((1-(4-Methoxy-2,6-dimethylphenylsulfonyl)pyrrolidin-2-yl)methoxy)-1-(4-methyl-1,4-diazepan-1-yl)ethanon
468 2-((1-(4-Methoxy-2,6-dimethylphenylsulfonyl)pyrrolidin-2-yl)methoxy)-1-(4-(5-(trifluormethyl)pyridin-2-yl)piperazin-1-yl)ethanon
469 1-(4-(3-Chlor-5-(trifluormethyl)pyridin-2-yl)piperazin-1-yl)-2-((1-(4-methoxy-2,6-dimethylphenylsulfonyl)pyrrolidin-2-yl)methoxy)ethanon
470 2-((1-(4-Methoxy-2,6-dimethylphenylsulfonyl)pyrrolidin-2-yl)methoxy)-N-(4-(2-(1-methyl-1H-benzo[d]imidazol-2-yl)ethyl)phenyl)acetamid
471 2-((1-(4-Methoxy-2,6-dimethylphenylsulfonyl)pyrrolidin-2-yl)methoxy)-N-(3-(piperidin-1-ylmethyl)phenyl)acetamid
472 N-(4-(2-(1H-Benzo[d]imidazol-2-yl)ethyl)phenyl)-2-((1-(4-methoxy-2,6-dimethylphenylsulfonyl)pyrrolidin-2-yl)methoxy)acetamid
473 2-((1-(4-Methoxy-2,6-dimethylphenylsulfonyl)pyrrolidin-2-yl)methoxy)-N-(2-(4-methylpiperazin-1-yl)benzyl)acetamid
474 2-((1-(4-Methoxy-2,6-dimethylphenylsulfonyl)pyrrolidin-2-yl)methoxy)-1-(4-(5-methyl-1H-benzo[d]imidazol-2-yl)piperidin-1-yl)ethanon
475 1-(2-(4-(Dimethylamino)phenyl)azepan-1-yl)-2-((1-(4-methoxy-2,6-dimethylphenylsulfonyl)pyrrolidin-2-yl)methoxy)ethanon
476 1-((R)-3-(Dimethylamino)pyrrolidin-1-yl)-2-((1-(4-methoxy-2,6-dimethylphenylsulfonyl)pyrrolidin-2-yl)methoxy)ethanon
477 N-(1-Benzylpiperidin-4-yl)-2-((1-(4-methoxy-2,6-dimethylphenylsulfonyl)pyrrolidin-2-yl)methoxy)acetamid
478 1-(4-Benzylpiperazin-1-yl)-2-((1-(2,5-dichlorthiophen-3-ylsulfonyl)pyrrolidin-2-yl)methoxy)ethanon
479 2-((2-(4-Methoxy-2,3,6-trimethylphenylsulfonyl)-1,2,3,4-tetrahydroisoquinolin-3-yl)methoxy)-1-(4-phenylpiperazin-1-yl)ethanon
480 1-(4-Benzylpiperazin-1-yl)-2-((1-(2,6-dichlorphenylsulfonyl)piperidin-2-yl)methoxy)ethanon
481 2-((1-(2,6-Dichlorphenylsulfonyl)piperidin-2-yl)methoxy)-1-(4-(3-(trifluormethyl)phenyl)piperazin-1-yl)ethanon
482 2-((1-(2,6-Dichlorphenylsulfonyl)piperidin-2-yl)methoxy)-N-ethyl-N-(pyridin-4-ylmethyl)acetamid
483 2-((1-(2,6-Dichlorphenylsulfonyl)piperidin-2-yl)methoxy)-1-(4-methyl-1,4-diazepan-1-yl)ethanon
484 2-((1-(2,6-Dichlorphenylsulfonyl)piperidin-2-yl)methoxy)-1-(4-(5- (trifluormethyl)pyridin-2-yl)piperazin-1-yl)ethanon
485 1-(4-(3-Chlor-5-(trifluormethyl)pyridin-2-yl)piperazin-1-yl)-2-((1-(2,6-dichlorphenylsulfonyl)piperidin-2-yl)methoxy)ethanon
486 N-Benzyl-2-((1-(2,6-dichlorphenylsulfonyl)piperidin-2-yl)methoxy)-N-(pyridin-2-yl)acetamid
487 2-((1-(2,6-Dichlorphenylsulfonyl)piperidin-2-yl)methoxy)-1-(4-(5-methyl-1H-benzo[d]imidazol-2-yl)piperidin-1-yl)ethanon
488 N-(1-Benzylpiperidin-4-yl)-2-((1-(2,6-dichlorphenylsulfonyl)piperidin-2-yl)methoxy)acetamid
489 N-Benzyl-2-((1-(2,6-dichlorphenylsulfonyl)piperidin-2-yl)methoxy)-N-(2-(dimethylamino)ethyl)acetamid
490 1-(2-((5-Ethylpyridin-2-yl)methyl)pyrrolidin-1-yl)-2-((1-(4-methoxy-2,6-dimethylphenylsulfonyl)pyrrolidin-2-yl)methoxy)ethanon
491 2-((1-(4-Methoxy-2,6-dimethylphenylsulfonyl)pyrrolidin-2-yl)methoxy)-1-(4-(2-methoxyphenyl)piperazin-1-yl)ethanon
492 1-(4-(Cyclohexylmethyl)piperazin-1-yl)-2-((1-(4-methoxy-2,6-dimethylphenylsulfonyl)pyrrolidin-2-yl)methoxy)ethanon
493 N-(3-Chlor-4-morpholinophenyl)-2-((1-(4-methoxy-2,6-dimethylphenylsulfonyl)pyrrolidin-2-yl)methoxy)acetamid
494 2-((1-(4-Methoxy-2,6-dimethylphenylsulfonyl)pyrrolidin-2-yl)methoxy)-N-(1-methyl-1 H-benzo[d]imidazol-5-yl)acetamid
495 N-(2-(Dimethylamino)-2-(4-(trifluormethyl)phenyl)ethyl)-2-((1-(4-methoxy-2,6-dimethylphenylsulfonyl)pyrrolidin-2-yl)methoxy)acetamid
496 2-((1-(4-Methoxy-2,6-dimethylphenylsulfonyl)pyrrolidin-2-yl)methoxy)-N-(2-(pyrrolidin-1-yl)-2-(4-(trifluormethyl)phenyl)ethyl)acetamid
497 2-((1-(2,6-Dichlorphenylsulfonyl)piperidin-2-yl)methoxy)-1-(2-(pyridin-3-yl)pyrrolidin-1-yl)ethanon
498 2-((1-(2,6-Dichlorphenylsulfonyl)piperidin-2-yl)methoxy)-1-(4-(2-methoxyphenyl)piperazin-1-yl)ethanon
499 1-(4-(Cyclohexylmethyl)piperazin-1-yl)-2-((1-(2,6-dichlorphenylsulfonyl)piperidin-2-yl)methoxy)ethanon
500 2-((1-(4-Methoxy-2,6-dimethylphenylsulfonyl)pyrrolidin-2-yl)methoxy)-N-(2-morpholino-1-phenylethyl)acetamid
501 2-((1-(4-Methoxy-2,6-dimethylphenylsulfonyl)pyrrolidin-2-yl)methoxy)-N-(1-methyl-1 H-indazol-6-yl)acetamid
502 N-(2-(2-Chlorphenyl)-2-(pyrrolidin-1-yl)ethyl)-2-((1-(4-methoxy-2,6-dimethylphenylsulfonyl)pyrrolidin-2-yl)methoxy)acetamid
503 1-(4-(3,5-Dimethoxyphenyl)piperazin-1-yl)-2-((1-(4-methoxy-2,6-dimethylphenylsulfonyl)pyrrolidin-2-yl)methoxy)ethanon
504 2-((1-(4-Methoxy-2,6-dimethylphenylsulfonyl)pyrrolidin-2-yl)methoxy)-N-(1-methyl-1H-benzo[d]imidazol-2-yl)acetamid
505 2-((1-(4-Methoxy-2,6-dimethylphenylsulfonyl)pyrrolidin-2-yl)methoxy)-N-(1-phenyl-2-(pyrrolidin-1-yl)ethyl)acetamid
506 3-(4-(2-((1-(2,6-Dichlorphenylsulfonyl)piperidin-2-yl)methoxy)acetyl)piperazin-1-yl)propanenitrile
507 N-Benzyl-2-((1-(4-methoxy-2,6-dimethylphenylsulfonyl)piperidin-2-yl)methoxy)-N-(4-methylpyridin-2-yl)acetamid
508 N-Benzyl-2-((1-(4-methoxy-2,6-dimethylphenylsulfonyl)piperidin-2-yl)methoxy)-N-(6-methylpyridin-2-yl)acetamid
509 2-((1-(4-Methoxy-2,6-dimethylphenylsulfonyl)piperidin-2-yl)methoxy)-1-(4-(3-(trifluormethyl)phenyl)piperazin-1-yl)ethanon
510 N-ethyl-2-((1-(4-methoxy-2,6-dimethylphenylsulfonyl)piperidin-2-yl)methoxy)-N-(pyridin-4-ylmethyl)acetamid
511 1-(4-(3-Chlorphenyl)piperazin-1-yl)-2-((1-(4-methoxy-2,6-dimethylphenylsulfonyl)piperidin-2-yl)methoxy)ethanon
512 1-(4-(3,4-Dimethylphenyl)piperazin-1-yl)-2-((1-(4-methoxy-2,6-dimethylphenylsulfonyl)piperidin-2-yl)methoxy)ethanon
513 2-(4-(2-((1-(4-Methoxy-2,6-dimethylphenylsulfonyl)piperidin-2-yl)methoxy)acetyl)piperazin-1-yl)nicotinonitrile
514 2-((1-(4-Methoxy-2,6-dimethylphenylsulfonyl)piperidin-2-yl)methoxy)-1-(4-(5-(trifluormethyl)pyridin-2-yl)piperazin-1-yl)ethanon
515 N-Benzyl-2-((1-(2,5-dichlorthiophen-3-ylsulfonyl)piperidin-2-yl)methoxy)-N-(6-methylpyridin-2-yl)acetamid
516 1-(4-(2-Fluorphenyl)piperazin-1-yl)-2-((1-(4-methoxy-2,6-dimethylphenylsulfonyl)pyrrolidin-2-yl)methoxy)ethanon
517 1-(4-Ethylpiperazin-1-yl)-2-((1-(4-methoxy-2,6-dimethylphenylsulfonyl)pyrrolidin-2-yl)methoxy)ethanon
518 2-((1-(4-Methoxy-2,6-dimethylphenylsulfonyl)pyrrolidin-2-yl)methoxy)-1-(4-(pyrrolidin-1-yl)piperidin-1-yl)ethanon
519 2-((1-(4-Methoxy-2,6-dimethylphenylsulfonyl)pyrrolidin-2-yl)methoxy)-1-(4-morpholinopiperidin-1-yl)ethanon
520 1-(1,4'-Bipiperidin-1'-yl)-2-((1-(4-methoxy-2,6-dimethylphenylsulfonyl)pyrrolidin-2-yl)methoxy)ethanon
521 N-(3-(4-Benzylpiperazin-1-yl)propyl)-2-((1-(4-methoxy-2,6-dimethylphenylsulfonyl)pyrrolidin-2-yl)methoxy)acetamid
522 2-((1-(4-Methoxy-2,6-dimethylphenylsulfonyl)pyrrolidin-2-yl)methoxy)-N-(3-(piperidin-1-yl)propyl)acetamid
523 2-((1-(4-Methoxy-2,6-dimethylphenylsulfonyl)pyrrolidin-2-yl)methoxy)-N-(4-(pyrrolidin-1-yl)butyl)acetamid
524 2-((1-(4-Methoxy-2,6-dimethylphenylsulfonyl)piperidin-2-yl)methoxy)-1-(4-(4-methylpiperazin-1-yl)piperidin-1-yl)ethanon
525 N-(3-(1H-Benzo[d]imidazol-2-yl)propyl)-2-((1-(4-methoxy-2,6-dimethylphenylsulfonyl)piperidin-2-yl)methoxy)acetamid
526 1-(4-(Benzo[d]thiazol-2-yl)piperazin-1-yl)-2-((1-(4-methoxy-2,6-dimethylphenylsulfonyl)piperidin-2-yl)methoxy)ethanon
527 2-((1-(2,6-Dichlorphenylsulfonyl)piperidin-2-yl)methoxy)-N-(4-(2-methyl-1H-imidazol-1-yl)phenyl)acetamid
528 2-((1-(2,6-Dichlorphenylsulfonyl)piperidin-2-yl)methoxy)-1-(2-(4-(dimethylamino)phenyl)pyrrolidin-1-yl)ethanon
529 1-(4-Benzyl-1,4-diazepan-1-yl)-2-((1-(2,6-dichlorphenylsulfonyl)piperidin-2-yl)methoxy)ethanon
530 2-((1-(2,6-Dichlorphenylsulfonyl)piperidin-2-yl)methoxy)-N-(4-(dimethylamino)benzyl)-N-isopropylacetamid
531 2-(4-(2-((1-(4-Methoxy-2,6-dimethylphenylsulfonyl)pyrrolidin-2-yl)methoxy)acetyl)-1,4-diazepan-1-yl)nicotinonitrile
532 2-((1-(4-Methoxy-2,6-dimethylphenylsulfonyl)piperidin-2-yl)methoxy)-N-methyl-N-(2-(piperidin-1-yl)ethyl)acetamid
533 2-((1-(4-Methoxy-2,6-dimethylphenylsulfonyl)piperidin-2-yl)methoxy)-1-(4-(2-(piperidin-1-yl)ethyl)piperidin-1-yl)ethanon
534 2-((1-(4-Methoxy-2,6-dimethylphenylsulfonyl)piperidin-2-yl)methoxy)-1-(2-(2-morpholinoethyl)piperidin-1-yl)ethanon
535 2-((1-(4-Methoxy-2,6-dimethylphenylsulfonyl)piperidin-2-yl)methoxy)-N-(5-methyl-1 H-pyrazol-3-yl)acetamid
536 2-((1-(4-Methoxy-2,6-dimethylphenylsulfonyl)piperidin-2-yl)methoxy)-N-(5-phenyl-1 H-pyrazol-3-yl)acetamid
537 N-(3-(4-Chlorphenyl)-1 H-pyrazol-5-yl)-2-((1-(4-methoxy-2,6-dimethylphenylsulfonyl)piperidin-2-yl)methoxy)acetamid
538 N-(4-(3,4-Dimethoxyphenyl)-1H-pyrazol-5-yl)-2-((1-(4-methoxy-2,6-dimethylphenylsulfonyl)piperidin-2-yl)methoxy)acetamid
539 2-((1-(4-Methoxy-2,6-dimethylphenylsulfonyl)piperidin-2-yl)methoxy)-N-(3-(thiophen-2-yl)-1 H-pyrazol-5-yl)acetamid
540 2-((1-(4-Methoxy-2,6-dimethylphenylsulfonyl)piperidin-2-yl)methoxy)-N-(5-(2-methoxyphenyl)-1 H-pyrazol-3-yl)acetamid
541 2-((1-(4-Methoxy-2,6-dimethylphenylsulfonyl)piperidin-2-yl)methoxy)-N-(5-(3-methoxyphenyl)-1 H-pyrazol-3-yl)acetamid
542 N-(5-(3-Fluorphenyl)-1 H-pyrazol-3-yl)-2-((1-(4-methoxy-2,6-dimethylphenylsulfonyl)piperidin-2-yl)methoxy)acetamid
543 2-((1-(4-Methoxy-2,6-dimethylphenylsulfonyl)piperidin-2-yl)methoxy)-N-(5-(4-methoxyphenyl)-1 H-pyrazol-3-yl)acetamid
544 2-((1-(4-Methoxy-2,6-dimethylphenylsulfonyl)piperidin-2-yl)methoxy)-N-(3-p-tolyl-1 H-pyrazol-5-yl)acetamid
545 2-((1-(4-Methoxy-2,6-dimethylphenylsulfonyl)piperidin-2-yl)methoxy)-N-(3-phenyl-1 H-pyrazol-5-yl)acetamid
546 2-((1-(4-Methoxy-2,6-dimethylphenylsulfonyl)piperidin-2-yl)methoxy)-N-(quinolin-2-yl)acetamid
547 2-((1-(4-Methoxyphenylsulfonyl)indolin-2-yl)methoxy)-1-(4-(2-(piperidin-1-yl)ethyl)piperidin-1-yl)ethanon
548 1-(4-(2-(2,5-Dimethyl-1 H-pyrrol-1-yl)ethyl)piperazin-1-yl)-2-((1-(4-methoxyphenylsulfonyl)indolin-2-yl)methoxy)ethanon
549 2-((1-(2,6-Dichlorphenylsulfonyl)piperidin-2-yl)methoxy)-N-(4-(morpholinomethyl)benzyl)acetamid
550 2-((1-(2,6-Dichlorphenylsulfonyl)piperidin-2-yl)methoxy)-1-(4-(4-(trifluormethyl)phenyl)piperazin-1-yl)ethanon
551 2-((1-(2,6-Dichlorphenylsulfonyl)piperidin-2-yl)methoxy)-1-(4-(3,5-dichlorpyridin-2-yl)piperazin-1-yl)ethanon
552 2-((1-(2,6-Dichlorphenylsulfonyl)piperidin-2-yl)methoxy)-1-(4-(2-hydroxyethyl)piperazin-1-yl)ethanon
553 1-(4-(Benzo[d]thiazol-2-yl)piperazin-1-yl)-2-((1-(2,6-dichlorphenylsulfonyl)piperidin-2-yl)methoxy)ethanon
554 1-(4-(6-Chlorbenzo[d]thiazol-2-yl)piperazin-1-yl)-2-((1-(2,6-dichlorphenylsulfonyl)piperidin-2-yl)methoxy)ethanon
555 N-(4-(4-Ethylpiperazin-1-yl)phenyl)-2-((1-(4-methoxyphenylsulfonyl)piperidin-2-yl)methoxy)acetamid
556 2-((1-(4-Methoxyphenylsulfonyl)piperidin-2-yl)methoxy)-N-(7-morpholinobenzo[c][1,2,5]oxadiazol-4-yl)acetamid
557 2-((1-(4-Methoxyphenylsulfonyl)piperidin-2-yl)methoxy)-N-(3-methyl-4-(2-(piperidin-1-yl)acetamido)phenyl)acetamid
558 2-((1-(4-Methoxyphenylsulfonyl)piperidin-2-yl)methoxy)-1-(4-(2-phenoxyethyl)piperazin-1-yl)ethanon
559 1-(4-(2-(2,5-Dimethyl-1H-pyrrol-1-yl)ethyl)piperazin-1-yl)-2-((1-(4-methoxy-2,6-dimethylphenylsulfonyl)piperidin-2-yl)methoxy)ethanon
560 1-(4-(3-(Dimethylamino)propyl)piperazin-1-yl)-2-((2-(4-methoxy-2,3,6-trimethylphenylsulfonyl)-1,2,3,4-tetrahydroisoquinolin-3-yl)methoxy)ethanon
561 N,N-bis(3-(Dimethylamino)propyl)-2-((1-(4-methoxy-2,6-dimethylphenylsulfonyl)piperidin-2-yl)methoxy)acetamid
562 2-((2-(4-Methoxy-2,3,6-trimethylphenylsulfonyl)-1,2,3,4-tetrahydroisoquinolin-3-yl)methoxy)-1-(4-(1-methylpiperidin-4-yl)piperazin-1-yl)ethanon
563 2-((2-(4-Methoxy-2,3,6-trimethylphenylsulfonyl)-1,2,3,4-tetrahydroisoquinolin-3-yl)methoxy)-1-(4-((1-methylpiperidin-4-yl)methyl)piperazin-1-yl)ethanon
564 2-((1-(2,6-Dichlorphenylsulfonyl)piperidin-2-yl)methoxy)-1-(4-(3-(dimethylamino)propyl)piperazin-1-yl)ethanon
565 2-((1-(2,6-Dichlorphenylsulfonyl)piperidin-2-yl)methoxy)-1-(4-(1-methylpiperidin-4-yl)piperazin-1-yl)ethanon
566 2-((1-(2,6-Dichlorphenylsulfonyl)pyrrolidin-2-yl)methoxy)-1-(4-(3-(dimethylamino)propyl)piperazin-1-yl)ethanon
567 2-((1-(2,6-Dichlorphenylsulfonyl)pyrrolidin-2-yl)methoxy)-1-(4-(1-methylpiperidin-4-yl)piperazin-1-yl)ethanon
568 1-(4-Benzylpiperazin-1-yl)-2-((1-(4-chlor-2,5-dimethylphenylsulfonyl)piperidin-2-yl)methoxy)ethanon
569 2-((1-(2,6-Dichlor-4-(trifluormethyl)phenylsulfonyl)pyrrolidin-2-yl)methoxy)-1-(4-(3-(dimethylamino)propyl)piperazin-1-yl)ethanon
570 2-((1-(2,6-Dichlor-4-(trifluormethyl)phenylsulfonyl)pyrrolidin-2-yl)methoxy)-1-(4-(1-methylpiperidin-4-yl)piperazin-1-yl)ethanon
571 1-(4-(1-Methylpiperidin-4-yl)piperazin-1-yl)-2-((1-(2-(trifluormethyl)phenylsulfonyl)piperidin-2-yl)methoxy)ethanon
572 2-((1-(Benzo[b]thiophen-3-ylsulfonyl)piperidin-2-yl)methoxy)-1-(4-(4-methylpiperazin-1-yl)piperidin-1-yl)ethanon
573 2-((1-(Benzo[b]thiophen-3-ylsulfonyl)piperidin-2-yl)methoxy)-1-(4-((1-methylpiperidin-4-yl)methyl)piperazin-1-yl)ethanon
574 N-((S)-1-Benzylpyrrolidin-3-yl)-2-((1-(2,6-dichlor-4-(trifluormethyl)phenylsulfonyl)piperidin-2-yl)methoxy)-N-methylacetamid
575 N-(4-(Dimethylamino)benzyl)-2-((1-(2,4,6-trichlorphenylsulfonyl)pyrrolidin-2-yl)methoxy)acetamid
576 N-(4-(dimethylamino)benzyl)-2-((1-(4-methoxy-2,6-dimethylphenylsulfonyl)pyrrolidin-2-yl)methoxy)acetamid
577 N-(4-(Dimethylamino)benzyl)-2-((1-(4-methoxy-2,6-dimethylphenylsulfonyl)piperidin-2-yl)methoxy)acetamid
578 N-(4-(Dimethylamino)benzyl)-2-((1-(2,4,6-trichlorphenylsulfonyl)piperidin-2-yl)methoxy)acetamid
579 2-((1-(4-Methoxy-2,6-dimethylphenylsulfonyl)pyrrolidin-2-yl)methoxy)-N-(4-morpholinobenzyl)acetamid
580 N-(4-Morpholinobenzyl)-2-((1-(2,4,6-trichlorphenylsulfonyl)pyrrolidin-2-yl)methoxy)acetamid
581 N-(4-Morpholinobenzyl)-2-((1-(2,4,6-trichlorphenylsulfonyl)piperidin-2-yl)methoxy)acetamid
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

15. Verwendung eines substituierten Sulfonamid-Derivats gemäß einem der Ansprüche 1 bis 14 zur Herstellung eines Arzneimittels zur Behandlung von Schmerz, insbesondere von akutem, neuropathischem oder chronischem Schmerz, von Migräne, Diabetes, Erkrankungen der Atemwege, entzündlichen Darmerkrankungen, neurologischen Erkrankungen, Entzündungen der Haut, rheumatischen Erkrankungen, septischem Schock, Reperfusionssyndrom, Fettleibigkeit und als Angiognese-Inhibitor.
